# EUROPEAN PATENT SPECIFICATION

(11) **EP 4 259 625 B1**
(45) Date of publication and mention of the grant of the patent: **05.03.2025**
(21) Application number: 21840313.7
(22) Date of filing: 13.12.2021
(51) Int. Cl.: C07D 417/12, A01N 43/78, C07D 417/14

(54) **MOLECULES HAVING CERTAIN PESTICIDAL UTILITIES, AND INTERMEDIATES, COMPOSITIONS, AND PROCESSES RELATED THERETO**
MOLEKÜLE MIT BESTIMMTEN PESTIZIDFUNKTIONEN SOWIE ZWISCHENPRODUKTE, ZUSAMMENSETZUNGEN UND VERFAHREN IM ZUSAMMENHANG DAMIT
MOLÉCULES PRÉSENTANT CERTAINES UTILITÉS PESTICIDES, ET INTERMÉDIAIRES, COMPOSITIONS ET PROCÉDÉS ASSOCIÉS

(30) Priority: 14.12.2020 US 202063124916 P
(43) Date of publication of application: 18.10.2023
(73) Proprietor: Corteva Agriscience LLC, Indianapolis, IN 46268 (US)
(72) Inventor: DEMETER, David A., Indianapolis, Indiana 46268 (US); ESGUERRA, Kenneth Virgel N., Indianapolis, Indiana 46268 (US); GIAMPIETRO, Natalie C., Indianapolis, Indiana 46268 (US); SCHULDT, Ryan Aaron, Indianapolis, Indiana 46268 (US); WATSON, Gerald B., Indianapolis, Indiana 46268 (US)
(74) Representative: f & e patent
(86) International application number: PCT/US2021/063008
(87) International publication number: WO 2022/132609

(56) References cited:
- WO-A1-2016/113271
- WO-A1-2019/005517
- WO-A1-2019/113006
- WO-A1-2020/163146

## Description

### CROSS REFERENCE TO RELATED APPLICATIONS

This application claims the benefit of U.S. Provisional Patent Application Serial No. 63/124,916 filed December 14, 2020.

### BACKGROUND

This disclosure relates to the field of molecules having pesticidal utility against pests in Phyla Nematoda, Arthropoda, and Mollusca, processes to produce such molecules and intermediates used in such processes, compositions containing such molecules, and processes of using such molecules against such pests. These molecules may be used, for example, as nematicides, acaricides, insecticides, miticides, and molluscicides. The scope of the present invention is defined in the appended claims.

"Many of the most dangerous human diseases are transmitted by insect vectors" (Rivero, A. et al., Insect Control of Vector-Borne Diseases: When is Insect Resistance a Problem? Public Library of Science Pathogens, 6(8) (2010)). Historically, vector-borne diseases, such as, malaria, dengue, yellow fever, plague, and louse-borne typhus, among others, were responsible for more human disease and death from the 1600s through the early 1900s than all other causes combined (Gubler D., Resurgent Vector-Borne Diseases as a Global Health Problem, Emerging Infectious Diseases, Vol.4, No. 3, July-September (1998)). Currently, vector-borne diseases are responsible for about 17% of the global parasitic and infectious diseases. It has been estimated that about 250 million people around the world have malaria and about 800,000 deaths occur each year - 85% of those deaths are children under the age of five. A further 250,000 to 500,000 cases of dengue hemorrhagic fever occur each year (Matthews, G., Integrated Vector Management: controlling vectors of malaria and other insect vector borne diseases (2011)). Vector control plays a critical role in the prevention and control of infectious diseases. However, insecticide resistance, including resistance to multiple insecticides, has arisen in all insect species that are major vectors of human diseases (Rivero, A. et al.).

Each year insects, plant pathogens, and weeds destroy more than 40% of all potential food production. This loss occurs despite the application of pesticides and the use of a wide array of non-chemical controls, such as crop rotations and biological controls. If just some of this food could be saved, it could be used to feed the more than three billion people in the world who are malnourished (Pimental, D., Pest Control in World Agriculture, Agricultural Sciences - Vol. II (2009)).

Plant parasitic nematodes are among the most widespread pests and are frequently one of the most insidious and costly. It has been estimated that losses attributable to nematodes are from about 9% in developed countries to about 15% in undeveloped countries. However, in the United States of America, a survey of 35 States on various crops indicated nematode-derived losses of up to 25% (Nicol, J. et al., Current Nematode Threats to World Agriculture, Genomic and Molecular Genetics of Plant -Nematode Interactions (Eds. Jones, J. et al.), Chapter 2, (2011)).

It is noted that gastropods (slugs and snails) are pests of less economic importance than insects or nematodes, but in certain areas, gastropods may reduce yields substantially, severely affecting the quality of harvested products, as well as transmitting human, animal, and plant diseases. While only a few dozen species of gastropods are serious regional pests, a handful of species are important pests on a world-wide scale. In particular, gastropods affect a wide variety of agricultural and horticultural crops, such as arable, pastoral, and fiber crops; vegetables; bush and tree fruits; herbs; and ornamentals (Speiser, B., Molluscicides, Encyclopedia of Pest Management (2002)).

Termites cause damage to all kinds of private and public structures, as well as to agricultural and forestry resources. In 2003, it was estimated that termites cause over US$20 billion in damage world-wide each year (Su, N.Y., Overview of the global distribution and control of the Formosan subterranean termite, Sociobiology 2003, 41, 177-192). Pesticides have been disclosed, for example, in WO 2019/113006 and WO 2019/005517.

Therefore, for many reasons, including the above reasons, a need exists for new pesticides.

### SUMMARY

Provided are compounds/molecules having a structure of **"Formula One"** 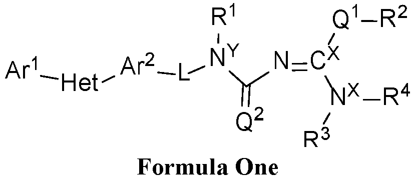 wherein:
**(A)** Ar¹ is selected from
   **(1)** furanyl, phenyl, pyridazinyl, pyridyl, pyridinonyl, pyrimidinyl, thienyl, or
   **(2)** substituted furanyl, substituted phenyl, substituted pyridazinyl, substituted pyridyl, substituted pyridinonyl, substituted pyrimidinyl, or substituted thienyl,
      wherein said substituted furanyl, substituted phenyl, substituted pyridazinyl, substituted pyridyl, substituted pyridazinyl, substituted pyridinonyl, substituted pyrimidinyl, and substituted thienyl have one or more substituents independently selected from H, F, Cl, Br, I, CN, OH, SH, SF₅, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1),
      wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) substituent may be optionally substituted with one or more substituents independently selected from H, F, Cl, Br, I, CN, OH, SH, SF₅, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)OC₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(B)** Het is a 5- or 6-membered, saturated or unsaturated, heterocyclic ring, containing one or more heteroatoms independently selected from nitrogen, sulfur, or oxygen, where said heterocyclic ring may also be substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O) C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, or S(=O)₂NR^{x}R^{y},
   wherein each alkyl, haloalkyl, cycloalkyl, halocycloalkyl, alkoxy, haloalkoxy, alkenyl, cycloalkenyl, haloalkenyl, alkynyl, phenyl, and phenoxy substituent may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)C(=O)NR^{x}R^{y}, (C₁-C₈ alkyl)NR^{x}R^{y}, C(=O)C(=O)(C₁-C₈ alkyl), C(=O)O(C₁-C₈ alkyl), C(=O)C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, or S(=O)₂NR^{x}R^{y};
**(C)** Ar² is selected from
   **(1)** furanyl, phenyl, pyridazinyl, pyridyl, pyrimidinyl, thienyl, or
   **(2)** substituted furanyl, substituted phenyl, substituted pyridazinyl, substituted pyridyl, substituted pyrimidinyl, or substituted thienyl,
      wherein said substituted furanyl, substituted phenyl, substituted pyridazinyl, substituted pyridyl, substituted pyrimidinyl, and substituted thienyl, have one or more substituents independently selected from H, F, Cl, Br, I, CN, OH, SH, SF₅, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1),
      wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) substituent may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(D)** R¹ is selected from H, CN, OH, SH, NO₂, C(=O)H, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, and (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1),
   wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, and (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(E)** R² is selected from **(J),** H, C₁-C₈ alkyl, C₃-C₈ cycloalkyl, C₂-C₈ alkenyl, C₂-C₈ alkynyl, C(=O)(C₁-C₈ alkyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), C₁-C₈ alkyl)S(=O)₂(C₁-C₈ alkyl), (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-OC(=O)-(C₁-C₈ alkyl), (C₁-C₈ alkyl)-O-C(=O)O-(C₁-C₈ alkyl), (C₁-C₈ alkyl)-O-C(=O)NR^{x}R^{y}, (C₁-C₈ alkyl)-C(=O)N(R^{x})(C₁-C₈ alkyl)-(Het-1), (C₁-C₈ alkyl)-C(=O)(Het-1), (C₁-C₈ alkyl)-C(=O)N(R^{x})(C₁-C₈ alkyl)N(R^{y})C(=O)OH, (C₁-C₈ alkyl)-C(=O)N(R^{x})(C₁-C₈ alkyl)N(R^{x})(R^{y}), (C₁-C₈ alkyl)-C(=O)N(R^{x})(C₁-C₈ alkyl)N(R^{y})C(=O)O-(C₁-C₈ alkyl), (C₁-C₈ alkyl)-C(=O)N(R^{x})(C₁-C₈ alkyl)(N(R^{y})C(=O)O-(C₁-C₈ alkyl)C(=O)OH, (C₁-C₈ alkyl)-C(=O)(Het-1)C(=O)O-(C₁-C₈ alkyl), (C₁-C₈ alkyl)-OC(=O)O-(C₁-C₈ alkyl), (C₁-C₈ alkyl)-OC(=O)-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-OC(=O)-(C₁-C₈ alkyl)N(R^{x})C(=O)O-(C₁-C₈ alkyl), (C₁-C₈ alkyl)-NR^{x}R^{y}, (C₁-C₈ alkyl)-S-(Het-1), (C₁-C₈ alkyl)S(Het-1), (C₁-C₈ alkyl)S(=O)(Het-1), (C₁-C₈ alkyl)S(=O)₂(Het-1), or (C₁-C₈ alkyl)-O-(Het-1),
   wherein each alkyl, cycloalkyl, phenyl, and (Het-1) are optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(F)** R³ has the following structure: 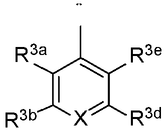 wherein
   X is N or CR^{3c};
   each R^{3a}, R^{3b}, R^{3c}, R^{3d}, and R^{3e} is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, or phenoxy;
   two of R^{3c}, R^{3d}, and R^{3e} form a 5- or 6-membered, saturated or unsaturated, ring having zero, one, or two heteroatoms selected from nitrogen, sulfur, and oxygen, and optionally substituted with at least one R¹⁴; and
   each R¹⁴ is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, or phenoxy;
**(G)** R⁴ is selected from **(J),** H, OH, SH, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-OC(=O)-(C₁-C₈ alkyl), (C₁-C₈ alkyl)-O-C(=O)O-(C₁-C₈ alkyl), (C₁-C₈ alkyl)-O-C(=O)NR^{x}R^{y}, (C₁-C₈ alkyl)-C(=O)N(R^{x})(C₁-C₈ alkyl)-(Het-1), (C₁-C₈ alkyl)-C(=O)(Het-1), (C₁-C₈ alkyl)-C(=O)N(R^{x})(C₁-C₈ alkyl)N(R^{y})C(=O)OH, (C₁-C₈ alkyl)-C(=O)N(R^{x})(C₁-C₈ alkyl)N(R^{x})(R^{y}), (C₁-C₈ alkyl)-C(=O)N(R^{x})(C₁-C₈ alkyl)N(R^{y})C(=O)O-(C₁-C₈ alkyl), (C₁-C₈ alkyl)-C(=O)N(R^{x})(C₁-C₈ alkyl)(N(R^{y})C(=O)O-(C₁-C₈ alkyl)C(=O)OH, (C₁-C₈ alkyl)-C(=O)(Het-1)C(=O)O-(C₁-C₈ alkyl), (C₁-C₈ alkyl)-OC(=O)O-(C₁-C₈ alkyl), (C₁-C₈ alkyl)-OC(=O)-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-OC(=O)-(C₁-C₈ alkyl)N(R^{x})C(=O)O-(C₁-C₈ alkyl), (C₁-C₈ alkyl)-NR^{x}R^{y}, (C₁-C₈ alkyl)-S-(Het-1), (C₁-C₈ alkyl)S(=O)(Het-1), (C₁-C₈ alkyl)S(=O)₂(Het-1), or (C₁-C₈ alkyl)-O-(Het-1),
   wherein each alkyl, haloalkyl, cycloalkyl, halocycloalkyl, cycloalkoxy, halocycloalkoxy, alkoxy, haloalkoxy, alkenyl, cycloalkenyl, haloalkenyl, alkynyl, phenyl, and (Het-1), are optionally substituted with one or more substituents independently selected from H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, halophenyl, phenoxy, and (Het-1);
**(H)** each of Q¹ and Q² is independently selected from O or S;
**(I)** R^{x} and R^{y} are independently selected from H, OH, SH, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-OC(=O)-(C₁-C₈ alkyl), (C₁-C₈ alkyl)-O-C(=O)O-(C₁-C₈ alkyl), (C₁-C₈ alkyl)-C(=O)(Het-1), (C₁-C₈ alkyl)-C(=O)(Het-1)C(=O)O-(C₁-C₈ alkyl), (C₁-C₈ alkyl)-OC(=O)O-(C₁-C₈ alkyl), (C₁-C₈ alkyl)-OC(=O)-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-S-(Het-1), (C₁-C₈ alkyl)S(=O)(Het-1), (C₁-C₈ alkyl)S(=O)₂(Het-1), or (C₁-C₈ alkyl)-O-(Het-1),
   wherein each alkyl, haloalkyl, cycloalkyl, halocycloalkyl, cycloalkoxy, halocycloalkoxy, alkoxy, haloalkoxy, alkenyl, cycloalkenyl, haloalkenyl, alkynyl, phenyl, and (Het-1), are optionally substituted with one or more substituents independently selected from H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, halophenyl, phenoxy, and (Het-1),
   or R^{x} and R^{y} together can optionally form a 5- to 7-membered saturated or unsaturated cyclic group which may contain one or more heteroatoms selected from nitrogen, sulfur, and oxygen, and where said cyclic group may be substituted with H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, substituted phenyl, phenoxy, and (Het-1);
**(J)** R² and R⁴ may be a 1- to 4-membered saturated or unsaturated, hydrocarbyl link, which may contain one or more heteroatoms selected from nitrogen, sulfur, and oxygen, and together with C^{x}(Q¹)(N^{x}) forms a 4- to 7-membered cyclic structure, wherein said hydrocarbyl link may optionally be substituted with one or more substituents independently selected from R⁵, R⁶, and R⁷, wherein each R⁵, R⁶, and R⁷ is selected from H, F, Cl, Br, I, CN, OH, SH, NO₂, NR^{x}R^{y}, C₁-C₈ alkyl, C₁-C₈ alkyl substituted with at least one OH, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, substituted phenyl, phenoxy, or (Het-1);
**(K)** (Het-1) is a 5- or 6-membered, saturated or unsaturated, heterocyclic ring, containing one or more heteroatoms independently selected from nitrogen, sulfur or oxygen, wherein said heterocyclic ring may also be substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₄ alkyl), S(=O)(C₁-C₄ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, and phenoxy,
   wherein each alkyl, cycloalkyl, alkoxy, alkenyl, alkynyl, phenyl, and phenoxy may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₄ alkyl), S(=O)(C₁-C₄ haloalkyl), OS(=O)₂(C₁-C₈ alkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, and phenoxy; and
**(L)** L is a bond which connects Ar² to N^{Y}

### DETAILED DESCRIPTION

### DEFINITIONS

The examples given in the definitions are generally non-exhaustive and must not be construed as limiting the molecules disclosed in this document. It is understood that a substituent should comply with chemical bonding rules and steric compatibility constraints in relation to the particular molecule to which it is attached.

"Alkenyl" means an acyclic, unsaturated (at least one carbon-carbon double bond), branched or unbranched, substituent consisting of carbon and hydrogen, for example, vinyl, allyl, butenyl, pentenyl, and hexenyl.

"Alkenyloxy" means an alkenyl further consisting of a carbon-oxygen single bond, for example, allyloxy, butenyloxy, pentenyloxy, hexenyloxy.

"Alkoxy" means an alkyl further consisting of a carbon-oxygen single bond, for example, methoxy, ethoxy, propoxy, isopropoxy, butoxy, isobutoxy, and tert-butoxy.

"Alkyl" means an acyclic, saturated, branched or unbranched, substituent consisting of carbon and hydrogen, for example, methyl, ethyl, propyl, isopropyl, butyl, and *tert*-butyl*.*

"Alkynyl" means an acyclic, unsaturated (at least one carbon-carbon triple bond), branched or unbranched, substituent consisting of carbon and hydrogen, for example, ethynyl, propargyl, butynyl, and pentynyl.

"Alkynyloxy" means an alkynyl further consisting of a carbon-oxygen single bond, for example, pentynyloxy, hexynyloxy, heptynyloxy, and octynyloxy.

"Aryl" means a cyclic, aromatic substituent consisting of hydrogen and carbon, for example, phenyl, naphthyl, and biphenyl.

"Cycloalkenyl" means a monocyclic or polycyclic, unsaturated (at least one carbon-carbon double bond) substituent consisting of carbon and hydrogen, for example, cyclobutenyl, cyclopentenyl, cyclohexenyl, norbornenyl, bicyclo[2.2.2]octenyl, tetrahydronaphthyl, hexahydronaphthyl, and octahydronaphthyl.

"Cycloalkenyloxy" means a cycloalkenyl further consisting of a carbon-oxygen single bond, for example, cyclobutenyloxy, cyclopentenyloxy, norbornenyloxy, and bicyclo[2.2.2]octenyloxy.

"Cycloalkyl" means a monocyclic or polycyclic, saturated substituent consisting of carbon and hydrogen, for example, cyclopropyl, cyclobutyl, cyclopentyl, norbornyl, bicyclo[2.2.2]octyl, and decahydronaphthyl.

"Cycloalkoxy" means a cycloalkyl further consisting of a carbon-oxygen single bond, for example, cyclopropyloxy, cyclobutyloxy, cyclopentyloxy, norbornyloxy, and bicyclo[2.2.2]octyloxy.

"Halo" means fluoro, chloro, bromo, and iodo.

"Haloalkoxy" means an alkoxy further consisting of, from one to the maximum possible number of identical or different, halos, for example, fluoromethoxy, trifluoromethoxy, 2,2-difluoropropoxy, chloromethoxy, trichloromethoxy, 1,1,2,2-tetrafluoroethoxy, and pentafluoroethoxy.

"Haloalkyl" means an alkyl further consisting of, from one to the maximum possible number of, identical or different, halos, for example, fluoromethyl, trifluoromethyl, 2,2-difluoropropyl, chloromethyl, trichloromethyl, and 1,1,2,2-tetrafluoroethyl.

"Heterocyclyl" means a cyclic substituent that may be fully saturated, partially unsaturated, or fully unsaturated, where the cyclic structure contains at least one carbon and at least one heteroatom, where said heteroatom is nitrogen, sulfur, or oxygen. Examples of aromatic heterocyclyls include, but are not limited to, benzofuranyl, benzoisothiazolyl, benzoisoxazolyl, benzoxazolyl, benzothienyl, benzothiazolyl, cinnolinyl, furanyl, indazolyl, indolyl, imidazolyl, isoindolyl, isoquinolinyl, isothiazolyl, isoxazolyl, oxadiazolyl, oxazolinyl, oxazolyl, phthalazinyl, pyrazinyl, pyrazolinyl, pyrazolyl, pyridazinyl, pyridyl, pyrimidinyl, pyrrolyl, quinazolinyl, quinolinyl, quinoxalinyl, tetrazolyl, thiazolinyl, thiazolyl, thienyl, triazinyl, and triazolyl. Examples of fully saturated heterocyclyls include, but are not limited to, piperazinyl, piperidinyl, morpholinyl, pyrrolidinyl, tetrahydrofuranyl, and tetrahydropyranyl. Examples of partially unsaturated heterocyclyls include, but are not limited to, 1,2,3,4-tetrahydro-quinolinyl, 4,5-dihydro-oxazolyl, 4,5-dihydro-1*H*-pyrazolyl, 4,5-dihydro-isoxazolyl, and 2,3-dihydro-[1,3,4]-oxadiazolyl.

In one embodiment, Ar¹ and Ar² are not *ortho* to each other (but may be *meta* or *para*, such as, for a five-membered ring they are 1,3 and for a 6-membered ring they are either 1,3 or 1,4).

In another embodiment Ar¹ is a substituted phenyl. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted phenyl that has one or more substituents selected from C₁-C₆ haloalkyl and C₁-C₆ haloalkoxy. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted phenyl that has one or more substituents selected from CN, SF₅, CH₃, CF₃, SCF₃, OCF₃, OCH₂CF₃, and OC₂F₅. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted phenyl that has one or more substituents selected from CF₃, OCF₃, and OC₂F₅. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted pyridyl. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted pyridyl that has one or more substituents selected from C₁-C₆ haloalkyl and C₁-C₆ haloalkoxy. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted pyridinonyl. This embodiment may be used in combination with the other embodiments of Het, Ar², R¹, R², R³, R⁴, Q¹, Q², R² and R⁴ hydrocarbyl links, and/or L.

In another embodiment Ar¹ is a substituted pyridinonyl that has one or more substituents selected from oxo, C₁-C₆ haloalkyl and C₁-C₆ haloalkoxy. This embodiment may be used in combination with the other embodiments of Het, Ar², R¹, R², R³, R⁴, Q¹, Q², R² and R⁴ hydrocarbyl links, and/or L.

In another embodiment Het is selected from benzofuranyl, benzoisothiazolyl, benzoisoxazolyl, benzoxazolyl, benzothienyl, benzothiazolyl, cinnolinyl, furanyl, indazolyl, indolyl, imidazolyl, isoindolyl, isoquinolinyl, isothiazolyl, isoxazolyl, oxadiazolyl, oxazolinyl, oxazolyl, phthalazinyl, pyrazinyl, pyrazolinyl, pyrazolyl, pyridazinyl, pyridyl, pyrimidinyl, pyrrolyl, quinazolinyl, quinolinyl, quinoxalinyl, tetrazolyl, thiazolinyl, thiazolyl, thienyl, triazinyl, triazolyl, piperazinyl, piperidinyl, morpholinyl, pyrrolidinyl, tetrahydrofuranyl, tetrahydropyranyl, 1,2,3,4-tetrahydro-quinolinyl, 4,5-dihydro-oxazolyl, 4,5-dihydro-1H-pyrazolyl, 4,5-dihydro-isoxazolyl, and 2,3-dihydro-[1,3,4]-oxadiazolyl.

In another embodiment Het is triazolyl. This embodiment may be used in combination with the other embodiments of Ar¹, Ar², R¹, R², R³, R⁴, Q¹, Q², R² and R⁴ hydrocarbyl links, and/or L.

In another embodiment Het is 1,2,4-triazolyl. This embodiment may be used in combination with the other embodiments of Ar¹, Ar², R¹, R², R³, R⁴, Q¹, Q², R² and R⁴ hydrocarbyl links, and/or L.

In another embodiment Het is oxadiazolyl. This embodiment may be used in combination with the other embodiments of Ar¹, Ar², R¹, R², R³, R⁴, Q¹, Q², R² and R⁴ hydrocarbyl links, and/or L.

In another embodiment Het is 1,3,4-oxadiazolyl. This embodiment may be used in combination with the other embodiments of Ar¹, Ar², R¹, R², R³, R⁴, Q¹, Q², R² and R⁴ hydrocarbyl links, and/or L.

In another embodiment Het is pyrazolyl. This embodiment may be used in combination with the other embodiments of Ar¹, Ar², R¹, R², R³, R⁴, Q¹, Q², R² and R⁴ hydrocarbyl links, and/or L.

In another embodiment Ar² is phenyl. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted phenyl. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted phenyl that has one or more substituents selected from C₁-C₆ alkyl. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted phenyl that has one or more substituents wherein said substituent is H, F, Cl, Br, I, CN, OH, OCH₃, OCH₂OCH₃, CHF₂, CF₃, CH₂CH₃, CH(CH₃)₂, and CH₃. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted pyridyl. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted pyridyl that has one or more substituents selected from C₁-C₆ alkyl. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted phenyl that has one or more substituents wherein said substituent is CH₃. This embodiment may be used in combination with the other embodiments of Ar¹, Het, R¹, R², R³, R⁴, Q¹, Q², R² and R⁴ hydrocarbyl links, and/or L.

In another embodiment Ar² is a substituted phenyl that has one or more substituents wherein said substituent is F. This embodiment may be used in combination with the other embodiments of Ar¹, Het, R¹, R², R³, R⁴, Q¹, Q², R² and R⁴ hydrocarbyl links, and/or L.

In another embodiment R¹ is H. This embodiment may be used in combination with the other embodiments of Ar¹, Het, Ar², R², R³, R⁴, Q¹, Q², R² and R⁴ hydrocarbyl links, and/or L.

In another embodiment R² is **(J),** H, C₁-C₆ alkyl, C₁-C₆ alkyl-O-C(=O)C₁-C₆ alkyl, C₁-C₆ alkyl-O-C(=O)N(R^{x}R^{y}), or (C₁-C₆ alkyl)S-(Het-1). This embodiment may be used in combination with the other embodiments of Ar¹, Het, Ar², R¹, R³, R⁴, Q¹, Q², R² and R⁴ hydrocarbyl links, and/or L.

In another embodiment R² is **(J),** H, CH₃, C₁-C₆ alkyl, CH₂OC(=O)CH(CH₃)₂, CH₂OC(=O)N(H)(C(=O)OCH₂Ph), or CH₂S(3,4,5-trimethoxy-2-tetrahydropyran). This embodiment may be used in combination with the other embodiments of Ar¹, Het, Ar², R¹, R³, R⁴, Q¹, R² and R⁴ hydrocarbyl links, and/or L.

In another embodiment R³ has a structure of Formula R3A or Formula R3B: 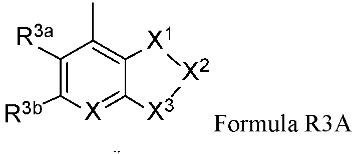 or 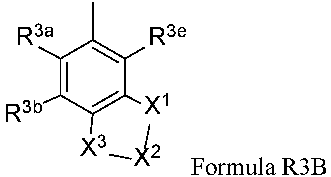 wherein
X is N or CR^{3c};
X¹ is O, CH, CH₂, N, N(R¹⁴), S, C(O), or S;
x² is O, CH, CH₂, N, N(R¹⁴), S, C(O), or S;
X³ is O, CH, CH₂, N, N(R¹⁴), S, C(O), S, O(CH₂), (CH₂)O, -N=CH-, -CH=N-, C(O)O, OC(O), -N(R¹⁴)-CH₂-, -CH₂-N(R¹⁴)-, -N(R¹⁴)-CH-, -CH-N(R¹⁴)-, -CH=CH-, or -CH₂-CH₂-;
R^{3a}, R^{3b}, R^{3c}, and R¹⁴ is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, or phenoxy.

In another embodiment R³ is selected from one of groups R³-1 to R³-33 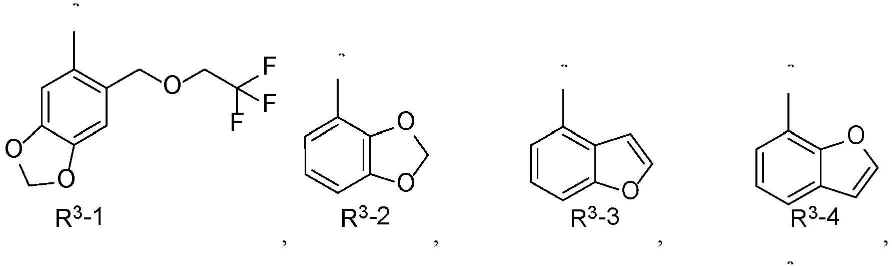 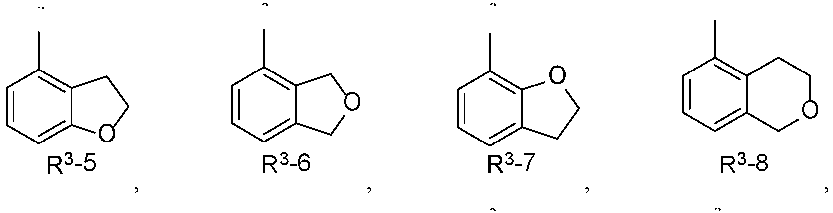 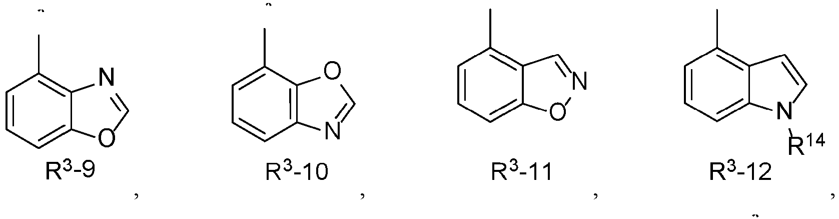 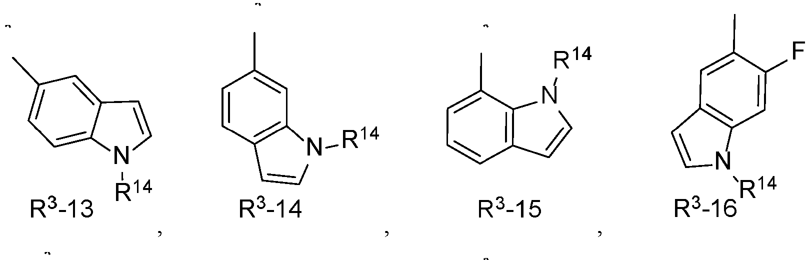 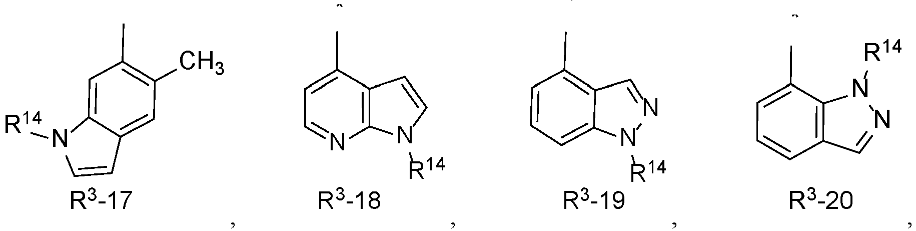 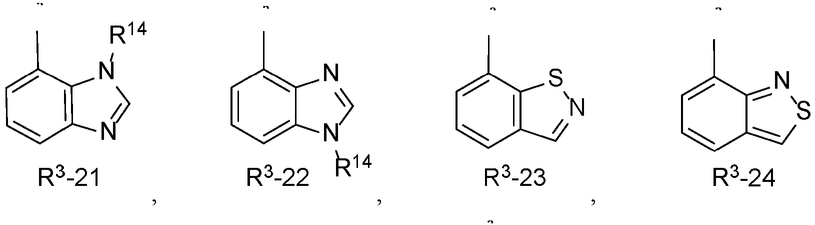 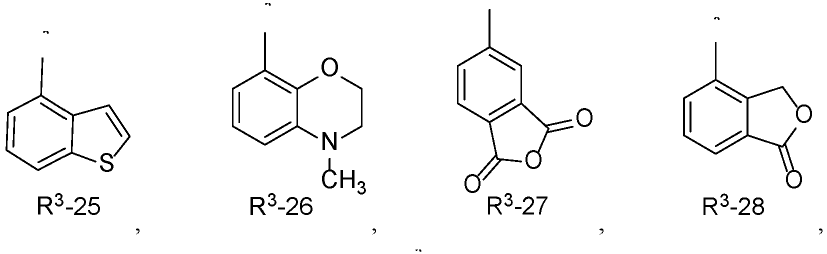 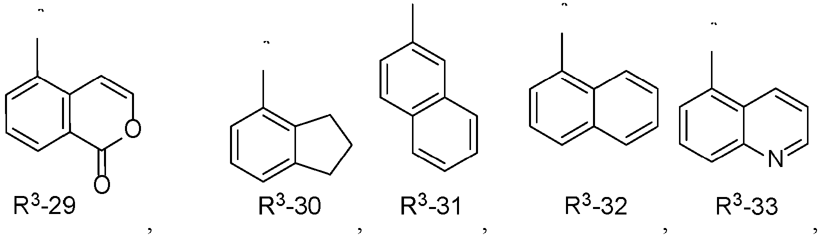 wherein R¹⁴, if applicable to group R³, is selected from the group consisting of H and (C₁-C₈ alkyl). This embodiment may be used in combination with the other embodiments of Ar¹, Het, Ar², R¹, R², R⁴, Q¹, Q², R² and R⁴ hydrocarbyl links, and/or L.

In another embodiment R³ is selected from one of the following groups R³ 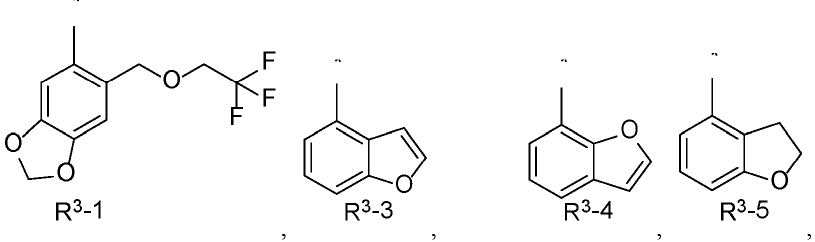 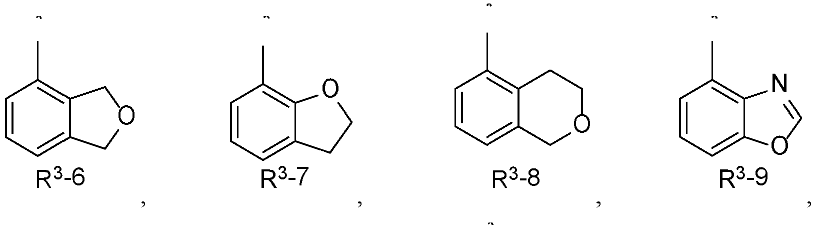 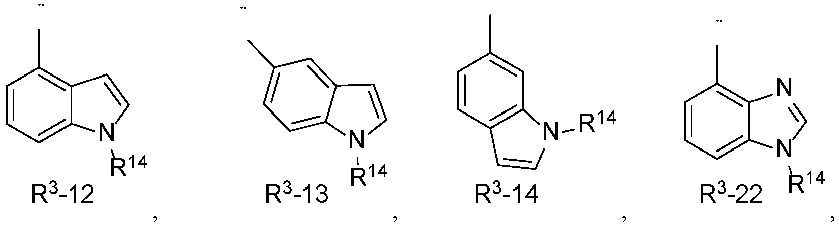 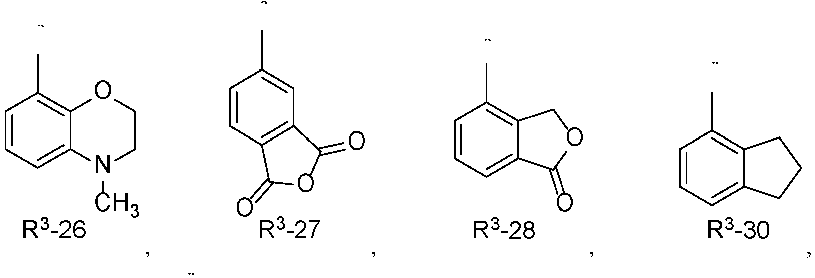 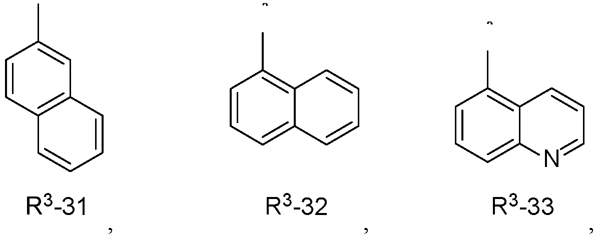 wherein R¹⁴, if applicable to group R³, is selected from the group consisting of H and CH₃. This embodiment may be used in combination with the other embodiments of Ar¹, Het, Ar², R¹, R², R⁴, Q¹, Q², R² and R⁴ hydrocarbyl links, and/or L.

In another embodiment R⁴ is H. This embodiment may be used in combination with the other embodiments of Ar¹, Het, Ar², R¹, R², R³, Q¹, Q², and/or L.

In another embodiment Q¹ is O. This embodiment may be used in combination with the other embodiments of Ar¹, Het, Ar², Q², R¹, R², R³, R⁴, R² and R⁴ hydrocarbyl links, and/or L.

In another embodiment Q¹ is S. This embodiment may be used in combination with the other embodiments of Ar¹, Het, Ar², Q², R¹, R², R³, R⁴, R² and R⁴ hydrocarbyl links, and/or L.

In another embodiment Q² is O. This embodiment may be used in combination with the other embodiments of Ar¹, Het, Ar², Q¹, R¹, R², R³, R⁴, R² and R⁴ hydrocarbyl links, and/or L.

In another embodiment Q² is S. This embodiment may be used in combination with the other embodiments of Ar¹, Het, Ar², Q¹, R¹, R², R³, R⁴, R² and R⁴ hydrocarbyl links, and/or L.

In another embodiment R² and R⁴ is a hydrocarbyl link wherein said hydrocarbyl link is substituted with oxo or C₁-C₆ alkyl. This embodiment may be used in combination with the other embodiments of Ar¹, Het, Ar², R¹, R², R³, R⁴, Q¹, Q², and/or L.

In another embodiment R² and R⁴ is a hydrocarbyl link wherein said hydrocarbyl link is CH₂C(=O), C(C(OH)(CH₃)₂)C(=O), C(cyclopropyl)C(=O), C(CH₃)₂C(=O), CFHC(=O), CBrHC(=O), CH(CH₃)C(=O), CH₂CH₂, CH₂C(OH)(CH₃), CH₂CH₂CH₂, CH₂CH₂C(=O), CH₂CH(CH₃)CH₂, N(CH₃)C(=O), N(CH₂CH₃)C(=O), CH=C(CH₃), or CH₂CH(CH₃). This embodiment may be used in combination with the other embodiments of Ar¹, Het, Ar², R¹, R², R³, R⁴, Q¹, Q², and/or L.

In one embodiment, also provided are compound disclosed herein, wherein:
**(A)** Ar¹ is a substituted phenyl, a substituted pyridazinyl, a substituted pyridyl, a substituted pyridinonyl, or a substituted pyrimidinyl, wherein said substituted phenyl, substituted pyridyl, and substituted pyrimidinyl have one or more substituents independently selected from H, CN, SF₅, oxo, C₁-C₆ alkyl, C₁-C₆ alkoxy, S(C₁-C₆ haloalkyl), C₁-C₆ haloalkyl and C₁-C₆ haloalkoxy;
**(B)** Het is triazolyl or pyrazolyl;
**(C)** Ar² is phenyl, pyridyl, pyrimidinyl, substituted phenyl, substituted pyridyl, or substituted pyrimidinyl, wherein said substituted phenyl, substituted pyridyl, and substituted pyrimidinyl have one or more substituents independently selected from H, F, Cl, Br, I, CN, NO₂, NR^{x}R^{y}, OH, C₁-C₆ alkoxy, (C₁-C₆ alkyl)O(C₁-C₆ alkyl), C₁-C₆ haloalkyl, and C₁-C₆ alkyl;
**(D)** R¹ is H, C₁-C₆ alkyl, C₃-C₆ cycloalkyl, or C₂-C₆ alkenyl, wherein said alkyl, cycloalkyl, or alkenyl is optionally substituted with a C₁-C₆ alkoxy, C₁-C₆ haloalkoxy, C₃-C₆ cycloalkyl, or C₃-C₆ halocycloalkyl;
**(E)** R² is selected from **(J),** H, C₁-C₆ alkyl, or C₁-C₆ haloalkyl;
**(F)** R³ is selected from one of the following groups R³ 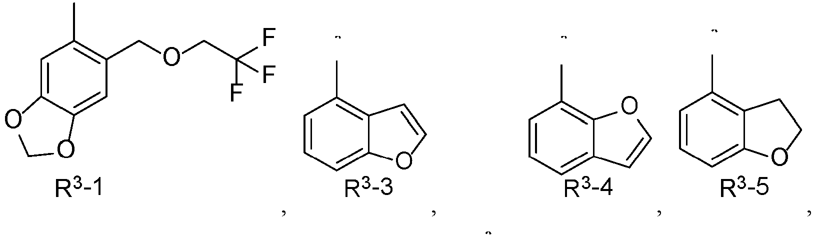 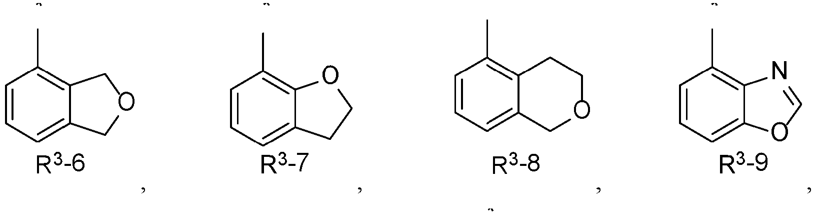 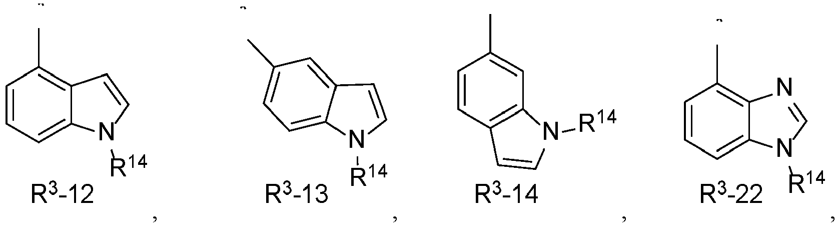 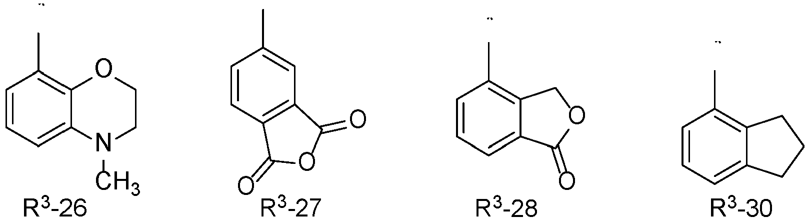 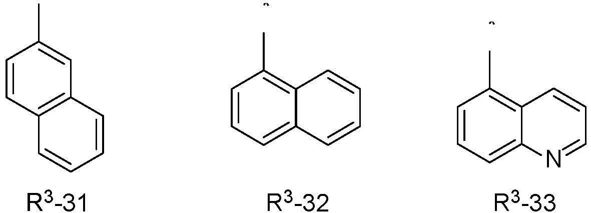 wherein R¹⁴, if applicable to group R³, is selected from the group consisting of H and CH₃;
**(G)** R⁴ is selected from **(J),** H, C₁-C₆ alkyl, or C₁-C₆ haloalkyl;
**(H)** Q¹ is S and Q² is O;
**(I)** R^{x} and R^{y} are independently selected from H, C(=O)(C₁-C₆ alkyl), C₁-C₆ alkyl, C₁-C₆ haloalkyl, C₃-C₆ cycloalkyl, C₃-C₆ halocycloalkyl, C₂-C₆ alkenyl, C₃-C₆ cycloalkenyl, C₂-C₆ haloalkenyl, C₂-C₆ alkynyl, and phenyl;
**(J)** R² and R⁴ may be a 1- to 4-membered saturated or unsaturated, hydrocarbyl link, which may contain one or more heteroatoms selected from nitrogen, sulfur, and oxygen, and together with C^{x}(Q¹)(N^{x}) forms a cyclic structure, wherein said hydrocarbyl link may optionally be substituted with one or more substituents independently selected from R⁵, R⁶, and R⁷, wherein each R⁵, R⁶, and R⁷ is selected from H, F, Cl, Br, I, CN, NO₂, NR^{x}R^{y}, OH, SH, S(C₁-C₆ alkyl), S(C₁-C₆ haloalkyl), C₁-C₆ alkyl, C₁-C₆ haloalkyl, C₁-C₆ alkoxy, C₁-C₆ haloalkoxy, C₃-C₆ cycloalkyl, C₃-C₆ halocycloalkyl, phenyl, and oxo; and

In another embodiment, the compound provided herein has a structure of the following formula **("Formula Two"):**

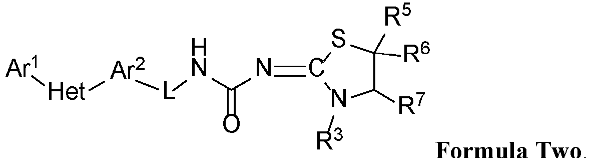

In another embodiment, the compound provided herein has a structure selected from the compounds listed in Table 1.

In another embodiment, the compound provided herein has the structure of Formula Two and is selected from the compounds listed in Table 1.

In one embodiment, also provided is a process comprising applying the compound provided herein to a locus to control a pest, in an amount sufficient to control such pest.

In a further embodiment, wherein said pest is beet armyworm (BAW), cabbage looper (CL), or yellow fever mosquito (YFM).

In another embodiment, the compound provided herein has at least one ²H or at least one ¹⁴C.

In another embodiment, also provided is a composition comprising the compound provided herein and at least one other compound having insecticidal, herbicidal, acaricidal, nematicidal, or fungicidal activity.

In another embodiment, also provided is a composition comprising the compound provided herein and a seed.

In another embodiment, also provided is a process comprising applying the compound provided herein to a genetically modified plant, or genetically-modified seed, which has been genetically modified to express one or more specialized traits.

In another embodiment, also provided is a process comprising: orally administering; or topically applying; the compound provided herein, to a non-human animal, to control endoparasites and/or ectoparasites.

Many of the compounds of Formula One, Two, and/or Three may be depicted in two or more tautomeric forms such as when R¹, R², or R⁴, is H (see for example, "Scheme TAU" below). For the sake of simplifying the schemes, all molecules have been depicted as existing as a single tautomer. Any and all alternative tautomers are included within the scope of this Formula One, and no inference should be made as to whether the molecule exists as the tautomeric form in which it is drawn.

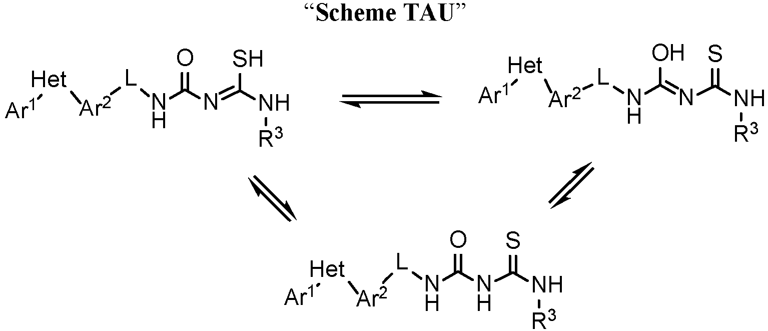

The compounds of Formula One, Two, and/or Three will generally have a molecular mass of about 400 Daltons to about 1200 Daltons. However, it is generally preferred if the molecular mass is from about 300 Daltons to about 1000 Daltons, and it is even more generally preferred if the molecular mass is from about 400 Daltons to about 750 Daltons.

### PREPARATION OF THIOBIURETS

Thiobiurets disclosed herein are prepared from the corresponding isocyanate, Ar¹-Het-Ar²-L-NCO (1-2). Usually, these isocyanates are not isolated, but are instead generated *in situ* from a suitable precursor and used directly in the preparation of a thiobiuret. One such suitable precursor is an amine (1-1) which can be converted into an isocyanate by using one of several common reagents such as phosgene, diphosgene, triphosgene, or carbonyldiimidazole (Scheme 1, step a), in a mixed solvent system such as dichloromethane and water or diethyl ether and water, in the presence of a base such as sodium bicarbonate or triethylamine, at temperatures from about -10°C to about 50 °C.

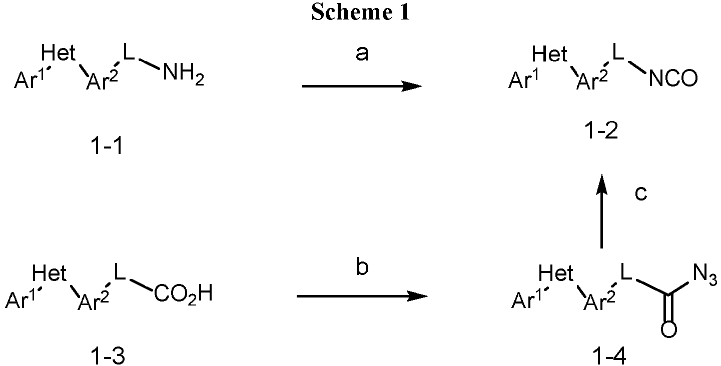

Alternatively, the isocyanates may be generated *via* a Curtius rearrangement of an acyl azide, Ar¹-Het-Ar²-L-C(=O)N₃ (1-4), which is, in turn, prepared from the corresponding carboxylic acid precursor, Ar¹-Het-Ar²-L-CO₂H (1-3). Formation of an acyl azide (Scheme 1, step b) occurs either by treatment of the acid with ethyl chloroformate and sodium azide in the presence of an amine base such as triethylamine, or with diphenylphosphoryl azide in the presence of an amine base such as triethylamine. The acyl azide is then made to undergo a Curtius rearrangement (which may need to be thermally induced), leading to the corresponding isocyanate (1-2). Depending on the nature of the particular acyl azide, this rearrangement may occur spontaneously at ambient temperature, or it may require heating from about 40 °C to about 100°C in a suitable solvent, such as toluene, or acetonitrile, or an ethereal solvent such as dioxane or tetrahydrofuran. Azides of arylacetic acids are known, though frequently, due to their reactivity, they are not isolated as pure solids. Accordingly, the acyl azide intermediate is not always fully characterized, but may simply be heated directly without characterization, to generate the isocyanate.

An isocyanate, Ar¹-Het-Ar²-L-NCO (1-2), can be treated directly with an N-aryl thiourea (2-1) in the presence of about 0.1 to about 2 equivalents of an inorganic base such as cesium carbonate or sodium hydride, resulting in the formation of a thiobiuret (2-2, Scheme 2). The reaction can be performed at temperatures from about 0 °C to about 100 °C, preferably from about 20 °C to about 80 °C, in an aprotic solvent or solvent mixture chosen from acetonitrile, acetone, toluene, tetrahydrofuran, 1,2-dichloroethane, dichloromethane, or mixtures thereof, but use of acetonitrile is preferred.

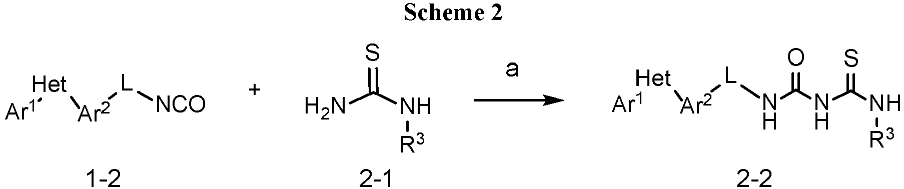

Thiobiurets (2-2) generated *in situ* can be converted directly without purification into a variety of cyclized analogs (Scheme 3), or they can be isolated from the reaction medium prior to cyclization. Cyclization can be achieved by treatment with an α-halo ester such as methyl bromoacetate to form 2-imino 1,3-thiazolin-4-ones (3-1, step a) unsubstituted or mono- or di-substituted with R⁵; vicinal dihalides such as 1-bromo-2-chloroethane or 1,2-dichloroethane, to form 2-imino-1,3-thiazolines (3-2, step b) unsubstituted or mono-substituted with R⁵ or R⁶; α-halo ketones such as chloroacetone to form 2-imino-1,3-thiazoles (3-3, step c) substituted with R⁵ or R⁶; or 1,3-dihalopropanes such as 1-bromo-3-chloro-propane to form 2-imino-1,3-thiazinanes (3-4, step d) unsubstituted or mono-substituted with R⁵ or R⁶ or unsubstituted or mono- or di-substituted with R⁷. With step a, use of sodium acetate in a protic solvent such as ethanol or methanol, at temperatures ranging from about 20 °C to about 70 °C is preferred. With step b, use of an inorganic base such as potassium carbonate in a solvent such as acetonitrile or (preferably) 2-butanone, at a temperature between about 0 °C and about 80 °C, is preferred.

An alternative method for preparing analogs having the general structure 3-1' is described in Scheme 3a. Intermediate 4-imino-3-arylthiazolidinone-2-one (3-1a, step a) is reacted directly with an isocyanate (1-2), in the presence of about 0.1 to about 2 equivalents of an inorganic base such as cesium carbonate or sodium hydride to form cyclized thiobiuret (3-1'). The reaction can be performed at temperatures from about 0 °C to about 100 °C, preferably from about 20 °C to about 80 °C, in an aprotic solvent or solvent mixture chosen from acetonitrile, acetone, toluene, tetrahydrofuran, 1,2-dichloroethane, dichloromethane, or mixtures thereof, but use of acetonitrile is preferred.

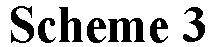

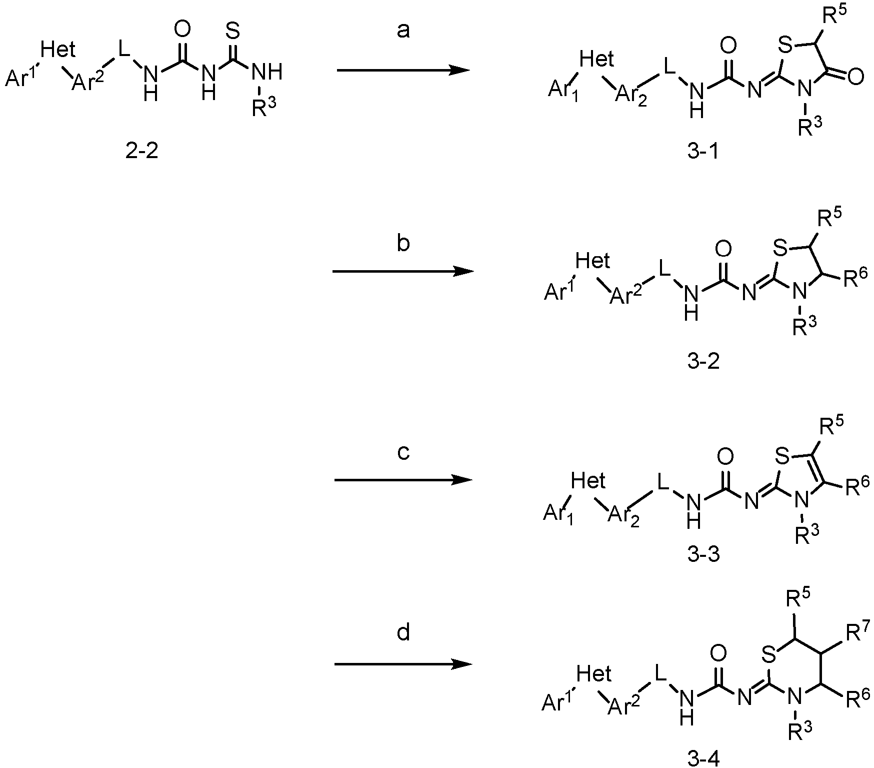

Alternatively, the 4-imino-3-arylthiazolidinone-2-one (3-1a) may be reacted with 4-nitrophenyl chloroformate (step b), forming a 4-nitrophenyl carbamate intermediate (3-2a). This reaction is conducted with equimolar quantities of the imine and the chloroformate, in a polar aprotic solvent such as tetrahydrofuran or dioxane, and in the presence of from about 0.1 to about 2 equivalents of an inorganic base such as cesium carbonate or potassium carbonate, preferably at room temperature. The intermediate (3-2a) may be isolated by filtration from inorganic salts and evaporation of solvent, or it can be used directly in step c. In step c, treatment of 3-2a with a primary or secondary alkyl amine Ar¹-Het-Ar²-L-NHR¹, wherein R¹ is H or alkyl, respectively, may generate cyclized thiobiuret (3-1'). Step c may also be conducted in the presence of an inorganic base such as cesium carbonate or potassium carbonate, from about 0.1 to about 2 equivalents, preferably about 1 to about 1.2 equivalents; it is also most conveniently undertaken at room temperature, although it may be undertaken at temperatures from about 0 °C to about 100 °C.

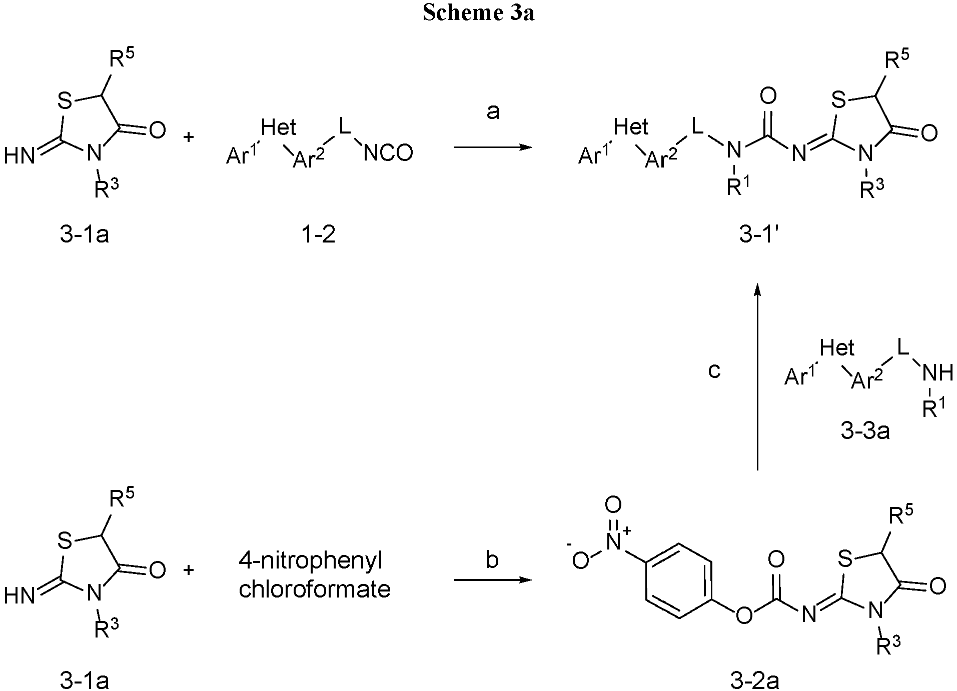

Alternatively, treatment of a primary or secondary alkyl amine (3-3a, Ar¹-Het-Ar²-L-NHR¹), wherein R¹ is H or alkyl, respectively, with an activating agent such bis(2,5-dioxopyrrolidin-1-yl) carbonate in the presence of an organic base such as pyridine, preferably from about 1 to about 1.2 equivalents, triphosgene in the presence of an organic base such as *N,N-diisopropylethylamine,* or 4-nitrophenyl carbonochloridate in an aprotic solvent such as dichloromethane may generate *in situ* an activated amine (not shown), which is reacted with 4-imino-3-arylthiazolidinone-2-one (3-1a) with or without an organic base such as *N,N-*diisopropylethylamine to afford the cyclized thiobiuret (3-1') as in step a, Scheme 3a'.

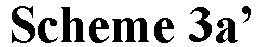

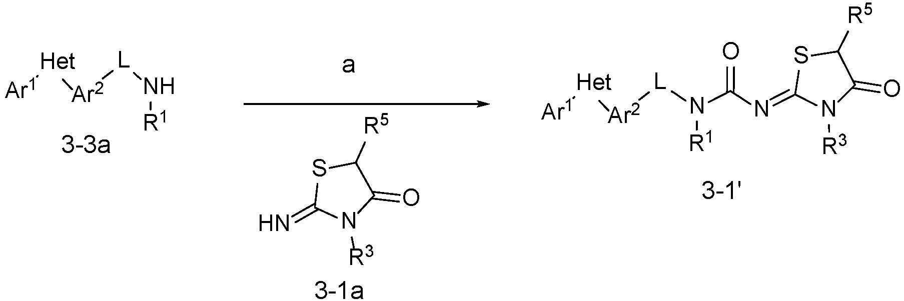

Thiobiurets (2-2) can also be converted into novel S-alkylated analogs as described in Scheme 3b. For example, reaction of a thiobiuret 2-2 with an alkyl iodide (step a), in a protic solvent such as ethanol, and in the presence of a base such as sodium acetate, at temperatures from about 0 °C to about 60 °C, results in formation of an S-R² substituted product (3-1b). A variation of the reaction conditions described in Scheme 3, step c, employs careful control of reaction conditions to ensure that the temperature does not exceed 20 °C. Under these conditions, 4-hydroxy-2-iminothiazolidines (3-2b, step b) may be isolated.

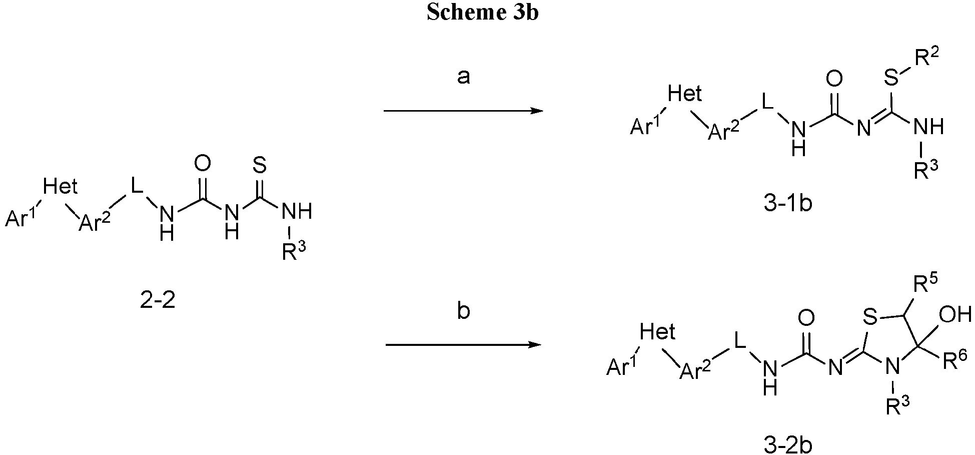

Analogs of Formula One wherein R² and R⁴ are cyclized to form a 2-(R⁵)-4-(R³)-5-imino-1,2,4-thiadiazolidin-3-one (3-4c) may be constructed as described in Scheme 3c. Following the work described by Kaugers, *et al* (*J. Org. Chem.* **1992**, 57, 1671), an N-arylamino 1,2,3,4-thiatriazole (3-1c), prepared in one step from the corresponding N³-aryl thiosemicarbazone by oxidation with sodium nitrite, is treated with an alkyl isocyanate to form 3-2c. Treatment of 3-2c with a base such as sodium methoxide in methanol at room temperature (step b) results in cleavage of the urea bond and formation of a 2-(R⁵)-4-(R³)-5-imino-1,2,4-thiadiazolidin-3-one (3-3c). This imine may then be treated with an isocyanate under conditions equivalent to those described in Scheme 3a, step a, to form 3-4c.

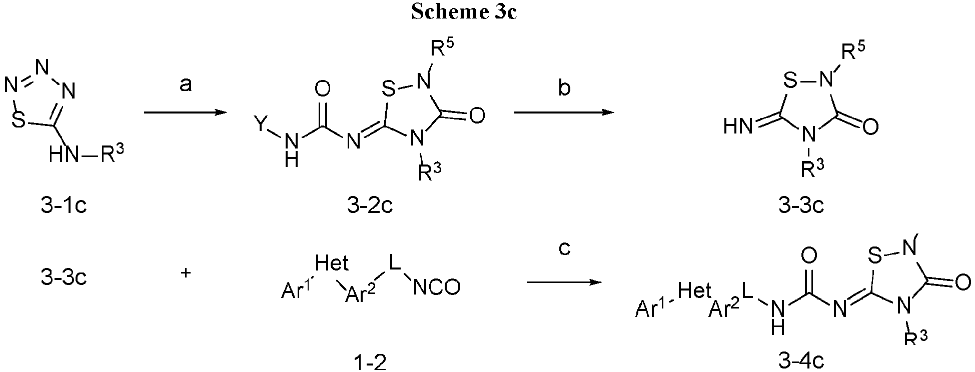

### PREPARATION OF TRIARYL-INTERMEDIATES

Compounds of Formula One, Two, and/or Three can be prepared by making a triaryl intermediate, Ar¹-Het-Ar², and then linking it to an appropriate intermediate to form a desired compound. A wide variety of triaryl intermediates can be used to prepare compounds of Formula One, Two, and/or Three, provided that such triaryl intermediates contain a suitable functional group on Ar² to which the rest of the desired functional group can be attached. Suitable functional groups include an amino, amino via nitro, isocyanate, carboxyl, or a halogen (preferably bromo or iodo). These triaryl intermediates can be prepared by methods previously described in the chemical literature, including Crouse, et al., WO2009102736.

The triaryl aldehydes used as precursors in preparation of the compounds of Formula One, Two, and/or Three can be prepared according to procedures described in Crouse, et al., US 2012/0202688 A1. Some of the procedures described above require use of halo-aryl intermediates, Ar¹-Het-Ph-Br, which are novel intermediates. These may be prepared as described in Scheme 4. 3-(4-Bromophenyl)-1,2,4-triazole (4-2, step a) is prepared in two steps from 4-bromobenzamide (4-1) under conditions described previously (Crouse, et al., WO2009102736). This triazole can then be coupled to an aryl halide (R = C₁-C₆ haloalkoxy) such as 4-trifluoromethoxyphenyl bromobenzene, in the presence of cesium carbonate or potassium phosphate, in a polar aprotic solvent such as dimethylformamide. This reaction is catalyzed by a copper salt such as copper(I) iodide and a chelator such as 8-hydroxyquinoline, both present in about 0.05 to about 0.25 equivalents, at a temperature ranging between about 80 °C and about 140 °C, to form the 1-aryl-3-(4-bromophenyl) triazole (4-4, step b).

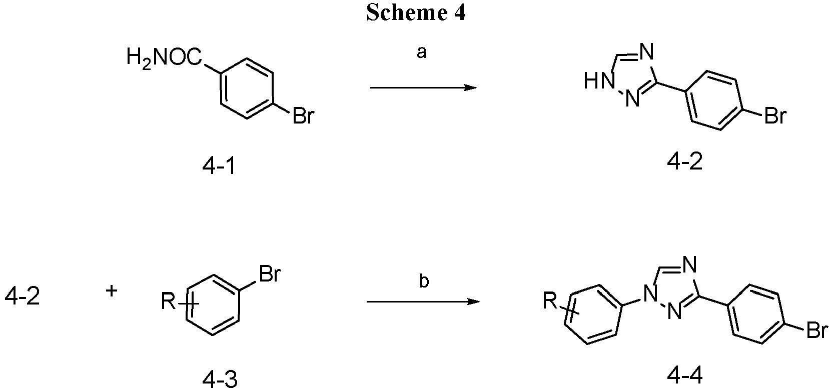

### PREPARATION OF 1-ATOM LINKED INTERMEDIATES

Compounds of Formula One, Two, and/or Three wherein L is a one-carbon linker, can be prepared from acid or amine intermediates described in Scheme 5 and Scheme 6, respectively. Acid precursors Ar¹-Het-Ar²-L-CO₂H, unsubstituted or mono- or di-substituted with R⁸; can be prepared as shown in the Scheme 5. Boronic esters (5-2, step a) can be prepared using Miyaura conditions from halophenyl esters (5-1). Coupling of the boronate esters with a bromo-heterocycle (5-3, step b) can be accomplished using a palladium catalyst and phosphine ligand, in the presence of a base, such as sodium bicarbonate, potassium phosphate, or cesium fluoride, in a suitable solvent system, such as dioxane/water, at temperatures from about 50 °C to about 120 °C to form triaryl ester intermediates (5-4, step c). Among palladium catalysts, tetrakis(triphenylphosphine) palladium(0) is preferred, although other well-known palladium catalysts may be used. Saponification of the ester may be achieved by using a strong base such as sodium hydroxide or lithium hydroxide in methanol or ethanol with or without tetrahydrofuran/water to furnish the desired carboxylic acid (5-5, step c).

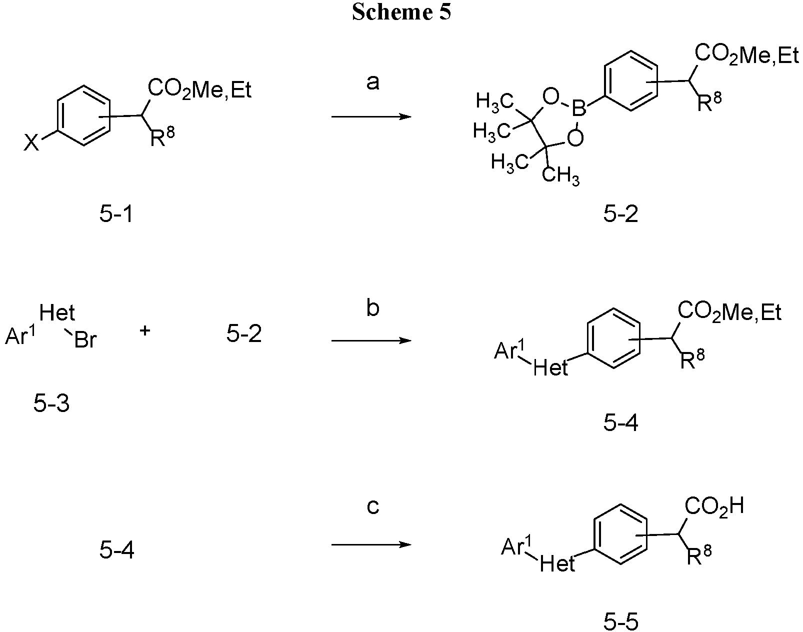

Amine precursors Ar¹-Het-Ar²-L-NH₂, unsubstituted or mono- or di-substituted with R⁸, can be prepared as shown in the Scheme 6. Halobenzyl amines (6-1) may be protected using benzyl chloroformate in the presence of a base such as triethylamine in an aprotic solvent such as dichloromethane from about -10 °C to about 10 °C to provide *N*-carboxybenzyl (Cbz) protected benzyl amines (6-2, step a). Alternatively, other *N*-protecting groups such as tert-butoxycarbonyl (BOC) or 9-fluorenylmethylcarbonyl (Fmoc) may be employed in step a using similar conditions described above for Cbz. The Cbz protected boronic ester 6-3 can be prepared using Miyaura conditions (step b). Coupling of the boronate esters with a bromo-heterocycle (5-3) can be accomplished using a palladium catalyst and phosphine ligand, in the presence of a base, such as sodium bicarbonate, potassium phosphate, or cesium fluoride, in a suitable solvent system, such as dioxane/water, at temperatures from about 50 °C to about 120 °C to form N-protected aminoalkylphenyl intermediates (6-4, step c). Removal of the Cbz group can be accomplished under acidic conditions with a strong acid such as hydrogen bromide, followed by free basing with a base such as sodium bicarbonate or sodium hydroxide, to furnish the free amine precursors Ar¹-Het-Ar²-L-NH₂ (6-5, step d). Similar methods could be applied to compounds wherein L is greater than 1-carbon.

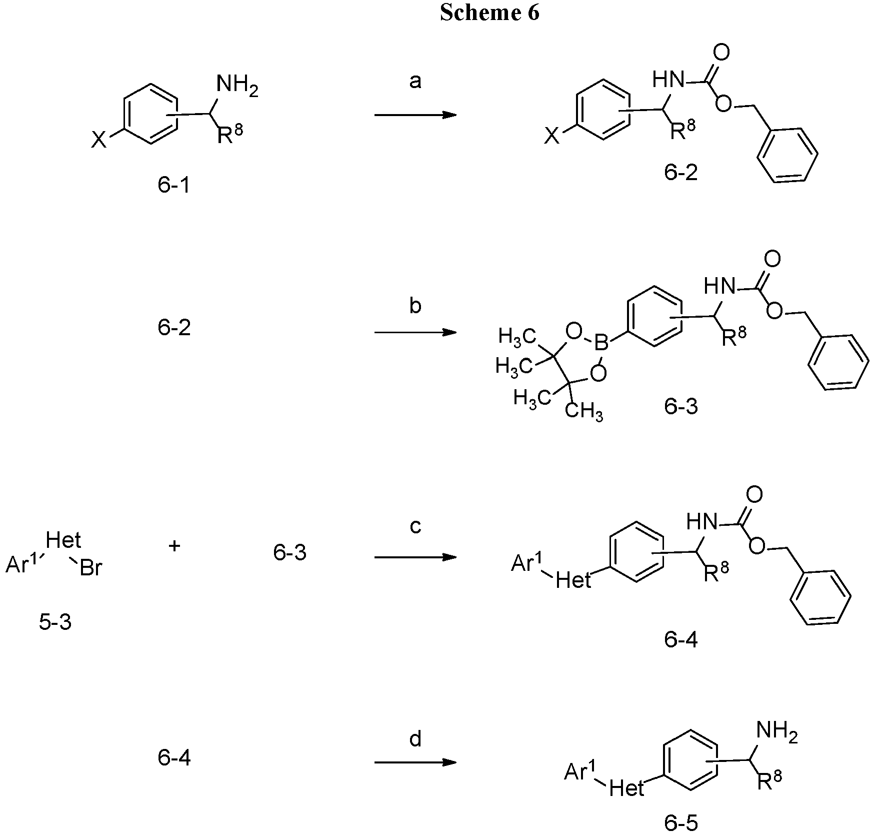

### PREPARATION OF ETHYL LINKED INTERMEDIATES

Preparation of compounds wherein L is a two-atom group is described in Schemes 7 to Schemes 9. Condensation of the aldehyde (7-1, R⁹ = H) (described in US 2012/0202688 A1) with reagents such as ethyl diethylphosphonoacetate or a Wittig reagent such as ethyl 2-(triphenylphosphoranylidene)propanoate) or α-substituted acetates such as ethyl 2-fluoroacetate or ethyl 2-cyanoacetate in the presence of a suitable base such as sodium hydride or n-butyl lithium in aprotic solvents such as tetrahydrofuran or diethyl ether at temperatures from about - 78 °C to about 20 °C can be used to prepare acrylic esters (7-2, step a) unsubstituted or mono-substituted with R⁹ and R¹⁰. Saponification of the resultant ester may be achieved by using a strong base such as sodium hydroxide in methanol or ethanol with or without tetrahydrofuran/water to furnish the vinyl carboxylic acid (7-3, step b). In some cases, the partial condensation of aldehyde (7-1, R⁹ = H) may result in the isolation of the alcohol intermediate (7-4, step c) especially when R¹⁰ is electron withdrawing. Substitution of this alcohol with nucleophilic reagents such as Deoxo-Fluor^{®} (step d) followed by saponification as described above (step e) can generate highly substituted ethyl carboxylic acids (7-5) additionally substituted with R¹¹, wherein R¹¹ is defined as R⁸ above. When the saturated linkage is preferred, the acrylate ester (7-2) can be converted to the corresponding cyclopropane (7-6, step f) unsubstituted or mono- or di-substituted with R¹²; with sulfur ylides such as those formed *in situ* from trimethyl sulfonium iodide in the presence of an inorganic base such as sodium hydride in a polar aprotic solvent such as dimethyl sulfoxide or tetrahydrofuran. Likewise, the acrylate ester (7-2) can be reduced to the parent alkane (7-8, step h) using hydrogen gas and a palladium catalyst. Both the cyclopropane and the alkane can be hydrolyzed under basic conditions described above to generate the free carboxylic acids 7-7 (step g) and 7-9 (step i), respectively.

In a similar manner, condensation of the ketone (7-1, R⁹ = alkyl) (described in WO 2011017504 A1) with either ethyl diethylphosphonoacetate or a Wittig reagent such as ethyl 2-(triphenylphosphoranylidene)propanoate or α-substituted alkyl esters such as ethyl 2-fluoroacetate or ethyl 2-cyanoacetate under similar conditions described above may generate the α-alkyl acrylate esters 7-2 or alcohols 7-4. Subsequent treatment of 7-2 or 7-4 as described above for R⁹ = H may lead to either the corresponding unsaturated (7-3) or saturated (7-5, 7-7, 7-9) carboxylic acids.

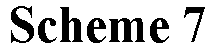

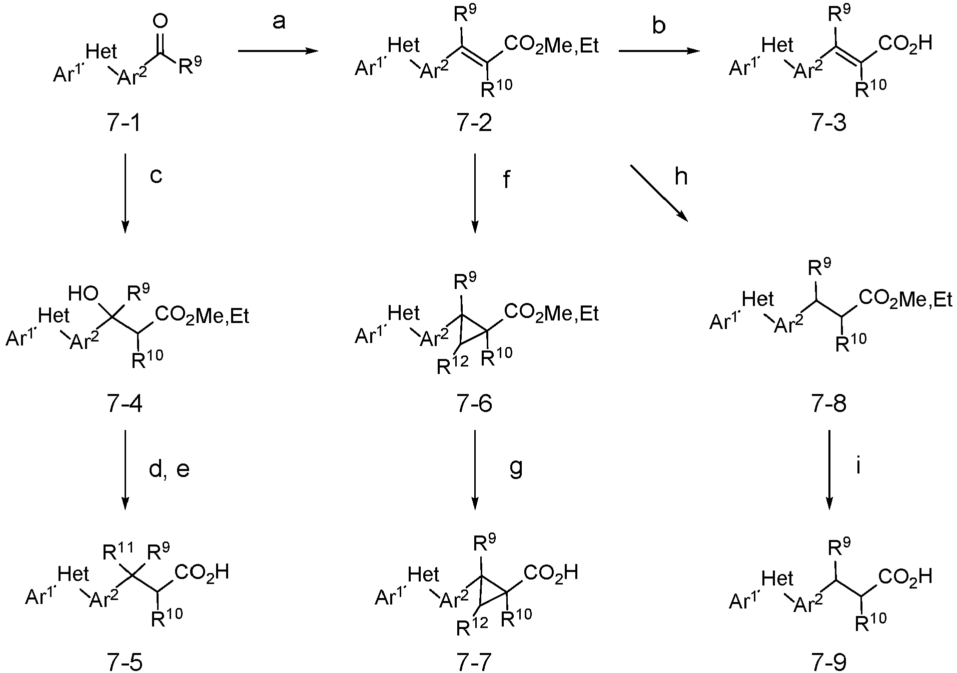

Alternatively, compounds wherein L is a 2-carbon linker may also be prepared as shown in Scheme 8. Using conditions first described by Molander et al. Org. Lett. 2007, 9, pp 203-206, coupling of a bromide Ar¹-Het-Ar²-Br (8-1, step a), with potassium (2*-*((*tert-*butoxycarbonyl)amino)ethyl)trifluoroborate in the presence of a palladium catalyst such as palladium(II) acetate, and a base such as cesium carbonate, at temperatures from about 80 °C to about 120 °C, results in the formation of the corresponding 2-(*tert*-butoxycarbonyl)amino)ethyl derivative 8-2. Further treatment of this material with from about 1 to about 5 equivalents of an acid such as trifluoroacetic acid or hydrogen chloride, in an aprotic solvent such as dichloromethane or dioxane at temperatures from about 0 °C to about 50 °C, results in the cleavage of the tert-butoxycarbonyl group and formation of the trifluoroacetic or hydrochloric acid salts, respectively, of the amine Ar¹-Het-Ar²-L-NH₂ (8-3, step b).

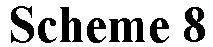

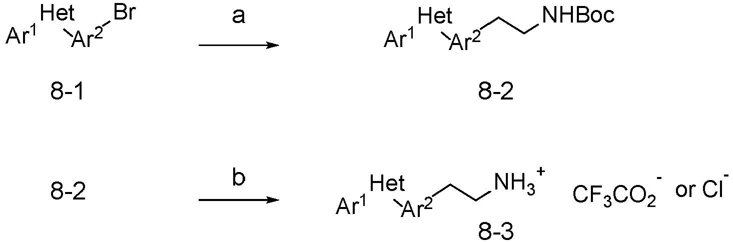

Aminoalkyl precursors Ar¹-Het-Ar²-L-NH₂, wherein L is 2-carbon atoms, mono- or di-substituted with R⁹, wherein R⁹ is defined as above; and unsubstituted or mono-substituted with R¹⁰, wherein R¹⁰ is defined as above, can be prepared as shown in Scheme 9. Halophenyl carbinols 9-1, wherein Y can be selected from Cl, Br, or I, unsubstituted at R⁹ and R¹⁰ are available commercially. Carbinols 9-1 that are mono- or di-substituted at R⁹ can be prepared from the corresponding halophenyl acetate (9-I, step a) in similar fashion to that described by Shin et al. Bioorg. Med. Chem. Lett. 2008, 18, pp 4424-4427 followed by reduction with a metal hydride such as lithium aluminum hydride in an ethereal solvent such as tetrahydrofuran or diethyl ether at temperatures at or below about 0 °C. Both 9-I and 9-II may be further mono-substituted (step b or step c) with R¹⁰ *via* reduction to the corresponding aldehyde with a metal hydride such as diisobutylaluminum hydride and further treatment with a Grignard reagent in a similar fashion to that described by Brimble et al. Org. Lett. 2012, 14, pp 5820-5823. Carbinols 9-1 can be treated with phthalimide under Mitsunobu conditions to generate *N*-phthalimido intermediates 9-2 (step d). The halide can be converted into a boronic ester under Miyaura conditions to form boronate esters (9-3, step e). Coupling of the boronate esters with a bromo-heterocycle can be accomplished using a palladium catalyst, such tetrakis(triphenylphosphine) palladium(0), in the presence of a base, such as sodium bicarbonate, in a suitable solvent system, such as dioxane/water, at temperatures from about 50 °C to about 120 °C to provide *N-*phthalimido intermediates 9-4 (step f). Deprotection using hydrazine and methanol, or other suitable solvent can furnish the amine 9-5 (step g).

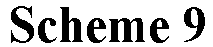

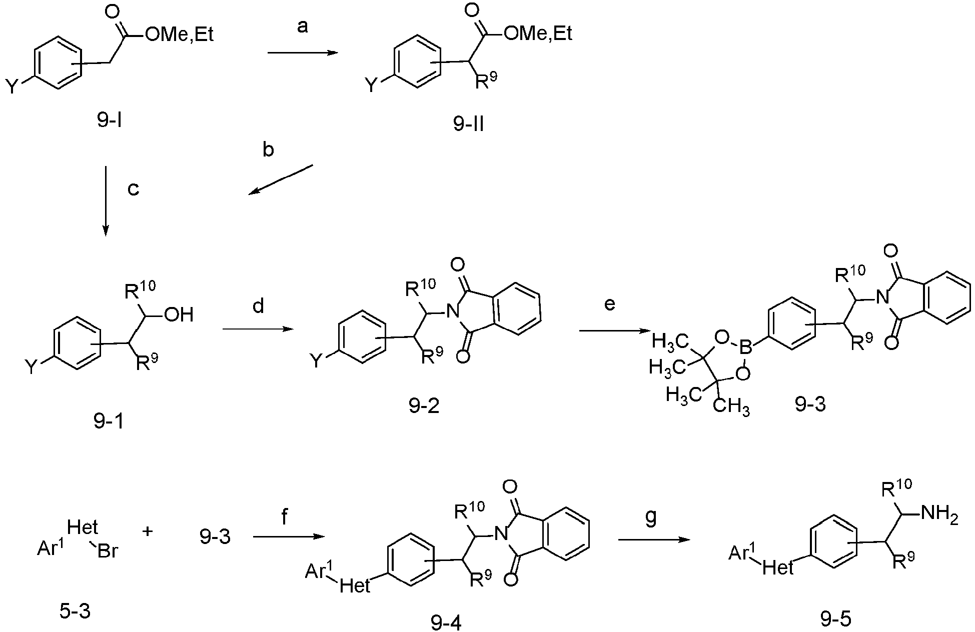

Alternatively, compounds wherein L is a 2-atom linker may also be prepared as shown in Scheme 9a. Olefination of aldehyde (7-1, R⁹ = H, step a) may be achieved with methylenetriphenylphosphorane which can be prepared from methyl triphenylphosphonium iodide in the presence of a base such as sodium hydride or 1,8-diazabicycloundec-7-ene in an aprotic solvent such as tetrahydrofuran or dichloromethane at temperatures of about -78 °C to about 40 °C. Further treatment of this material (9-2a) with a hydroborating reagent such as 9-borabicyclo(3.3.1)nonane in an aprotic solvent such as tetrahydrofuran followed by oxidation with an oxidant such as hydrogen peroxide can generate ethyl alcohol 9-3a (step b). Carbinols 9-3a can be treated with phthalimide under Mitsunobu conditions to generate *N*-phthalimido intermediates 9-5a (step c), wherein R¹⁰ = H. Deprotection using hydrazine and methanol, or other suitable solvent can furnish the amine 9-6a (step f). Additionally, 9-3a may be further mono-substituted (step d) with R¹⁰, wherein R¹⁰ is defined as above, *via* oxidation to the corresponding aldehyde under Swern conditions followed by addition of a Grignard reagent such as described above (Scheme 9). Carbinols 9-4a can be further treated with phthalimide under Mitsunobu conditions to generate *N*-phthalimido intermediates 9-5a (step e). Deprotection using hydrazine and methanol, or other suitable solvent can furnish the amine 9-6a (step f).

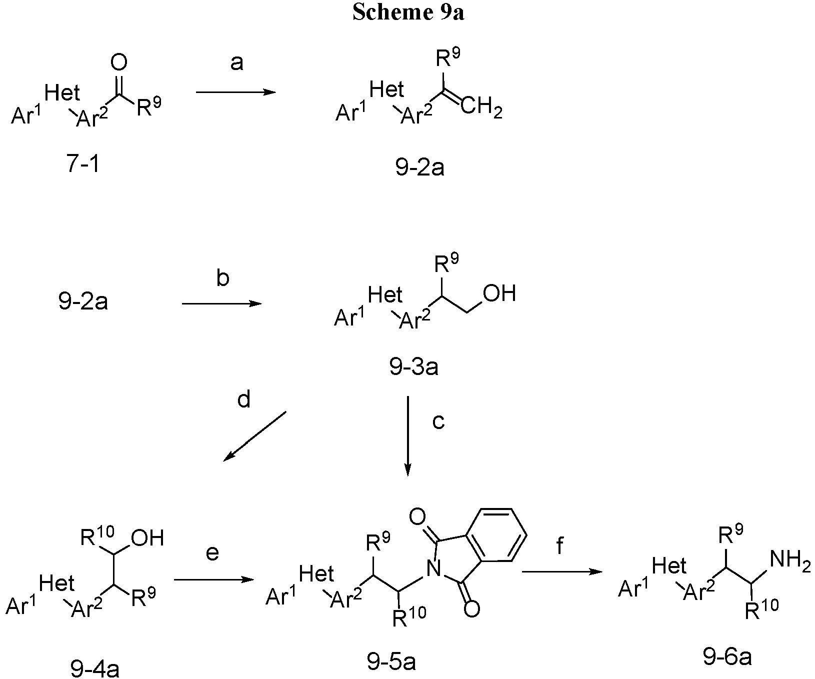

Scheme 9b outlines an alternative route for constructing analogs wherein the linker L is a two-atom linker. Copper-catalyzed arylation of 2,4-pentane-2,4-dione with 8-1 (*J. Am. Chem. Soc.* **2010,** *132*, 8273) may provide the substituted acetone intermediate 9-1b (step a). Reductive amination (step b), using any of a variety of conditions familiar to those skilled in the art, may generate amine 9-2b, which may be converted into the target molecules using conditions described previously in Scheme 2. When a linker contains a chiral center, such as with intermediate 9-2b, these intermediates may be separated into their pure isomeric forms either by means of a chiral column, or by fractional crystallization of the salt prepared from a chiral acid such as (+) and (-) tartaric acid.

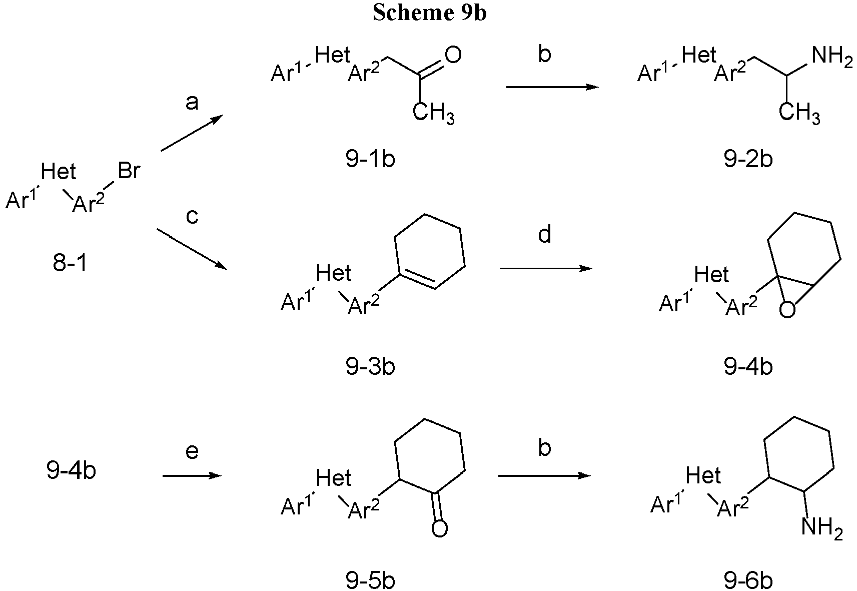

Construction of analogs wherein the ethyl linking group is part of a 6-membered ring may also be accomplished starting from bromide 8-1. Coupling of 8-1 with 2-cyclohex-1-enyl-4,4,5,5-tetramethyl-1,3,2-dioxaborolane (Scheme 9b, step c) under standard Suzuki coupling conditions can lead to alkene 9-3b. Epoxidation with standard reagents, such as *meta-*chloroperoxybenzoic acid (step d), followed by acid-catalyzed rearrangement using indium trichloride (J. Org.Chem. 1998, 63, 8212) may generate ketone 9-5b. Reductive amination and conversion into the target molecules can be accomplished using conditions described above.

### PREPARATION OF PROPYL LINKED INTERMEDIATES

Preparation of compounds wherein L is a three-atom group is described in Schemes 10 and 11. Aminoalkyl precursors Ar¹-Het-Ar²-L-NH₂, wherein L is 3-carbon atoms, mono- or di-substituted with R⁹, wherein R⁹ is defined as above; and unsubstituted or mono-substituted with R¹⁰, wherein R¹⁰ is defined as above; can be prepared as shown in Scheme 10. Halophenyl carbinol 10-1, wherein Z is Br and R⁹ and R¹⁰ are H, is available commercially. Carbinols 10-1 that are mono- or di-substituted at R⁹ can be prepared from the corresponding halophenyl acetate (10-I, step a) in similar fashion to that described by Shin et al. Bioorg. Med. Chem. Lett. 2008, 18, pp 4424-4427, followed by reduction with a metal hydride such as lithium aluminum hydride in an ethereal solvent such as tetrahydrofuran at temperatures at or below about 0 °C. Both 10-I and 10-II may be further mono-substituted (step b or step c) with R¹⁰ *via* reduction to the corresponding aldehyde with a metal hydride such as diisobutylaluminum hydride and further treatment with a Grignard reagent such as methylmagnesium bromide in a similar fashion to that described by Brimble et al. Org. Lett. 2012, 14, pp 5820-5823. Carbinols 10-1 can be treated with phthalimide under Mitsunobu conditions to generate *N*-phthalimido intermediates 10-2 (step d).

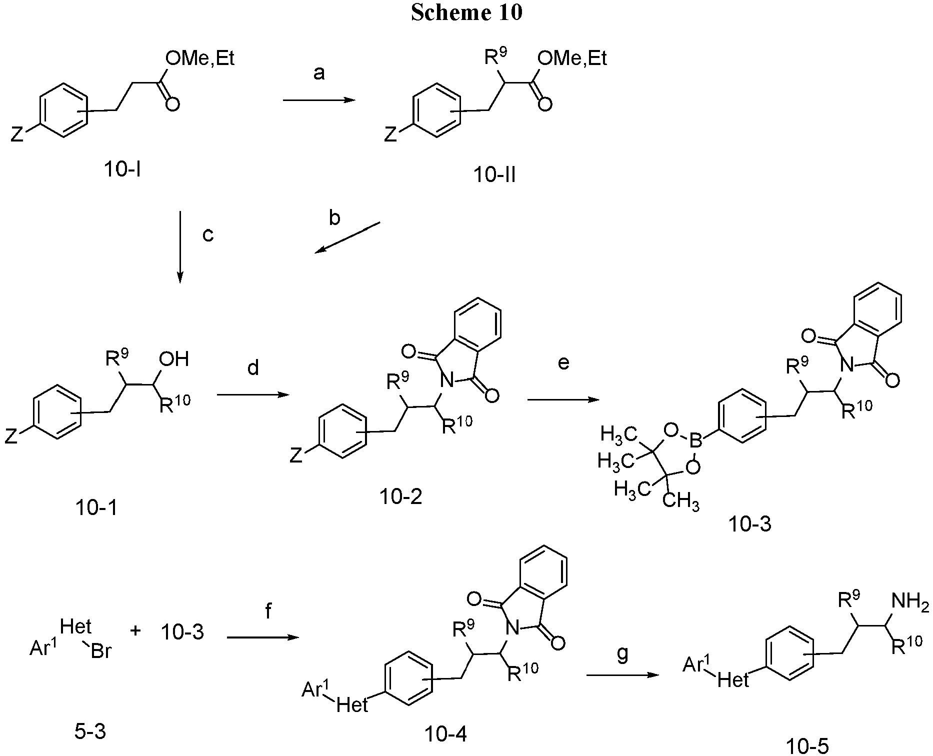

The halide can be converted into a boronic ester under Miyaura conditions (10-3, step e). Coupling of the boronate esters with a bromo-heterocycle can be accomplished using a palladium catalyst, such tetrakis(triphenylphosphine) palladium(0), in the presence of a base, such as sodium bicarbonate, in a suitable solvent system, such as dioxane/water, at temperatures from about 50 °C to about 120 °C to provide N-phthalimido intermediates 10-4 (step f). Deprotection using hydrazine and methanol, or other suitable solvent can furnish the amine 10-5 (step g).

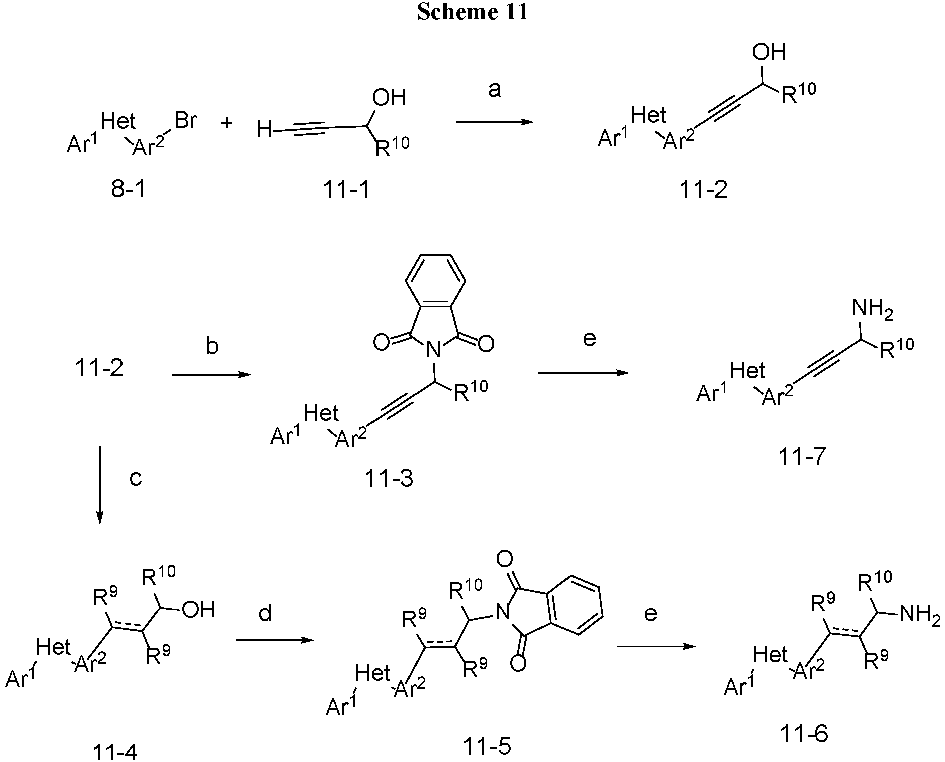

Alternatively, compounds wherein L is a 3-atom linker may also be prepared as shown in Scheme 11. Bromide Ar¹-Het-Ar²-Br (8-1) can be coupled with an appropriate alkynyl alcohol (11-1, step a) unsubstituted or mono-substituted with R¹⁰, wherein R¹⁰ is defined as above; in the presence of a palladium catalyst such as bistriphenylphosphine dichloropalladium(II), copper(I) iodide, and a base such as triethylamine, at temperatures from about 50 °C to about 120 °C, to generate the corresponding alkynyl alcohol derivatives 11-2. The resultant carbinols 11-2 can be treated with phthalimide under Mitsunobu conditions to generate *N*-phthalimido intermediates 11-3 (step b) which can be converted to amines (11-7, step e) using hydrazine and methanol or other suitable solvent. Carbinols 11-2 can be reduced using a transition metal catalyst, such as palladium under an atmosphere of hydrogen to provide alkenyl or fully saturated alkyl carbinols 11-4 unsubstituted at R¹⁰. Additionally, carbinols 11-2 can be treated with a metal hydride such as lithium aluminum hydride to provide the (*E*)-alkenyl carbinol 11-4. Likewise, carbinol 11-2 may be protected with a protecting group such as *tert*-butyl diphenyl silane and treated with a hydrometallation reagent such as Schwartz' reagent followed by an electrophile quench, with, for example, elemental iodine or N-bromosuccinimide (NBS). Alternatively, the carbinol 11-2 may be treated with a transmetallation reagent such as pinacol diboron for further use in transition metal-catalyzed coupling reactions, such as Suzuki or Negishi, to prepare carbinols 11-4 mono-or di-substituted with R⁹, wherein R⁹ is defined as above (step c). Following deprotection, the resultant carbinols 11-4 can be treated with phthalimide under Mitsunobu conditions to generate N-phthalimido intermediates 11-5 (step d) which can be converted to an amine (11-6, step e) using hydrazine and methanol or other suitable solvent.

### PREPARATION OF BUTYL LINKED INTERMEDIATES

Compounds wherein L is a 4-atom linker may be prepared as shown in Scheme 12. Bromide Ar¹-Het-Ar²-Br (8-1) can be coupled with an appropriate alkynyl alcohol (12-1, step a) unsubstituted or mono-substituted with R¹⁰, wherein R¹⁰ is defined as above; mono- or di-substituted with R⁹, wherein R⁹ is defined as above; in the presence of a palladium catalyst such as bistriphenylphosphine dichloropalladium(II), copper(I) iodide, and a base such as triethylamine, at temperatures from about 50 °C to about 120 °C, to generate the corresponding alkynyl alcohol derivatives 12-2. The resultant carbinols 12-2 can be treated with phthalimide under Mitsunobu conditions (step b) to generate N-phthalimido intermediates 12-3 which can be converted to an amine (12-7, step e) using hydrazine and methanol or other suitable solvents. Carbinols 12-2 can be reduced using a transition metal catalyst, such as palladium under an atmosphere of hydrogen to provide alkenyl or fully saturated alkyl carbinols 12-4 (step c) unsubstituted at R¹³. Additionally, carbinols 12-2 can be treated with a metal hydride such as lithium aluminum hydride to provide the (E)-alkenyl carbinols 12-4 (step c). Likewise, carbinol 12-2 may be protected with a protecting group such as *tert*-butyl diphenyl silane and treated with a hydrometallation reagent such as Schwartz' reagent followed by an electrophile quench, with, for example, elemental iodine or NBS. Alternatively, the carbinol 12-2 may be treated with a transmetallation reagent such as pinacol diboron for further use in transition metal-catalyzed coupling reactions, such as Suzuki or Negishi, to prepare carbinols 12-4 mono- or di-substituted with R¹³, wherein R¹³ is defined as R⁸ above (step c).

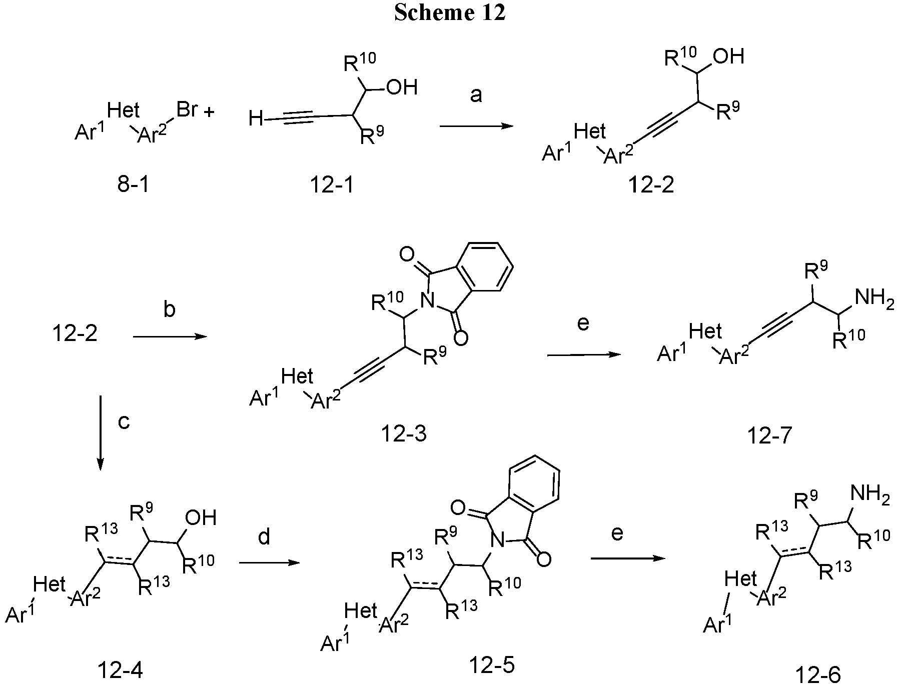

Following deprotection, the resultant carbinols 12-4 can be treated with phthalimide under Mitsunobu conditions (step d) to generate N-phthalimido intermediates 12-5 which can be converted to an amine (12-6, step e) using hydrazine and methanol or other suitable solvent.

### PREPARATION OF SUBSTITUTED THIOBIURETS

2-Imino 1,3-thiazolin-4-ones (3-1) may be further functionalized using a variety of conditions. When treated with Selectfluor^{®} and 9-fluorenone in anhydrous acetonitrile (*J*. *Am. Chem. Soc.* 2013, *135,* 17494), molecules having the formula 3-1 may be converted into the mono-fluoro analogs (13-1).

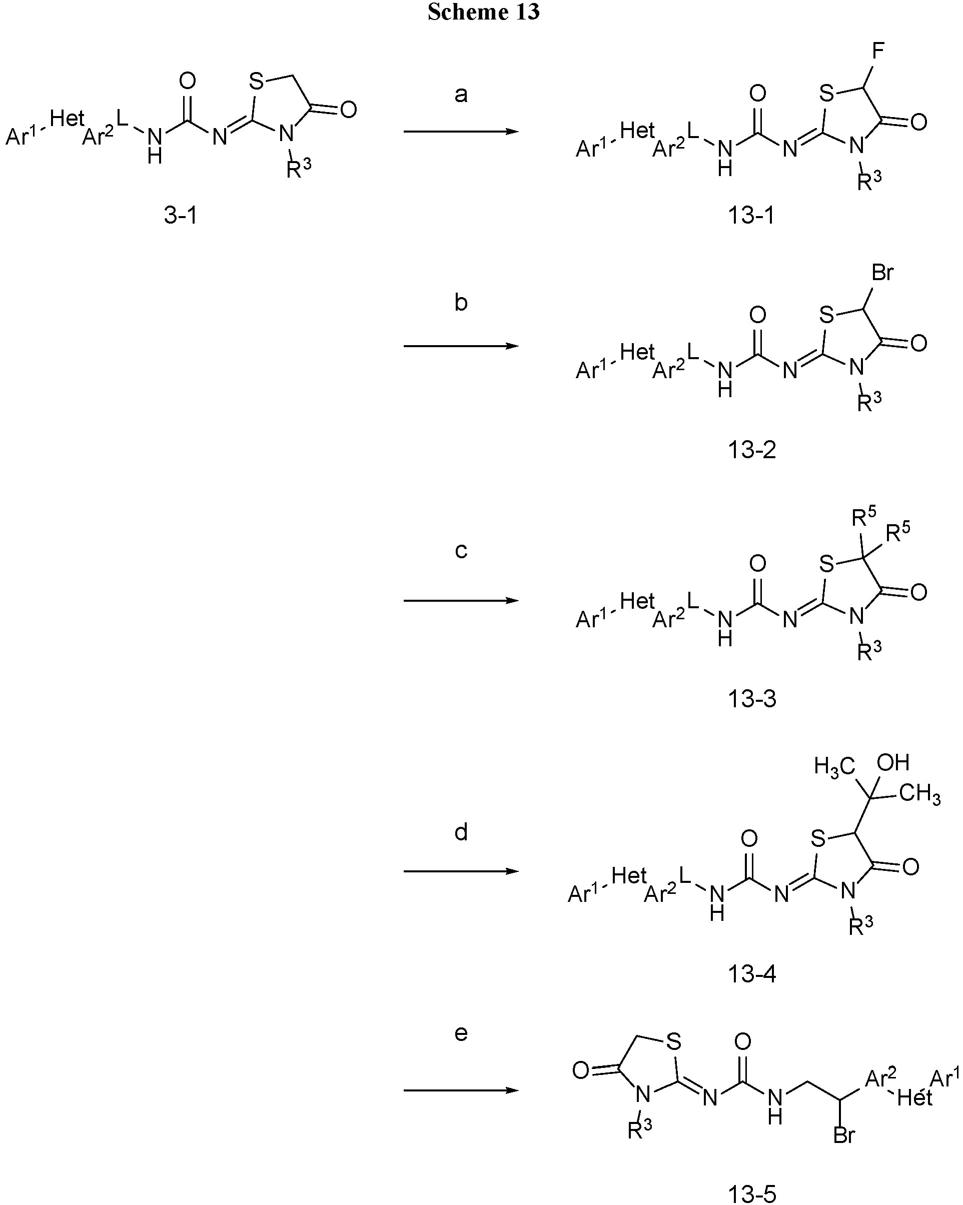

Treatment with molecular bromine in an aprotic solvent such as dichloromethane at from about 0 °C to about 30 °C (step b) may result in mono-bromination on the thiazolinone ring (13-2). Alkylation (step c), using at least 2 equivalents of an alkylating agent R⁵-I and a strong base such as sodium hydride or lithium diisopropylamide in a polar aprotic solvent such as dimethylformamide or tetrahydrofuran may lead to a di-alkylated product (13-3). Treatment with a ketone or an aldehyde and an inorganic base such as potassium carbonate or cesium carbonate may result in the formation of a carbinol (13-4). For analogs of compounds of the formula 3-1 wherein L is a -CH₂CH₂- group, free-radical bromination using N-bromosuccinimide and a free radical initiator such as azobis (isobutyronitrile) in carbon tetrachloride at about 30 °C to about 77 °C may lead to the mono-brominated product (13-5) wherein the bromine is incorporated into the ethyl linker.

The compounds of Formula One disclosed herein may be synthesized using methodology familiar to one skilled in the art, as well methods given in Fischer at al., United States Patent Application Publication 20140274688 A1 and Baum, et al., WO2016033025 A1, using known or commercially available starting materials.

### EXAMPLES

The following examples further illustrate the invention but, of course, should not be construed as in any way limiting its scope.

Starting materials, reagents, and solvents that were obtained from commercial sources were used without further purification. Anhydrous solvents were purchased as Sure/Seal^{™} from Aldrich and were used as received. Melting points were obtained on a Thomas Hoover Unimelt capillary melting point apparatus or an OptiMelt Automated Melting Point System from Stanford Research Systems and are uncorrected. Examples using "room temperature (or rt)" were conducted in climate controlled laboratories with temperatures ranging from about 20 °C to about 24 °C. Molecules are given their known names, named according to naming programs within ISIS Draw, ChemDraw or ACD Name Pro. If such programs are unable to name a molecule, the molecule is named using conventional naming rules. ¹H NMR spectral data are in ppm (δ) and were recorded at 300, 400 or 500 MHz; ¹³C NMR spectral data are in ppm (δ) and were recorded at 101 or 126 MHz; and ¹⁹F NMR spectral data are in ppm (δ) and were recorded at 376 or 471 MHz unless otherwise stated.

### EXAMPLE 1

### Preparation of (Z)-1-(2-methyl-4-(1-(4-(trifluoromethoxy)phenyl))-1H-1,2,4-triazol-3-yl)phenyl)-3-(4-oxo-3-(6-((2,2,2-trifluoroethoxy)methyl)benzo[d][1,3]dioxol-5-yl)thiazolidin-2-ylidene)urea (F1)

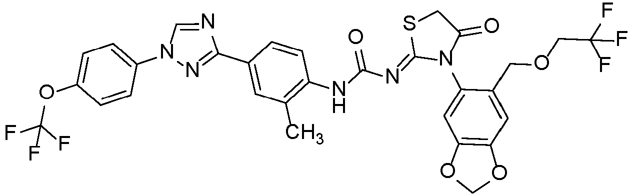

To 2-methyl-4-(1-(4-(trifluoromethoxy)phenyl)-1*H*-1,2,4-triazol-3-yl)aniline **(C2;** 50 milligrams (mg), 0.150 millimoles (mmol)) and bis(2,5-dioxopyrrolidin-1-yl) carbonate (46 mg, 0.179 mmol) in dichloromethane (DCM; 1.02 mL) was added pyridine (14.5 microliters (µL), 0.179 mmol), and the reaction mixture was stirred at 23 °C for 30 minutes. 2-Imino-3-(6-((2,2,2-trifluoroethoxy)methyl)benzo[*d*][1,3]dioxol-5-yl)thiazolidin-4-one (C26; 52 mg, 0.150 mmol), sodium bicarbonate (101 mg, 1.20 mmol) and water (1.02 mL) were added sequentially, and the reaction mixture was allowed to continue stirring at room temperature for 1 hour (h). The reaction mixture was filtered through a phase separator, rinsing with DCM. The filtrate was concentrated onto silica. Purification via flash chromatography (silica; ethyl acetate (EtOAc)/DCM-Hexanes, 1:1) yielded the title compound as an off-white solid (86 mg, 80%).

*The following compounds were prepared in accordance with the procedure in Example 1.* (Z)-1-(2-Fluoro-4-(1-(4-(trifluoromethoxy)phenyl)-1*H*-1,2,4-triazol-3-yl)phenyl)-3-(4-oxo-3-(6-((2,2,2-trifluoroethoxy)methyl)benzo[d][1,3]dioxol-5-yl)thiazolidin-2-ylidene)urea **(F2)**

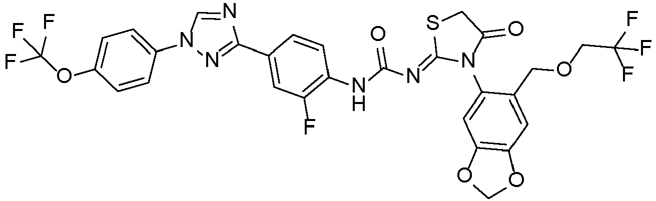

The title compound was prepared and was isolated as a white solid (61 mg, 57%). (*Z*)-1-(2-Fluoro-4-(1-(4-(pentafluoro-λ⁶-sulfaneyl)phenyl)-1*H*-1,2,4-triazol-3-yl)phenyl)-3-(4-oxo-3-(6-((2,2,2-trifluoroethoxy)methyl)benzo[*d*][1,3]dioxol-5-yl)thiazolidin-2-ylidene)urea **(F3)**

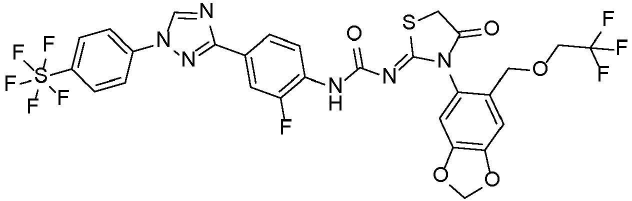

The title compound was prepared and was isolated as a white solid (51 mg, 50%). (*Z*)-1-(3-(Benzofuran-4-yl)-4-oxothiazolidin-2-ylidene)-3-(2-fluoro-4-(1-(4-(trifluoromethoxy)phenyl)-1*H*-1,2,4-triazol-3-yl)phenyl)urea **(F4)**

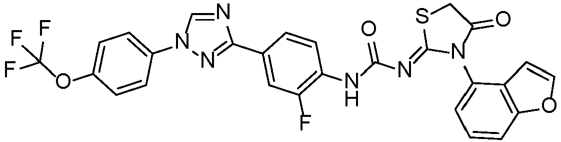

The title compound was prepared and was isolated as a yellow solid (31 mg, 23%). (*Z*)-1-(3-(Benzofuran-7-yl)-4-oxothiazolidin-2-ylidene)-3-(2-fluoro-4-(1-(4-(trifluoromethoxy)phenyl)-1H-1,2,4-triazol-3-yl)phenyl)urea **(F5)**

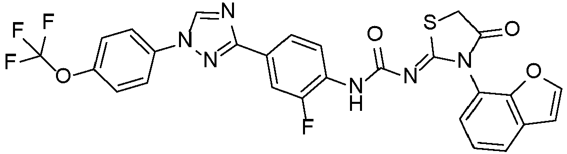

The title compound was prepared and was isolated as a yellow solid (35 mg, 26%). (*Z*)-1-(3-(2,3-Dihydrobenzofuran-4-yl)-4-oxothiazolidin-2-ylidene)-3-(2-fluoro-4-(1-(4-(trifluoromethoxy)phenyl)-1*H*-1,2,4-triazol-3-yl)phenyl)urea **(F6)**

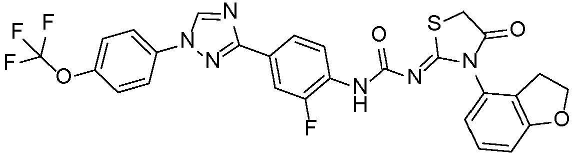

The title compound was prepared and was isolated as a white solid (35 mg, 26%). (*Z*)-1-(3-(Benzo[*d*]oxazol-4-yl)-4-oxothiazolidin-2-ylidene)-3-(2-fluoro-4-(1-(4-(trifluoromethoxy)phenyl)-1*H*-1,2,4-triazol-3-yl)phenyl)urea **(F7)**

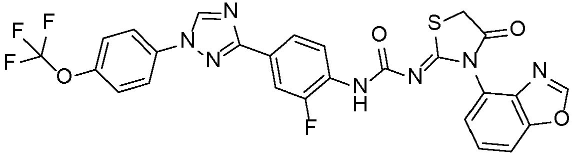

The title compound was prepared and was isolated as an off-white solid (33 mg, 27%). (*Z*)-1-(3-(1,3-Dihydroisobenzofuran-4-yl)-4-oxothiazolidin-2-ylidene)-3-(2-fluoro-4-(1-(4-(trifluoromethoxy)phenyl)-1*H*-1,2,4-triazol-3-yl)phenyl)urea **(F8)**

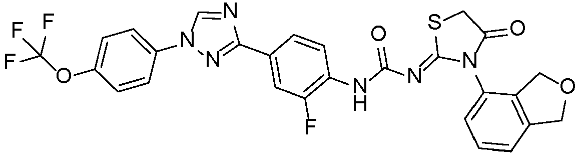

The title compound was prepared and was isolated as a white oil (53 mg, 36%). (*Z*)-1-(3-(2,3-Dihydrobenzofuran-7-yl)-4-oxothiazolidin-2-ylidene)-3-(2-fluoro-4-(1-(4-(trifluoromethoxy)phenyl)-1*H*-1,2,4-triazol-3-yl)phenyl)urea **(F9)**

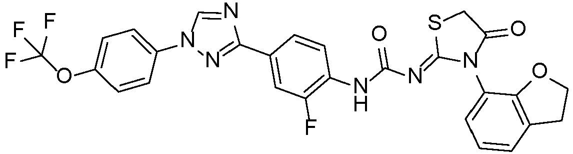

The title compound was prepared and was isolated as a yellow solid (146 mg, 47%). (Z)-1-(3-(2,3-Dihydro-1*H*-inden-4-yl)-4-oxothiazolidin-2-ylidene)-3-(2-fluoro-4-(1-(4-(trifluoromethoxy)phenyl)-1*H*-1,2,4-triazol-3-yl)phenyl)urea **(F10)**

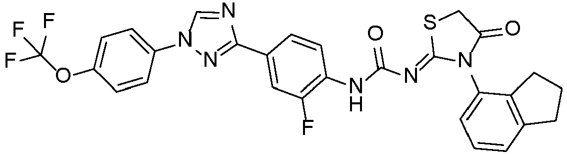

The title compound was prepared and was isolated as a white solid (55 mg, 28%). (*Z*)-1-(3-(1,3-Dioxo-1,3-dihydroisobenzofuran-5-yl)-4-oxothiazolidin-2-ylidene)-3-(2-fluoro-4-(1-(4-(trifluoromethoxy)phenyl)-1*H*-1,2,4-triazol-3-yl)phenyl)urea **(F11)**

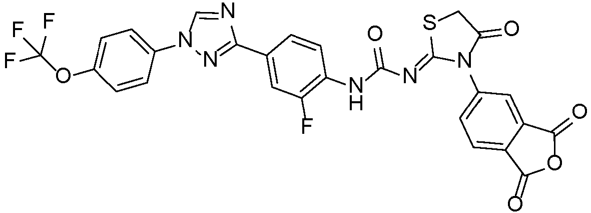

The title compound was prepared and was isolated as a white solid (23 mg, 14%). (*Z*)-1-(2-Fluoro-4-(1-(4-(trifluoromethoxy)phenyl)-1*H*-1,2,4-triazol-3-yl)phenyl)-3-(3-(4-methyl-3,4-dihydro-2H-benzo[b][1,4]oxazin-8-yl)-4-oxothiazolidin-2-ylidene)urea **(F12)**

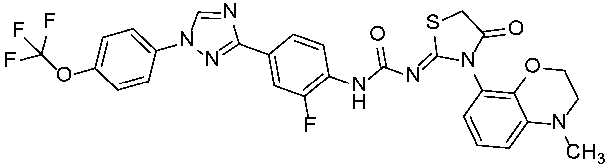

The title compound was prepared and was isolated as a yellow solid (21 mg, 8.1%). (*Z*)-1-(2-Fluoro-4-(1-(4-(trifluoromethoxy)phenyl)-1*H*-1,2,4-triazol-3-yl)phenyl)-3-(3-(naphthalen-2-yl)-4-oxothiazolidin-2-ylidene)urea **(F13)**

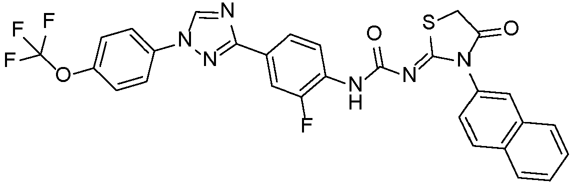

The title compound was prepared and was isolated as an orange solid (88 mg, 67%). (*Z*)-1-(2-Fluoro-4-(1-(4-(trifluoromethoxy)phenyl)-1*H*-1,2,4-triazol-3-yl)phenyl)-3-(3-(1-methyl-1*H*-indol-6-yl)-4-oxothiazolidin-2-ylidene)urea **(F14)**

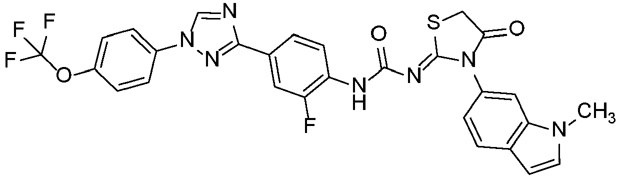

The title compound was prepared and was isolated as a yellow solid (51 mg, 67%). (*Z*)-1-(2-Fluoro-4-(1-(4-(trifluoromethoxy)phenyl)-1*H*-1,2,4-triazol-3-yl)phenyl)-3-(3-(1-methyl-1*H*-indol-4-yl)-4-oxothiazolidin-2-ylidene)urea **(F15)**

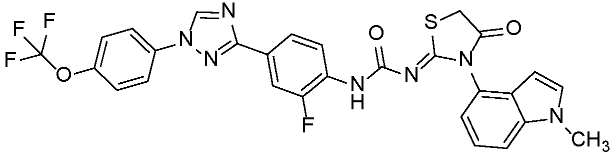

The title compound was prepared and was isolated as a yellow solid (80 mg, 51%). (*Z*)-1-(2-Fluoro-4-(1-(4-(trifluoromethoxy)phenyl)-1*H*-1,2,4-triazol-3-yl)phenyl)-3-(3-(naphthalen-1-yl)-4-oxothiazolidin-2-ylidene)urea **(F16)**

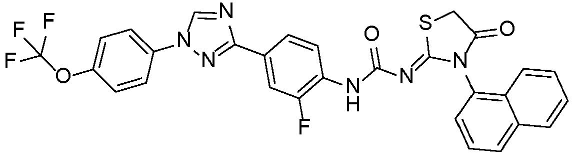

The title compound was prepared and was isolated as a yellow solid (70 mg, 53%). (*Z*)-1-(2-Fluoro-4-(1-(4-(trifluoromethoxy)phenyl)-1*H*-1,2,4-triazol-3-yl)phenyl)-3-(3-(1-methyl-1H-benzo[d]imidazol-4-yl)-4-oxothiazolidin-2-ylidene)urea **(F17)**

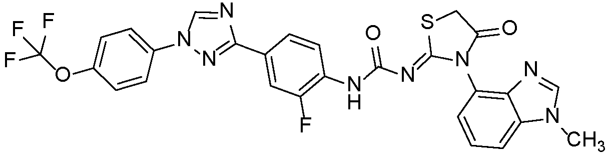

The title compound was prepared and was isolated as a yellow powder (26 mg, 56%). (*Z*)-1-(2-Fluoro-4-(1-(4-(trifluoromethoxy)phenyl)-1*H*-1,2,4-triazol-3-yl)phenyl)-3-(3-(isochroman-5-yl)-4-oxothiazolidin-2-ylidene)urea **(F18)**

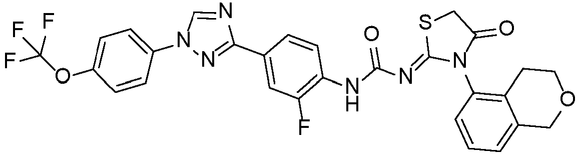

The title compound was prepared and was isolated as a yellow solid (43 mg, 17%). (Z)-1-(2-Fluoro-4-(1-(4-(trifluoromethoxy)phenyl)-1*H*-1,2,4-triazol-3-yl)phenyl)-3-(4-oxo-3-(1-oxo-1,3-dihydroisobenzofuran-4-yl)thiazolidin-2-ylidene)urea **(F19)**

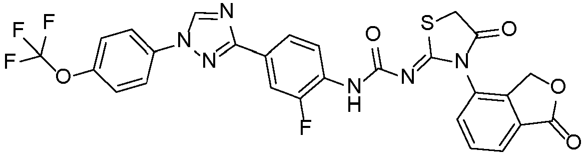

The title compound was prepared and was isolated as a white solid (39 mg, 32%). (*Z*)-1-(3-(Benzofuran-4-yl)-4-oxothiazolidin-2-ylidene)-3-(2-fluoro-4-(1-(4-(perfluoroethyl)phenyl)-1*H*-1,2,4-triazol-3-yl)phenyl)urea **(F20)**

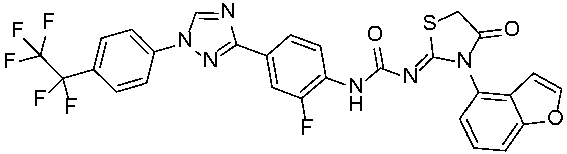

The title compound was prepared and was isolated as a yellow oil (33 mg, 62%). (*Z*)-1-(3-(Benzofuran-7-yl)-4-oxothiazolidin-2-ylidene)-3-(2-fluoro-4-(1-(4-(perfluoroethyl)phenyl)-1*H*-1,2,4-triazol-3-yl)phenyl)urea **(F21)**

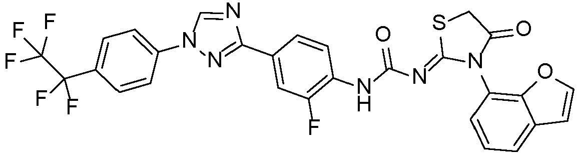

The title compound was prepared and was isolated as a yellow oil (34 mg, 64%). (*Z*)-1-(3-(Benzofuran-4-yl)-4-oxothiazolidin-2-ylidene)-3-(2-methyl-4-(1-(4-(perfluoroethoxy)phenyl)-1*H*-1,2,4-triazol-3-yl)phenyl)urea **(F22)**

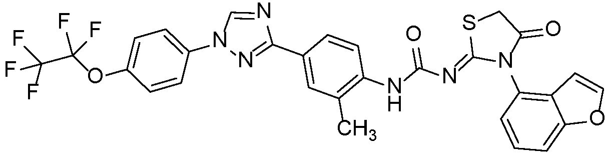

The title compound was prepared and was isolated as a white solid (30 mg, 57%). (*Z*)-1-(3-(Benzofuran-7-yl)-4-oxothiazolidin-2-ylidene)-3-(2-methyl-4-(1-(4-(perfluoroethoxy)phenyl)-1*H*-1,2,4-triazol-3-yl)phenyl)urea **(F23)**

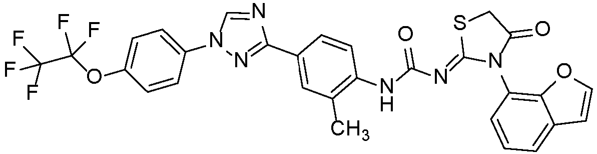

The title compound was prepared and was isolated as a white solid (32 mg, 61%). (*Z*)-1-(2-Fluoro-4-(1-(4-(perfluoroethyl)phenyl)-1*H*-1,2,4-triazol-3-yl)phenyl)-3-(3-(1-methyl-1*H*-indol-5-yl)-4-oxothiazolidin-2-ylidene)urea **(F24)**

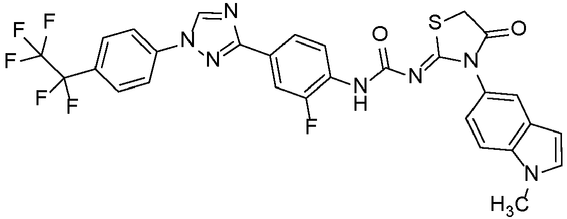

The title compound was prepared and was isolated as a yellow oil (20 mg, 35%). (*Z*)-1-(3-(2,3-Dihydrobenzofuran-4-yl)-4-oxothiazolidin-2-ylidene)-3-(2-methyl-4-(1-(4-(perfluoroethoxy)phenyl)-1*H*-1,2,4-triazol-3-yl)phenyl)urea **(F25)**

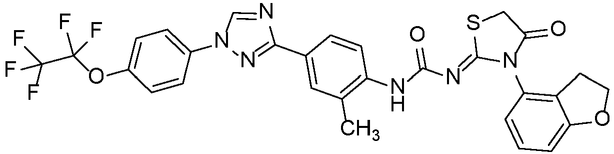

### (Z)-1-(3-(2,3-dihydrobenzofuran-4-yl)-4-oxothiazolidin-2-ylidene)-3-(2-methyl-4-(1-(4-(perfluoroethoxy)phenyl)-1H-1,2,4-triazol-3-yl)phenyl)urea

The title compound was prepared and was isolated as a yellow solid (32 mg, 55%). (*Z*)-1-(2-Methyl-4-(1-(4-(perfluoroethoxy)phenyl)-1*H*-1,2,4-triazol-3-yl)phenyl)-3-(3-(naphthalen-1-yl)-4-oxothiazolidin-2-ylidene)urea **(F26)**

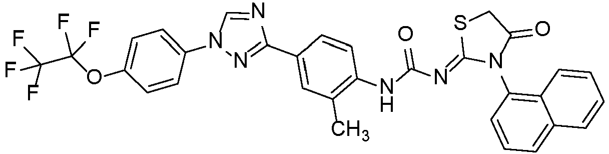

The title compound was prepared and was isolated as a yellow oil (30 mg, 56%). (*Z*)-1-(3-(1-Methyl-1*H*-indol-4-yl)-4-oxothiazolidin-2-ylidene)-3-(2-methyl-4-(1-(4-(perfluoroethoxy)phenyl)-1*H*-1,2,4-triazol-3-yl)phenyl)urea **(F27)**

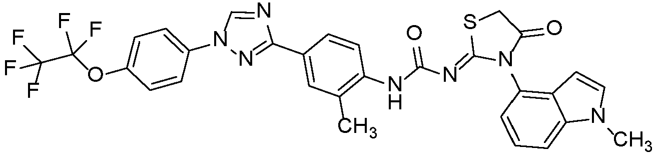

The title compound was prepared and was isolated as a yellow oil (35 mg, 66%). (*Z*)-1-(3-(2,3-Dihydrobenzofuran-7-yl)-4-oxothiazolidin-2-ylidene)-3-(2-methyl-4-(1-(4-(perfluoroethoxy)phenyl)-1*H*-1,2,4-triazol-3-yl)phenyl)urea **(F28)**

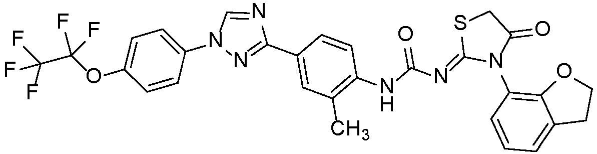

The title compound was prepared and was isolated as a yellow solid (29 mg, 55%). (*Z*)-1-(2-Methyl-4-(1-(4-(perfluoroethoxy)phenyl)-1*H*-1,2,4-triazol-3-yl)phenyl)-3-(3-(naphthalen-2-yl)-4-oxothiazolidin-2-ylidene)urea **(F29)**

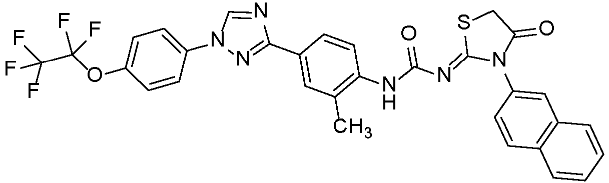

The title compound was prepared and was isolated as a yellow solid (27 mg, 51%). (*Z*)-1-(2-Fluoro-4-(1-(4-(perfluoroethyl)phenyl)-1*H*-1,2,4-triazol-3-yl)phenyl)-3-(3-(1-methyl-1*H*-indol-4-yl)-4-oxothiazolidin-2-ylidene)urea **(F30)**

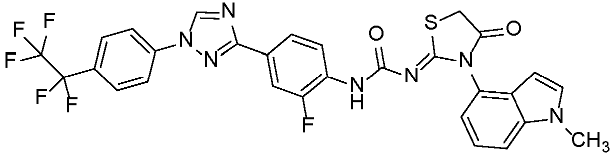

The title compound was prepared and was isolated as a yellow solid (26 mg, 49%). (*Z*)-1-(3-(1-Methyl-1*H*-indol-5-yl)-4-oxothiazolidin-2-ylidene)-3-(2-methyl-4-(1-(4-(perfluoroethoxy)phenyl)-1*H*-1,2,4-triazol-3-yl)phenyl)urea **(F31)**

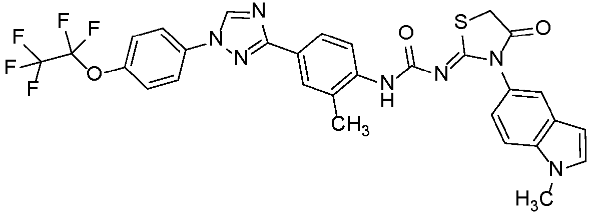

The title compound was prepared and was isolated as a yellow solid (32 mg, 60%). (*Z*)-1-(2-Methyl-4-(1-(4-(perfluoroethoxy)phenyl)-1*H*-1,2,4-triazol-3-yl)phenyl)-3-(4-oxo-3-(quinolin-5-yl)thiazolidin-2-ylidene)urea **(F32)**

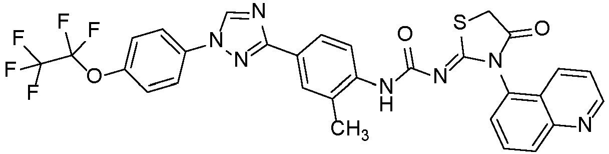

The title compound was prepared and was isolated as a white solid (31 mg, 57%). (Z)-1-(2-Fluoro-4-(1-(4-(trifluoromethyl)phenyl)-1*H*-1,2,4-triazol-3-yl)phenyl)-3-(4-oxo-3-(quinolin-5-yl)thiazolidin-2-ylidene)urea **(F33)**

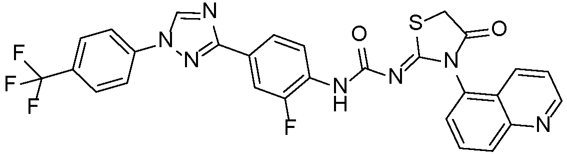

The title compound was prepared and was isolated as a white solid (32 mg, 55%). (*Z*)-1-(2-Fluoro-4-(1-(4-(perfluoroethyl)phenyl)-1*H*-1,2,4-triazol-3-yl)phenyl)-3-(4-oxo-3-(quinolin-5-yl)thiazolidin-2-ylidene)urea **(F34)**

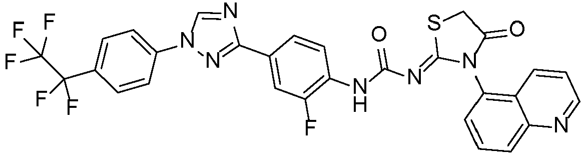

The title compound was prepared and was isolated as a white solid (29 mg, 50%).

### EXAMPLE 2

### Preparation of 2-fluoro-4-(1-(4-(trifluoromethoxy)phenyl)-1H-1,2,4-triazol-3-yl)aniline (C1)

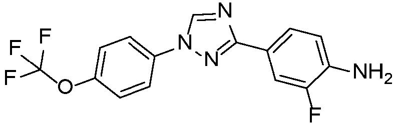

To two separate 10-20 mL microwave vials were added 3-bromo-1-(4-(trifluoromethoxy)phenyl)-1*H*-1,2,4-triazole (prepared as in Fischer at al., United States Patent Application Publication 20140274688 A1; 0.5 g, 1.623 mmol), 2-fluoro-4-(4,4,5,5-tetramethyl-1,3,2-dioxaborolan-2-yl)aniline (0.385 g, 1.623 mmol), Pd(PPh₃)₄ (0.188 g, 0.162 mmol), and sodium bicarbonate (0.409 g, 4.87 mmol). The solids were dissolved in dioxane (6.49 mL) and water (1.623 mL). The vials were placed in a Biotage^{®} microwave reactor and were heated at 140 °C for 30 min each. The solutions were cooled to room temperature, diluted with EtOAc, and washed with water. The organic layers were dried, filtered, and concentrated. Purification by flash chromatography (silica; EtOAc-hexanes) yielded the title compound as a gummy yellow semi-solid (718 mg, 62%): ¹H NMR (400 MHz, CDCl₃) δ 8.51 (s, 1H), 7.88 - 7.71 (m, 4H), 7.41 - 7.33 (m, 2H), 6.85 (dd, *J* = 9.0, 8.2 Hz, 1H), 3.93 (s, 2H); ¹⁹F NMR (376 MHz, CDCl₃) δ - 58.04, -135.37; ESIMS *m*/*z* 339 ([M+H]⁺).

*The following compounds were prepared in accordance with the procedure in Example 2.* 2-Methyl-4-(1-(4-(trifluoromethoxy)phenyl)-1*H*-1,2,4-triazol-3-yl)aniline **(C2)**

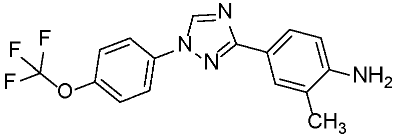

The title compound was prepared and was isolated as a yellow solid (700 mg, 63%): ¹H NMR (400 MHz, CDCl₃) δ 8.50 (d, *J* = 1.3 Hz, 1H), 7.97 - 7.83 (m, 2H), 7.83 - 7.70 (m, 2H), 7.36 (d, *J* = 8.6 Hz, 2H), 6.75 (d, *J* = 8.2 Hz, 1H), 3.81 (s, 2H), 2.24 (s, 3H); ¹⁹F NMR (376 MHz, CDCl₃) δ -58.04; ESIMS *m*/*z* 335 ([M+H]⁺).

### 2-Fluoro-4-(1-(4-(trifluoromethyl)phenyl)-1H-1,2,4-triazol-3-yl)aniline (C3)

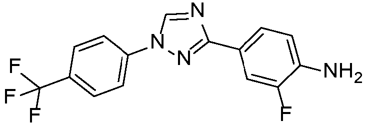

The title compound was prepared and was isolated as a pale yellow solid (659 mg, 59%): ¹H NMR (400 MHz, CDCl₃) δ 8.60 (s, 1H), 7.96 - 7.71 (m, 6H), 6.85 (dd, *J* = 9.0, 8.2 Hz, 1H), 3.94 (s, 2H); ¹⁹F NMR (376 MHz, CDCl₃) δ -62.48, -135.33; HRMS-ESI *m*/*z* ([M+H]⁺) calcd for C₁₅H₁₀F₄N₄, 323.0914; found, 323.0927.

### 2-Fluoro-4-(1-(4-(pentafluoro-λ⁶-sulfaneyl)phenyl)-1H-1,2,4-triazol-3-yl)aniline (C4)

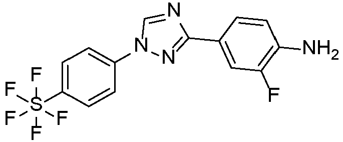

The title compound was prepared and was isolated as an off-white solid (1.3 g, 61%): ¹H NMR (400 MHz, CDCl₃) δ 8.60 (s, 1H), 7.99 - 7.75 (m, 6H), 6.95 - 6.78 (m, 1H), 3.95 (s, 2H); ¹⁹F NMR (376 MHz, CDCl₃) δ -135.26.

### 2-Methyl-4-(1-(4-(perfluoroethoxy)phenyl)-1H-1,2,4-triazol-3-yl)aniline (C5)

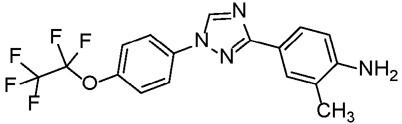

The title compound was prepared and was isolated as a white solid (1.25 g, 55%): ¹H NMR (400 MHz, CDCl₃) δ 8.51 (d, *J* = 1.5 Hz, 1H), 7.95 - 7.73 (m, 4H), 7.37 (d, *J* = 8.4 Hz, 2H), 6.75 (d, *J =* 8.1 Hz, 1H), 3.81 (s, 2H), 2.25 (s, 3H); ¹⁹F NMR (376 MHz, CDCl₃) δ -85.90, - 87.85; ESIMS *m*/*z* 385 ([M+H]⁺).

### 2-Fluoro-4-(1-(4-(perfluoroethyl)phenyl)-1H-1,2,4-triazol-3-yl)aniline (C6)

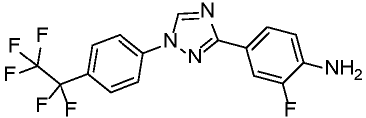

The title compound was prepared and was isolated as a white solid (0.42 g, 73%): ¹H NMR (400 MHz, CDCl₃) δ 8.61 (s, 1H), 7.96 - 7.70 (m, 6H), 6.86 (dd, *J* = 8.9, 8.2 Hz, 1H), 3.95 (s, 2H); ¹⁹F NMR (376 MHz, CDCl₃) δ -84.71, -114.86, -135.28; ESIMS *m*/*z* 373 ([M+H]⁺).

### EXAMPLE 3

### Preparation of 3-bromo-1-(4-(pentafluoro-λ⁶-sulfaneyl)phenyl)-1H-1,2,4-triazole (C7)

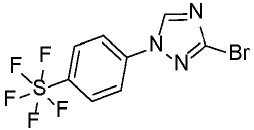

To a 250 mL round-bottomed flask were added 3-bromo-1*H*-1,2,4-triazole (3.92 g, 26.5 mmol), cesium carbonate (8.63 g, 26.5 mmol) and DMSO (58.9 mL). (4-Bromophenyl)pentafluoro-λ⁶-sulfane (5 g, 17.7 mmol) and copper(I) iodide (0.673 g, 3.53 mmol) were added sequentially. The reaction mixture was pumped and purged with nitrogen gas and heated to an internal temperature of 130 °C overnight. The reaction mixture was allowed to cool to room temperature and was diluted with EtOAc and a small amount of water. The mixture was extracted with EtOAc (3x). The organic extracts were combined, washed with brine, dried over Na₂SO₄, filtered, and concentrated under reduced pressure. Purification by column chromatography afforded the title compound as a white solid (1.54 g, 24%): ¹H NMR (400 MHz, CDCl₃) δ 8.52 (s, 1H), 7.98 - 7.87 (m, 2H), 7.79 (d, *J* = 8.8 Hz, 2H).

### EXAMPLE 4

### Preparation of 3-bromo-1-(4-(pentafluoro-λ⁶-sulfaneyl)phenyl)-1H-1,2,4-triazole (C7)

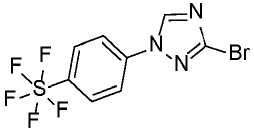

To a 2-5 mL microwave vial was added pentafluoro(4-fluorophenyl)-λ⁶-sulfane (1.27 mL, 9.00 mmol), DMF (12.9 mL), 3-bromo-1*H*-1,2,4-triazole (1.998 g, 13.5 mmol), and potassium carbonate (1.87 g, 13.5 mmol). The reaction mixture was stirred at 95 °C overnight. The reaction mixture was cooled to room temperature, and the DMF was concentrated under a stream of nitrogen. The solids were made into a slurry in DCM and loaded onto a silica cartridge. Purification by silica gel column chromatography eluting with 0 - 40% EtOAc-hexanes yielded the title compound as a white solid (2 g, 62%): ¹H NMR (400 MHz, CDCl₃) δ 8.52 (s, 1H), 7.99 - 7.89 (m, 2H), 7.79 (d, *J* = 8.7 Hz, 2H); ESIMS *m*/*z* 351 ([M+H]⁺).

*The following compounds were prepared in accordance with the procedure in Example 4.* 3-Bromo-1-(4-(perfluoroethyl)phenyl)-1*H*-1,2,4-triazole (**C8**)

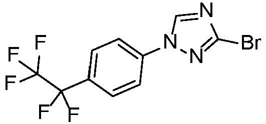

The title compound was prepared and was isolated as a white solid (1.17 g, 24%): ¹H NMR (400 MHz, CDCl₃) δ 8.53 (s, 1H), 7.88 - 7.72 (m, 4H); ¹⁹F NMR (376 MHz, CDCl₃) δ - 84.68, -114.97; ESIMS *m*/*z* 343 ([M+H]⁺).

### EXAMPLE 5

### Preparation of 3-(benzofuran-4-yl)-2-iminothiazolidin-4-one (C9)

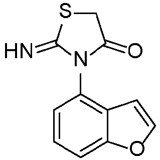

*N*-(Benzofuran-4-yl)-2-chloroacetamide (**C2**7; 100 mg, 0.477 mmol) and potassium thiocyanate (46.4 mg, 0.477 mmol) were added to a dried 10-mL vial equipped with a Teflon-coated stir bar and the vial was capped with a rubber septum. The vial was evacuated and backfilled with nitrogen gas three times. Degassed and dry acetone was added by syringe, and the resulting mixture was stirred at reflux for 12 h. The reaction mixture was cooled to room temperature, and cesium carbonate (406 mg, 1.193 mmol) was added in one portion and the resulting reaction mixture was stirred for 10 minutes at room temperature. At this point, the reaction mixture turned red. The reaction mixture was filtered over Celite^{®} and concentrated *in vacuo.* Purification of the residue with flash chromatography (0 - 60% EtOAc-hexanes) provided the title compound as a yellow oil (97 mg, 70%): ¹H NMR (500 MHz, CDCl₃) δ 7.94 (br s, 1H), 7.64 (d, *J* = 27.6 Hz, 2H), 7.43 (t, *J* = 8.1 Hz, 1H), 7.18 (d, *J* = 7.8 Hz, 1H), 6.61 (dd, *J* = 2.3, 1.0 Hz, 1H), 4.21 - 3.99 (m, 2H); ¹³C NMR (126 MHz, CDCl₃) δ 171.23, 170.87, 155.92, 145.99, 130.10, 125.70, 124.73, 122.83, 112.75, 104.50, 53.50.

*The following compounds were prepared in accordance with the procedure in Example 5.* 3-(Benzofuran-7-yl)-2-iminothiazolidin-4-one (**C10**)

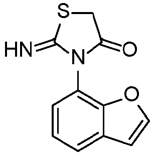

The title compound was prepared and was isolated as a white solid (105 mg, 90%): ¹H NMR (500 MHz, CDCl₃) δ 7.91 (s, 1H), 7.70 (s, 1H), 7.67 - 7.58 (m, 1H), 7.37 (t*, J* = 7.8 Hz, 1H), 7.25 - 7.14 (m, 1H), 6.85 (s, 1H), 4.25 - 4.09 (m, 2H); ¹³C NMR (126 MHz, CDCl₃) δ 171.20, 170.70, 149.80, 145.52, 124.31, 123.48, 122.84, 107.18, 34.37 ppm.

### 3-(2,3-Dihydrobenzofuran-4-yl)-2-iminothiazolidin-4-one (C11)

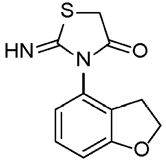

The title compound was prepared and was isolated as a yellow oil (151 mg, 65%): ¹H NMR (400 MHz, CDCl₃) δ 7.91 (s, 1H), 7.25 (s, 1H), 6.87 (s, 1H), 6.72 (s, 1H), 4.61 (t, *J* = 8.7 Hz, 2H), 4.19 - 3.96 (m, 2H), 3.11 (s, 2H); ¹³C NMR (101 MHz, CDCl₃) δ 170.89, 170.37, 157.25, 130.85, 129.88, 129.17, 119.64, 110.36, 71.33, 60.41, 34.32.

### 3-(Benzo[d]oxazol-4-yl)-2-iminothiazolidin-4-one (C12)

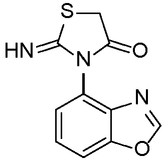

The title compound was prepared and was isolated as a white solid (31 mg, 25%): ¹H NMR (400 MHz, CDCl₃) δ 9.21 (s, 1H), 8.23 (d, *J* = 6.4 Hz, 1H), 8.02 (s, 1H), 7.34 - 7.29 (m, 2H), 3.63 (s, 2H); ¹³C NMR (101 MHz, CDCl₃) δ 165.03, 154.57, 151.25, 150.05, 131.02, 129.90, 126.56, 118.44, 113.79, 105.62, 27.51.

### 3-(1,3-Dihydroisobenzofuran-4-yl)-2-iminothiazolidin-4-one (C13)

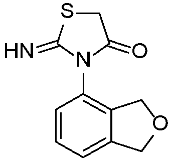

The title compound was prepared and was isolated as a yellow oil (70 mg, 60%): ¹H NMR (500 MHz, CDCl₃) δ 7.93 (s, 1H), 7.44 (t, *J* = 7.7 Hz, 1H), 7.33 (d, *J* = 7.5 Hz, 1H), 7.15 (d, *J* = 7.8 Hz, 1H), 5.18 (d, *J* = 2.2 Hz, 2H), 5.04 - 4.96 (m, 2H), 4.09 (s, 2H); ¹³C NMR (126 MHz, CDCl₃) δ 171.62, 170.33, 141.81, 137.83, 129.10, 126.94, 122.25, 73.90, 72.28, 34.10.

### 3-(2,3-Dihydrobenzofuran-7-yl)-2-iminothiazolidin-4-one (C14)

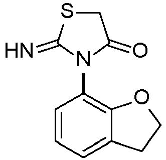

The title compound was prepared and was isolated as a yellow oil (82 mg, 70%): ¹H NMR (500 MHz, CDCl₃) δ 7.84 (s, 1H), 7.28 (d, *J* = 6.8 Hz, 1H), 7.02 - 6.93 (m, 2H), 4.64 (td, *J* = 8.8, 2.1 Hz, 2H), 4.05 (t, *J* = 15.9 Hz, 2H), 3.30 (t, *J* = 8.5 Hz, 2H); ¹³C NMR (126 MHz, CDCl₃) δ 171.21, 170.51, 155.87, 129.71, 127.67, 126.46, 121.33, 72.27, 29.84.

### 3-(2,3-Dihydro-1H-inden-4-yl)-2-iminothiazolidin-4-one (C15)

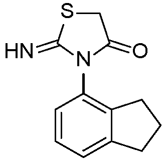

The title compound was prepared and was isolated as a yellow oil (126 mg, 54%): ¹H NMR (400 MHz, CDCl₃) δ 7.85 (s, 1H), 7.30 (d, *J* = 8.5 Hz, 2H), 6.99 (d, *J* = 7.4 Hz, 1H), 4.09 (d, *J* = 17.3 Hz, 2H), 3.00 (t, *J* = 7.5 Hz, 2H), 2.77 (s, 2H), 2.1¹ (qd, *J* = 7.3, 2.4 Hz, 2H); ¹³C NMR (101 MHz, CDCl₃) δ 171.18, 142.23, 127.79, 125.64, 117.26, 60.41, 33.19, 30.55, 24.64. 5-(2-Imino-4-oxothiazolidin-3-yl)isobenzofuran-1,3-dione (**C16**)

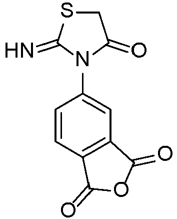

The title compound was prepared and was isolated as a white solid (83 mg, 34%): ¹H NMR (500 MHz, CDCl₃) δ 8.08 - 7.98 (m, 1H), 7.69 (t, *J* = 7.7 Hz, 1H), 7.62 (d, *J* = 7.8 Hz, 1H), 5.24 (d, *J* = 17.8 Hz, 2H), 4.14 (s, 1H); ¹³C NMR (126 MHz, CDCl₃) δ 173.65, 170.14, 169.98, 158.93, 144.32, 133.82, 130.37, 127.94, 126.74, 68.76.

### 2-Imino-3-(4-methyl-3,4-dihydro-2H-benzo[b][1,4]oxazin-8-yl)thiazolidin-4-one (C17)

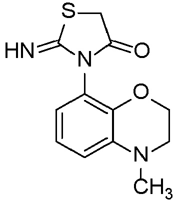

The title compound was prepared and was isolated as a yellow oil (95 mg, 52%): ¹H NMR (500 MHz, CDCl₃) δ 7.85 (s, 1H), 6.86 (d, *J* = 8.2 Hz, 1H), 6.46 (s, 2H), 4.30 (s, 2H), 4.03 (s, 2H), 3.30 (s, 2H), 2.87 (s, 3H); ESIMS *m*/*z* 264 ([M+H]⁺).

### 2-Imino-3-(1-methyl-1H-benzo[d]imidazol-4-yl)thiazolidin-4-one (C18)

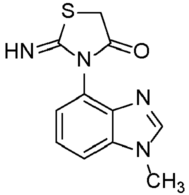

The title compound was prepared and was isolated as a yellow oil (59 mg, 32%): ¹H NMR (500 MHz, CDCl₃) δ 9.42 (s, 1H), 8.23 (d, *J* = 7.9 Hz, 1H), 7.81 (s, 1H), 7.31 (t, *J* = 8.0 Hz, 1H), 7.15 (d, *J* = 7.9 Hz, 1H), 3.85 (s, 3H), 3.61 (s, 2H); ¹³C NMR (126 MHz, CDCl₃) δ 165.73, 142.47, 134.64, 128.93, 128.31, 123.82, 110.98, 105.26, 49.24, 47.99. 2-Imino-3-(isochroman-5-yl)thiazolidin-4-one (**C19**)

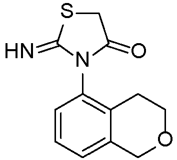

The title compound was prepared and was isolated as a yellow oil (77 mg, 42%): ¹H NMR (500 MHz, CDCl₃) δ 7.91 (d, *J* = 27.6 Hz, 1H), 7.44 - 7.27 (m, 1H), 7.07 - 6.87 (m, 1H), 4.70 - 4.31 (m, 2H), 4.11 - 4.01 (m, 2H), 4.01 - 3.82 (m, 2H), 2.88 (d, *J* = 17.2 Hz, 2H); ESIMS *m*/*z* 249 ([M+H]⁺).

### 2-Imino-3-(1-oxo-1,3-dihydroisobenzofuran-4-yl)thiazolidin-4-one (C20)

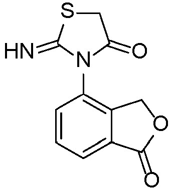

The title compound was prepared and was isolated as a yellow oil (83 mg, 45%): ¹H NMR (500 MHz, CDCl₃) δ 7.42 - 7.31 (m, 2H), 6.93 (dd, *J* = 6.1, 2.5 Hz, 1H), 5.18 (s, 2H), 3.81 (s, 2H); ESIMS *m*/*z* 249 ([M+H]⁺).

### 2-Imino-3-(naphthalen-2-yl)thiazolidin-4-one (C21)

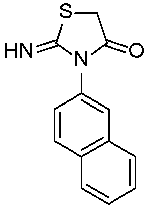

The title compound was prepared and was isolated as a yellow oil (86 mg, 47%): ¹H NMR (500 MHz, CDCl₃) δ 7.98 (d, *J* = 8.6 Hz, 1H), 7.87 (t, *J* = 8.2 Hz, 2H), 7.76 (d, *J* = 16.8 Hz, 1H), 7.54 (s, 2H), 7.31 (dd, *J* = 18.2, 8.4 Hz, 1H), 4.11 (d, *J* = 2.9 Hz, 2H); ESIMS *m*/*z* 243 ([M+H]⁺).

### 2-Imino-3-(naphthalen-1-yl)thiazolidin-4-one (C22)

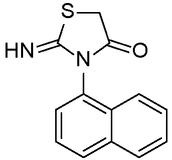

The title compound was prepared and was isolated as a yellow oil (96 mg, 52%): ¹H NMR (500 MHz, CDCl₃) δ 7.99 (d, *J* = 8.3 Hz, 1H), 7.94 (dd, *J* = 5.4, 3.8 Hz, 1H), 7.64 - 7.51 (m, 4H), 7.43 - 7.32 (m, 1H), 4.54 - 4.15 (m, 2H); ESIMS *m*/*z* 243 ([M+H]⁺).

### 2-Imino-3-(1-methyl-1H-indol-4-yl)thiazolidin-4-one (C23)

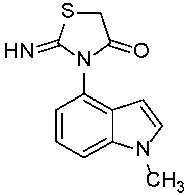

The title compound was prepared and was isolated as a yellow oil (83 mg, 45%): ¹H NMR (500 MHz, CDCl₃) δ 7.44 (d, *J* = 3.2 Hz, 1H), 7.33 (t, *J* = 7.9 Hz, 1H), 7.11 (q, *J* = 3.8 Hz, 1H), 7.02 (d, *J* = 7.4 Hz, 1H), 6.25 (dd, *J* = 19.6, 3.0 Hz, 1H), 4.21 - 4.05 (m, 2H), 3.81 (s, 3H); ESIMS *m*/*z* 246 ([M+H]⁺).

### 2-Imino-3-(1-methyl-1H-indol-6-yl)thiazolidin-4-one (C24)

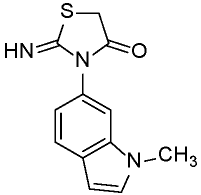

The title compound was prepared and was isolated as a yellow oil (77 mg, 42%): ¹H NMR (500 MHz, CDCl₃) δ 7.73 (d, *J* = 8.3 Hz, 1H), 7.21 (s, 1H), 7.13 (s, 1H), 6.92 (d, *J* = 8.1 Hz, 1H), 6.53 (d, *J* = 2.5 Hz, 1H), 4.08 (s, 2H), 3.78 (d, *J* = 2.0 Hz, 3H); ESIMS *m*/*z* 246 ([M+H]⁺).

### 2-Imino-3-(quinolin-5-yl)thiazolidin-4-one (C25)

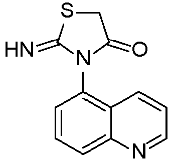

The title compound was prepared and was isolated as a white solid (362 mg, 78%): ¹H NMR (400 MHz, CDCl₃) δ 8.98 (s, 1H), 8.27 (d, *J* = 8.0 Hz, 1H), 7.94 (d, *J* = 8.4 Hz, 1H), 7.86 (t, *J* = 7.9 Hz, 1H), 7.54 - 7.38 (m, 3H), 4.33 - 4.07 (m, 2H); ¹³C NMR (101 MHz, CDCl₃) δ 151.66, 149.49, 130.90, 130.81, 129.26, 125.30, 122.61, 121.99, 72.08.

### 2-Imino-3-(6-((2,2,2-trifluoroethoxy)methyl)benzo[d][1,3]dioxol-5-yl)thiazolidin-4-one (C26)

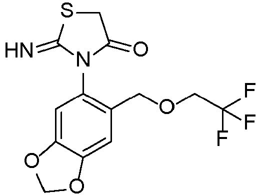

The title compound was prepared and was isolated as a white foam (575 mg, 83%): ¹H NMR (300 MHz, CDCl₃) δ 7.85 (s, 1H), 6.96 (s, 1H), 6.67 (s, 1H), 6.05 (d, *J* = 6.1 Hz, 2H), 4.58 - 4.31 (m, 2H), 4.07 (s, 2H), 3.71 (q, *J* = 8.8 Hz, 2H); ¹⁹F NMR (376 MHz, CDCl₃) δ -73.75; ESIMS 349 *m*/*z* ([M+H]⁺).

### EXAMPLE 6

### Preparation of N-(benzofuran-4-yl)-2-chloroacetamide (C27)

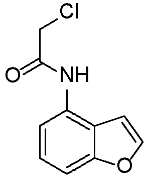

Benzofuran-4-amine (400 mg, 3.00 mmol) and sodium bicarbonate (631 mg, 7.51 mmol) were added to a dried 10-mL vial equipped with a Teflon-coated stir bar and the vial was capped with a rubber septum. The vial was evacuated and backfilled with nitrogen gas three times. Degassed and dry DCM (10 mL) was added by syringe, followed by 2-chloroacetyl chloride (287 µL, 3.60 mmol). The reaction mixture was stirred at 23 °C for 1.5 h and was diluted with saturated sodium bicarbonate and DCM. The phases were separated, and the organic layer was collected and concentrated *in vacuo* to afford a white powder residue. Purification of the residue by flash chromatography (0 - 60% EtOAc-hexanes) provided the title compound as a white solid (643 mg, 97%): ¹H NMR (400 MHz, CDCl₃) δ 8.47 (s, 1H), 7.72 (d, *J* = 7.8 Hz, 1H), 7.64 (d, *J* = 2.3 Hz, 1H), 7.38 (d, *J* = 8.3 Hz, 1H), 7.30 (t, *J* = 8.0 Hz, 1H), 6.77 (dd, *J* = 2.3, 1.0 Hz, 1H), 4.27 (s, 2H); ¹³C NMR (101 MHz, CDCl₃) δ 163.73, 155.54, 144.87, 129.24, 129.15, 125.27, 124.88, 120.24, 120.13, 115.08, 109.10, 103.50, 43.02.

*The following compounds were prepared in accordance with the procedure in Example 6. N*-(Benzofuran-7-yl)-2-chloroacetamide (**C28**)

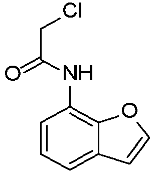

The title compound was prepared and was isolated as a white solid (650 mg, 98%): ¹H NMR (400 MHz, CDCl₃) δ 8.78 (s, 1H), 8.12 (dd, *J* = 8.0, 1.1 Hz, 1H), 7.64 (d, *J* = 2.1 Hz, 1H), 7.40 (dd, *J* = *7.9,* 1.1 Hz, 1H), 7.23 (d, *J* = 7.9 Hz, 1H), 6.81 (d, *J* = 2.2 Hz, 1H), 4.27 (s, 2H); ¹³C NMR (101 MHz, CDCl₃) δ 163.90, 145.05, 144.78, 127.82, 123.51, 122.23, 117.62, 115.31, 107.41, 42.95.

### N-(Benzo[d]oxazol-4-yl)-2-chloroacetamide (C29)

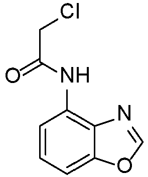

The title compound was prepared and was isolated as a white solid (785 mg, 95%): ¹H NMR (400 MHz, CDCl₃) δ 9.20 (s, 1H), 8.29 (dd, *J* = 7.6, 1.4 Hz, 1H), 8.08 (s, 1H), 7.45 - 7.34 (m, 2H), 4.27 (s, 2H); ¹³C NMR (101 MHz, CDCl₃) δ 164.11, 151.82, 150.13, 130.58, 129.31, 126.49, 114.09, 107.08, 42.96.

### 2-Chloro-N-(1,3-dihydroisobenzofuran-4-yl)acetamide (C30)

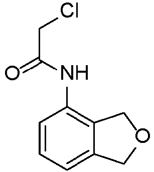

The title compound was prepared and was isolated as a white solid (742 mg, 90%): ¹H NMR (400 MHz, CDCl₃) δ 8.05 (s, 1H), 7.54 (d, *J* = 8.0 Hz, 1H), 7.30 (t, *J* = 7.7 Hz, 1H), 7.10 (dd, *J* = 7.5, 1.1 Hz, 1H), 5.14 (tt, *J* = 2.3, 0.9 Hz, 2H), 5.10 (d, *J* = 2.2 Hz, 2H), 4.20 (s, 2H); ¹³C NMR (101 MHz, CDCl₃) δ 163.79, 140.98, 131.64, 130.29, 128.69, 120.82, 118.55, 73.97, 72.15, 42.79.

### 2-Chloro-N-(2,3-dihydrobenzofuran-7-yl)acetamide (C31)

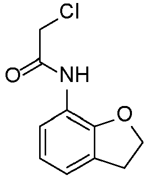

The title compound was prepared and was isolated as a white solid (593 mg, 90%): ¹H NMR (400 MHz, CDCl₃) δ 8.37 (s, 1H), 7.98 (dd, *J* = 8.1, 1.2 Hz, 1H), 6.99 (dq, *J =* 7.4, 1.1 Hz, 1H), 6.85 (t, *J* = 7.8 Hz, 1H), 4.64 (t, *J* = 8.7 Hz, 2H), 4.18 (s, 2H), 3.30 - 3.21 (m, 2H); ¹³C NMR (101 MHz, CDCl₃) δ 163.70, 149.42, 126.92, 121.23, 121.00, 120.95, 118.97, 72.09, 42.90, 30.25.

### 2-Chloro-N-(2,3-dihydro-1H-inden-4-yl)acetamide (C32)

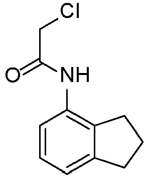

The title compound was prepared and was isolated as a white solid (812 mg, 98%): ¹H NMR (400 MHz, CDCl₃) δ 8.17 (s, 1H), 7.79 (d, *J* = 8.0 Hz, 1H), 7.18 (t, *J* = 7.7 Hz, 1H), 7.07 (d, *J* = 7.4 Hz, 1H), 4.21 (s, 2H), 2.97 (t, *J* = 7.5 Hz, 2H), 2.86 (t, *J* = 7.4 Hz, 2H), 2.14 (p, *J =* 7.5 Hz, 2H); ¹³C NMR (101 MHz, CDCl₃) δ 163.54, 145.53, 134.24, 132.82, 127.43, 121.53, 118.39, 43.07, 33.17, 29.75, 24.82.

### 2-Chloro-N-(1,3-dioxo-1,3-dihydroisobenzofuran-5-yl)acetamide (C33)

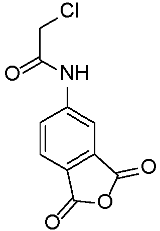

The title compound was prepared and was isolated as a white solid (734 mg, 95%): ¹H NMR (400 MHz, CDCl₃) δ 8.37 (s, 1H), 7.82 (d, *J* = 7.5 Hz, 1H), 7.72 - 7.65 (m, 1H), 7.57 (t, *J* = 7.7 Hz, 1H), 4.26 (s, 2H); ¹³C NMR (101 MHz, CDCl₃) δ 170.48, 163.97, 139.23, 130.95, 130.12, 127.65, 127.34, 123.63, 69.32, 42.60.

### 2-Chloro-N-(4-methyl-3,4-dihydro-2H-benzo[b][1,4]oxazin-8-yl)acetamide (C34)

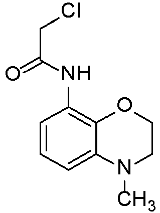

The title compound was prepared and was isolated as a white solid (798 mg, 98%): ¹H NMR (400 MHz, CDCl₃) δ 8.07 (s, 1H), 6.98 (d, *J* = 2.3 Hz, 1H), 6.72 (d, *J* = 8.4 Hz, 1H), 6.65 (dd, *J* = 8.5, 2.4 Hz, 1H), 4.28 - 4.26 (m, 2H), 4.16 (s, 2H), 3.28 - 3.25 (m, 2H), 2.89 (s, 3H); ¹³C NMR (101 MHz, CDCl₃) δ 163.53, 141.73, 136.76, 130.55, 115.78, 110.09, 105.21, 77.35, 77.23, 77.03, 76.71, 64.75, 48.95, 42.92, 38.67.

### 2-Chloro-N-(1-methyl-1H-indol-6-yl)acetamide (C35)

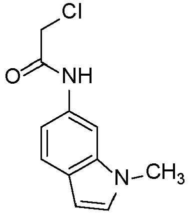

The title compound was prepared and was isolated as a white solid (786 mg, 98%): ¹H NMR (500 MHz, CDCl₃) δ 8.33 (s, 1H), 7.93 (s, 1H), 7.55 (d, *J* = 8.4 Hz, 1H), 7.04 (d, *J* = 3.1 Hz, 1H), 6.93 (dd, *J* = 8.4, 1.9 Hz, 1H), 6.45 (d, *J* = 2.8 Hz, 1H), 4.22 (s, 2H), 3.77 (s, 3H); ¹³C NMR (126 MHz, CDCl₃) δ 163.63, 136.68, 131.19, 129.54, 126.09, 121.10, 112.85, 101.54, 100.97, 43.03, 32.99.

### 2-Chloro-N-(1-methyl-1H-indol-4-yl)acetamide (C36)

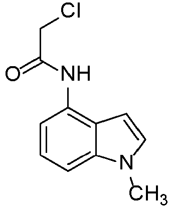

The title compound was prepared and was isolated as a white solid (697 mg, 87%): ¹H NMR (500 MHz, CDCl₃) δ 8.55 (s, 1H), 7.77 (d, *J* = 7.6 Hz, 1H), 7.26 - 7.15 (m, 2H), 7.05 (d, *J* = 3.2 Hz, 1H), 6.43 (d, *J* = 3.1 Hz, 1H), 4.24 (s, 2H), 3.78 (s, 3H); ¹³C NMR (126 MHz, CDCl₃) δ 163.60, 137.42, 128.73, 122.11, 120.61, 111.07, 106.83, 96.58, 43.18, 33.07.

### 2-Chloro-N-(1-methyl-1H-benzo[d]imidazol-4-yl)acetamide (C37)

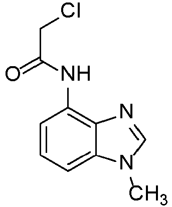

The title compound was prepared and was isolated as a white solid (776 mg, 97%): ¹H NMR (400 MHz, CDCl₃) δ 9.44 (s, 1H), 8.21 (d, *J* = 7.9 Hz, 1H), 7.81 (s, 1H), 7.31 (t, *J* = 8.0 Hz, 1H), 7.17 (dd, *J* = 8.2, 0.7 Hz, 1H), 4.26 (s, 2H), 3.85 (s, 3H); ¹³C NMR (101 MHz, CDCl₃) δ 164.11, 142.50, 134.65, 134.46, 128.79, 123.79, 111.24, 105.64, 53.45, 31.28.

### 2-Chloro-N-(isochroman-5-yl)acetamide (C38)

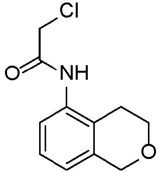

The title compound was prepared and was isolated as a white solid (780 mg, 98%): ¹H NMR (400 MHz, CDCl₃) δ 7.91 (s, 1H), 7.46 (d, *J* = 7.9 Hz, 1H), 7.22 (t, *J* = 7.8 Hz, 1H), 7.05 (d, *J* = 7.6 Hz, 1H), 4.72 (s, 2H), 4.22 (s, 2H), 3.96 (t, *J =* 5.7 Hz, 2H), 2.89 (t, *J* = 5.7 Hz, 2H); ¹³C NMR (101 MHz, CDCl₃) δ 164.17, 134.68, 131.56, 128.38, 127.29, 126.96, 121.83, 64.90, 64.68, 42.96, 28.28.

### 2-Chloro-N-(1-oxo-1,3 -dihydroisobenzofuran-4-yl)acetamide (C39)

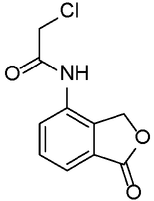

The title compound was prepared and was isolated as a dark yellow solid (406 mg, 85%): ¹H NMR (500 MHz, CDCl₃) δ 8.33 (s, 1H), 7.83 (d, *J* = 7.5 Hz, 1H), 7.68 (d, *J* = 7.8 Hz, 1H), 7.57 (t, *J* = 7.7 Hz, 1H), 5.36 (s, 2H), 4.26 (s, 2H); ¹³C NMR (101 MHz, CDCl₃) δ 173.04, 171.75, 165.73, 141.85, 139.44, 132.05, 131.56, 130.41, 130.09, 127.94, 127.13, 126.07, 122.96, 119.58, 114.69, 70.06, 68.70, 42.5.

### 2-Chloro-N-(naphthalen-1-yl)acetamide (C40)

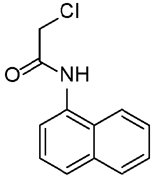

The title compound was prepared and was isolated as a white solid (791 mg, 98%): ¹H NMR (500 MHz, CDCl₃) δ 8.77 (s, 1H), 7.99 (d, *J* = 7.5 Hz, 1H), 7.89 (dd, *J* = 13.8, 8.1 Hz, 2H), 7.76 (d, *J* = 8.3 Hz, 1H), 7.61 - 7.47 (m, 3H), 4.35 (s, 2H); ¹³C NMR (126 MHz, CDCl₃) δ 164.29, 134.11, 131.12, 128.91, 126.94, 126.74, 126.49, 126.25, 125.71, 120.56, 120.19, 43.35.

### 2-Chloro-N-(naphthalen-2-yl)acetamide (C41)

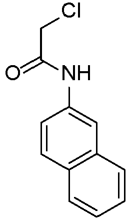

The title compound was prepared and was isolated as a white solid (783 mg, 95%): ¹H NMR (500 MHz, CDCl₃) δ 8.38 (s, 1H), 8.21 (d, *J* = 1.7 Hz, 1H), 7.86 - 7.78 (m, 3H), 7.54 - 7.40 (m, 3H), 4.24 (s, 2H); ¹³C NMR (126 MHz, CDCl₃) δ 163.92, 134.10, 133.69, 131.08, 129.03, 127.80, 127.64, 126.73, 125.51, 119.68, 117.20, 42.97.

### 2-Chloro-N-(quinolin-5-yl)acetamide (C42)

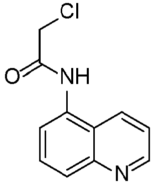

The title compound was prepared and was isolated as a white solid (1.58 g, 98%): ¹H NMR (400 MHz, Methanol-*d*₄) δ 8.90 (dd, *J* = 4.3, 1.6 Hz, 1H), 8.53 - 8.46 (m, 1H), 8.00 (d, *J* = 8.4 Hz, 1H), 7.86 - 7.73 (m, 2H), 7.61 (dd, *J* = 8.6, 4.3 Hz, 1H), 4.40 (s, 2H); ¹³C NMR (101 MHz, Methanol-*d*₄) δ 167.57, 150.09, 147.77, 132.77, 132.03, 129.27, 126.59, 124.11, 123.38, 121.18, 42.32.

### 2-Chloro-N-(6-((2,2,2-trifluoroethoxy)methyl)benzo[d][1,3]dioxol-5-yl)acetamide (C43)

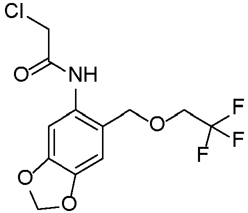

The title compound was prepared and was isolated as a white solid (612 mg, 55%): ¹H NMR (400 MHz, CDCl₃) δ 9.04 (s, 1H), 7.58 (s, 1H), 6.72 (s, 1H), 6.00 (s, 2H), 4.61 (s, 2H), 4.19 (s, 2H), 3.85 (q, *J* = 8.5 Hz, 2H); ¹⁹F NMR (376 MHz, CDCl₃) δ -73.50; ESIMS 326 *m*/*z* ([M+H]⁺).

### EXAMPLE 7

### Preparation of 6-((2,2,2-trifluoroethoxy)methyl)benzo[d][1,3]dioxol-5-amine (C44)

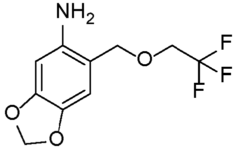

To a pressure bottle were added 5-nitro-6-((2,2,2-trifluoroethoxy)methyl)benzo [*d*][1,3]dioxole (977 mg, 3.50 mmol), palladium hydroxide (246 mg, 0.350 mmol), and ethyl acetate (17.50 mL). The suspension was reacted in a Parr shaker and shaken under hydrogen gas for 2 h. The suspension was filtered over a pad of Celite^{®} and the filtrate was concentrated under reduced pressure to afford the title compound as a white solid (846 mg, 92%): ¹H NMR (400 MHz, CDCl₃) δ 6.58 (s, 1H), 6.30 (s, 1H), 5.87 (s, 2H), 4.58 (s, 2H); the NH₂ peak was not observed; ¹⁹F NMR (376 MHz, CDCl₃) δ -73.77; ESIMS 250 *m*/*z* ([M+H]⁺).

### EXAMPLE 8

### Preparation of 5-nitro-6-((2,2,2-trifluoroethoxy)methyl)benzo[d][1,3]dioxole (C45)

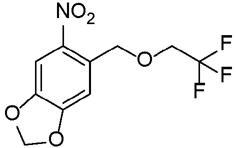

To a 250 mL round bottom flask were added THF (45 mL) and sodium hydride (568 mg, 14.20 mmol). 2,2,2-Trifluoroethyl trifluoromethanesulfonate (2.207 mL, 15.22 mmol) was added to the suspension and the reaction mixture was stirred and cooled to 0 °C. A solution of (6-nitrobenzo[*d*][1,3]dioxol-5-yl)methanol (2 g, 10.14 mmol) in THF (45 mL) was added to the reaction mixture via an addition funnel. The reaction mixture was stirred at room temperature for 3 h. The reaction mixture was concentrated under reduced pressure. The resulting slurry was dissolved in EtOAc and water. The mixture was extracted with EtOAc twice. The organic extracts were combined, washed with brine, dried over sodium sulfate, filtered over paper, and concentrated under reduced pressure. Purification of the resulting material by column chromatography yielded the title compound as a clear oil (977 mg, 34%): ¹H NMR (400 MHz, CDCl₃) δ 7.65 (s, 1H), 7.25 (d, *J* = 0.9 Hz, 1H), 6.15 (s, 2H), 5.03 (s, 2H), 4.00 (q, *J* = 8.5 Hz, 2H); ¹⁹F NMR (376 MHz, CDCl₃) δ -74.07; ESIMS 280 *m*/*z* ([M+H]⁺).

The following molecules having a structure according to Formula One and listed in Table 1 may be made according to the aforementioned examples and reported conditions.

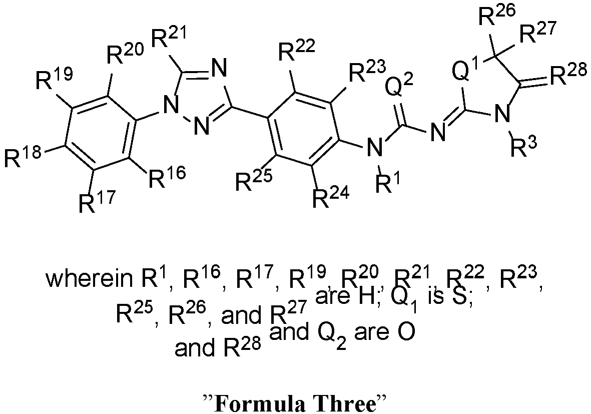

| **Table 1** | | | | |
|---|---|---|---|---|
| Cmpd ID | R¹⁸ | R²⁴ | R³ | R¹⁴ |
| F1 | OCF₃ | CH₃ | R³-1 | -- |
| F2 | OCF₃ | F | R³-1 | -- |
| F3 | SF₅ | F | R³-1 | -- |
| F4 | OCF₃ | F | R³-3 | -- |
| F5 | OCF₃ | F | R³-4 | -- |
| F6 | OCF₃ | F | R³-5 | -- |
| F7 | OCF₃ | F | R³-9 | -- |
| F8 | OCF₃ | F | R³-6 | -- |
| F9 | OCF₃ | F | R³-7 | -- |
| F10 | OCF₃ | F | R³-30 | -- |
| F11 | OCF₃ | F | R³-27 | -- |
| F12 | OCF₃ | F | R³-26 | -- |
| F13 | OCF₃ | F | R³-31 | -- |
| F14 | OCF₃ | F | R³-14 | CH₃ |
| F15 | OCF₃ | F | R³-12 | CH₃ |
| F16 | OCF₃ | F | R³-32 | -- |
| F17 | OCF₃ | F | R³-22 | CH₃ |
| F18 | OCF₃ | F | R³-8 | -- |
| F19 | OCF₃ | F | R³-28 | -- |
| F20 | CF₂CF₃ | F | R³-3 | -- |
| F21 | CF₂CF₃ | F | R³-4 | -- |
| F22 | OCF₂CF₃ | CH₃ | R³-3 | -- |
| F23 | OCF₂CF₃ | CH₃ | R³-4 | -- |
| F24 | CF₂CF₃ | F | R³-13 | CH₃ |
| F25 | OCF₂CF₃ | CH₃ | R³-5 | -- |
| F26 | OCF₂CF₃ | CH₃ | R³-32 | -- |
| F27 | OCF₂CF₃ | CH₃ | R³-12 | CH₃ |
| F28 | OCF₂CF₃ | CH₃ | R³-7 | -- |
| F29 | OCF₂CF₃ | CH₃ | R³-31 | -- |
| F30 | CF₂CF₃ | F | R³-12 | CH₃ |
| F31 | OCF₂CF₃ | CH₃ | R³-13 | CH₃ |
| F32 | OCF₂CF₃ | CH₃ | R³-33 | -- |
| F33 | CF₃ | F | R³-33 | -- |
| F34 | CF₂CF₃ | F | R³-33 | -- |

**Table 2: Analytical Data for Compounds in Table 1**

| **Cmpd. No.** | **MASS SPEC** | **NMR** |
|---|---|---|
| **F1** | HRMS-ESI (*m*/*z*) [M+H]⁺ calcd for C₃₀H₂₂F₆N₆O₆S, 709.1298; found, 709.1295 | ¹H NMR (400 MHz, CDCl₃) δ 8.54 (s, 1H), 8.19 (d, *J* = 8.5 Hz, 1H), 8.08 - 7.95 (m, 2H), 7.84 - 7.73 (m, 2H), 7.38 (d, *J* = 8.5 Hz, 2H), 7.09 (s, 1H), 7.00 (s, 1H), 6.68 (s, 1H), 6.10 (dd, *J* = 21.1, 1.4 Hz, 2H), 4.51 (d, *J* = 12.4 Hz, 1H), 4.41 (d, *J* = 12.4 Hz, 1H), 3.93 (d, *J* = 1.6 Hz, 2H), 3.75 (q, *J* = 8.7 Hz, 2H), 2.29 (s, 3H); |
| | | ¹⁹F NMR (376 MHz, CDCl₃) δ -58.02, -73.73 |
| **F2** | HRMS-ESI (*m*/*z*) [M+H]⁺ calcd for C₂₉H₁₉F₇N₆O₆S, 713.1048; found, 713.1041 | ¹H NMR (400 MHz, CDCl₃) δ 8.54 (s, 1H), 8.48 (t, *J* = 8.4 Hz, 1H), 7.98 (d, *J* = 8.8 Hz, 1H), 7.90 (dd, *J* = 12.0, 1.9 Hz, 1H), 7.84 - 7.71 (m, 2H), 7.52 (d, *J* = 2.9 Hz, 1H), 7.45 - 7.34 (m, 2H), 6.99 (s, 1H), 6.67 (s, 1H), 6.12 (dd, *J* = 16.5, 1.4 Hz, 2H), 4.50 (d, *J* = 12.4 Hz, 1H), 4.40 (d, *J* = 12.5 Hz, 1H), 3.95 (d, *J* = 2.1 Hz, 2H), 3.73 (qd, *J* = 8.7, 3.7 Hz, 2H); |
| | | ¹⁹F NMR (376 MHz, CDCl₃) δ -58.02, -73.74, -131.42 |
| **F3** | HRMS-ESI (*m*/*z*) [M+H]⁺ calcd for C₂₈H₁₉F₉N₆O₅S₂, 755.0787; found, 755.0778 | ¹H NMR (400 MHz, CDCl₃) δ 8.64 (s, 1H), 8.49 (t, *J* = 8.4 Hz, 1H), 7.98 (d, *J* = 8.6 Hz, 1H), 7.96 - 7.81 (m, 4H), 7.53 (d, *J* = 3.1 Hz, 1H), 6.99 (s, 1H), 6.67 (s, 1H), 6.12 (dd, *J* = 16.6, 1.4 Hz, 2H), 4.50 (d, *J* = 12.4 Hz, 1H), 4.41 (d, *J* = 12.4 Hz, 1H), 4.03 - 3.88 (m, 3H), 3.73 (qd, *J* = 8.7, 3.4 Hz, 2H) |
| **F4** | ESIMS *m*/*z* 597 ([M+H]⁺) | ¹H NMR (500 MHz, CDCl₃) δ 8.53 (s, 1H), 8.47 (t, *J* = 8.3 Hz, 1H), 7.96 (d, *J* = 8.3 Hz, 1H), 7.86 (dd, *J* = 12.0, 1.8 Hz, 1H), 7.82 - 7.75 (m, 2H), 7.70 (d, *J* = 2.3 Hz, 1H), 7.67 (d, *J* = 8.4 Hz, 1H), 7.53 - 7.45 (m, 2H), 7.38 (d, *J* = 8.5 Hz, 2H), 7.20 (d, *J* = 0.8 Hz, 1H), 6.60 (dd, *J* = 2.2, 1.0 Hz, 1H), 4.05 (d, *J* = 1.1 Hz, 2H); |
| | | ¹³C NMR (126 MHz, CDCl₃) δ 171.74, 170.20, 159.33, 155.85, 146.16, 141.59, 135.46, 127.07, 125.53, 124.55, 122.48, 122.43, 121.22, 120.25, 112.90, 104.49, 60.41, 53.43, 33.16; |
| | | ¹⁹F NMR (471 MHz, CDCl₃) δ -58.42, -131.04 |
| **F5** | ESIMS *m*/*z* 597 ([M+H]⁺) | ¹H NMR (500 MHz, CDCl₃) δ 8.59 - 8.41 (m, 1H), 7.98 - 7.84 (m, 2H), 7.81 - 7.72 (m, 2H), 7.70 - 7.60 (m, 2H), 7.47 - 7.31 (m, 3H), 7.24 - 7.15 (m, 1H), 6.90 - 6.79 (m, 2H), 4.20 - 4.10 (m, 2H); |
| | | ¹³C NMR (126 MHz, CDCl₃) δ 171.52, 171.18, 170.22, 159.33, 149.51, 145.71, 145.48, 141.59, 135.46, 129.65, 124.35, 124.02, 123.40, 123.01, 122.81, 122.42, 121.21, 118.81, 107.19, 53.43; |
| | | ¹⁹F NMR (471 MHz, CDCl₃) δ -57.41, -130.76 |
| **F6** | ESIMS *m*/*z 599* ([M+H]⁺) | ¹H NMR (500 MHz, CDCl₃) δ 8.54 (s, 1H), 8.49 (t, *J* = 8.3 Hz, 1H), 7.97 (d, *J* = 8.7 Hz, 1H), 7.92 - 7.85 (m, 1H), 7.82 - 7.76 (m, 2H), 7.59 (d, *J* = 3.0 Hz, 1H), 7.35 (dd, *J* = 30.4, 7.3 Hz, 2H), 7.06 - 6.93 (m, 2H), 4.65 (t, *J* = 8.8 Hz, 2H), 4.06 - 3.87 (m, 2H), 3.41 - 3.24 (m, 2H); ¹³C NMR (126 MHz, CDCl₃) δ 171.54, 170.38, 162.17, 159.34, 155.51, 141.59, 135.47, 129.57, 127.39, 126.34, 123.69, 122.42, 121.22, 121.00, 116.99, 116.01, 112.13, 72.32, 53.43, 32.95, 29.92; |
| | | ¹⁹F NMR (471 MHz, CDCl₃) δ -57.87, -131.27 |
| **F7** | | ¹H NMR (500 MHz, Acetone-*d*₆) δ 9.14 (s, 1H), 8.56 (d, *J* = 26.1 Hz, 1H), 8.36 (s, 1H), 8.10 (d, *J* = 9.0 Hz, 2H), 7.99 (d, *J* = 8.3 Hz, 1H), 7.93 - 7.78 (m, 2H), 7.71 - 7.53 (m, 3H), 7.48 (d, *J=* 7.9 Hz, 1H), 4.21 (q, *J* = 18.1 Hz, 2H); |
| | | ¹³C NMR (126 MHz, Acetone-*d*₆) δ 173.57, 155.99, 152.77, 149.30, 144.91, 140.27, 138.42, 129.96, 127.45, 126.73, 124.30, 123.03, 113.76, 89.29; |
| | | ¹⁹F NMR (471 MHz, Acetone-*d*₆) δ -58.64, -127.87 |
| **F8** | ESIMS *m*/*z* 597 ([M-H]⁻) | ¹H NMR (500 MHz, CDCl₃) δ 8.65 - 8.37 (m, 1H), 8.11 - 7.90 (m, 1H), 7.90 - 7.68 (m, 3H), 7.56 - 7.27 (m, 5H), 7.00 (s, 1H), 4.99 - 4.39 (m, 2H), 3.96 - 3.44 (m, 2H), 2.41 (d, *J* = 20.7 Hz, 2H); |
| | | ¹³C NMR (126 MHz, CDCl₃) δ 172.18, 171.17, 162.35, 159.29, 150.98, 148.45, 141.59, 139.50, 135.47, 133.72, 133.65, 133.20, 132.54, 131.68, 131.49, 130.75, 130.21, 129.52, 128.64, 128.52, 127.83, 126.46, 126.26, 126.18, 125.81, 125.21, 125.17, 124.06, 123.76, 122.82, 122.42, 121.21, 120.19, 113.04, 70.64, 69.24, 67.98; |
| | | ¹⁹F NMR (471 MHz, CDCl₃) δ -58.02, -131.09 |
| **F9** | | ¹H NMR (500 MHz, CDCl₃) δ 8.56 - 8.43 (m, 1H), 8.02 - 7.85 (m, 2H), 7.82 - 7.74 (m, 1H), 7.58 (dd, *J* = 15.7, 2.8 Hz, 1H), 7.38 (d, *J* = 8.6 Hz, 1H), 7.27 (d, *J* = 8.5 Hz, 1H), 7.22 (d, *J* = 19.7 Hz, 1H), 6.96 - 6.88 (m, 1H), 6.86 (s, 1H), 6.74 (d, *J* = 7.9 Hz, 1H), 4.64 (dq, *J* = 24.7, 8.6 Hz, 2H), 4.14 - 3.98 (m, 2H), 3.11 (td, *J* = 8.7, 4.1 Hz, 2H); |
| | | ¹³C NMR (126 MHz, CDCl₃) δ 171.44, 169.97, 162.27, 161.64, 159.37, 151.13, 148.46, 141.61, 135.46, 131.37, 129.19, 127.69, 125.79, 122.92, 122.43, 121.22, 120.31, 119.64, 119.47, 113.16, 110.54, 71.33, 60.41, 53.44, 33.07; |
| | | ¹⁹F NMR (471 MHz, CDCl₃) δ -58.02, -130.83 |
| **F10** | | ¹H NMR (500 MHz, CDCl₃) δ 8.54 (s, 1H), 8.49 (t, *J* = 8.3 Hz, 1H), 7.97 (d, *J* = 8.7 Hz, 1H), 7.88 (dd, *J* = 12.0, 1.5 Hz, 1H), 7.82 - 7.74 (m, 2H), 7.60 - 7.52 (m, 1H), 7.42 - 7.34 (m, 3H), 7.32 (t, *J* = 7.6 Hz, 1H), 7.01 (d, *J* = 7.6 Hz, 1H), 3.97 (s, 2H), 3.05 (dhept, *J* = 16.6, 7.1 Hz, 2H), 2.77 (t, *J* = 7.4 Hz, 2H), 2.17 - 2.07 (m, 2H); |
| | | ¹³C NMR (126 MHz, CDCl₃) δ 171.78, 171.17, 170.33, 162.30, 159.51, 153.03, 151.09, 148.47, 146.78, 142.13, 141.59, 135.47, 131.00, 127.56, 126.27, 125.83, 125.30, 122.89, 122.42, 121.22, 120.29, 113.11, 112.94, 60.41, 53.43, 33.22, 33.06, 30.74; |
| | | ¹⁹F NMR (471 MHz, CDCl₃) δ -58.02, -130.97 |
| **F11** | | ¹H NMR (500 MHz, CDCl₃) δ 8.65 - 8.41 (m, 2H), 8.09 (d, *J* = 7.6 Hz, 1H), 8.00 - 7.85 (m, 2H), 7.81 - 7.71 (m, 2H), 7.61 (d, *J* = 7.7 Hz, 1H), 7.48 (d, *J* = 11.3 Hz, 1H), 7.38 (d, *J* = 6.7 Hz, 1H), 7.09 - 6.99 (m, 1H), 4.81 (d, *J* = 12.6 Hz, 1H), 4.04 (s, 2H); |
| | | ¹³C NMR (126 MHz, CDCl₃) δ 171.00, 168.80, 166.89, 160.90, 158.74, 153.30, 148.30, 146.47, 144.04, 141.47, 133.74, 130.56, 129.71, 127.01, 122.92, 122.40, 121.23, 121.16, 112.53, 85.99; |
| | | ¹⁹F NMR (471 MHz, CDCl₃) δ -58.02, -130.93 |
| **F12** | | ¹H NMR (500 MHz, CDCl₃) δ 8.54 (s, 1H), 8.48 (t, *J* = 8.3 Hz, 1H), 7.96 (d, *J* = 8.8 Hz, 1H), 7.89 (dd, *J* = 12.0, 1.7 Hz, 1H), 7.81 - 7.75 (m, 2H), 7.65 (d, *J* = 2.4 Hz, 1H), 7.38 (d, *J* = 8.6 Hz, 2H), 6.87 (d, *J* = 8.3 Hz, 1H), 6.50 (dd, *J* = 8.3, 2.4 Hz, 1H), 6.46 (d, *J* = 2.4 Hz, 1H), 4.39 - 4.30 (m, 2H), 3.94 (s, 2H), 3.37 - 3.29 (m, 2H), 2.90 (s, 3H); |
| | | ¹³C NMR (126 MHz, CDCl₃) δ 172.60, 171.29, 171.16, 162.31, 159.63, 148.44, 144.72, 141.60, 137.31, 135.48, 127.91, 122.42, 121.21, 120.29, 116.85, 116.38, 112.94, 111.13, 53.43, 31.59, 22.66, 21.0; |
| | | ¹⁹F NMR (471 MHz, CDCl₃) δ -58.43, -130.96 |
| **F13** | | ¹H NMR (500 MHz, CDCl₃) δ 8.52 (s, 1H), 8.48 (td, *J* = 8.3, 5.4 Hz, 1H), 8.00 (d, *J* = 8.7 Hz, 1H), 7.99 - 7.89 (m, 3H), 7.86 (dt, *J* = 12.0, 2.0 Hz, 1H), 7.81 - 7.74 (m, 3H), 7.63 - 7.54 (m, 2H), 7.50 (dd, *J* = 14.4, 2.6 Hz, 1H), 7.40 - 7.28 (m, 3H), 4.02 (s, 2H); |
| | | ¹³C NMR (126 MHz, CDCl₃) δ 174.93, 172.45, 171.17, 169.68, 162.40, 159.49, 159.34, 153.13, 151.19, 148.58, 141.71, 135.58, 133.47, 133.42, 132.06, 131.78, 129.76, 129.68, 128.49, 128.06, 127.86, 127.49, 127.37, 127.01, 126.36, 125.11, 124.98, 123.02, 122.54, 121.55, 121.32, 120.38, 119.50, 113.23, 113.07, 73.73; |
| | | ¹⁹F NMR (471 MHz, CDCl₃) δ -58.25, -130.81 |
| **F14** | ESIMS *m*/*z* 610 ([M+H]⁺) | ¹H NMR (500 MHz, CDCl₃) δ 8.52 (s, 1H), 8.51 - 8.45 (m, 1H), 7.95 (d, *J* = 9.2 Hz, 1H), 7.85 (dd, *J* = 12.0, 1.7 Hz, 1H), 7.81 - 7.71 (m, 3H), 7.53 (dd, *J* = 15.3, 2.5 Hz, 1H), 7.37 (d, *J* = 8.6 Hz, 2H), 7.24 - 7.21 (m, 1H), 7.18 (dd, *J* = 7.3, 3.0 Hz, 1H), 6.98 - 6.92 (m, 1H), 6.57 (d, *J* = 2.8 Hz, 1H), 4.00 (s, 2H), 3.82 (d, *J* = 2.7 Hz, 3H); |
| | | ¹³C NMR (126 MHz, CDCl₃) δ 173.00, 171.72, 162.43, 159.72, 153.13, 151.19, 148.56, 141.71, 136.67, 135.60, 130.96, 129.31, 128.59, 128.06, 127.97, 126.31, 123.00, 122.54, 121.85, 121.55, 121.32, 120.38, 119.50, 118.85, 113.21, 113.05, 109.16, 101.66, 73.71, 60.53; |
| | | ¹⁹F NMR (471 MHz, CDCl₃) δ -57.97, -130.94 |
| **F15** | ESIMS *m*/*z* 610 ([M+H]⁺) | ¹H NMR (500 MHz, CDCl₃) δ 8.52 (s, 1H), 8.47 (t, *J* = 8.3 Hz, 1H), 7.94 (d, *J* = 8.5 Hz, 1H), 7.83 (dd, *J* = 12.0, 1.7 Hz, 1H), 7.79 - 7.74 (m, 2H), 7.47 (dd, *J* = 8.1, 3.7 Hz, 2H), 7.39 - 7.32 (m, 3H), 7.11 (dd, *J* = 6.5, 3.2 Hz, 1H), 7.04 (d, *J* = 7.4 Hz, 1H), 6.29 - 6.20 (m, 1H), 4.04 (d, *J* = 6.5 Hz, 2H), 3.85 (d, *J* = 4.5 Hz, 3H); |
| | | ¹³C NMR (126 MHz, CDCl₃) δ 174.71, 172.10, 171.17, 170.67, 162.29, 159.64, 152.95, 151.01, 148.42, 141.58, 138.05, 135.46, 130.47, 130.35, 127.95, 127.87, 126.66, 126.10, 126.04, 125.45, 122.86, 122.40, 121.65, 121.42, 121.18, 120.23, 119.37, 119.14, 113.05, 112.88, 111.05, 110.93, 98.22, 73.63, 73.43, 55.37; |
| | | ¹⁹F NMR (471 MHz, CDCl₃) δ -58.01, -130.93 |
| **F16** | ESIMS *m*/*z* 607 ([M+H]⁺) | ¹H NMR (500 MHz, CDCl₃) δ 8.54 (d, *J* = 2.8 Hz, 1H), 8.47 (q, *J* = 8.4 Hz, 1H), 7.97 (d, *J* = 9.4 Hz, 1H), 7.95 - 7.87 (m, 1H), 7.83 - 7.74 (m, 2H), 7.66 - 7.49 (m, 4H), 7.40 - 7.32 (m, 4H), 7.31 - 7.26 (m, 2H), 3.89 - 3.45 (m, 2H); |
| | | ¹³C NMR (126 MHz, CDCl₃) δ 171.85, 171.17, 171.08, 162.29, 159.34, 153.03, 151.09, 148.46, 141.61, 140.88, 138.27, 135.47, 132.60, 130.99, 130.06, 128.90, 128.71, 128.52, 128.39, 127.97, 127.89, 127.81, 126.27, 126.21, 122.89, 122.42, 121.43, 121.21, 120.23, 119.37, 113.11, 112.94, 32.67; |
| | | ¹⁹F NMR (471 MHz, CDCl₃) δ -57.94, -131.10 |
| **F17** | | ¹H NMR (500 MHz, DMSO-*d*₆) δ 9.54 (s, 1H), 9.40 (s, 1H), 8.07 (d, *J* = 9.0 Hz, 2H), 7.84 (d, *J* = 35.6 Hz, 3H), 7.62 (d, *J* = 8.7 Hz, 2H), 6.79 - 6.48 (m, 4H), 4.26 (s, 2H), 4.03 (s, 3H); |
| | | ¹⁹F NMR (471 MHz, DMSO-*d*₆) δ -56.94, -121.60 |
| **F18** | | ¹H NMR (400 MHz, CDCl₃) δ 8.55 (d, *J* = 4.9 Hz, 1H), 8.51 - 8.29 (m, 1H), 8.03 - 7.83 (m, 2H), 7.84 - 7.74 (m, 2H), 7.62 - 7.50 (m, 1H), 7.45 - 7.28 (m, 4H), 7.01 (d, *J* = 8.0 Hz, 1H), 4.66 - 4.49 (m, 2H), 4.13 - 3.83 (m, 4H), 3.16 - 2.84 (m, 2H); |
| | | ¹³C NMR (126 MHz, CDCl₃) δ 171.74, 171.09, 170.06, 161.52, 159.26, 153.00, 148.47, 141.60, 135.46, 132.65, 131.56, 130.71, 127.50, 126.04, 122.43, 121.22, 120.27, 119.40, 114.93, 64.97, 64.43, 60.41; |
| | | ¹⁹F NMR (471 MHz, CDCl₃) δ -57.49, -131.00 |
| **F19** | | ¹H NMR (400 MHz, Acetone-*d*₆) δ 9.16 (s, 1H), 8.80 - 8.31 (m, 2H), 8.18 - 8.06 (m, 3H), 8.01 (d, *J* = 8.5 Hz, 1H), 7.91 (dd, *J* = 12.4, 1.7 Hz, 1H), 7.64 - 7.52 (m, 4H), 5.44 (s, 2H), 2.82 (s, 2H); |
| | | ¹³C NMR (126 MHz, CDCl₃) δ 206.16, 171.12, 161.87, 153.84, 152.56, 151.63, 148.78, 145.87, 138.53, 137.10, 135.86, 130.77, 127.70, 127.14, 125.78, 123.40, 122.13, 122.03, 121.01, 120.86, 114.42, 69.63; |
| | | ¹⁹F NMR (376 MHz, Acetone-*d*₆) δ -58.79, -132.20 |
| **F20** | ESIMS m/z 631 ([M+H]⁺) | ¹H NMR (400 MHz, CDCl₃) δ 8.64 (s, 1H), 8.48 (t, *J* = 8.4 Hz, 1H), 7.98 (d, *J* = 8.7 Hz, 1H), 7.95 - 7.85 (m, 3H), 7.77 (d, *J* = 8.6 Hz, 2H), 7.72 - 7.64 (m, 2H), 7.52 - 7.45 (m, 2H), 7.20 (d, *J* = 7.5 Hz, 1H), 6.61 (d, *J* = 1.4 Hz, 1H), 4.06 (s, 2H); |
| | | ¹⁹F NMR (376 MHz, CDCl₃) δ -84.70, -114.25, -130.95 |
| **F21** | ESIMS *m*/*z* 631 ([M+H]⁺) | ¹H NMR (400 MHz, CDCl₃) δ 8.63 (s, 1H), 8.48 (t, *J* = 8.3 Hz, 1H), 8.01 - 7.83 (m, 4H), 7.81 - 7.73 (m, 3H), 7.64 (d, *J* = 2.1 Hz, 1H), 7.46 (d, *J* = 2.7 Hz, 1H), 7.40 (t, *J* = 7.8 Hz, 1H), 7.24 (d, *J* = 7.8 Hz, 1H), 6.89 (d, *J* = 2.2 Hz, 1H), 4.11 - 3.96 (m, 2H); |
| | | ¹⁹F NMR (376 MHz, CDCl₃) δ -84.74, -113.72, -131.10 |
| **F22** | ESIMS *m*/*z* 643 ([M+H]⁺) | ¹H NMR (400 MHz, CDCl₃) δ 8.54 (s, 1H), 8.20 (d, *J* = 8.5 Hz, 1H), 8.03 (d, *J* = 8.5 Hz, 1H), 7.97 (s, 1H), 7.80 (d, *J* = 9.0 Hz, 2H), 7.70 (d, *J* = 2.2 Hz, 1H), 7.66 (d, *J* = 8.3 Hz, 1H), 7.46 (q, *J* = 7.6 Hz, 1H), 7.39 (d, *J* = 8.9 Hz, 2H), 7.21 (t, *J* = 7.5 Hz, 1H), 7.05 (s, 1H), 6.63 (d, *J* = 1.4 Hz, 1H), 4.04 (s, 2H), 2.22 (s, 3H); ¹⁹F NMR (376 MHz, CDCl₃) δ -85.89, -87.85 |
| **F23** | | ¹H NMR (400 MHz, CDCl₃) δ 8.54 (s, 1H), 8.20 (d, *J* = 8.5 Hz, 1H), 7.96 (s, 1H), 7.84 - 7.73 (m, 3H), 7.64 (dd, *J* = 14.3, 2.0 Hz, 2H), 7.41 - 7.34 (m, 3H), 7.05 (s, 1H), 6.88 (d, *J* = 2.2 Hz, 1H), 4.10 - 3.96 (m, 2H), 2.22 (s, 3H); ¹⁹F NMR (376 MHz, CDCl₃) δ -85.89, -87.85 |
| **F24** | ESIMS *m*/*z* 642 ([M-H]⁻) | ¹H NMR (400 MHz, CDCl₃) δ 8.65 - 8.60 (m, 1H), 8.01 - 7.85 (m, 4H), 7.82 - 7.68 (m, 4H), 7.56 (d, *J* = 28.2 Hz, 1H), 7.22 - 7.13 (m, 2H), 7.04 - 6.83 (m, 2H), 6.55 (dd, *J* = 21.2, 2.5 Hz, 1H), 4.16 (s, 2H), 3.81 (d, *J* = 13.2 Hz, 3H); |
| | | ¹⁹F NMR (376 MHz, CDCl₃) δ -84.71, -114.85, -135.27 |
| **F25** | ESIMS *m*/*z* 664 ([M]⁺) | ¹H NMR (400 MHz, CDCl₃) δ 8.55 (s, 1H), 8.21 (d, *J* = 8.5 Hz, 1H), 8.03 (d, *J* = 8.5 Hz, 1H), 7.99 (s, 1H), 7.83 - 7.76 (m, 2H), 7.39 (d, *J* = 8.9 Hz, 2H), 7.32 (d, *J* = 6.3 Hz, 1H), 7.20 (s, 1H), 7.00 (dt, *J* = 15.1, 7.6 Hz, 2H), 4.71 - 4.59 (m, 2H), 4.08 - 3.85 (m, 2H), 3.33 (t, *J* = 8.6 Hz, 2H), 2.29 (s, 3H); |
| | | ¹⁹F NMR (376 MHz, CDCl₃) δ -85.96, -87.85 |
| **F26** | ESIMS *m*/*z* 653 ([M+H]⁺) | ¹H NMR (400 MHz, CDCl₃) δ 8.52 (s, 1H), 8.18 (dd, *J* = 12.0, 8.6 Hz, 1H), 8.06 - 7.96 (m, 3H), 7.93 (s, 1H), 7.77 (d, *J* = 9.0 Hz, 2H), 7.68 - 7.61 (m, 1H), 7.60 - 7.54 (m, 3H), 7.44 (d, *J* = 6.4 Hz, 1H), 7.37 (d, *J* = 8.8 Hz, 2H), 7.01 (d, *J* = 9.0 Hz, 1H), 4.20 - 4.01 (m, 2H), 2.14 (s, 3H); |
| | | ¹⁹F NMR (376 MHz, CDCl₃) δ -85.89, -87.93 |
| **F27** | ESIMS *m*/*z* 656 ([M+H]⁺) | ¹H NMR (400 MHz, CDCl₃) δ 8.53 (s, 1H), 8.21 (dd, *J* = 12.6, 8.5 Hz, 1H), 8.02 (d, *J* = 8.5 Hz, 1H), 7.95 (s, 1H), 7.85 - 7.73 (m, 2H), 7.47 (d, *J* = 8.3 Hz, 1H), 7.37 (t, *J* = 10.0 Hz, 2H), 7.17 - 7.10 (m, 2H), 7.05 (dd, *J* = 17.8, 7.4 Hz, 1H), 6.28 (dd, *J* = 7.0, 3.1 Hz, 1H), 4.19 - 3.99 (m, 2H), 3.84 (d, *J* = 11.3 Hz, 3H), 2.19 (s, 3H); |
| | | ¹⁹F NMR (376 MHz, CDCl₃) δ -86.00, -87.60 |
| **F28** | ESIMS *m*/*z* 645 ([M+H]⁺) | ¹H NMR (400 MHz, CDCl₃) δ 8.55 (s, 1H), 8.21 (d, *J* = 8.5 Hz, 1H), 8.05 (t, *J* = 10.2 Hz, 1H), 7.99 (s, 1H), 7.80 (d, *J* = 9.0 Hz, 2H), 7.39 (d, *J* = 8.8 Hz, 2H), 7.31 (d, *J* = 6.9 Hz, 1H), 7.20 (s, 1H), 7.00 (dt, *J* = 15.1, 7.6 Hz, 2H), 4.73 - 4.55 (m, 2H), 4.02 - 3.85 (m, 2H), 3.33 (t, *J* = 8.7 Hz, 2H), 2.29 (s, 3H); |
| | | ¹⁹F NMR (376 MHz, CDCl₃) δ -85.96, -87.92 |
| **F29** | | ¹H NMR (400 MHz, CDCl₃) δ 8.54 (s, 1H), 8.28 - 8.17 (m, 1H), 8.02 (t, *J* = 8.5 Hz, 2H), 7.99 - 7.91 (m, 3H), 7.80 (d, *J* = 9.1 Hz, 3H), 7.59 (t, *J* = 3.2 Hz, 2H), 7.43 - 7.35 (m, 3H), 7.10 (s, 1H), 4.02 (s, 2H), 2.22 (s, 3H); ¹⁹F NMR (376 MHz, CDCl₃) δ -85.89, -87.85 |
| **F30** | ESIMS *m*/*z* 644 ([M+H]⁺) | ¹H NMR (400 MHz, CDCl₃) δ 8.63 (s, 1H), 8.54 - 8.45 (m, 1H), 7.96 (d, *J* = 9.1 Hz, 1H), 7.90 (d, *J* = 8.7 Hz, 2H), 7.85 (dd, *J* = 12.0, 1.6 Hz, 1H), 7.76 (d, *J* = 8.6 Hz, 2H), 7.54 - 7.45 (m, 2H), 7.41 - 7.34 (m, 1H), 7.13 (d, *J* = 3.2 Hz, 1H), 7.09 - 7.02 (m, 1H), 6.27 (d, *J* = 2.5 Hz, 1H), 4.05 (d, *J* = 4.2 Hz, 2H), 3.86 (s, 3H); |
| | | ¹⁹F NMR (376 MHz, CDCl₃) δ -85.20, -113.72, -130.23 |
| **F31** | ESIMS *m*/*z* 656 ([M+H]⁺) | ¹H NMR (400 MHz, CDCl₃) δ 8.54 (s, 1H), 8.21 (d, *J* = 8.5 Hz, 1H), 8.02 (d, *J* = 8.6 Hz, 1H), 7.96 (s, 1H), 7.79 (d, *J* = 9.0 Hz, 2H), 7.75 (d, *J* = 8.4 Hz, 1H), 7.38 (d, *J* = 8.9 Hz, 2H), 7.17 (d, *J* = 7.3 Hz, 2H), 6.98 (dd, *J* = 8.3, 1.7 Hz, 1H), 6.57 (d, *J* = 2.6 Hz, 1H), 3.99 (s, 2H), 3.83 (s, 3H), 2.23 (s, 3H); |
| | | ¹⁹F NMR (376 MHz, CDCl₃) δ -85.96, -87.85 |
| **F32** | | ¹H NMR (400 MHz, CDCl₃) δ 9.01 (s, 1H), 8.54 (s, 1H), 8.31 (d, *J* = 8.0 Hz, 1H), 8.17 (d, *J* = 8.4 Hz, 1H), 8.02 (d, *J* = 8.5 Hz, 1H), 7.99 - 7.85 (m, 3H), 7.79 (d, *J* = 8.5 Hz, 2H), 7.52 (d, *J* = 7.3 Hz, 2H), 7.38 (d, *J* = 8.2 Hz, 2H), 6.95 (s, 1H), 4.11 (d, *J* = 4.6 Hz, 2H), 2.16 (s, 3H); |
| | | ¹³C NMR (126 MHz, CDCl₃) δ 172.24, 170.08, 163.16, 159.40, 151.15, 148.93, 147.53, 141.47, 137.12, 135.73, 131.80, 131.31, 130.60, 129.07, 128.51, 127.37, 127.33, 126.19, 125.22, 125.02, 123.07, 122.23, 121.20, 120.73, 60.42, 54.39, 30.94, 17.74; |
| | | ¹⁹F NMR (376 MHz, CDCl₃) δ -84.81, -87.86 |
| **F33** | | ¹H NMR (400 MHz, Acetone-*d*₆) δ 9.14 (s, 1H), 8.98 (d, *J* = 3.4 Hz, 1H), 8.32 (t, *J* = 9.3 Hz, 1H), 8.28 - 8.18 (m, 2H), 8.09 (d, *J* = 8.9 Hz, 2H), 7.98 (d, *J* = 8.2 Hz, 1H), 7.92 (t, *J* = 8.0 Hz, 1H), 7.81 (d, *J* = 11.5 Hz, 1H), 7.68 (t, *J* = 6.3 Hz, 1H), 7.56 (t, *J* = 8.4 Hz, 3H), 4.46 - 4.03 (m, 2H); |
| | | ¹⁹F NMR (376 MHz, Acetone-*d*₆) δ -58.57, -128.59 |
| **F34** | | ¹H NMR (400 MHz, Acetone-*d*₆) δ 9.28 (s, 1H), 8.98 (s, 1H), 8.34 (s, 1H), 8.23 (t, *J* = 8.1 Hz, 5H), 8.00 (d, *J* = 9.4 Hz, 1H), 7.93 (d, *J* = 8.3 Hz, 3H), 7.83 (d, *J =* 11.1 Hz, 1H), 7.69 (d, *J* = 6.5 Hz, 1H), 7.56 (dd, *J* = 8.5, 4.1 Hz, 1H), 4.37 - 4.14 (m, 2H); |
| | | ¹⁹F NMR (376 MHz, Acetone-*d*₆) δ -85.52, -115.02, - 128.70 |

### EXAMPLE A

### BIOASSAYS ON BEET ARMYWORM (Spodoptera exigua, "BAW") AND Cabbage Looper (Trichoplusia ni, "CL")

BAW has few effective parasites, diseases, or predators to lower its population. BAW infests many weeds, trees, grasses, legumes, and field crops. In various places, it is of economic concern upon asparagus, cotton, corn, soybeans, tobacco, alfalfa, sugar beets, peppers, tomatoes, potatoes, onions, peas, sunflowers, and citrus, among other plants. CL is a member of the moth family Noctuidae. It is found throughout the world. It attacks cabbage, cauliflower, broccoli, Brussel sprouts, tomatoes, cucumbers, potatoes, kale, turnips, mustard, peppers, eggplant, watermelons, melons, squash, cantaloupe, peas, beans, collards, lettuce, spinach, celery, parsley, beets, peas, alfalfa, soybeans, and cotton. This species is very destructive to plants due to its voracious consumption of leaves. In the case of cabbage, however, they feed not only on the wrapper leaves, but also may bore into the developing head. The larvae consume three times their weight in plant material daily. The feeding sites are marked by large accumulations of sticky, wet fecal material.

Consequently, because of the above factors, control of these pests is important. Furthermore, molecules that control these pests (BAW and CL), which are known as chewing pests, are useful in controlling other pests that chew on plants.

Certain molecules disclosed in this document were tested against BAW and CL using procedures described in the following examples. In the reporting of the results, the **"BAW & CL Rating Table"** was used (See Table Section).

### BIOASSAYS ON BAW

Bioassays on BAW were conducted using a 128-well diet tray assay. one to five second instar BAW larvae were placed in each well (3 mL) of the diet tray that had been previously filled with 1 mL of artificial diet to which 50 µg/cm² of the test compound (dissolved in 50 µL of 90: 10 acetone-water mixture) had been applied (to each of eight wells) and then allowed to dry. Trays were covered with a clear self-adhesive cover and held at 25 °C, 14:10 light-dark cycle for five to seven days. Percent mortality was recorded for the larvae in each well; activity in the eight wells was then averaged. The results are indicated in the table entitled **"Table ABC: Biological Results"** (See Table Section).

### BIOASSAYS ON CL

Bioassays on CL were conducted using a 128-well diet tray assay. one to five second instar CL larvae were placed in each well (3 mL) of the diet tray that had been previously filled with 1 mL of artificial diet to which 50 µg/cm² of the test compound (dissolved in 50 µL of 90: 10 acetone-water mixture) had been applied (to each of eight wells) and then allowed to dry. Trays were covered with a clear self-adhesive cover and held at 25 °C, 14:10 light-dark cycle for five to seven days. Percent mortality was recorded for the larvae in each well; activity in the eight wells was then averaged. The results are indicated in the table entitled **"Table ABC: Biological Results"** (See Table Section).

### EXAMPLE B

### BIOASSAYS ON Yellow Fever Mosquito (Aedes aegypti, "YFM")

YFM prefers to feed on humans during the daytime and is most frequently found in or near human habitations. YFM is a vector for transmitting several diseases. It is a mosquito that can spread the dengue fever and yellow fever viruses. Yellow fever is the second most dangerous mosquito-borne disease after malaria. Yellow fever is an acute viral hemorrhagic disease, and up to 50% of severely affected persons without treatment will die from yellow fever. There are an estimated 200,000 cases of yellow fever, causing 30,000 deaths, worldwide each year. Dengue fever is a nasty, viral disease; it is sometimes called "breakbone fever" or "break-heart fever" because of the intense pain it can produce. Dengue fever kills about 20,000 people annually. Consequently, because of the above factors control of this pest is important. Furthermore, molecules that control this pest (YFM), which is known as a sucking pest, are useful in controlling other pests that cause human and animal suffering.

Certain molecules disclosed in this document were tested against YFM using procedures described in the following paragraph. In the reporting of the results, the **"YFM Rating Table"** was used (See Table Section).

Master plates containing 400 µg of a molecule dissolved in 100 µL of dimethyl sulfoxide (DMSO) (equivalent to a 4000 ppm solution) are used. A master plate of assembled molecules contains 15 µL per well. To this plate, 135 µL of a 90: 10 water:acetone mixture is added to each well. A robot (Biomek^{®} NXP Laboratory Automation Workstation) is programmed to dispense 15 µL aspirations from the master plate into an empty 96-well shallow plate ("daughter" plate). There are 6 reps ("daughter" plates) created per master. The created daughter plates are then immediately infested with YFM larvae.

The day before plates are to be treated, mosquito eggs are placed in Millipore water containing liver powder to begin hatching (4 g. into 400 mL). After the daughter plates are created using the robot, they are infested with 220 µL of the liver powder/larval mosquito mixture (about 1 day-old larvae). After plates are infested with mosquito larvae, a non-evaporative lid is used to cover the plate to reduce drying. Plates are held at room temperature for 3 days prior to grading. After 3 days, each well is observed and scored based on mortality.

**TABLE SECTION**

| **BAW & CL Rating Table** | |
|---|---|
| % Control (or Mortality) | Rating |
| 50-100 | A |
| More than 0 - Less than 50 | B |
| Not Tested | C |
| No activity noticed in this bioassay | D |

| **YFM Rating Table** | |
|---|---|
| % Control (or Mortality) | Rating |
| 80-100 | A |
| More than 0 - Less than 80 | B |
| Not Tested | C |
| No activity noticed in this bioassay | D |

**Table ABC: Biological Results**

| Cmpd ID | BAW | CL | YFM |
|---|---|---|---|
| F1 | A | A | C |
| F2 | A | A | C |
| F3 | A | A | C |
| F4 | A | A | C |
| F5 | A | A | C |
| F6 | C | C | C |
| F7 | A | A | C |
| F8 | A | A | C |
| F9 | A | A | C |
| F10 | A | A | C |
| F11 | D | A | C |
| F12 | A | A | A |
| F13 | A | A | D |
| F14 | A | A | A |
| F15 | A | A | A |
| F16 | A | A | A |
| F17 | C | C | C |
| F18 | A | A | A |
| F19 | A | D | D |
| F20 | A | A | D |
| F21 | A | A | A |
| F22 | A | A | A |
| F23 | A | A | A |
| F24 | C | C | D |
| F25 | A | A | C |
| F26 | A | A | C |
| F27 | A | A | A |
| F28 | C | C | A |
| F29 | A | A | D |
| F30 | C | C | A |
| F31 | C | C | A |
| F32 | A | A | A |
| F33 | A | A | A |
| F34 | C | C | A |

### AGRICULTURALLY ACCEPTABLE ACID ADDITION SALTS, SALT DERIVATIVES, SOLVATES, ESTER DERIVATIVES, POLYMORPHS, ISOTOPES, AND RADIONUCLIDES

Compounds of Formula One, Two, and/or Three may be formulated into agriculturally acceptable acid addition salts. By way of a non-limiting example, an amine function can form salts with hydrochloric, hydrobromic, sulfuric, phosphoric, acetic, benzoic, citric, malonic, salicylic, malic, fumaric, oxalic, succinic, tartaric, lactic, gluconic, ascorbic, maleic, aspartic, benzenesulfonic, methanesulfonic, ethanesulfonic, hydroxyl-methanesulfonic, and hydroxyethanesulfonic acids. Additionally, by way of a non-limiting example, an acid function can form salts including those derived from alkali or alkaline earth metals and those derived from ammonia and amines. Examples of preferred cations include sodium, potassium, and magnesium.

Compounds of Formula One, Two, and/or Three may be formulated into salt derivatives. By way of a non-limiting example, a salt derivative can be prepared by contacting a free base with a sufficient amount of the desired acid to produce a salt. A free base may be regenerated by treating the salt with a suitable dilute aqueous base solution such as dilute aqueous sodium hydroxide, potassium carbonate, ammonia, and sodium bicarbonate. As an example, in many cases, a pesticide, such as 2,4-D, is made more water-soluble by converting it to its dimethylamine salt.

Compounds of Formula One, Two, and/or Three may be formulated into stable complexes with a solvent, such that the complex remains intact after the non-complexed solvent is removed. These complexes are often referred to as "solvates." However, it is particularly desirable to form stable hydrates with water as the solvent.

Compounds of Formula One, Two, and/or Three may be made into ester derivatives. These ester derivatives can then be applied in the same manner as the molecules disclosed in this document are applied.

Compounds of Formula One, Two, and/or Three may be made as various crystal polymorphs. Polymorphism is important in the development of agrochemicals since different crystal polymorphs or structures of the same molecule can have vastly different physical properties and biological performances.

Compounds of Formula One, Two, and/or Three may be made with different isotopes. Of particular importance are molecules having ²H (also known as deuterium) in place of ¹H.

Compounds of Formula One, Two, and/or Three may be made with different radionuclides. Of particular importance are molecules having ¹⁴C.

### STEREOISOMERS

Compounds of Formula One, Two, and/or Three may exist as one or more stereoisomers. Thus, certain molecules can be produced as racemic mixtures. It will be appreciated by those skilled in the art that one stereoisomer may be more active than the other stereoisomers. Individual stereoisomers may be obtained by known selective synthetic procedures, by conventional synthetic procedures using resolved starting materials, or by conventional resolution procedures. Certain molecules disclosed in this document can exist as two or more isomers. The various isomers include geometric isomers, diastereomers, and enantiomers. Thus, the molecules disclosed in this document include geometric isomers, racemic mixtures, individual stereoisomers, and optically active mixtures. It will be appreciated by those skilled in the art that one isomer may be more active than the others. The structures disclosed in the present disclosure are drawn in only one geometric form for clarity but are intended to represent all geometric forms of the molecule.

### FORMULATIONS

A pesticide is rarely suitable for application in its pure form. It is usually necessary to add other substances so that the pesticide can be used at the required concentration and in an appropriate form, permitting ease of application, handling, transportation, storage, and maximum pesticide activity. Thus, pesticides are formulated into, for example, baits, concentrated emulsions, dusts, emulsifiable concentrates, fumigants, gels, granules, microencapsulations, seed treatments, suspension concentrates, suspoemulsions, tablets, water soluble liquids, water dispersible granules or dry flowables, wettable powders, and ultra-low volume solutions. For further information on formulation types see "Catalogue of Pesticide Formulation Types and International Coding System" Technical Monograph n°2, 5th Edition by CropLife International (2002).

Pesticides are applied most often as aqueous suspensions or emulsions prepared from concentrated formulations of such pesticides. Such water-soluble, water-suspendable, or emulsifiable formulations are either solids, usually known as wettable powders, or water dispersible granules, or liquids usually known as emulsifiable concentrates, or aqueous suspensions. Wettable powders, which may be compacted to form water dispersible granules, comprise an intimate mixture of the pesticide, a carrier, and surfactants. The concentration of the pesticide is usually from about 10% to about 90% by weight. The carrier is usually selected from among the attapulgite clays, the montmorillonite clays, the diatomaceous earths, or the purified silicates. Effective surfactants, comprising from about 0.5% to about 10% of the wettable powder, are found among sulfonated lignins, condensed naphthalenesulfonates, naphthalenesulfonates, alkylbenzenesulfonates, alkyl sulfates, and non-ionic surfactants such as ethylene oxide adducts of alkyl phenols.

Emulsifiable concentrates of pesticides comprise a convenient concentration of a pesticide, such as from about 50 to about 500 grams per liter of liquid dissolved in a carrier that is either a water-miscible solvent or a mixture of water-immiscible organic solvent and emulsifiers. Useful organic solvents include aromatics, especially xylenes and petroleum fractions, especially the high-boiling naphthalenic and olefinic portions of petroleum such as heavy aromatic naphtha. Other organic solvents may also be used, such as the terpenic solvents including rosin derivatives, aliphatic ketones such as cyclohexanone, and complex alcohols such as 2-ethoxyethanol. Suitable emulsifiers for emulsifiable concentrates are selected from conventional anionic and non-ionic surfactants.

Aqueous suspensions comprise suspensions of water-insoluble pesticides dispersed in an aqueous carrier at a concentration in the range from about 5% to about 50% by weight. Suspensions are prepared by finely grinding the pesticide and vigorously mixing it into a carrier comprised of water and surfactants. Ingredients, such as inorganic salts and synthetic or natural gums may also be added, to increase the density and viscosity of the aqueous carrier. It is often most effective to grind and mix the pesticide at the same time by preparing the aqueous mixture and homogenizing it in an implement such as a sand mill, ball mill, or piston-type homogenizer.

Pesticides may also be applied as granular compositions that are particularly useful for applications to the soil. Granular compositions usually contain from about 0.5% to about 10% by weight of the pesticide, dispersed in a carrier that comprises clay or a similar substance. Such compositions are usually prepared by dissolving the pesticide in a suitable solvent and applying it to a granular carrier which has been pre-formed to the appropriate particle size, in the range of from about 0.5 to about 3 mm. Such compositions may also be formulated by making a dough or paste of the carrier and compound and crushing and drying to obtain the desired granular particle size.

Dusts containing a pesticide are prepared by intimately mixing the pesticide in powdered form with a suitable dusty agricultural carrier, such as kaolin clay, ground volcanic rock, and the like. Dusts can suitably contain from about 1% to about 10% of the pesticide. They can be applied as a seed dressing or as a foliage application with a dust blower machine.

It is equally practical to apply a pesticide in the form of a solution in an appropriate organic solvent, usually petroleum oil, such as the spray oils, which are widely used in agricultural chemistry.

Pesticides can also be applied in the form of an aerosol composition. In such compositions the pesticide is dissolved or dispersed in a carrier, which is a pressure-generating propellant mixture. The aerosol composition is packaged in a container from which the mixture is dispensed through an atomizing valve.

Pesticide baits are formed when the pesticide is mixed with food or an attractant or both. When the pests eat the bait, they also consume the pesticide. Baits may take the form of granules, gels, flowable powders, liquids, or solids. They can be used in pest harborages.

Fumigants are pesticides that have a relatively high vapor pressure and hence can exist as a gas in sufficient concentrations to kill pests in soil or enclosed spaces. The toxicity of the fumigant is proportional to its concentration and the exposure time. They are characterized by a good capacity for diffusion and act by penetrating the pest's respiratory system or being absorbed through the pest's cuticle. Fumigants are applied to control stored product pests under gas proof sheets, in gas sealed rooms or buildings or in special chambers.

Pesticides can be microencapsulated by suspending the pesticide particles or droplets in plastic polymers of various types. By altering the chemistry of the polymer or by changing factors in the processing, microcapsules can be formed of various sizes, solubility, wall thicknesses, and degrees of penetrability. These factors govern the speed with which the active ingredient within is released, which in turn, affects the residual performance, speed of action, and odor of the product.

Oil solution concentrates are made by dissolving pesticide in a solvent that will hold the pesticide in solution. Oil solutions of a pesticide usually provide faster knockdown and kill of pests than other formulations due to the solvents themselves having pesticidal action and the dissolution of the waxy covering of the integument increasing the speed of uptake of the pesticide. Other advantages of oil solutions include better storage stability, better penetration of crevices, and better adhesion to greasy surfaces.

Another embodiment is an oil-in-water emulsion, wherein the emulsion comprises oily globules which are each provided with a lamellar liquid crystal coating and are dispersed in an aqueous phase, wherein each oily globule comprises at least one compound which is agriculturally active, and is individually coated with a monolamellar or oligolamellar layer comprising: (1) at least one non-ionic lipophilic surface-active agent, (2) at least one non-ionic hydrophilic surface-active agent and (3) at least one ionic surface-active agent, wherein the globules having a mean particle diameter of less than 800 nanometers. Further information on the embodiment is disclosed in U.S. patent publication 20070027034 published February 1, 2007, having Patent Application serial number 11/495,228. For ease of use, this embodiment will be referred to as "OIWE".

For further information consult "Insect Pest Management" 2nd Edition by D. Dent, copyright CAB International (2000). Additionally, for more detailed information consult "Handbook of Pest Control - The Behavior, Life History, and Control of Household Pests" by Arnold Mallis, 9th Edition, copyright 2004 by GIE Media Inc.

### OTHER FORMULATION COMPONENTS

Generally, when the molecules disclosed in Formula One are used in a formulation, such formulation can also contain other components. These components include, but are not limited to, (this is a non-exhaustive and non-mutually exclusive list) wetters, spreaders, stickers, penetrants, buffers, sequestering agents, drift reduction agents, compatibility agents, anti-foam agents, cleaning agents, and emulsifiers. A few components are described forthwith.

A wetting agent is a substance that when added to a liquid increases the spreading or penetration power of the liquid by reducing the interfacial tension between the liquid and the surface on which it is spreading. Wetting agents are used for two main functions in agrochemical formulations: during processing and manufacture to increase the rate of wetting of powders in water to make concentrates for soluble liquids or suspension concentrates; and during mixing of a product with water in a spray tank to reduce the wetting time of wettable powders and to improve the penetration of water into water-dispersible granules. Examples of wetting agents used in wettable powder, suspension concentrate, and water-dispersible granule formulations are: sodium lauryl sulfate; sodium dioctyl sulfosuccinate; alkyl phenol ethoxylates; and aliphatic alcohol ethoxylates.

A dispersing agent is a substance which adsorbs onto the surface of particles and helps to preserve the state of dispersion of the particles and prevents them from reaggregating. Dispersing agents are added to agrochemical formulations to facilitate dispersion and suspension during manufacture, and to ensure the particles redisperse into water in a spray tank. They are widely used in wettable powders, suspension concentrates and water-dispersible granules. Surfactants that are used as dispersing agents have the ability to adsorb strongly onto a particle surface and provide a charged or steric barrier to reaggregation of particles. The most commonly used surfactants are anionic, non-ionic, or mixtures of the two types. For wettable powder formulations, the most common dispersing agents are sodium lignosulfonates. For suspension concentrates, very good adsorption and stabilization are obtained using polyelectrolytes, such as sodium naphthalene sulfonate formaldehyde condensates. Tristyrylphenol ethoxylate phosphate esters are also used. Non-ionics such as alkylarylethylene oxide condensates and EO-PO block copolymers are sometimes combined with anionics as dispersing agents for suspension concentrates. In recent years, new types of very high molecular weight polymeric surfactants have been developed as dispersing agents. These have very long hydrophobic 'backbones' and a large number of ethylene oxide chains forming the 'teeth' of a 'comb' surfactant. These high molecular weight polymers can give very good long-term stability to suspension concentrates because the hydrophobic backbones have many anchoring points onto the particle surfaces. Examples of dispersing agents used in agrochemical formulations are: sodium lignosulfonates; sodium naphthalene sulfonate formaldehyde condensates; tristyrylphenol ethoxylate phosphate esters; aliphatic alcohol ethoxylates; alkyl ethoxylates; EO-PO block copolymers; and graft copolymers.

An emulsifying agent is a substance which stabilizes a suspension of droplets of one liquid phase in another liquid phase. Without the emulsifying agent the two liquids would separate into two immiscible liquid phases. The most commonly used emulsifier blends contain alkylphenol or aliphatic alcohol with twelve or more ethylene oxide units and the oil-soluble calcium salt of dodecylbenzenesulfonic acid. A range of hydrophile-lipophile balance ("HLB") values from 8 to 18 will normally provide good stable emulsions. Emulsion stability can sometimes be improved by the addition of a small amount of an EO-PO block copolymer surfactant.

A solubilizing agent is a surfactant which will form micelles in water at concentrations above the critical micelle concentration. The micelles are then able to dissolve or solubilize water-insoluble materials inside the hydrophobic part of the micelle. The types of surfactants usually used for solubilization are non-ionics, sorbitan monooleates, sorbitan monooleate ethoxylates, and methyl oleate esters.

Surfactants are sometimes used, either alone or with other additives such as mineral or vegetable oils as adjuvants to spray-tank mixes to improve the biological performance of the pesticide on the target. The types of surfactants used for bioenhancement depend generally on the nature and mode of action of the pesticide. However, they are often non-ionics such as: alkyl ethoxylates; linear aliphatic alcohol ethoxylates; aliphatic amine ethoxylates.

A carrier or diluent in an agricultural formulation is a material added to the pesticide to give a product of the required strength. Carriers are usually materials with high absorptive capacities, while diluents are usually materials with low absorptive capacities. Carriers and diluents are used in the formulation of dusts, wettable powders, granules and water-dispersible granules.

Organic solvents are used mainly in the formulation of emulsifiable concentrates, oil-in-water emulsions, suspoemulsions, and ultra-low volume formulations, and to a lesser extent, granular formulations. Sometimes mixtures of solvents are used. The first main groups of solvents are aliphatic paraffinic oils such as kerosene or refined paraffins. The second main group (and the most common) comprises the aromatic solvents such as xylene and higher molecular weight fractions of C9 and C10 aromatic solvents. Chlorinated hydrocarbons are useful as cosolvents to prevent crystallization of pesticides when the formulation is emulsified into water. Alcohols are sometimes used as cosolvents to increase solvent power. Other solvents may include vegetable oils, seed oils, and esters of vegetable and seed oils.

Thickeners or gelling agents are used mainly in the formulation of suspension concentrates, emulsions and suspoemulsions to modify the rheology or flow properties of the liquid and to prevent separation and settling of the dispersed particles or droplets. Thickening, gelling, and anti-settling agents generally fall into two categories, namely water-insoluble particulates and water-soluble polymers. It is possible to produce suspension concentrate formulations using clays and silicas. Examples of these types of materials, include, but are not limited to, montmorillonite, bentonite, magnesium aluminum silicate, and attapulgite. Water-soluble polysaccharides have been used as thickening-gelling agents for many years. The types of polysaccharides most commonly used are natural extracts of seeds and seaweeds or are synthetic derivatives of cellulose. Examples of these types of materials include, but are not limited to, guar gum; locust bean gum; carrageenam; alginates; methyl cellulose; sodium carboxymethyl cellulose (SCMC); hydroxyethyl cellulose (HEC). Other types of anti-settling agents are based on modified starches, polyacrylates, polyvinyl alcohol and polyethylene oxide. Another good anti-settling agent is xanthan gum.

Microorganisms can cause spoilage of formulated products. Therefore, preservation agents are used to eliminate or reduce their effect. Examples of such agents include but are not limited to: propionic acid and its sodium salt; sorbic acid and its sodium or potassium salts; benzoic acid and its sodium salt; *p*-hydroxybenzoic acid sodium salt; methyl *p*-hydroxybenzoate; and 1,2-benzisothiazolin-3-one (BIT).

The presence of surfactants often causes water-based formulations to foam during mixing operations in production and in application through a spray tank. In order to reduce the tendency to foam, anti-foam agents are often added either during the production stage or before filling into bottles. Generally, there are two types of anti-foam agents, namely silicones and non-silicones. Silicones are usually aqueous emulsions of dimethyl polysiloxane, while the non-silicone anti-foam agents are water-insoluble oils, such as octanol and nonanol, or silica. In both cases, the function of the anti-foam agent is to displace the surfactant from the air-water interface.

"Green" agents (*e.g.,* adjuvants, surfactants, solvents) can reduce the overall environmental footprint of crop protection formulations. Green agents are biodegradable and generally derived from natural and/or sustainable sources, *e.g.,* plant and animal sources. Specific examples are: vegetable oils, seed oils, and esters thereof, also alkoxylated alkyl polyglucosides.

For further information, see "Chemistry and Technology of Agrochemical Formulations" edited by D.A. Knowles, copyright 1998 by Kluwer Academic Publishers. Also see "Insecticides in Agriculture and Environment - Retrospects and Prospects" by A.S. Perry, I. Yamamoto, I. Ishaaya, and R. Perry, copyright 1998 by Springer-Verlag.

### PESTS

In general, the compounds of Formula One, Two, and/or Three may be used to control pests *e.g.,* ants, aphids, beetles, bristletails, cockroaches, crickets, earwigs, fleas, flies, grasshoppers, leafhoppers, lice, locusts, mites, moths, nematodes, scales, symphylans, termites, thrips, ticks, wasps, and whiteflies.

In another embodiment, the compounds of Formula One, Two, and/or Three may be used to control pests in the **Phyla Nematoda** and/or **Arthropoda.**

In another embodiment, the compounds of Formula One, Two, and/or Three may be used to control pests in the **Subphyla Chelicerata, Myriapoda,** and/or **Hexapoda.**

In another embodiment, the compounds of Formula One, Two, and/or Three may be used to control pests in the **Classes of Arachnida, Symphyla,** and/or **Insecta.**

In another embodiment, the compounds of Formula One, Two, and/or Three may be used to control pests of the **Order Anoplura.** A non-exhaustive list of particular genera includes, but is not limited to, *Haematopinus* spp., *Hoplopleura* spp., *Linognathus* spp., *Pediculus* spp., and *Polyplax* spp. A non-exhaustive list of particular species includes, but is not limited to, *Haematopinus asini, Haematopinus suis, Linognathus setosus, Linognathus ovillus, Pediculus humanus capitis, Pediculus humanus humanus,* and *Pthirus pubis.*

In another embodiment, the compounds of Formula One, Two, and/or Three may be used to control pests in the **Order Coleoptera.** A non-exhaustive list of particular genera includes, but is not limited to, *Acanthoscelides* spp., *Agriotes* spp., *Anthonomus* spp., *Apion* spp., *Apogonia* spp., *Aulacophora* spp., *Bruchus* spp., *Cerosterna* spp., *Cerotoma* spp., *Ceutorhynchus* spp., *Chaetocnema* spp., *Colaspis* spp., *Ctenicera* spp., *Curculio* spp., *Cyclocephala* spp., *Diabrotica* spp., *Hypera* spp., *Ips* spp., *Lyctus* spp., *Megascelis* spp., *Meligethes* spp., *Otiorhynchus* spp., *Pantomorus* spp., *Phyllophaga* spp., *Phyllotreta* spp., *Rhizotrogus* spp., *Rhynchites* spp., *Rhynchophorus* spp., *Scolytus* spp., *Sphenophorus* spp., *Sitophilus* spp., and *Tribolium* spp. A non-exhaustive list of particular species includes, but is not limited to, *Acanthoscelides obtectus, Agrilus planipennis, Anoplophora glabripennis, Anthonomus grandis, Ataenius spretulus, Atomaria linearis, Bothynoderes punctiventris, Bruchus pisorum, Callosobruchus maculatus, Carpophilus hemipterus, Cassida vittata, Cerotoma trifurcata, Ceutorhynchus assimilis, Ceutorhynchus napi, Conoderus scalaris, Conoderus stigmosus, Conotrachelus nenuphar, Cotinis nitida, Crioceris asparagi, Cryptolestes ferrugineus, Cryptolestes pusillus, Cryptolestes turcicus, Cylindrocopturus adspersus, Deporaus marginatus, Dermestes lardarius, Dermestes maculatus, Epilachna varivestis, Faustinus cubae, Hylobius pales, Hypera postica, Hypothenemus hampei, Lasioderma serricorne, Leptinotarsa decemlineata, Liogenys fuscus, Liogenys suturalis, Lissorhoptrus oryzophilus, Maecolaspis joliveti, Melanotus communis, Meligethes aeneus, Melolontha melolontha, Oberea brevis, Oberea linearis, Oryctes rhinoceros, Oryzaephilus mercator, Oryzaephilus surinamensis, Oulema melanopus, Oulema oryzae, Phyllophaga cuyabana, Popillia japonica, Prostephanus truncatus, Rhyzopertha dominica,, Sitona lineatus, Sitophilus granarius, Sitophilus oryzae, Sitophilus zeamais, Stegobium paniceum, Tribolium castaneum, Tribolium confusum, Trogoderma variabile,* and *Zabrus tenebrioides.*

In another embodiment, the compounds of Formula One, Two, and/or Three may be used to control pests of the **Order Dermaptera.**

In another embodiment, the compounds of Formula One, Two, and/or Three may be used to control pests of the **Order Blattaria.** A non-exhaustive list of particular species includes, but is not limited to, *Blattella germanica, Blatta orientalis, Parcoblatta pennsylvanica, Periplaneta americana, Periplaneta australasiae, Periplaneta brunnea, Periplaneta fuliginosa, Pycnoscelus surinamensis,* and *Supella longipalpa.*

In another embodiment, the compounds of Formula One, Two, and/or Three may be used to control pests of the **Order Diptera.** A non-exhaustive list of particular genera includes, but is not limited to, *Aedes* spp., *Agromyza* spp., *Anastrepha* spp., *Anopheles* spp., *Bactrocera* spp., *Ceratitis* spp., *Chrysops* spp., *Cochliomyia* spp., *Contarinia* spp., *Culex* spp., *Dasineura* spp., *Delia* spp., *Drosophila* spp., *Fannia* spp., *Hylemyia* spp., *Liriomyza* spp., *Musca* spp., *Phorbia* spp., *Tabanus* spp., and *Tipula* spp. A non-exhaustive list of particular species includes, but is not limited to, *Agromyza frontella, Anastrepha suspensa, Anastrepha ludens, Anastrepha obliqa, Bactrocera cucurbitae, Bactrocera dorsalis, Bactrocera invadens, Bactrocera zonata, Ceratitis capitata, Dasineura brassicae, Delia platura, Fannia canicularis, Fannia scalaris, Gasterophilus intestinalis, Gracillia perseae, Haematobia irritans, Hypoderma lineatum, Liriomyza brassicae, Melophagus ovinus, Musca autumnalis, Musca domestica, Oestrus ovis, Oscinella frit, Pegomya betae, Psila rosae, Rhagoletis cerasi, Rhagoletis pomonella, Rhagoletis mendax, Sitodiplosis mosellana,* and *Stomoxys calcitrans.*

In another embodiment, the compounds of Formula One, Two, and/or Three may be used to control pests of the **Order Hemiptera.** A non-exhaustive list of particular genera includes, but is not limited to, *Adelges* spp., *Aulacaspis* spp., *Aphrophora* spp., *Aphis* spp., *Bemisia* spp., *Ceroplastes* spp., *Chionaspis* spp., *Chrysomphalus* spp., *Coccus* spp., *Empoasca* spp., *Lepidosaphes* spp., *Lagynotomus* spp., *Lygus* spp., *Macrosiphum* spp., *Nephotettix* spp., *Nezara* spp., *Philaenus* spp., *Phytocoris* spp., *Piezodorus* spp., *Planococcus* spp., *Pseudococcus* spp., *Rhopalosiphum* spp., *Saissetia* spp., *Therioaphis* spp., *Toumeyella* spp., *Toxoptera* spp., *Trialeurodes* spp., *Triatoma* spp. and *Unaspis* spp. A non-exhaustive list of particular species includes, but is not limited to, *Acrosternum hilare, Acyrthosiphon pisum, Aleyrodes proletella, Aleurodicus dispersus, Aleurothrixus floccosus, Amrasca biguttula biguttula, Aonidiella aurantii, Aphis gossypii, Aphis glycines, Aphis pomi, Aulacorthum solani, Bemisia argentifolii, Bemisia tabaci, Blissus leucopterus, Brachycorynella asparagi, Brevennia rehi, Brevicoryne brassicae, Calocoris norvegicus, Ceroplastes rubens, Cimex hemipterus, Cimex lectularius, Dagbertus fasciatus, Dichelops furcatus, Diuraphis noxia, Diaphorina citri, Dysaphis plantaginea, Dysdercus suturellus, Edessa meditabunda, Eriosoma lanigerum, Eurygaster maura, Euschistus heros, Euschistus servus, Helopeltis antonii, Helopeltis theivora, Icerya purchasi, Idioscopus nitidulus, Laodelphax striatellus, Leptocorisa oratorius, Leptocorisa varicornis, Lygus hesperus, Maconellicoccus hirsutus, Macrosiphum euphorbiae, Macrosiphum granarium, Macrosiphum rosae, Macrosteles quadrilineatus, Mahanarva frimbiolata, Metopolophium dirhodum, Mictis longicornis, Myzus persicae, Nephotettix cinctipes, Neurocolpus longirostris, Nezara viridula, Nilaparvata lugens, Parlatoria pergandii, Parlatoria ziziphi, Peregrinus maidis, Phylloxera vitifoliae, Physokermes piceae,, Phytocoris californicus, Phytocoris relativus, Piezodorus guildinii, Poecilocapsus lineatus, Psallus vaccinicola, Pseudacysta perseae, Pseudococcus brevipes, Quadraspidiotus perniciosus, Rhopalosiphum maidis, Rhopalosiphum padi, Saissetia oleae, Scaptocoris castanea, Schizaphis graminum, Sitobion avenae, Sogatella furcifera, Trialeurodes vaporariorum, Trialeurodes abutiloneus, Unaspis yanonensis,* and *Zulia entrerriana.*

In another embodiment, the compounds of Formula One, Two, and/or Three may be used to control pests of the **Order Hymenoptera.** A non-exhaustive list of particular genera includes, but is not limited to, *Acromyrmex* spp., *Atta* spp., *Camponotus* spp., *Diprion* spp., *Formica* spp., *Monomorium* spp., *Neodiprion* spp., *Pogonomyrmex* spp., *Polistes* spp., *Solenopsis* spp., *Vespula* spp., and *Xylocopa* spp. A non-exhaustive list of particular species includes, but is not limited to, *Athalia rosae, Atta texana, Iridomyrmex humilis, Monomorium minimum, Monomorium pharaonis, Solenopsis invicta, Solenopsis geminata, Solenopsis molesta, Solenopsis richtery, Solenopsis xyloni,* and *Tapinoma sessile.*

In another embodiment, the compounds of Formula One, Two, and/or Three may be used to control pests of the **Order Isoptera.** A non-exhaustive list of particular genera includes, but is not limited to, *Coptotermes* spp., *Cornitermes* spp., *Cryptotermes* spp., *Heterotermes* spp., *Kalotermes* spp., *Incisitermes* spp., *Macrotermes* spp., *Marginitermes* spp., *Microcerotermes* spp., *Procornitermes* spp., *Reticulitermes* spp., *Schedorhinotermes* spp., and *Zootermopsis* spp. A non-exhaustive list of particular species includes, but is not limited to, *Coptotermes curvignathus, Coptotermes frenchi, Coptotermes formosanus, Heterotermes aureus, Microtermes obesi, Reticulitermes banyulensis, Reticulitermes grassei, Reticulitermes flavipes, Reticulitermes hageni, Reticulitermes hesperus, Reticulitermes santonensis, Reticulitermes speratus, Reticulitermes tibialis,* and *Reticulitermes virginicus.*

In another embodiment, the compounds of Formula One, Two, and/or Three may be used to control pests of the **Order Lepidoptera.** A non-exhaustive list of particular genera includes, but is not limited to, *Adoxophyes* spp., *Agrotis* spp., *Argyrotaenia* spp., *Cacoecia* spp., *Caloptilia* spp., *Chilo* spp., *Chrysodeixis* spp., *Colias* spp., *Crambus* spp., *Diaphania* spp., *Diatraea* spp., *Earias* spp., *Ephestia* spp., *Epimecis* spp., *Feltia* spp., *Gortyna* spp., *Helicoverpa* spp., *Heliothis* spp., *Indarbela* spp., *Lithocolletis* spp., *Loxagrotis* spp., *Malacosoma* spp., *Peridroma* spp., *Phyllonorycter* spp., *Pseudaletia* spp., *Sesamia* spp., *Spodoptera* spp., *Synanthedon* spp., and *Yponomeuta* spp. A non-exhaustive list of particular species includes, but is not limited to, *Achaea janata, Adoxophyes orana, Agrotis ipsilon, Alabama argillacea, Amorbia cuneana, Amyelois transitella, Anacamptodes defectaria, Anarsia lineatella, Anomis sabulifera, Anticarsia gemmatalis, Archips argyrospila, Archips rosana, Argyrotaenia citrana, Autographa gamma, Bonagota cranaodes, Borbo cinnara, Bucculatrix thurberiella, Capua reticulana, Carposina niponensis, Chlumetia transversa, Choristoneura rosaceana, Cnaphalocrocis medinalis, Conopomorpha cramerella, Cossus cossus, Cydia caryana, Cydia funebrana, Cydia molesta, Cydia nigricana, Cydia pomonella, Darna diducta, Diatraea saccharalis, Diatraea grandiosella, Earias insulana, Earias vittella, Ecdytolopha aurantianum, Elasmopalpus lignosellus, Ephestia cautella, Ephestia elutella, Ephestia kuehniella, Epinotia aporema, Epiphyas postvittana, Erionota thrax, Eupoecilia ambiguella, Euxoa auxiliaris, Grapholita molesta, Hedylepta indicata, Helicoverpa armigera, Helicoverpa zea, Heliothis virescens, Hellula undalis, Keiferia lycopersicella, Leucinodes orbonalis, Leucoptera coffeella, Leucoptera malifoliella, Lobesia botrana, Loxagrotis albicosta, Lymantria dispar, Lyonetia clerkella, Mahasena corbetti, Mamestra brassicae, Maruca testulalis, Metisa plana, Mythimna unipuncta, Neoleucinodes elegantalis, Nymphula depunctalis, Operophtera brumata, Ostrinia nubilalis, Oxydia vesulia, Pandemis cerasana, Pandemis heparana, Papilio demodocus, Pectinophora gossypiella, Peridroma saucia, Perileucoptera coffeella, Phthorimaea operculella, Phyllocnistis citrella, Pieris rapae, Plathypena scabra, Plodia interpunctella, Plutella xylostella, Polychrosis viteana, Prays endocarpa, Prays oleae, Pseudaletia unipuncta, Pseudoplusia includens, Rachiplusia nu, Scirpophaga incertulas, Sesamia inferens, Sesamia nonagrioides, Setora nitens, Sitotroga cerealella, Sparganothis pilleriana, Spodoptera exigua, Spodoptera frugiperda, Spodoptera eridania, Thecla basilides, Tineola bisselliella, Trichoplusia ni, Tuta absoluta, Zeuzera coffeae,* and *Zeuzera pyrina.*

In another embodiment, the compounds of Formula One, Two, and/or Three may be used to control pests of the **Order Mallophaga.** A non-exhaustive list of particular genera includes, but is not limited to, *Anaticola* spp., *Bovicola* spp., *Chelopistes* spp., *Goniodes* spp., *Menacanthus* spp., and *Trichodectes* spp. A non-exhaustive list of particular species includes, but is not limited to, *Bovicola bovis, Bovicola caprae, Bovicola ovis, Chelopistes meleagridis, Goniodes dissimilis, Goniodes gigas, Menacanthus stramineus, Menopon gallinae,* and *Trichodectes canis.*

In another embodiment, the compounds of Formula One, Two, and/or Three may be used to control pests of the **Order Orthoptera.** A non-exhaustive list of particular genera includes, but is not limited to, *Melanoplus* spp., and *Pterophylla* spp. A non-exhaustive list of particular species includes, but is not limited to, *Anabrus simplex, Gryllotalpa africana, Gryllotalpa australis, Gryllotalpa brachyptera, Gryllotalpa hexadactyla, Locusta migratoria, Microcentrum retinerve, Schistocerca gregaria,* and *Scudderia furcata.*

In another embodiment, the compounds of Formula One, Two, and/or Three may be used to control pests of the **Order Siphonaptera.** A non-exhaustive list of particular species includes, but is not limited to, *Ceratophyllus gallinae, Ceratophyllus niger, Ctenocephalides canis, Ctenocephalides felis,* and *Pulex irritans.*

In another embodiment, the compounds of Formula One, Two, and/or Three may be used to control pests of the **Order Thysanoptera.** A non-exhaustive list of particular genera includes, but is not limited to, Caliothrips spp., Frankliniella spp., *Scirtothrips* spp., and *Thrips* spp. A non-exhaustive list of particular sp. includes, but is not limited to, *Frankliniella fusca, Frankliniella occidentalis, Frankliniella schultzei, Frankliniella williamsi, Heliothrips haemorrhoidalis, Rhipiphorothrips cruentatus, Scirtothrips citri, Scirtothrips dorsalis,* and *Taeniothrips rhopalantennalis, Thrips hawaiiensis, Thrips nigropilosus, Thrips orientalis, Thrips tabaci.*

In another embodiment, the compounds of Formula One, Two, and/or Three may be used to control pests of the **Order Thysanura.** A non-exhaustive list of particular genera includes, but is not limited to, *Lepisma* spp. and *Thermobia* spp.

In another embodiment, the compounds of Formula One, Two, and/or Three may be used to control pests of the **Order Acarina.** A non-exhaustive list of particular genera includes, but is not limited to, *Acarus* spp., *Aculops* spp., *Boophilus* spp., *Demodex* spp., *Dermacentor* spp., *Epitrimerus* spp., *Eriophyes* spp., *Ixodes* spp., *Oligonychus* spp., *Panonychus* spp., *Rhizoglyphus* spp., and *Tetranychus* spp. A non-exhaustive list of particular species includes, but is not limited to, *Acarapis woodi, Acarus siro, Aceria mangiferae, Aculops lycopersici, Aculus pelekassi, Aculus schlechtendali, Amblyomma americanum, Brevipalpus obovatus, Brevipalpus phoenicis, Dermacentor variabilis, Dermatophagoides pteronyssinus, Eotetranychus carpini, Notoedres cati, Oligonychus coffeae, Oligonychus ilicis, Panonychus citri, Panonychus ulmi, Phyllocoptruta oleivora, Polyphagotarsonemus latus, Rhipicephalus sanguineus, Sarcoptes scabiei, Tegolophus perseaflorae, Tetranychus urticae,* and *Varroa destructor.*

In another embodiment, the compounds of Formula One, Two, and/or Three may be used to control pest of the **Order Symphyla.** A non-exhaustive list of particular sp. includes, but is not limited to, *Scutigerella immaculata.*

In another embodiment, the compounds of Formula One, Two, and/or Three may be used to control pests of the **Phylum Nematoda.** A non-exhaustive list of particular genera includes, but is not limited to, *Aphelenchoides* spp., *Belonolaimus* spp., *Criconemella* spp., *Ditylenchus* spp., *Heterodera* spp., *Hirschmanniella* spp., *Hoplolaimus* spp., *Meloidogyne* spp., *Pratylenchus* spp., and *Radopholus* spp. A non-exhaustive list of particular sp. includes, but is not limited to, *Dirofilaria immitis, Heterodera zeae, Meloidogyne incognita, Meloidogyne javanica, Onchocerca volvulus, Radopholus similis, and Rotylenchulus reniformis.*

### APPLICATIONS

Controlling pests of Phyla Nematoda, Arthropoda, and/or Mollusca generally means that pest populations, pest activity, or both, are reduced in a locus. This can come about when:
(a) pest populations are repulsed from a locus;
(b) pests are incapacitated in, or around, a locus; or
(c) pests are exterminated in, or around, a locus.
Of course, a combination of these results can occur. Generally, pest populations, activity, or both are desirably reduced more than fifty percent, preferably more than 90 percent, and most preferably more than 98 percent. Generally, the locus is not in, or on, a human; consequently, the locus is generally a non-human locus.

In another embodiment, the locus to which a molecule of Formula One is applied can be any locus that is inhabited, or that may become inhabited, or that may be traversed, by a pest of Phyla Nematoda, Arthropoda, and/or Mollusca. For example, the locus can be:
(a) where crops, trees, fruits, cereals, fodder species, vines, turf, and/or ornamental plants, are growing;
(b) where domesticated animals are residing;
(c) the interior or exterior surfaces of buildings (such as places where grains are stored);
(d) the materials of construction used in buildings (such as impregnated wood); and
(e) the soil around buildings.

Particular crop areas to use a molecule of Formula One include areas where apples, corn, sunflowers, cotton, soybeans, canola, wheat, rice, sorghum, barley, oats, potatoes, oranges, alfalfa, lettuce, strawberries, tomatoes, peppers, crucifers, pears, tobacco, almonds, sugar beets, beans and other valuable crops are growing, or the seeds thereof are going to be planted. It is also advantageous to use ammonium sulfate with a molecule of Formula One when growing various plants.

In another embodiment, compounds of Formula One, Two, and/or Three are generally used in amounts from about 0.0001 grams per hectare to about 5000 grams per hectare to provide control. In another embodiment, it is preferred that compounds of Formula One, Two, and/or Three are used in amounts from about 0.001 grams per hectare to about 500 grams per hectare. In another embodiment, it is more preferred that compounds of Formula One, Two, and/or Three are used in amounts from about 0.01 gram per hectare to about 50 grams per hectare.

The compounds of Formula One, Two, and/or Three may be used in mixtures, applied simultaneously or sequentially, alone or with other compounds to enhance plant vigor (*e.g.,* to grow a better root system, to better withstand stressful growing conditions). Such other compounds are, for example, compounds that modulate plant ethylene receptors, most notably 1-methylcyclopropene (also known as 1-MCP). Furthermore, such molecules may be used during times when pest activity is low, such as before the plants that are growing begin to produce valuable agricultural commodities. Such times include the early planting season when pest pressure is usually low.

The compounds of Formula One, Two, and/or Three can be applied to the foliar and fruiting portions of plants to control pests. The molecules will either come in direct contact with the pest, or the pest will consume the pesticide when eating leaf, fruit mass, or extracting sap, which contains the pesticide. The compounds of Formula One, Two, and/or Three can also be applied to the soil, and when applied in this manner, root and stem feeding pests can be controlled. The roots can absorb a molecule taking it up into the foliar portions of the plant to control above ground chewing and sap feeding pests.

Generally, with baits, the baits are placed in the ground where, for example, termites can come into contact with, and/or be attracted to, the bait. Baits can also be applied to a surface of a building, (horizontal, vertical, or slant surface) where, for example, ants, termites, cockroaches, and flies, can come into contact with, and/or be attracted to, the bait. Baits can comprise a molecule of Formula One.

The compounds of Formula One, Two, and/or Three can be encapsulated inside, or placed on the surface of a capsule. The size of the capsules can range from nanometer size (about 100-900 nanometers in diameter) to micrometer size (about 10-900 microns in diameter).

Because of the unique ability of the eggs of some pests to resist certain pesticides, repeated applications of the compounds of Formula One, Two, and/or Three may be desirable to control newly emerged larvae.

Systemic movement of pesticides in plants may be utilized to control pests on one portion of the plant by applying (for example by spraying an area) the compounds of Formula One, Two, and/or Three to a different portion of the plant. For example, control of foliar-feeding insects can be achieved by drip irrigation or furrow application, by treating the soil with for example pre- or post-planting soil drench, or by treating the seeds of a plant before planting.

Seed treatment can be applied to all types of seeds, including those from which plants genetically modified to express specialized traits will germinate. Representative examples include those expressing proteins toxic to invertebrate pests, such as *Bacillus thuringiensis* or other insecticidal toxins, those expressing herbicide resistance, such as "Roundup Ready" seed, or those with "stacked" foreign genes expressing insecticidal toxins, herbicide resistance, nutrition-enhancement, drought resistance, or any other beneficial traits. Furthermore, such seed treatments with the compounds of Formula One, Two, and/or Three may further enhance the ability of a plant to better withstand stressful growing conditions. This results in a healthier, more vigorous plant, which can lead to higher yields at harvest time. Generally, about 1 gram of the compounds of Formula One, Two, and/or Three to about 500 grams per 100,000 seeds is expected to provide good benefits, amounts from about 10 grams to about 100 grams per 100,000 seeds is expected to provide better benefits, and amounts from about 25 grams to about 75 grams per 100,000 seeds is expected to provide even better benefits.

It should be readily apparent that the compounds of Formula One, Two, and/or Three may be used on, in, or around plants genetically modified to express specialized traits, such as *Bacillus thuringiensis* or other insecticidal toxins, or those expressing herbicide resistance, or those with "stacked" foreign genes expressing insecticidal toxins, herbicide resistance, nutrition-enhancement, or any other beneficial traits.

The compounds of Formula One, Two, and/or Three may be used for controlling endoparasites and ectoparasites in the veterinary medicine sector or in the field of non-human animal keeping. The compounds of Formula One, Two, and/or Three are applied, such as by oral administration in the form of, for example, tablets, capsules, drinks, granules, by dermal application in the form of, for example, dipping, spraying, pouring on, spotting on, and dusting, and by parenteral administration in the form of, for example, an injection.

The compounds of Formula One, Two, and/or Three may also be employed advantageously in livestock keeping, for example, cattle, sheep, pigs, chickens, and geese. They may also be employed advantageously in pets such as, horses, dogs, and cats. Particular pests to control would be fleas and ticks that are bothersome to such animals. Suitable formulations are administered orally to the animals with the drinking water or feed. The dosages and formulations that are suitable depend on the species.

The compounds of Formula One, Two, and/or Three may also be used for controlling parasitic worms, especially of the intestine, in the animals listed above.

The compounds of Formula One, Two, and/or Three may also be employed in therapeutic methods for human health care. Such methods include, but are limited to, oral administration in the form of, for example, tablets, capsules, drinks, granules, and by dermal application.

Pests around the world have been migrating to new environments (for such pest) and thereafter becoming a new invasive species in such new environment. The compounds of Formula One, Two, and/or Three may also be used on such new invasive species to control them in such new environment.

The compounds of Formula One, Two, and/or Three may also be used in an area where plants, such as crops, are growing (e.g., pre-planting, planting, pre-harvesting) and where there are low levels (even no actual presence) of pests that can commercially damage such plants. The use of such molecules in such area is to benefit the plants being grown in the area. Such benefits, may include, but are not limited to, improving the health of a plant, improving the yield of a plant (e.g., increased biomass and/or increased content of valuable ingredients), improving the vigor of a plant (e.g., improved plant growth and/or greener leaves), improving the quality of a plant (e.g., improved content or composition of certain ingredients), and improving the tolerance to abiotic and/or biotic stress of the plant.

Before a pesticide can be used or sold commercially, such pesticide undergoes lengthy evaluation processes by various governmental authorities (local, regional, state, national, and international). Voluminous data requirements are specified by regulatory authorities and must be addressed through data generation and submission by the product registrant or by a third party on the product registrant's behalf, often using a computer with a connection to the World Wide Web. These governmental authorities then review such data and if a determination of safety is concluded, provide the potential user or seller with product registration approval. Thereafter, in that locality where the product registration is granted and supported, such user or seller may use or sell such pesticide.

A molecule according to Formula One can be tested to determine its efficacy against pests. Furthermore, mode of action studies can be conducted to determine if said molecule has a different mode of action than other pesticides. Thereafter, such acquired data can be disseminated, such as by the internet, to third parties.

The headings in this document are for convenience only and must not be used to interpret any portion hereof.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced within the scope of the appended claims.

## Claims

1. A compound having a structure of the following formula ("Formula One") 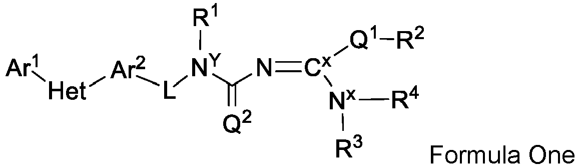 wherein:
(A) Ar¹ is selected from
(1) furanyl, phenyl, pyridazinyl, pyridyl, pyridinonyl, pyrimidinyl, thienyl, or
(2) substituted furanyl, substituted phenyl, substituted pyridazinyl, substituted pyridyl, substituted pyridinonyl, substituted pyrimidinyl, or substituted thienyl,
wherein said substituted furanyl, substituted phenyl, substituted pyridazinyl, substituted pyridyl, substituted pyridazinyl, substituted pyridinonyl, substituted pyrimidinyl, and substituted thienyl have one or more substituents independently selected from H, F, Cl, Br, I, CN, OH, SH, SF₅, NO₂, NR^{×}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{×}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1),
wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) substituent may be optionally substituted with one or more substituents independently selected from H, F, Cl, Br, I, CN, OH, SH, SF₅, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)OC₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) substituent may be optionally substituted with one or more substituents independently selected from H, F, Cl, Br, I, CN, OH, SH, SF₅, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)OC₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(B)** Het is a 5- or 6-membered, saturated or unsaturated, heterocyclic ring, containing one or more heteroatoms independently selected from nitrogen, sulfur, or oxygen, where said heterocyclic ring may also be substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O) C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, or S(=O)₂NR^{x}R^{y},
wherein each alkyl, haloalkyl, cycloalkyl, halocycloalkyl, alkoxy, haloalkoxy, alkenyl, cycloalkenyl, haloalkenyl, alkynyl, phenyl, and phenoxy substituent may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)C(=O)NR^{x}R^{y}, (C₁-C₈ alkyl)NR^{x}R^{y}, C(=O)C(=O)(C₁-C₈ alkyl), C(=O)O(C₁-C₈ alkyl), C(=O)C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, or S(=O)₂NR^{x}R^{y};
**(C)** Ar² is selected from
**(1)** furanyl, phenyl, pyridazinyl, pyridyl, pyrimidinyl, thienyl, or
**(2)** substituted furanyl, substituted phenyl, substituted pyridazinyl, substituted pyridyl, substituted pyrimidinyl, or substituted thienyl,
wherein said substituted furanyl, substituted phenyl, substituted pyridazinyl, substituted pyridyl, substituted pyrimidinyl, and substituted thienyl, have one or more substituents independently selected from H, F, Cl, Br, I, CN, OH, SH, SF₅, NO₂, NR^{×}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{×}R^{y}, S(=O)NR^{×}R^{y}, S(=O)₂NR^{x}R^{y}, or (Het-1),
wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) substituent may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)O(C₁-C₈ haloalkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{×}R^{y}, S(=O)NR^{×}R^{y}, S(=O)₂NR^{x}R^{y}, or (Het-1);
**(D)** R¹ is selected from H, CN, OH, SH, NO₂, C(=O)H, NR^{×}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{×}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, and (C₁-C₈ alkyl)-O-phenyl, phenyl, phenoxy, Si(C₁-C₈ alkyl)₃, SNR^{×}R^{y}, S(=O)NR^{×}R^{y}, S(=O)₂NR^{x}R^{y}, or (Het-1),
wherein each alkyl, haloalkyl, cycloalkyl, halocycloalkyl, alkoxy, haloalkoxy, alkenyl, cycloalkenyl, haloalkenyl, alkynyl, phenyl, phenoxy, and (Het-1) may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, and (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(E)** R² is selected from **(J),** H, C₁-C₈ alkyl, C₃-C₈ cycloalkyl, C₂-C₈ alkenyl, C₂-C₈ alkynyl, C(=O)(C₁-C₈ alkyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), C₁-C₈ alkyl)S(=O)₂(C₁-C₈ alkyl), (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-OC(=O)-(C₁-C₈ alkyl), (C₁-C₈ alkyl)-O-C(=O)O-(C₁-C₈ alkyl), (C₁-C₈ alkyl)-O-C(=O)NR^{x}R^{y}, (C₁-C₈ alkyl)-C(=O)N(R^{×})(C₁-C₈ alkyl)-(Het-1), (C₁-C₈ alkyl)-C(=O)(Het-1), (C₁-C₈ alkyl)-C(=O)N(R^{×})(C₁-C₈ alkyl)N(R^{y})C(=O)OH, (C₁-C₈ alkyl)-C(=O)N(R^{×})(C₁-C₈ alkyl)N(R^{x})(R^{y}), (C₁-C₈ alkyl)-C(=O)N(R^{×})(C₁-C₈ alkyl)N(R^{y})C(=O)O-(C₁-C₈ alkyl), (C₁-C₈ alkyl)-C(=O)N(R^{x})(C₁-C₈ alkyl)(N(R^{y})C(=O)O-(C₁-C₈ alkyl)C(=O)OH, (C₁-C₈ alkyl)-C(=O)(Het-1)C(=O)O-(C₁-C₈ alkyl), (C₁-C₈ alkyl)-OC(=O)O-(C₁-C₈ alkyl), (C₁-C₈ alkyl)-OC(=O)-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-OC(=O)-(C₁-C₈ alkyl)N(R^{x})C(=O)O-(C₁-C₈ alkyl), (C₁-C₈ alkyl)-NR^{x}R^{y}, (C₁-C₈ alkyl)-S-(Het-1), (C₁-C₈ alkyl)S(Het-1), (C₁-C₈ alkyl)S(=O)(Het-1), (C₁-C₈ alkyl)S(=O)₂(Het-1), or (C₁-C₈ alkyl)-O-(Het-1), wherein each alkyl, cycloalkyl, phenyl, and (Het-1) are optionally substituted with one or more substituents independently selected from H, F, Cl, Br, I, CN, OH, SH, NO₂, NR^{×}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, phenoxy, Si(C₁-C₈ alkyl)₃, SNR^{x}R^{y}, S(=O)NR^{x}R^{y}, S(=O)₂NR^{x}R^{y}, or (Het-1);
**(F)** R³ has the following structure: 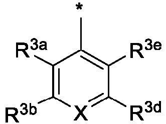 wherein
X is N or CR^{3c};
each R^{3a}, R^{3b}, R^{3c}, R^{3d}, and R^{3e} is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, or phenoxy;
two of R^{3c}, R^{3d}, and R^{3e} forms a 5- or 6-membered, saturated or unsaturated, ring having zero, one, or two heteroatoms selected from nitrogen, sulfur, and oxygen, and optionally substituted with at least one R¹⁴; and
each R¹⁴ is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, or phenoxy;
**(G)** R⁴ is selected from **(J),** H, OH, SH, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), C(=O)(C₁-C₈ alkyl)C(=O)O(C₁-C₈ alkyl), (C₁-C₈ alkyl)C(=O)O(C₁-C₈ alkyl), phenyl, (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-OC(=O)-(C₁-C₈ alkyl), (C₁-C₈ alkyl)-O-C(=O)O-(C₁-C₈ alkyl), (C₁-C₈ alkyl)-O-C(=O)NR^{x}R^{y}, (C₁-C₈ alkyl)-C(=O)N(R^{x})(C₁-C₈ alkyl)-(Het-1), (C₁-C₈ alkyl)-C(=O)(Het-1), (C₁-C₈ alkyl)-C(=O)N(R^{x})(C₁-C₈ alkyl)N(R^{y})C(=O)OH, (C₁-C₈ alkyl)-C(=O)N(R^{x})(C₁-C₈ alkyl)N(R^{x})(R^{y}), (C₁-C₈ alkyl)-C(=O)N(R^{x})(C₁-C₈ alkyl)N(R^{y})C(=O)O-(C₁-C₈ alkyl), (C₁-C₈ alkyl)-C(=O)N(R^{x})(C₁-C₈ alkyl)(N(R^{y})C(=O)O-(C₁-C₈ alkyl)C(=O)OH, (C₁-C₈ alkyl)-C(=O)(Het-1)C(=O)O-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-OC(=O)-(C₁-C₈ alkyl)N(R^{x})C(=O)O-(C₁-C₈ alkyl), (C₁-C₈ alkyl)-NR^{x}R^{y}, (C₁-C₈ alkyl)-S-(Het-1), (C₁-C₈ alkyl)S(=O)(Het-1), (C₁-C₈ alkyl)S(=O)₂(Het-1), or (C₁-C₈ alkyl)-O-(Het-1),
wherein each alkyl, haloalkyl, cycloalkyl, halocycloalkyl, cycloalkoxy, halocycloalkoxy, alkoxy, haloalkoxy, alkenyl, cycloalkenyl, haloalkenyl, alkynyl, phenyl, and (Het-1), are optionally substituted with one or more substituents independently selected from H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, halophenyl, phenoxy, and (Het-1);
**(H)** each of Q¹ and Q² is independently selected from O or S;
**(I)** R^{x} and R^{y} are independently selected from H, OH, SH, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, (C₁-C₈ alkyl)phenyl, (C₁-C₈ alkyl)-O-phenyl, C(=O)(Het-1), (Het-1), (C₁-C₈ alkyl)-(Het-1), (C₁-C₈ alkyl)-C(=O)-(C₁-C₈ alkyl), (C₁-C₈ alkyl)-C(=O)(Het-1), (C₁-C₈ alkyl)-C(=O)(Het-1)C(=O)O-(C₁-C₈ alkyl), (C₁-C₈ alkyl)-OC(=O)O-(C₁-C₈ alkyl), (C₁-C₈ alkyl)-OC(=O)-(C₁-C₈ alkyl), (C₁-C₈ alkyl)-OC(=O)-(C₃-C₈ cycloalkyl), (C₁-C₈ alkyl)-OC(=O)-(Het-1), (C₁-C₈ alkyl)-S-(Het-1), (C₁-C₈ alkyl)S(=O)(Het-1), (C₁-C₈ alkyl)S(=O)₂(Het-1), or (C₁-C₈ alkyl)-O-(Het-1),
wherein each alkyl, haloalkyl, cycloalkyl, halocycloalkyl, cycloalkoxy, halocycloalkoxy, alkoxy, haloalkoxy, alkenyl, cycloalkenyl, haloalkenyl, alkynyl, phenyl, and (Het-1), are optionally substituted with one or more substituents independently selected from H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)OH, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈alkyl)S(C₁-C₈ alkyl), (C₁-C₈alkyl)S(=O)(C₁-C₈ alkyl), (C₁-C₈alkyl)S(=O)₂(C₁-C₈ alkyl), (C₁-C₈alkyl)OC(=O)(C₁-C₈ alkyl), (C₁-C₈alkyl)OC(=O)O(C₁-C₈ alkyl), C(=O)(C₁-C₈ alkyl)C(=O)O(C₁-C₈ alkyl), (C₁-C₈alkyl)C(=O)O(C₁-C₈ alkyl), (C₁-C₈alkyl)C(=O)(C₁-C₈ alkyl), (C₁-C₈ alkyl)phenyl, (C₁-C₈alkyl)-O-phenyl, phenyl, halophenyl, phenoxy, and (Het-1),
or R^{x} and R^{y} together can optionally form a 5- to 7-membered saturated or unsaturated cyclic group which may contain one or more heteroatoms selected from nitrogen, sulfur, and oxygen, and where said cyclic group may be substituted with H, F, Cl, Br, I, CN, OH, SH, NO₂, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈alkyl)O(C₁-C₈ alkyl), (C₁-C₈ alkyl)S(C₁-C₈ alkyl), (C₁-C₈alkyl)S(=O)(C₁-C₈ alkyl), (C₁-C₈ alkyl)S(=O)₂(C₁-C₈ alkyl), (C₁-C₈alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈alkyl)C(=O)(C₁-C₈ alkyl), (C₁-C₈ alkyl)phenyl, (C₁-C₈alkyl)-O-phenyl, phenyl, substituted phenyl, phenoxy, and (Het-1);
**(J)** R² and R⁴ may be a 1- to 4-membered saturated or unsaturated, hydrocarbyl link, which may contain one or more heteroatoms selected from nitrogen, sulfur, and oxygen, and together with C^{x}(Q¹)(N^{x}) forms a 4-to 7-membered cyclic structure, wherein said hydrocarbyl link may optionally be substituted with one or more substituents independently selected from R⁵, R⁶, and R⁷, wherein each R⁵, R⁶, and R⁷ is selected from H, F, Cl, Br, I, CN, OH, SH, NO₂, NR^{x}R^{y}, C₁-C₈ alkyl, C₁-C₈ alkyl substituted with at least one OH, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, substituted phenyl, phenoxy, or (Het-1);
**(K)** (Het-1) is a 5- or 6-membered, saturated or unsaturated, heterocyclic ring, containing one or more heteroatoms independently selected from nitrogen, sulfur or oxygen, wherein said heterocyclic ring may also be substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, and phenoxy,
wherein each alkyl, cycloalkyl, alkoxy, alkenyl, alkynyl, phenyl, and phenoxy may be optionally substituted with one or more substituents independently selected from H, F, Cl, Br, I, CN, OH, SH, NO₂, NR^{x}R^{y}, C₁-C₈ alkyl, C₁-C₈ haloalkyl, C₃-C₈ cycloalkyl, C₃-C₈ halocycloalkyl, C₃-C₈ cycloalkoxy, C₃-C₈ halocycloalkoxy, C₁-C₈ alkoxy, C₁-C₈ haloalkoxy, C₂-C₈ alkenyl, C₃-C₈ cycloalkenyl, C₂-C₈ haloalkenyl, C₂-C₈ alkynyl, S(C₁-C₈ alkyl), S(C₃-C₈ cycloalkyl), S(C₁-C₈ haloalkyl), S(C₃-C₈ halocycloalkyl), S(=O)(C₁-C₈ alkyl), S(=O)(C₁-C₈ haloalkyl), OS(=O)₂(C₁-C₈ alkyl), S(=O)₂(C₁-C₈ alkyl), S(=O)₂(C₁-C₈ haloalkyl), OS(=O)₂(C₁-C₈ alkyl), OS(=O)₂(C₁-C₈ haloalkyl), C(=O)H, C(=O)NR^{x}R^{y}, (C₁-C₈ alkyl)NR^{x}R^{y}, C(=O)(C₁-C₈ alkyl), C(=O)O(C₁-C₈ alkyl), C(=O)(C₁-C₈ haloalkyl), C(=O)O(C₁-C₈ haloalkyl), C(=O)(C₃-C₈ cycloalkyl), C(=O)O(C₃-C₈ cycloalkyl), C(=O)(C₂-C₈ alkenyl), C(=O)O(C₂-C₈ alkenyl), (C₁-C₈ alkyl)O(C₁-C₈ alkyl), (C₁-C₈ alkyl)S(C₁-C₈ alkyl), (C₁-C₈ alkyl)S(=O)(C₁-C₈ alkyl), (C₁-C₈ alkyl)S(=O)₂(C₁-C₈ alkyl), (C₁-C₈ alkyl)OC(=O)(C₁-C₈ alkyl), (C₁-C₈ alkyl)OC(=O)O(C₁-C₈ alkyl), C(=O)(C₁-C₈ alkyl)C(=O)O(C₁-C₈ alkyl), (C₁-C₈ alkyl)C(=O)O(C₁-C₈ alkyl), (C₁-C₈ alkyl)C(=O)(C₁-C₈ alkyl), (C₁-C₈ alkyl)phenyl, (C₁-C₈ alkyl)-O-phenyl, phenyl, and phenoxy; and
**(L)** L is a bond which connects Ar² to N^{Y}.

2. The compound of claim 1, wherein R³ has a structure of Formula R3A or Formula R3B: 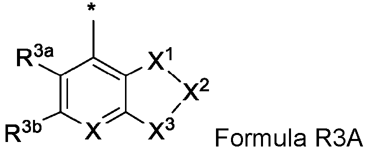 or 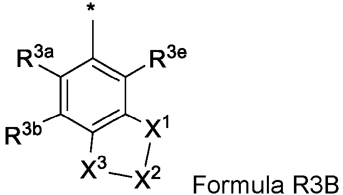 wherein
X is N or CR^{3c};
X¹ is O, CH, CH₂, N, N(R¹⁴), S, C(O), or S;
X² is O, CH, CH₂, N, N(R¹⁴), S, C(O), or S;
X³ is O, CH, CH₂, N, N(R¹⁴), S, C(O), S, O(CH₂), (CH₂)O, -N=CH-, -CH=N-, C(O)O, OC(O), -N(R¹⁴)-CH₂-, -CH₂-N(R¹⁴)-, -N(R¹⁴)-CH-, -CH-N(R¹⁴)-, - CH=CH-, or -CH₂-CH₂-;
R^{3a}, R^{3b}, R^{3c}, and R¹⁴ is independently H, F, Cl, Br, I, CN, OH, SH, NO₂, C₁-C₈ alkyl, C₁-C₈ haloalkyl, C₁-C₈ alkoxy, C₁-C₈ haloalkoxy, C(=O)(C₁-C₈ alkyl), C(=O)O(C₁-C₈ alkyl), C(=O)(C₁-C₈ haloalkyl), C(=O)O(C₁-C₈ haloalkyl), (C₁-C₈ alkyl)O(C₁-C₈ alkyl), (C₁-C₈ alkyl)S(C₁-C₈ alkyl), (C₁-C₈ haloalkyl)O(C₁-C₈ haloalkyl), (C₁-C₈ haloalkyl)S(C₁-C₈ haloalkyl), (C₁-C₈ alkyl)O(C₁-C₈ haloalkyl), (C₁-C₈ haloalkyl)O(C₁-C₈ alkyl), (C₁-C₈ alkyl)S(C₁-C₈ haloalkyl), (C₁-C₈ haloalkyl)S(C₁-C₈ alkyl), (C₁-C₈ alkyl)C(=O)(C₁-C₈ alkyl), (C₁-C₈ alkyl)phenyl, (C₁-C₈alkyl)-O-phenyl, phenyl, or phenoxy.

3. The compound of claim 1, wherein:
**(A)** Ar¹ is a substituted phenyl, a substituted pyridyl, a substituted pyridazinyl, a substituted pyridinonyl, or a substituted pyrimidinyl, wherein said substituted phenyl, substituted pyridyl, substituted pyridazinyl, substituted pyridinonyl, and substituted pyrimidinyl have one or more substituents independently selected from H, CN, SF₅, C₁-C₆ alkyl, C₁-C₆ alkoxy, S(C₁-C₆ haloalkyl), C₁-C₆ haloalkyl and C₁-C₆ haloalkoxy;
**(B)** Het is triazolyl or pyrazolyl;
**(C)** Ar² is phenyl, pyridyl, pyrimidinyl, substituted phenyl, substituted pyridyl, or substituted pyrimidinyl, wherein said substituted phenyl, substituted pyridyl, and substituted pyrimidinyl have one or more substituents independently selected from H, F, Cl, Br, I, CN, NO₂, NR^{x}R^{y}, OH, C₁-C₆ alkoxy, (C₁-C₆ alkyl)O(C₁-C₈ alkyl), C₁-C₆ haloalkyl, and C₁-C₆ alkyl;
**(D)** R¹ is H, C₁-C₆ alkyl, C₃-C₆ cycloalkyl, or C₂-C₆ alkenyl, wherein said alkyl, cycloalkyl, or alkenyl is optionally substituted with a C₁-C₆ alkoxy, C₁-C₆ haloalkoxy, C₃-C₆ cycloalkyl, or C₃-C₆ halocycloalkyl;
**(E)** R² is selected from **(J),** H, C₁-C₆ alkyl, or C₁-C₆ haloalkyl;
**(F)** R³ is selected from one of the following groups R³ 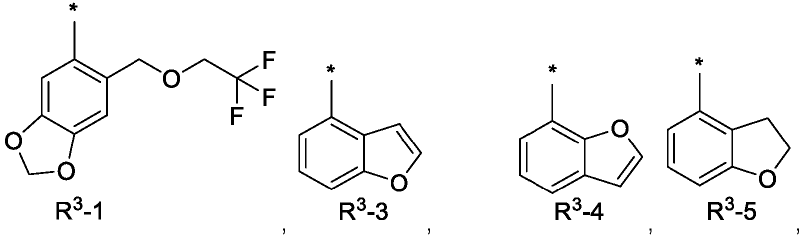 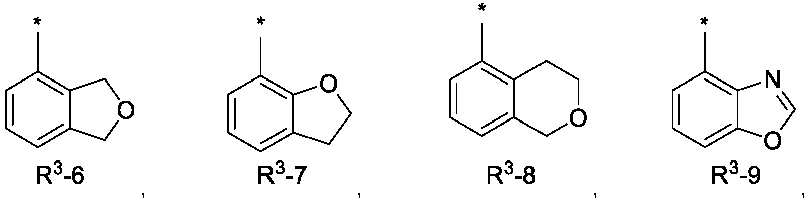 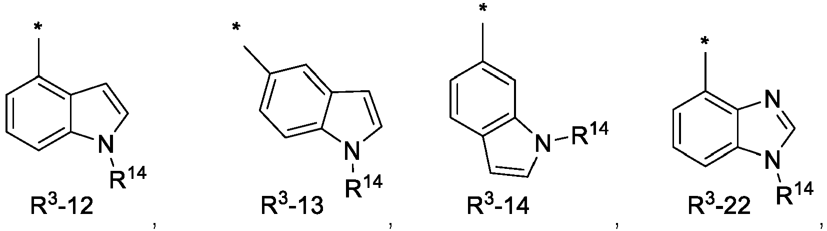 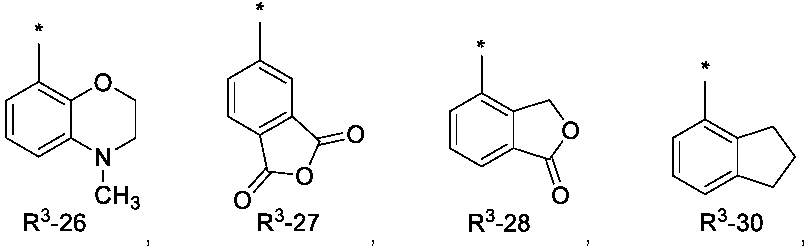 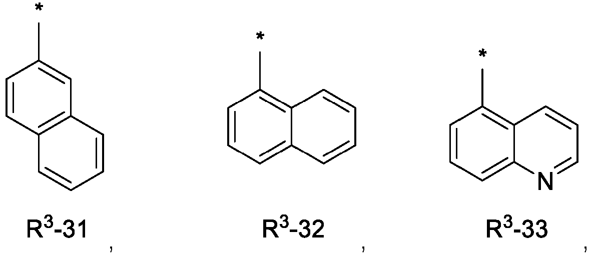 wherein R¹⁴, if applicable to group R³, is selected from the group consisting of H and C₁-C₆ alkyl;
**(G)** R⁴ is selected from (J), H, C₁-C₆ alkyl, or C₁-C₆ haloalkyl;
**(H)** Q¹ is S and Q² is O;
**(I)** R^{x} and R^{y} are independently selected from H, C(=O)(C₁-C₆ alkyl), C(=O)O(C₁-C₆ alkyl), C₁-C₆ alkyl, C₁-C₆ haloalkyl, C₃-C₆ cycloalkyl, C₃-C₆ halocycloalkyl, C₂-C₆ alkenyl, C₃-C₆ cycloalkenyl, C₂-C₆ haloalkenyl, C₂-C₆ alkynyl, and phenyl; and
**(J)** R² and R⁴ may be a 1- to 4-membered saturated or unsaturated, hydrocarbyl link, which may contain one or more heteroatoms selected from nitrogen, sulfur, and oxygen, and together with C^{x}(Q¹)(N^{x}) forms a cyclic structure, wherein said hydrocarbyl link may optionally be substituted with one or more substituents independently selected from R⁵, R⁶, and R⁷, wherein each R⁵, R⁶, and R⁷ is selected from H, F, Cl, Br, I, CN, NO₂, NR^{x}R^{y}, OH, SH, S(C₁-C₆ alkyl), S(C₁-C₆ haloalkyl), C₁-C₆ alkyl, C₁-C₆ alkyl substituted with OH, C₁-C₆ haloalkyl, C₁-C₆ alkoxy, C₁-C₆ haloalkoxy, C₃-C₆ cycloalkyl, C₃-C₆ halocycloalkyl, phenyl, and oxo.

4. The compound of claim 1, having a structure of **Formula Two:**

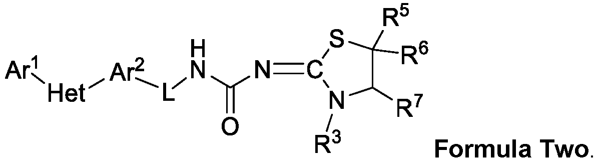

5. The compound of claim 1, having a structure selected from compounds listed in Table 1.

6. The compound of claim 1, wherein
**(A)** Ar¹ is a substituted phenyl, a substituted pyridyl, or a substituted pyrimidinyl, wherein said substituted phenyl, substituted pyridyl, and substituted pyrimidinyl have one or more substituents independently selected from H, CN, SF₅, C₁-C₆ alkyl, C₁-C₆ alkoxy, C₁-C₆ haloalkyl and C₁-C₆ haloalkoxy;
**(B)** Het is triazolyl or pyrazolyl;
**(C)** Ar² is phenyl, pyridyl, pyrimidinyl, substituted phenyl, substituted pyridyl, or substituted pyrimidinyl, wherein said substituted phenyl, substituted pyridyl, and substituted pyrimidinyl have one or more substituents independently selected from H, F, Cl, Br, I, CN, NO₂, NR^{x}R^{y}, OH, C₁-C₆ alkoxy, (C₁-C₆ alkyl)O(C₁-C₈ alkyl), C₁-C₆ haloalkyl, and C₁-C₆ alkyl;
**(D)** R¹ is H, C₁-C₆ alkyl, C₃-C₆ cycloalkyl, or C₂-C₆ alkenyl, wherein said alkyl, cycloalkyl, or alkenyl is optionally substituted with a C₁-C₆ alkoxy, C₁-C₆ haloalkoxy, C₃-C₆ cycloalkyl, or C₃-C₆ halocycloalkyl;
**(E)** R² is selected from **(J),** H, C₁-C₆ alkyl, or C₁-C₆ haloalkyl;
**(F)** R³ is a selected from one of the following groups R³ 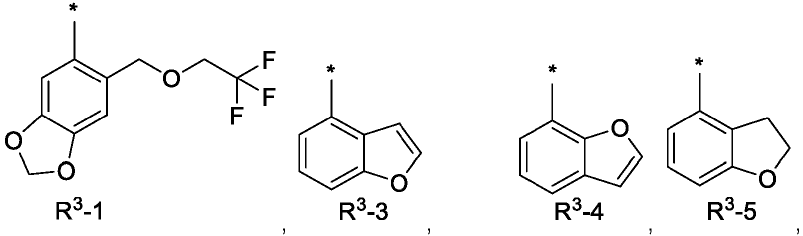 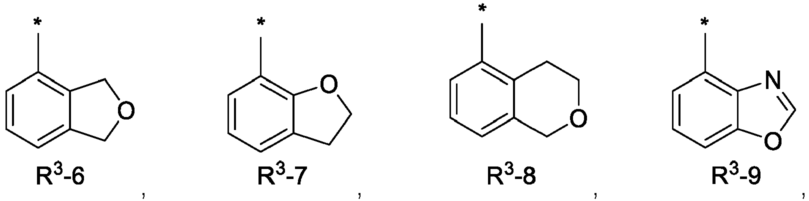 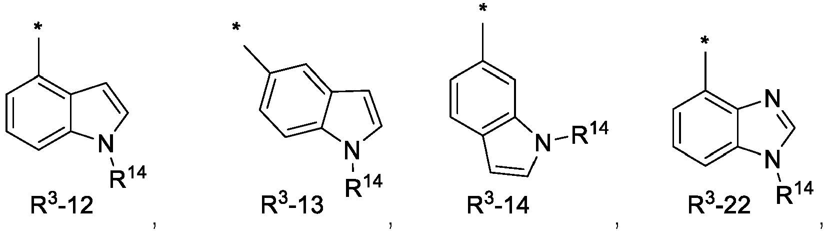 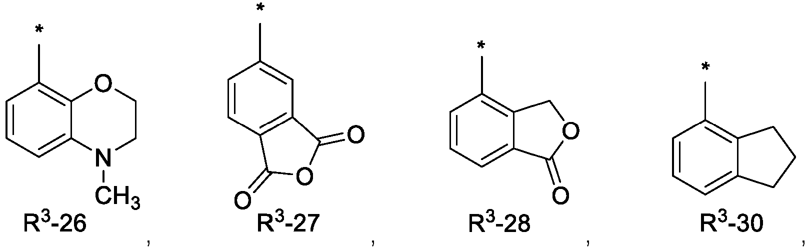 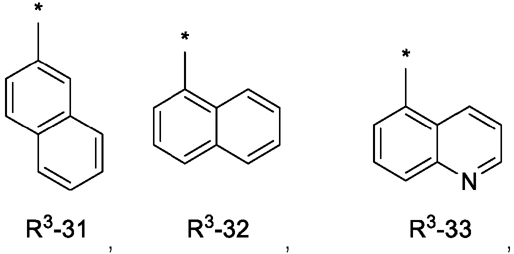 wherein R¹⁴, if applicable to group R³, is selected from the group consisting of H and CH₃;
**(G)** R⁴ is selected from **(J),** H, C₁-C₆ alkyl, or C₁-C₆ haloalkyl;
**(H)** Q¹ is S and Q² is O;
**(I)** R^{x} and R^{y} are independently selected from H, C(=O)(C₁-C₆ alkyl), C₁-C₆ alkyl, C₁-C₆ haloalkyl, C₃-C₆ cycloalkyl, C₃-C₆ halocycloalkyl, C₂-C₆ alkenyl, C₃-C₆ cycloalkenyl, C₂-C₆ haloalkenyl, C₂-C₆ alkynyl, and phenyl; and
**(J)** R² and R⁴ may be a 1- to 4-membered saturated or unsaturated, hydrocarbyl link, which may contain one or more heteroatoms selected from nitrogen, sulfur, and oxygen, and together with C^{x}(Q¹)(N^{x}) forms a cyclic structure, wherein said hydrocarbyl link may optionally be substituted with one or more substituents independently selected from R⁵, R⁶, and R⁷, wherein each R⁵, R⁶, and R⁷ is selected from H, F, Cl, Br, I, CN, NO₂, NR^{x}R^{y}, OH, SH, S(C₁-C₆ alkyl), S(C₁-C₆ haloalkyl), C₁-C₆ alkyl, C₁-C₆ haloalkyl, C₁-C₆ alkoxy, C₁-C₆ haloalkoxy, C₃-C₆ cycloalkyl, C₃-C₆ halocycloalkyl, phenyl, and oxo.

7. A compound of claim 1 wherein 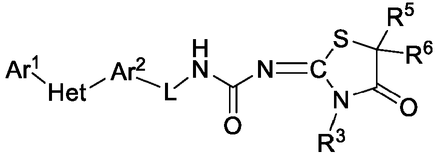
**(A)** Ar¹ is a substituted phenyl wherein said substituted phenyl has one or more substituents independently selected from H, SF₅, C₁-C₈ haloalkyl, and C₁-C₈ haloalkoxy;
**(B)** Het is a 1,2,3-triazolyl;
**(C)** Ar² is a substituted phenyl wherein said substituted phenyl has one or more substituents independently selected from H, F, Cl, and CH₃;
**(D)** R⁵ and R⁶ are H;
**(E)** R³ is selected from is a selected from one of the following groups R³ 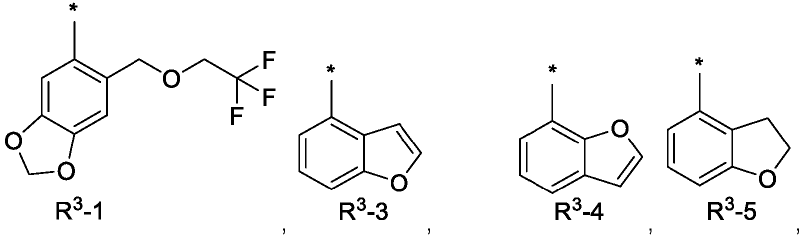 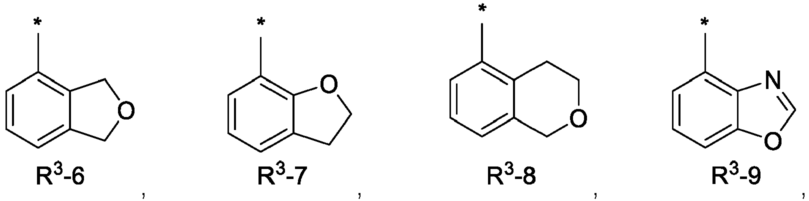 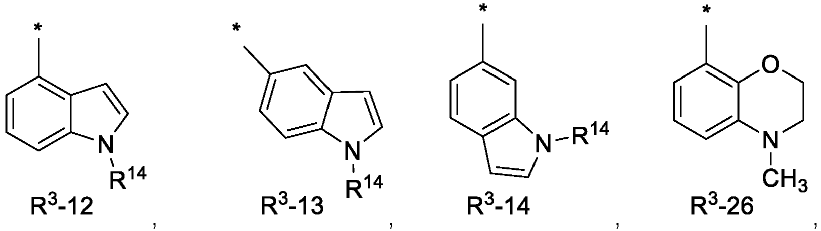 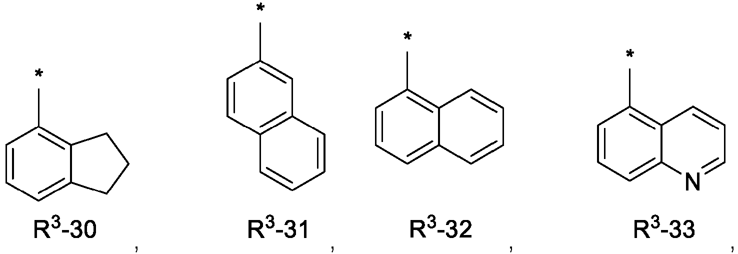 wherein R¹⁴, if applicable to group R³, is selected from the group consisting of H and CH₃.

8. The compound of claim 1, having one of the following structures

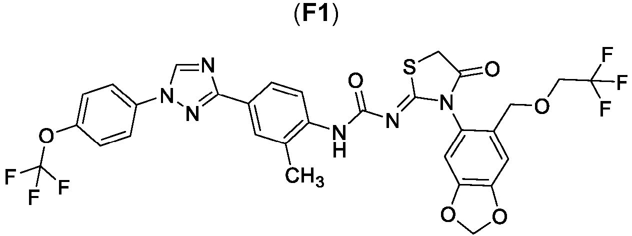

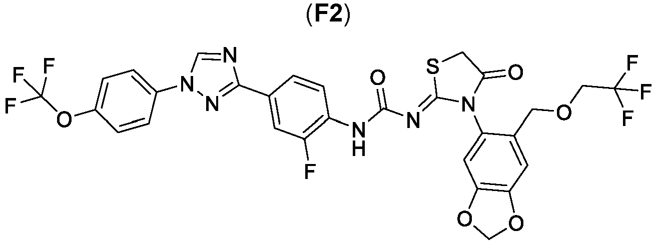

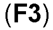

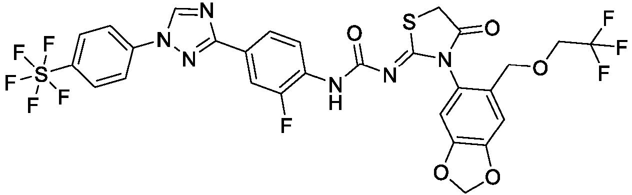

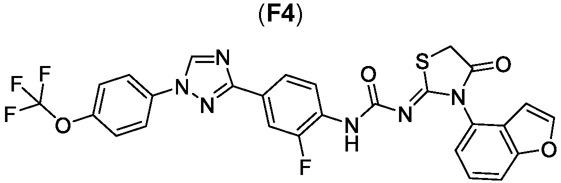

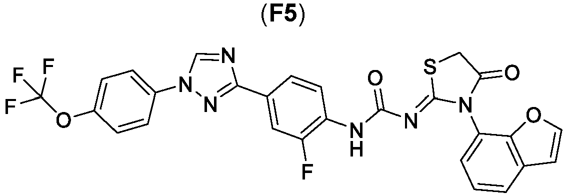

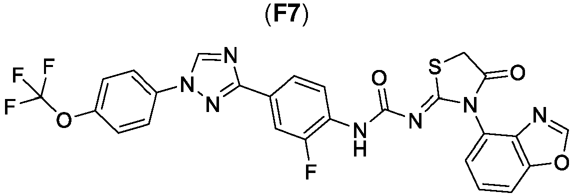

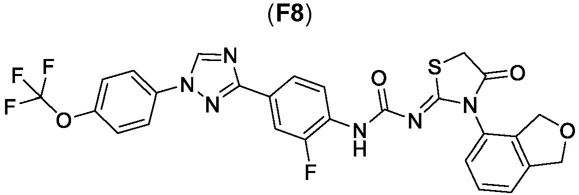

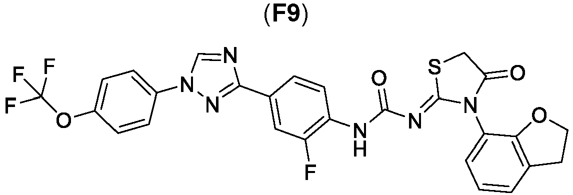

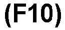

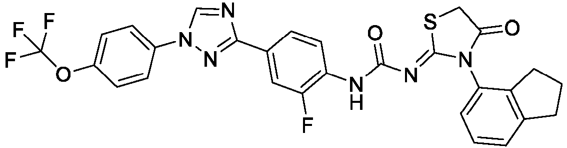

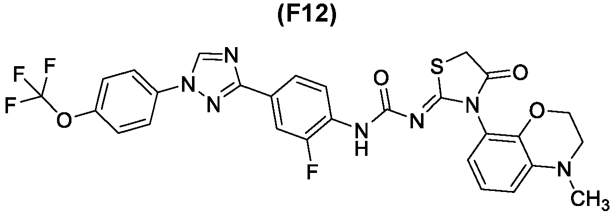

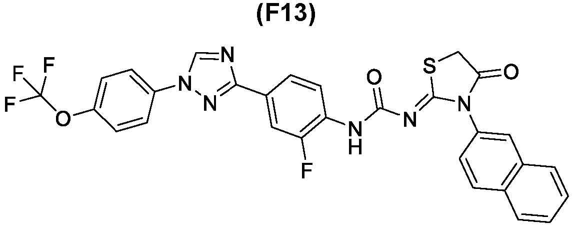

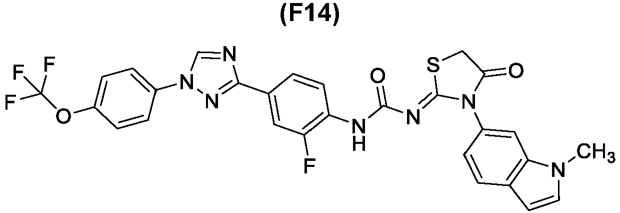

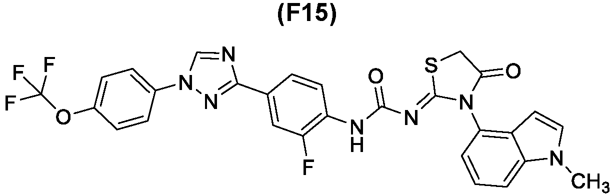

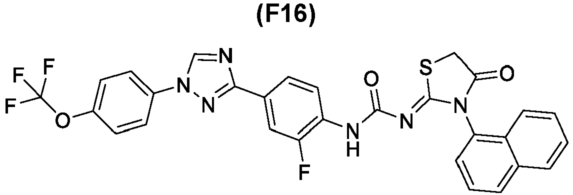

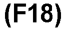

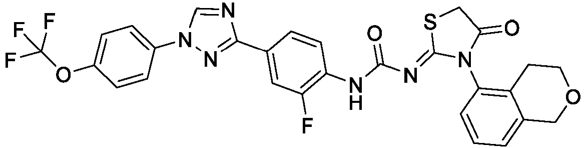

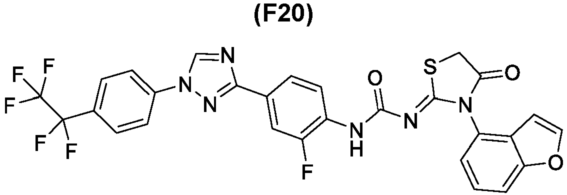

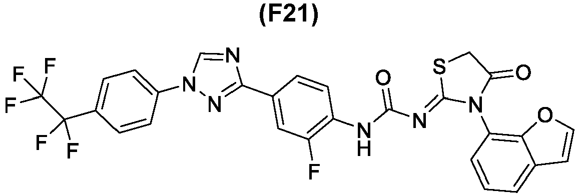

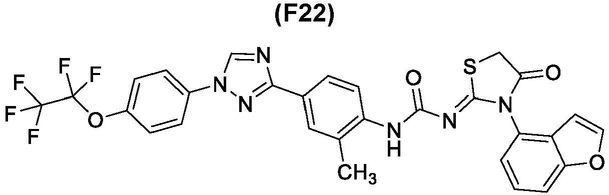

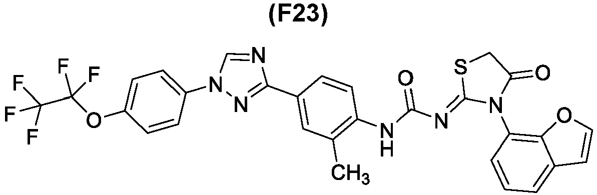

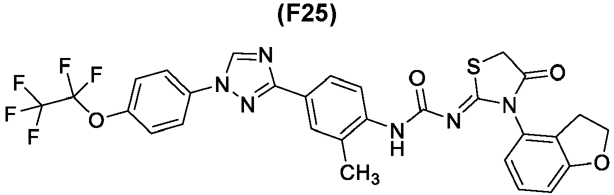

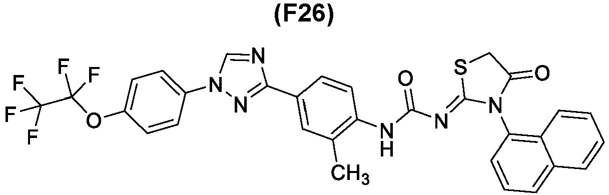

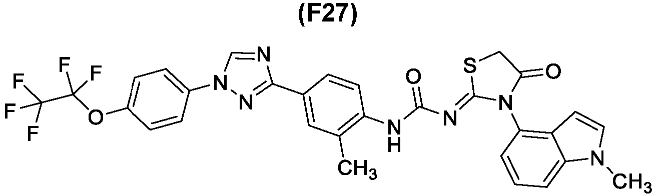

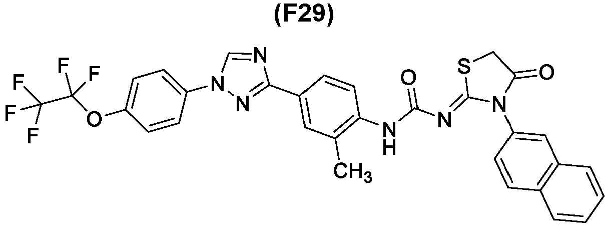

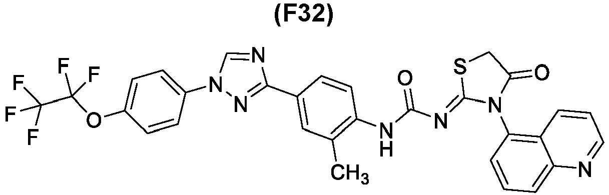

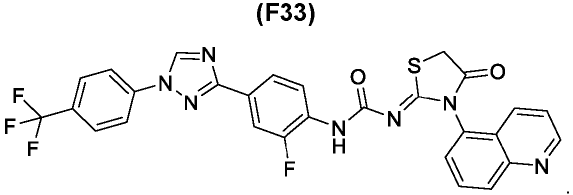

9. A compound of claim 1 for use in controlling endoparasites and ectoparasites in the veterinary medicine sector or in human health care.

10. A non-therapeutic process comprising applying the compound of claim 1 to a locus to control a pest, in an amount sufficient to control such pest.

11. The non-therapeutic process of claim 10, wherein said pest is beet armyworm (BAW), cabbage looper (CL), or yellow fever mosquito (YFM).

## Patentansprüche

1. Verbindung mit einer Struktur der folgenden Formel ("Formel Eins") 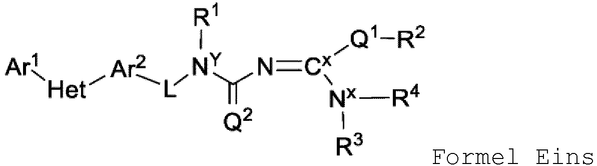 wobei:
(A) Ar¹ ausgewählt ist aus
(1) Furanyl, Phenyl, Pyridazinyl, Pyridyl, Pyridinonyl, Pyrimidinyl, Thienyl oder
(2) substituiertem Furanyl, substituiertem Phenyl, substituiertem Pyridazinyl, substituiertem Pyridyl, substituiertem Pyridinonyl, substituiertem Pyrimidinyl oder substituiertem Thienyl,
wobei das substituierte Furanyl, substituierte Phenyl, substituierte Pyridazinyl, substituierte Pyridyl, substituierte Pyridazinyl, substituierte Pyridinonyl, substituierte Pyrimidinyl und substituierte Thienyl einen oder mehrere Substituenten aufweisen, die unabhängig aus H, F, Cl, Br, I, CN, OH, SH, SF₅, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)O(C₁-C₈-halogenalkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)( C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind,
wobei jeder Alkyl-, Halogenalkyl-, Cycloalkyl-, Halogencycloalkyl-, Alkoxy-, Halogenalkoxy-, Alkenyl-, Cycloalkenyl-, Halogenalkenyl-, Alkinyl-, Phenyl-, Phenoxy- und (Het-1)-Substituent gegebenenfalls durch einen oder mehrere Substituenten substituiert sein kann, die unabhängig aus H, F, Cl, Br, I, CN, OH, SH, SF₅, NO₂, NR^{x}R^{y}, C₁-C₈-Alkyl, C₁-C₈-Halogenalkyl , C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)O(C₁-C₈-Halogenalkyl), (C₁-C₈-Alkyl)S(C₁-C₈-alkyl), (C₁-C₈-Alkyl)S(=O)(C₁-C₈-alkyl), C₁-C₈-Alkyl)S(=O)₂(C₁-C₈-alkyl), (C₁-C₈-Alkyl) OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)OC₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind;
wobei jeder Alkyl-, Halogenalkyl-, Cycloalkyl-, Halogencycloalkyl-, Alkoxy-, Halogenalkoxy-, Alkenyl-, Cycloalkenyl-, Halogenalkenyl-, Alkinyl-, Phenyl-, Phenoxy- und (Het-1)-Substituent gegebenenfalls durch einen oder mehrere Substituenten substituiert sein kann, die unabhängig aus H, F, Cl, Br, I, CN, OH, SH, SF₅, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S (C₁-C₈-Alkyl), S (C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)O(C₁-C₈-halogenalkyl), (C₁-C₈-Alkyl)S(C₁-C₈-alkyl), (C₁-C₈-Alkyl)S(=O)(C₁-C₈-alkyl), C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl) C(=O)OC₁-C₈-alkyl) , (C₁-C₈-Alkyl)C (=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind;
(B) Het für einen 5- oder 6-gliedrigen, gesättigten oder ungesättigten, heterocyclischen Ring mit einem oder mehreren Heteroatomen, die unabhängig aus Stickstoff, Schwefel oder Sauerstoff ausgewählt sind, steht, wobei der heterocyclische Ring auch durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl) , S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), ( C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl) OC(=O)O(C₁-C₈-alkyl), C(=O)(C₁-C₈-Alkyl)C(=O)O(C₁-C₈-alkyl) , (C₁-C₈-Alkyl)C(=O)O(C₁-C₈-alkyl), (C₁-C₈-Alkyl)C(=O)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y} oder S(=O)₂NR^{x}R^{y} ausgewählt sind,
wobei jeder Alkyl-, Halogenalkyl-, Cycloalkyl-, Halogencycloalkyl-, Alkoxy-, Halogenalkoxy-, Alkenyl-, Cycloalkenyl-, Halogenalkenyl-, Alkinyl-, Phenyl- und Phenoxysubstituent gegebenenfalls durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂ (C₁-C₈-Halogenalkyl), C(=O)C(=O)NR^{x}R^{y}, (C₁-C₈-Alkyl) NR^{x}R^{y}, C(=O)C(=O)(C₁-C₈-Alkyl), C(=O)O(C₁-C₈-Alkyl), C(=O)C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S (=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y} oder S(=O)₂NR^{x}R^{y} ausgewählt sind;
(C)Ar² ausgewählt ist aus
(1) Furanyl, Phenyl, Pyridazinyl, Pyridyl, Pyrimidinyl, Thienyl oder
(2) substituiertem Furanyl, substituiertem Phenyl, substituiertem Pyridazinyl, substituiertem Pyridyl, substituiertem Pyrimidinyl oder substituiertem Thienyl, wobei das substituierte Furanyl, substituierte Phenyl, substituierte Pyridazinyl, substituierte Pyridyl, substituierte Pyrimidinyl und substituierte Thienyl einen oder mehrere Substituenten aufweisen, die unabhängig aus H, F, Cl, Br, I, CN, OH, SH, SF₅, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)O(C₁-C₈-halogenalkyl) , (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈)-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl) C(=O)O(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind,
wobei jeder Alkyl-, Halogenalkyl-, Cycloalkyl-, Halogencycloalkyl-, Alkoxy-, Halogenalkoxy-, Alkenyl-, Cycloalkenyl-, Halogenalkenyl-, Alkinyl-, Phenyl-, Phenoxy- und (Het-1)-Substituent gegebenenfalls durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S (C₁-C₈-Alkyl) , S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl, C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)O(C₁-C₈-halogenalkyl), (C₁-C₈-Alkyl)S(C₁-C₈-alkyl), (C₁-C₈-Alkyl)S(=O)(C₁-C₈-alkyl), C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)OC₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind;
(D) R¹ aus H, CN, OH, SH, NO₂, C(=O)H, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl) S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl)phenyl und (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt ist,
wobei jedes Alkyl, Halogenalkyl, Cycloalkyl, Halogencycloalkyl, Alkoxy, Halogenalkoxy, Alkenyl, Cycloalkenyl, Halogenalkenyl, Alkinyl, Phenyl, Phenoxy und (Het-1) gegebenenfalls durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl)phenyl und (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind;
(E) R² aus (J), H, C₁-C₈-Alkyl, C₃-C₈-Cycloalkyl, C₂-C₈-Alkenyl, C₂-C₈-Alkinyl, C (=0) (C₁-C₈-Alkyl), (C₁-C₈-Alkyl)O(C₁-C₈-alkyl), (C₁-C₈-Alkyl)S(C₁-C₈-alkyl), (C₁-C₈-Alkyl) S(=O)(C₁-C₈-alkyl), C₁-C₈-Alkyl)S(=O)₂(C₁-C₈-alkyl), (C₁-C₈-Alkyl)phenyl, (C₁-C₈-Alkyl)-O-phenyl, C(=O)(Het-1), (Het-1), (C₁-C₈-Alkyl)-(Het-1), (C₁-C₈-Alkyl)-C(=O)-(C₁-C₈-alkyl), (C₁-C₈-Alkyl)-OC(=O)-(C₁-C₈-alkyl), (C₁-C₈-Alkyl)-O-C(=O)O-(C₁-C₈-alkyl), (C₁-C₈-Alkyl)-O-C(=O)NR^{x}R^{y}, (C₁-C₈-Alkyl)-C(=O)N(R^{x})(C₁-C₈-alkyl)-(Het-1), (C₁-C₈-Alkyl)-C(=O)(Het-1), (C₁-C₈-Alkyl)-C(=O)N(R^{x})(C₁-C₈-alkyl)N(R^{y})C(=O)OH, (C₁-C₈-Alkyl)-C(=O)N(R^{x})(C₁-C₈-alkyl)N(R^{x})(R^{y}), (C₁-C₈-Alkyl)-C(=O)N(R^{x})(C₁-C₈-alkyl)N(R^{y})C(=O)O-(C₁-C₈-alkyl), (C₁-C₈-Alkyl)-C(=O)N(R^{x})(C₁-C₈-alkyl)(N(R^{y})C(=O)O-(C₁-C₈-alkyl)C(=O)OH, (C₁-C₈-Alkyl)-C(=O)(Het-1)C(=O)O-(C₁-C₈-alkyl), (C₁-C₈-Alkyl)-OC(=O)O-(C₁-C₈-alkyl), (C₁-C₈-Alkyl)-OC(=O)-(C₁-C₈-alkyl), (C₁-C₈-Alkyl)-OC(=O)-(C₃-C₈-cycloalkyl), (C₁-C₈-Alkyl)-OC(=O)-(Het-1), (C₁-C₈-Alkyl)-OC(=O)-(C₁-C₈-alkyl)N(R^{x})C(=O)O-(C₁-C₈-alkyl), (C₁-C₈-Alkyl)-NR^{x}R^{y}, (C₁-C₈-Alkyl)-S-(Het-1), (C₁-C₈-Alkyl)S(Het-1), (C₁-C₈-Alkyl)S(=O)(Het-1), (C₁-C₈ -Alkyl)S(=O)₂(Het-1) oder (C₁-C₈-Alkyl)-O-(Het-1) ausgewählt ist,
wobei jedes Alkyl, Cycloalkyl, Phenyl und (Het-1) gegebenenfalls durch einen oder mehrere Substituenten substituiert ist, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkyl, C₂-C₈-Alkinyl, S(C₁-C₈-Alkyl, S(C₃-C₈-Cycloalkyl), S(C₁-C₈-Halogenalkyl, S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)OH, C(=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, Phenoxy, Si(C₁-C₈-Alkyl)₃, SNR^{x}R^{y}, S(=O)NR^{x}R^{y}, S(=O)₂NR^{x}R^{y} oder (Het-1) ausgewählt sind;
(F) R³ die folgende Struktur aufweist: 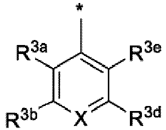 wobei
X für N oder CR^{3c} steht;
R^{3a}, R^{3b}, R^{3c}, R^{3d} und R^{3e} jeweils unabhängig für H, F, Cl, Br, I, CN, OH, SH, NO₂, C₁-C₈-Alkyl, C₁-C₈-Halogenalkyl, C₁-C₈-Alkoxy, C₁-C₈-Halogenalkoxy, C(=O)(C₁-C₈-Alkyl), C(=O)O(C₁-C₈-Alkyl), C(=O)(C₁-C₈-Halogenalkyl), C(=O)O(C₁-C₈-Halogenalkyl), (C₁-C₈-Alkyl)O(C₁-C₈-alkyl), (C₁-C₈-Alkyl)S(C₁-C₈-alkyl), (C₁-C₈-Halogenalkyl)O(C₁-C₈-halogenalkyl), (C₁-C₈-Halogenalkyl)S(C₁-C₈-halogenalkyl), (C₁-C₈-Alkyl)O(C₁-C₈-halogenalkyl), (C₁-C₈-Halogenalkyl)O(C₁-C₈-alkyl), (C₁-C₈-Alkyl)S(C₁-C₈-halogenalkyl), (C₁-C₈-Halogenalkyl)S(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl oder Phenoxy stehen;
zwei von R^{3c}, R^{3d} und R^{3e} einen 5- oder 6-gliedrigen, gesättigten oder ungesättigten Ring, der null, ein oder zwei Heteroatome, die aus Stickstoff, Schwefel und Sauerstoff ausgewählt sind, aufweist und gegebenenfalls durch mindestens ein R¹⁴ substituiert ist; und
R¹⁴ jeweils unabhängig für H, F, Cl, Br, I, CN, OH, SH, NO₂, C₁-C₈-Alkyl, C₁-C₈-Halogenalkyl, C₁-C₈-Alkoxy, C₁-C₈-Halogenalkoxy, C(=O)(C₁-C₈ alkyl), C(=O)O(C₁-C₈-Alkyl), C(=O)(C₁-C₈-Halogenalkyl), C(=O)O(C₁-C₈-Halogenalkyl), (C₁-C₈-Alkyl)O(C₁-C₈-alkyl), (C₁-C₈-Alkyl)S(C₁-C₈-alkyl), (C₁-C₈-Halogenalkyl)O(C₁-C₈-halogenalkyl), (C₁-C₈-Halogenalkyl)S(C₁-C₈-halogenalkyl), (C₁-C₈-Alkyl)O(C₁-C₈-halogenalkyl), (C₁-C₈-Halogenalkyl)O(C₁-C₈-alkyl), (C₁-C₈-Alkyl)S(C₁-C₈-halogenalkyl), (C₁-C₈-Halogenalkyl)S(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl oder Phenoxy steht;
(G) R⁴ aus (J), H, OH, SH, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogenalkyl, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S (C₁-C₈-Alkyl), S(C₃-C₈-Cycloalkyl), S(C₁-C₈-Halogenalkyl) , S(C₃-C₈-Halogencycloalkyl), S (=0) (C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), C(=O)(C₁-C₈-Alkyl)C(=O)O(C₁-C₈-alkyl), (C₁-C₈-Alkyl)C(=O)O(C₁-C₈-alkyl), Phenyl, (C₁-C₈-Alkyl)phenyl, (C₁-C₈-Alkyl)-O-phenyl, C(=O)(Het-1), (Het-1), (C₁-C₈-Alkyl)-(Het-1), (C₁-C₈-Alkyl)-C(=O)-(C₁-C₈-alkyl), (C₁-C₈-Alkyl)-OC(=O)-(C₁-C₈-alkyl), (C₁-C₈-Alkyl)-O-C(=O)O-(C₁-C₈-alkyl), (C₁-C₈-Alkyl)-O-C(=O)NR^{x}R^{y}, (C₁-C₈-Alkyl)-C(=O)N(R^{x})(C₁-C₈-alkyl)-(Het-1), (C₁-C₈-Alkyl)-C(=O)(Het-1), (C₁-C₈-Alkyl)-C(=O)N(R^{x})(C₁-C₈-alkyl)N(R^{y})C(=O)OH, (C₁-C₈-Alkyl)-C(=O)N(R^{x})(C₁-C₈-alkyl)N(R^{x})(R^{y}), (C₁-C₈-Alkyl)-C(=O)N(R^{x})(C₁-C₈-alkyl)N(R^{y})C(=O)O-(C₁-C₈-alkyl), (C₁-C₈-Alkyl)-C(=O)N(R^{x})(C₁-C₈-alkyl)(N(R^{y})C(=O)O-(C₁-C₈-alkyl)C(=O)OH, (C₁-C₈-Alkyl)-C(=O)(Het-1)C(=O)O-(C₁-C₈-alkyl), (C₁-C₈-Alkyl)-OC(=O)-(C₃-C₈-cycloalkyl), (C₁-C₈-Alkyl)-OC(=O)-(Het-1), (C₁-C₈-Alkyl)-OC(=O)-(C₁-C₈-alkyl)N(R^{x})C(=O)O-(C₁-C₈-alkyl), (C₁-C₈-Alkyl)-NR^{x}R^{y}, (C₁-C₈-Alkyl)-S-(Het-1), (C₁-C₈-Alkyl)S(=O)(Het-1), (C₁-C₈-Alkyl)S(=O)₂(Het-1) oder (C₁-C₈-Alkyl)-O- (Het-1) ausgewählt ist,
wobei jedes Alkyl, Halogenalkyl, Cycloalkyl, Halogencycloalkyl, Cycloalkoxy, Halogencycloalkoxy, Alkoxy, Halogenalkoxy, Alkenyl, Cycloalkenyl, Halogenalkenyl, Alkinyl, Phenyl und (Het-1) gegebenenfalls durch einen oder mehrere Substituenten substituiert ist, die aus H, F, Cl, Br, I, CN, OH, SH, NO₂, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-Cₑ-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)OH, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl) C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl) phenyl, (C₁-C₈-Alkyl)-O-phenyl, Phenyl, Halogenphenyl, Phenoxy und (Het-1) ausgewählt sind;
(H) Q¹ und Q² jeweils unabhängig aus O oder S ausgewählt sind;
(I) R^{x} und R^{y} unabhängig aus H, OH, SH, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S (C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl) phenyl, (C₁-C₈-Alkyl)-O-phenyl, Phenyl, (C₁-C₈-Alkyl) phenyl, (C₁-C₈-Alkyl)-O-phenyl, C(=O)(Het-1), (Het-1), (C₁-C₈-Alkyl)-(Het-1), (C₁-C₈-Alkyl)-C(=O)-(C₁-C₈-alkyl), (C₁-C₈-Alkyl)-C(=O)(Het-1), (C₁-C₈-Alkyl)-C(=O)(Het-1)C(=O)O-(C₁-C₈-alkyl), (C₁-C₈-Alkyl)-OC(=O)O-(C₁-C₈-alkyl), (C₁-C₈-Alkyl)-OC(=O)-(C₁-C₈-alkyl), (C₁-C₈-Alkyl)-OC(=O)-(C₃-C₈-cycloalkyl), (C₁-C₈-Alkyl)-OC(=O)-(Het-1), (C₁-C₈-Alkyl)-S-(Het-1), (C₁-C₈-Alkyl)S(=O)(Het-1), (C₁-C₈-Alkyl)S(=O)₂(Het-1) oder (C₁-C₈-Alkyl)-O- (Het-1) ausgewählt sind,
wobei jedes Alkyl, Halogenalkyl, Cycloalkyl, Halogencycloalkyl, Cycloalkoxy, Halogencycloalkoxy, Alkoxy, Halogenalkoxy, Alkenyl, Cycloalkenyl, Halogenalkenyl, Alkinyl, Phenyl und (Het-1) gegebenenfalls durch einen oder mehrere Substituenten substituiert sind, die unabhängig aus H, F, Cl, Br, I, CN, OH, SH, NO₂, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S (=0) (C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)OH, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl) C(=O)O(C₁-C₈-alkyl), (C₁-C₈-Alkyl)C(=O)O(C₁-C₈-alkyl), (C₁-C₈-Alkyl)C(=O)(C₁-C₈-alkyl), (C₁-C₈-Alkyl) phenyl, (C₁-C₈-Alkyl)-O-phenyl, Phenyl, Halogenphenyl, Phenoxy und (Het-1) ausgewählt sind,
oder R^{x} und R^{y} zusammen gegebenenfalls eine 5- bis 7-gliedrige gesättigte oder ungesättigte cyclische Gruppe bilden, die ein oder mehrere Heteroatome, die aus Stickstoff, Schwefel und Sauerstoff ausgewählt sind, enthalten kann, wobei die cyclische Gruppe durch H, F, Cl, Br, I, CN, OH, SH, NO₂, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl) OC(=O)O(C₁-C₈-alkyl), C(=O)(C₁-C₈-Alkyl)C(=O)O(C₁-C₈-alkyl), (C₁-C₈-Alkyl)C(=O)O(C₁-C₈-alkyl), (C₁-C₈-Alkyl) C (=0) (C₁-C₈-alkyl), (C₁-C₈-Alkyl)phenyl, (C₁-C₈-Alkyl)-O-phenyl, Phenyl, substituiertes Phenyl, Phenoxy und (Het-1) substituiert sein kann;
(J) R² und R⁴ für eine 1- bis 4-gliedrige gesättigte oder ungesättigte Hydrocarbylverknüpfung stehen können, die ein oder mehrere Heteroatome, die aus Stickstoff, Schwefel und Sauerstoff ausgewählt sind, enthalten kann und zusammen mit C^{X}(Q¹) (N^{X}) eine 4- bis 7-gliedrige cyclische Struktur bildet, wobei die Hydrocarbylverknüpfung gegebenenfalls durch einen oder mehrere Substituenten substituiert ist, die unabhängig aus R⁵, R⁶ und R⁷ ausgewählt sind, wobei R⁵, R⁶ und R⁷ jeweils aus H, F, Cl, Br, I, CN, OH, SH, NO₂, NR^{x}R^{y}, C₁-C₈-Alkyl, C₁-C₈-Alkyl, das durch mindestens ein OH substituiert ist, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), S(=O)₂(C₁-C₈-Alkyl), S(=O)₂(C₁-C₈-Halogenalkyl), OS(=O)₂(C₁-C₈-Alkyl), OS(=O)₂(C₁-C₈-Halogenalkyl), C(=O)H, C(=O)(C₁-C₈-Alkyl), C(=O)O(C₁-C₈-Alkyl), C(=O)(C₁-C₈-Halogenalkyl), C(=O)O(C₁-C₈-Halogenalkyl), C(=O)(C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O (C₁-C₈-alkyl), (C₁-C₈-Alkyl)S(C₁-C₈-alkyl), (C₁-C₈-Alkyl)S(=O)(C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl) C(=O)O(C₁-C₈-alkyl), (C₁-C₈-Alkyl)C (=O)O(C₁-C₈-alkyl), (C₁-C₈-Alkyl)C(=O)(C₁-C₈-alkyl), (C₁-C₈-Alkyl)phenyl, (C₁-C₈-Alkyl)-O-phenyl, Phenyl, substituiertem Phenyl, Phenoxy oder (Het-1) ausgewählt ist;
(K) (Het-1) für einen 5- oder 6-gliedrigen gesättigten oder ungesättigten, heterocyclischen Ring mit einem oder mehreren Heteroatomen, die unabhängig aus Stickstoff, Schwefel oder Sauerstoff ausgewählt sind, steht, wobei der heterocyclische Ring auch durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S (C₃-C₈-Halogencycloalkyl), S (=O) (C₁-C₈-Alkyl), S (=O) (C₁-C₈-Halogenalkyl), S(=O)₂(C₁-C₈-Alkyl) , S(=O)₂(C₁-C₈-Halogenalkyl), OS(=O)₂(C₁-C₈-Alkyl), OS(=O)₂(C₁-C₈-Halogenalkyl), C (=O)NR^{x}R^{y}, (C₁-C₈-Alkyl)NR^{x}R^{y}, C(=O)(C₁-C₈-Alkyl), C(=O)O(C₁-C₈-Alkyl), C(=O)(C₁-C₈-Halogenalkyl), C (=O) O (C₁-C₈-Halogenalkyl), C(=O)(C₃-C₈-Cycloalkyl), C (=O) O (C₃-C₈-Cycloalkyl), C(=O)(C₂-C₈-Alkenyl), C(=O)O(C₂-C₈-Alkenyl), (C₁-C₈-Alkyl)O(C₁-C₈-alkyl), (C₁-C₈-Alkyl)S(C₁-C₈-alkyl), (C₁-C₈-Alkyl) S (=O) (C₁-C₈-alkyl), (C₁-C₈-Alkyl)S(=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl)C(=O)O(C₁-C₈-alkyl), (C₁-C₈-Alkyl)C (=O) O (C₁-C₈-alkyl), (C₁-C₈-Alkyl) C (=O) (C₁-C₈-alkyl), (C₁-C₈-Alkyl)phenyl, (C₁-C₈-Alkyl)-O-phenyl, Phenyl und Phenoxy ausgewählt sind,
wobei jedes Alkyl, Cycloalkyl, Alkoxy, Alkenyl, Alkinyl, Phenyl und Phenoxy gegebenenfalls durch einen oder mehrere Substituenten substituiert sein kann, die unabhängig aus H, F, Cl, Br, I, CN, OH, SH, NO₂, NR^{x}R^{y}, C₁-C₈-Alkyl, C₁-C₈-Halogenalkyl, C₃-C₈-Cycloalkyl, C₃-C₈-Halogencycloalkyl, C₃-C₈-Cycloalkoxy, C₃-C₈-Halogencycloalkoxy, C₁-C₈-Alkoxy, C₁-C₈-Halogenalkoxy, C₂-C₈-Alkenyl, C₃-C₈-Cycloalkenyl, C₂-C₈-Halogenalkenyl, C₂-C₈-Alkinyl, S(C₁-C₈-Alkyl), S(C₃-C₈-Cycloalkyl), S(C₁-C₈-Halogenalkyl), S(C₃-C₈-Halogencycloalkyl), S(=O)(C₁-C₈-Alkyl), S(=O)(C₁-C₈-Halogenalkyl), OS(=O)₂(C₁-C₈-Alkyl), S(=O)₂(C₁-C₈-Alkyl), S(=O)₂(C₁-C₈-Halogenalkyl), OS(=O)₂(C₁-C₈-Alkyl), OS (=O)₂(C₁-C₈-Halogenalkyl), C(=O)H, C(=O)NR^{x}R^{y}, (C₁-C₈-Alkyl) NR^{x}R^{y}, C(=O)(C₁-C₈-Alkyl), C(=O)O(C₁-C₈-Alkyl), C (=O) (C₁-C₈-Halogenalkyl), C(=O)O(C₁-C₈-Halogenalkyl), C (=O) (C₃-C₈-Cycloalkyl), C(=O)O(C₃-C₈-Cycloalkyl), C(=O)(C₂-C₈-Alkenyl), C(=O)O(C₂-C₈-Alkenyl), (C₁-C₈-Alkyl)O (C₁-C₈-alkyl), (C₁-C₈-Alkyl)S(C₁-C₈-alkyl), (C₁-C₈-Alkyl)S (=O) (C₁-C₈-alkyl), (C₁-C₈-Alkyl)S (=O)₂(C₁-C₈-alkyl), (C₁-C₈-Alkyl)OC(=O)(C₁-C₈-alkyl), (C₁-C₈-Alkyl)OC(=O)O(C₁-C₈-alkyl), C(=O)(C₁-C₈-Alkyl) C (=O)O (C₁-C₈-alkyl), (C₁-C₈-Alkyl)C(=O)O(C₁-C₈-alkyl), (C₁-C₈-Alkyl)C (=O) (C₁-C₈-alkyl), (C₁-C₈-Alkyl)phenyl, (C₁-C₈-Alkyl)-O-phenyl, Phenyl und Phenoxy ausgewählt sind; und
(L) L für eine Bindung steht, die Ar² mit N^{Y} verbindet.

2. Verbindung nach Anspruch 1, wobei R³ eine Struktur der Formel R3A oder der Formel R3B aufweist: 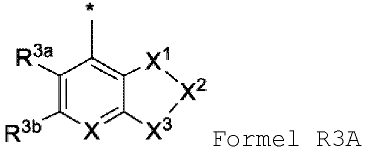 oder 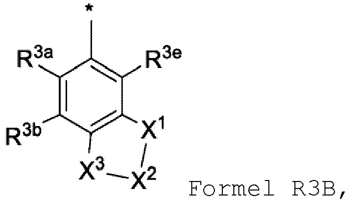 wobei
X für N oder CR^{3c} steht;
X¹ für O, CH, CH₂, N, N(R¹⁴), S, C(O) oder S steht;
X² für O, CH, CH₂, N, N(R¹⁴), S, C(O) oder S steht;
X³ für O, CH, CH₂, N, N(R¹⁴), S, C(O), S, O(CH₂), (CH₂)O, -N=CH-, -CH=N-, C(O)O, OC(O), -N(R¹⁴)-CH2-, -CH2-N(R¹⁴)-, -N(R¹⁴)-CH-, -CH-N(R¹⁴)-, -CH=CH- oder -CH₂-CH₂- steht;
R^{3a}, R^{3b}, R^{3c} und R¹⁴ unabhängig für H, F, Cl, Br, I, CN, OH, SH, NO₂, C₁-C₈-Alkyl, C₁-C₈-Halogenalkyl, C₁-C₈-Alkoxy, C₁-C₈-Halogenalkoxy, C(=O)(C₁-C₈-Alkyl), C(=O)O(C₁-C₈-Alkyl), C(=O)(C₁-C₈-Halogenalkyl), C(=O)O(C₁-C₈-Halogenalkyl), (C₁-C₈-Alkyl)O (C₁-C₈-alkyl), (C₁-C₈-Alkyl) S(C₁-C₈-alkyl), (C₁-C₈-Halogenalkyl)O(C₁-C₈-halogenalkyl), (C₁-C₈-Halogenalkyl)S(C₁-C₈-halogenalkyl), (C₁-C₈-Alkyl)O(C₁-C₈-halogenalkyl), (C₁-C₈-Halogenalkyl) O(C₁-C₈-alkyl), (C₁-C₈-Alkyl)S(C₁-C₈-halogenalkyl), (C₁-C₈-Halogenalkyl)S(C₁-C₈-alkyl), (C₁-C₈-Alkyl)C(=O)(C₁-C₈ alkyl), (C₁-C₈-Alkyl)phenyl, (C₁-C₈-Alkyl)-O-phenyl, Phenyl oder Phenoxy stehen.

3. Verbindung nach Anspruch 1, wobei:
(A) Ar¹ für ein substituiertes Phenyl, ein substituiertes Pyridyl, ein substituiertes Pyridazinyl, ein substituiertes Pyridinonyl oder ein substituiertes Pyrimidinyl steht, wobei das substituierte Phenyl, substituierte Pyridyl, substituierte Pyridazinyl, substituierte Pyridinonyl und substituierte Pyrimidinyl einen oder mehrere Substituenten aufweisen, die unabhängig aus H, CN, SF₅, C₁-C₆-Alkyl, C₁-C₆-Alkoxy, S(C₁-C₆-Halogenalkyl), C₁-C₆-Halogenalkyl und C₁-C₆-Halogenalkoxy ausgewählt sind;
(B) Het für Triazolyl oder Pyrazolyl steht;
(C) Ar² für Phenyl, Pyridyl, Pyrimidinyl, substituiertes Phenyl, substituiertes Pyridyl oder substituiertes Pyrimidinyl steht, wobei das substituierte Phenyl, substituierte Pyridyl und substituierte Pyrimidinyl einen oder mehrere Substituenten aufweisen, die unabhängig aus H, F, Cl, Br, I, CN, NO₂, NR^{x}R^{y}, OH, C₁-C₆-Alkoxy, (C₁-C₆-Alkyl)O(C₁-C₆-alkyl), C₁-C₆-Halogenalkyl und C₁-C₆-Alkyl ausgewählt sind;
(D) R¹ für H, C₁-C₆-Alkyl, C₃-C₆-Cycloalkyl oder C₂-C₆-Alkenyl steht, wobei das Alkyl, Cycloalkyl oder Alkenyl gegebenenfalls durch ein C₁-C₆-Alkoxy, C₁-C₆-Halogenalkoxy, C₃-C₆-Cycloalkyl oder C₃-C₆-Halogencycloalkyl substituiert ist;
(E) R² aus (J), H, C₁-C₆-Alkyl oder C₁-C₆-Halogenalkyl ausgewählt ist;
(F) R³ aus einer der folgenden Gruppen R³ ausgewählt ist: 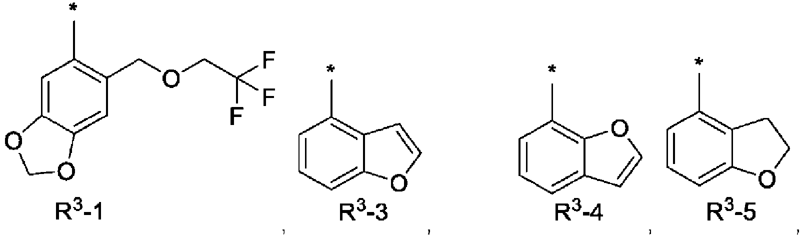 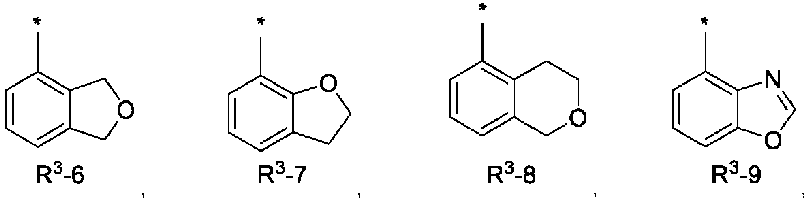 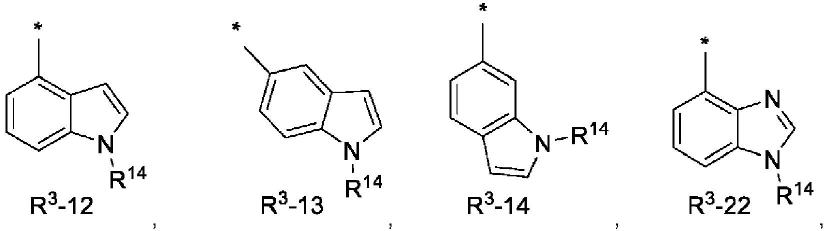 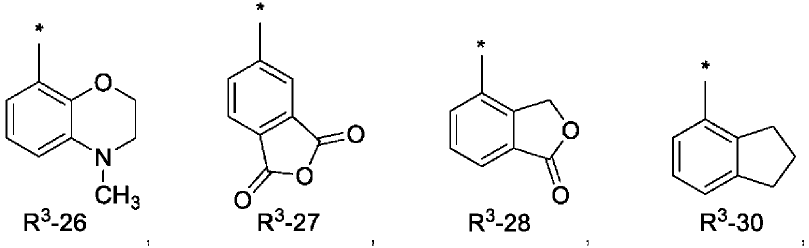 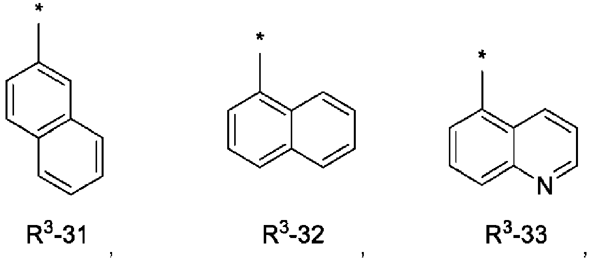 wobei R¹⁴, falls auf die Gruppe R³ anwendbar, aus der Gruppe bestehend aus H und C₁-C₆-Alkyl ausgewählt ist;
(G) R⁴ aus (J), H, C₁-C₆-Alkyl oder C₁-C₆-Halogenalkyl ausgewählt ist;
(H) Q¹ für S steht und Q² für O steht;
(I) R^{x} und R^{y} unabhängig aus H, C(=O)(C₁-C₆-Alkyl), C(=O)O(C₁-C₆-Alkyl), C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Cycloalkenyl, C₂-C₆-Halogenalkenyl, C₂-C₆-Alkinyl und Phenyl ausgewählt sind; und
(J) R² und R⁴ für eine 1- bis 4-gliedrige gesättigte oder ungesättigte Hydrocarbylverknüpfung stehen können, die ein oder mehrere Heteroatome, die aus Stickstoff, Schwefel und Sauerstoff ausgewählt sind, enthalten kann und zusammen mit C^{X}(Q¹)(N^{X}) eine cyclische Struktur bildet, wobei die Hydrocarbylverknüpfung gegebenenfalls durch einen oder mehrere Substituenten substituiert ist, die unabhängig aus R⁵, R⁶ und R⁷ ausgewählt sind, wobei R⁵, R⁶ und R⁷ jeweils aus H, F, Cl, Br, I, CN, NO₂, NR^{x}R^{y}, OH, SH, S (C₁-C₆-Alkyl), S(C₁-C₆-Halogenalkyl), C₁-C₆-Alkyl, C₁-C₆-Alkyl, das durch OH substituiert ist, C₁-C₆-Halogenalkyl, C₁-C₆-Alkoxy, C₁-C₆-Halogenalkoxy, C₃-C₆-Cycloalkyl, C₃-C₆-Halogencycloalkyl, Phenyl und Oxo ausgewählt sind.

4. Verbindung nach Anspruch 1 mit einer Struktur der Formel Zwei:

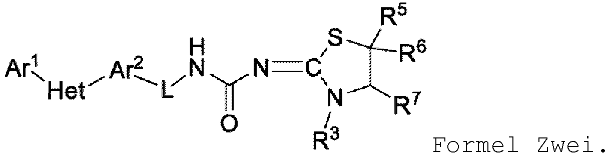

5. Verbindung nach Anspruch 1 mit einer Struktur, die aus den in Tabelle 1 aufgeführten Verbindungen ausgewählt ist.

6. Verbindung nach Anspruch 1, wobei
(A) Ar¹ für ein substituiertes Phenyl, ein substituiertes Pyridyl oder ein substituiertes Pyrimidinyl steht, wobei das substituierte Phenyl, substituierte Pyridyl und substituierte Pyrimidinyl einen oder mehrere Substituenten aufweisen, die unabhängig aus H, CN, SF₅, C₁-C₆-Alkyl, C₁-C₆-Alkoxy, C₁-C₆-Halogenalkyl und C₁-C₆-Halogenalkoxy ausgewählt sind;
(B) Het für Triazolyl oder Pyrazolyl steht;
(C) Ar² für Phenyl, Pyridyl, Pyrimidinyl, substituiertes Phenyl, substituiertes Pyridyl oder substituiertes Pyrimidinyl steht, wobei das substituierte Phenyl, substituierte Pyridyl und substituierte Pyrimidinyl einen oder mehrere Substituenten aufweisen, die unabhängig aus H, F, Cl, Br, I, CN, NO₂, NR^{x}R^{y}, OH, C₁-C₆-Alkoxy, (C₁-C₆-Alkyl)O(C₁-C₆-alkyl), C₁-C₆-Halogenalkyl und C₁-C₆-Alkyl ausgewählt sind;
(D) R¹ für H, C₁-C₆-Alkyl, C₃-C₆-Cycloalkyl oder C₂-C₆-Alkenyl steht, wobei das Alkyl, Cycloalkyl oder Alkenyl gegebenenfalls durch ein C₁-C₆-Alkoxy, C₁-C₆-Halogenalkoxy, C₃-C₆-Cycloalkyl oder C₃-C₆-Halogencycloalkyl substituiert ist;
(E) R² aus (J), H, C₁-C₆-Alkyl oder C₁-C₆-Halogenalkyl ausgewählt ist;
(F) R³ aus einer der folgenden Gruppen R³ ausgewählt ist: 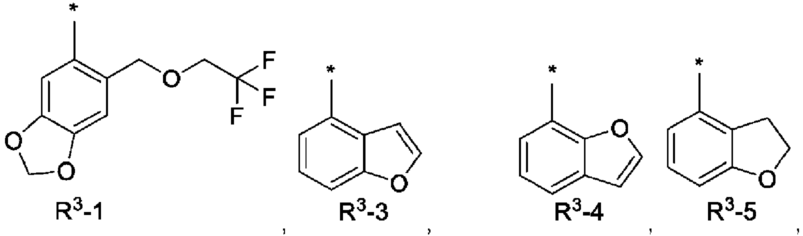 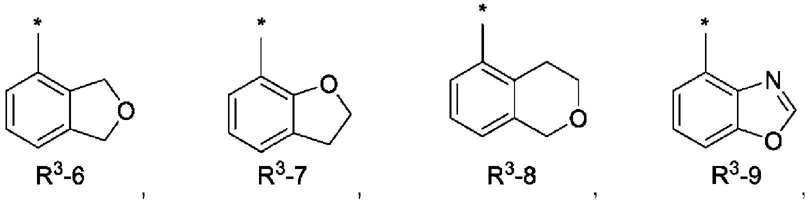 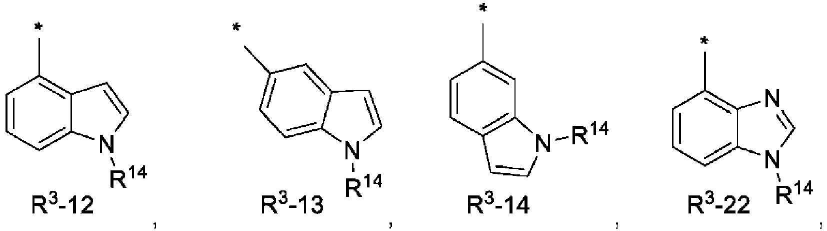 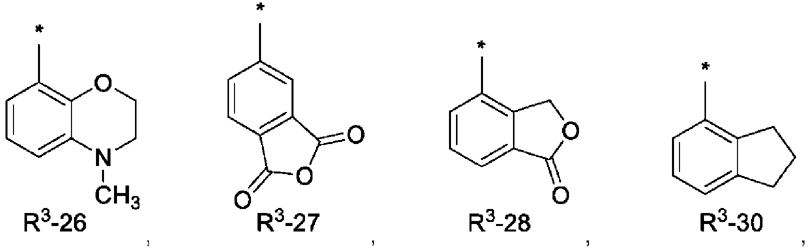 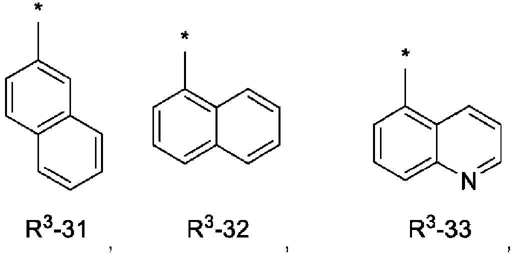 wobei R¹⁴, falls auf die Gruppe R³ anwendbar, aus der Gruppe bestehend aus H und CH₃ ausgewählt ist;
(G) R⁴ aus (J), H, C₁-C₆-Alkyl oder C₁-C₆-Halogenalkyl ausgewählt ist;
(H) Q¹ für S steht und Q² für O steht;
(I) R^{x} und R^{y} unabhängig aus H, C(=O)(C₁-C₆-Alkyl), C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Cycloalkenyl, C₂-C₆-Halogenalkenyl, C₂-C₆-Alkinyl und Phenyl ausgewählt sind; und
(J) R² und R⁴ für eine 1- bis 4-gliedrige gesättigte oder ungesättigte Hydrocarbylverknüpfung stehen können, die ein oder mehrere Heteroatome, die aus Stickstoff, Schwefel und Sauerstoff ausgewählt sind, enthalten kann und zusammen mit C^{X}(Q¹) (N^{X}) eine cyclische Struktur bildet, wobei die Hydrocarbylverknüpfung gegebenenfalls durch einen oder mehrere Substituenten substituiert ist, die unabhängig aus R⁵, R⁶ und R⁷ ausgewählt sind, wobei R⁵, R⁶ und R⁷ jeweils aus H, F, Cl, Br, I, CN, NO₂, NR^{x}R^{y}, OH, SH, S (C₁-C₆-Alkyl), S(C₁-C₆-Halogenalkyl), C₁-C₆-Alkyl, C₁-C₆-Halogenalkyl, C₁-C₆-Alkoxy, C₁-C₆-Halogenalkoxy, C₃-C₆-Cycloalkyl, C₃-C₆-Halogencycloalkyl, Phenyl und Oxo ausgewählt sind.

7. Verbindung nach Anspruch 1, wobei 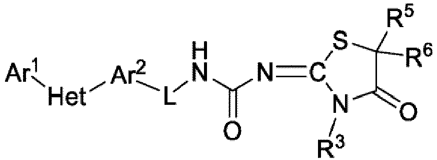
(A) Ar¹ für ein substituiertes Phenyl steht, wobei das substituierte Phenyl einen oder mehrere Substituenten aufweist, die unabhängig aus H, SF₅, C₁-C₈-Halogenalkyl und C₁-C₈-Halogenalkoxy ausgewählt sind;
(B) Het für ein 1,2,3-Triazolyl steht;
(C) Ar² für ein substituiertes Phenyl steht, wobei das substituierte Phenyl einen oder mehrere Substituenten aufweist, die unabhängig aus H, F, Cl und CH₃ ausgewählt sind;
(D) R⁵ und R⁶ für H stehen;
(E) R³ aus einer der folgenden Gruppen R³ ausgewählt ist: 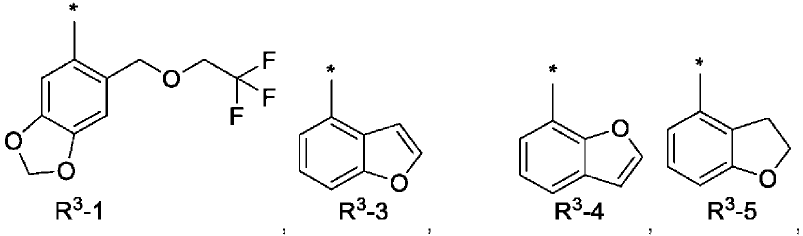 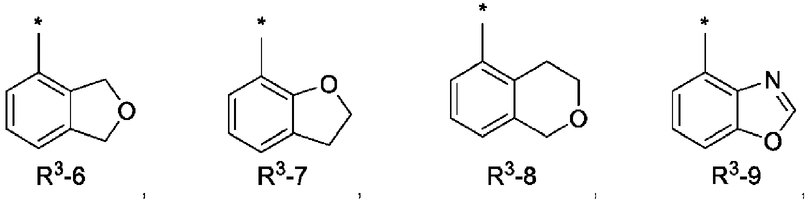 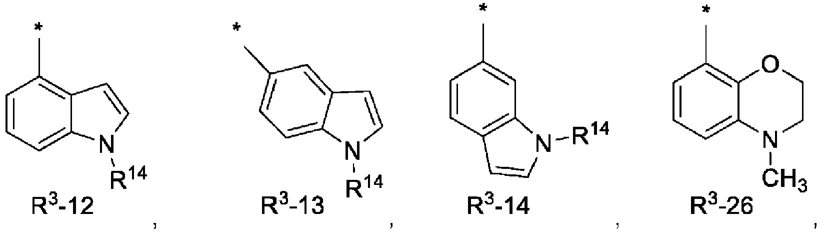 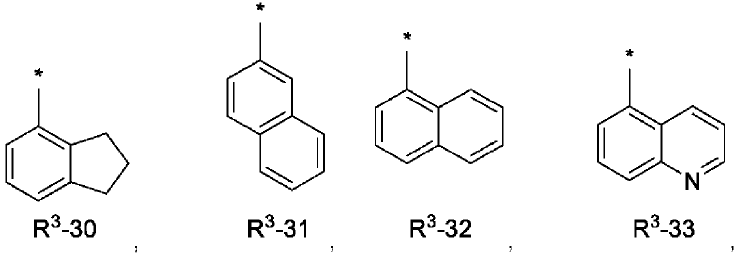 wobei R¹⁴, falls auf die Gruppe R³ anwendbar, aus der Gruppe bestehend aus H und CH₃ ausgewählt ist.

8. Verbindung nach Anspruch 1 mit einer der folgenden Strukturen:

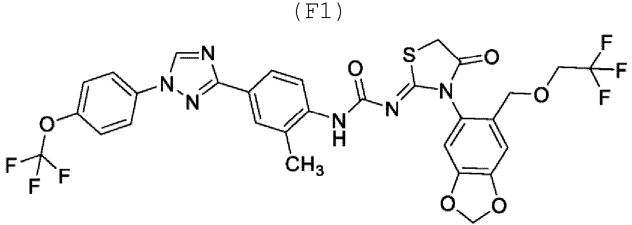

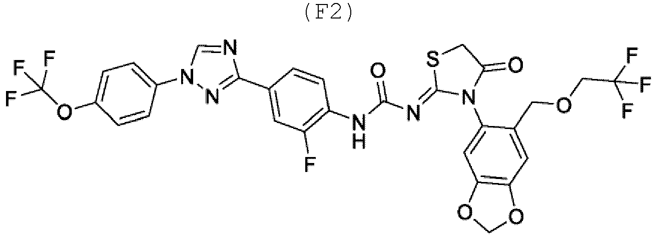

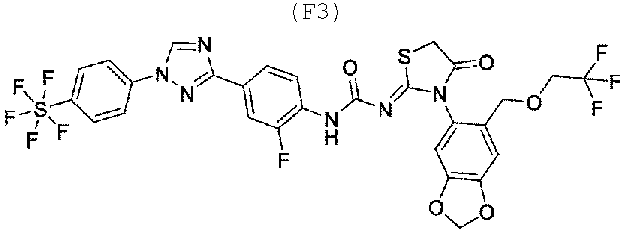

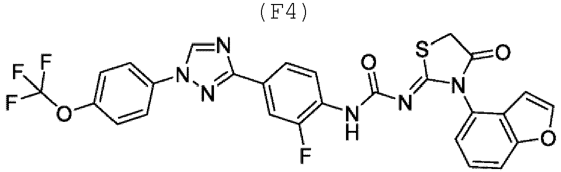

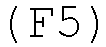

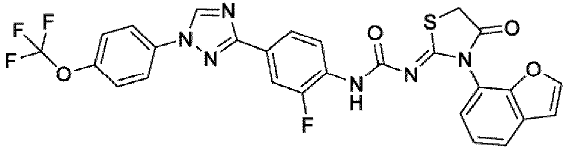

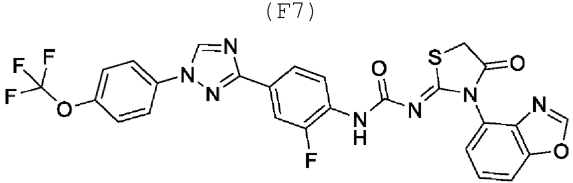

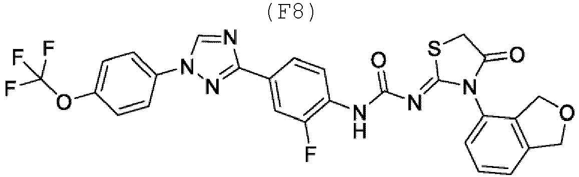

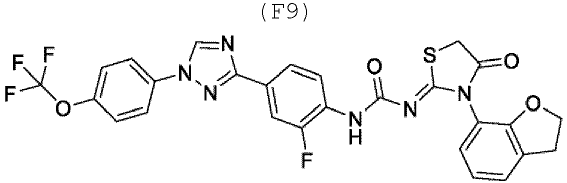

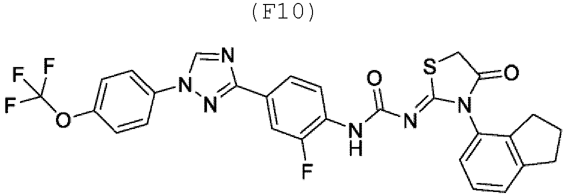

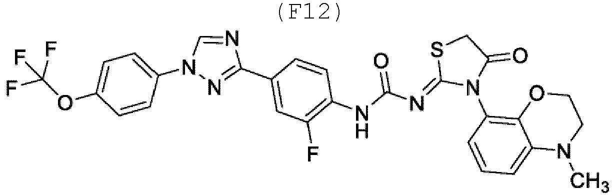

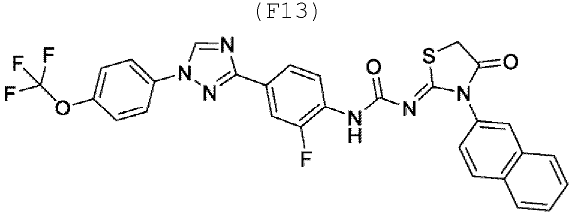

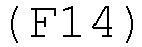

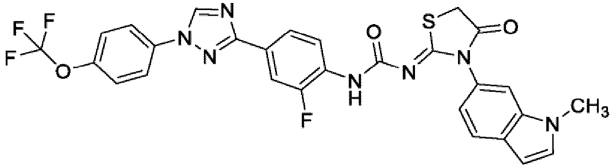

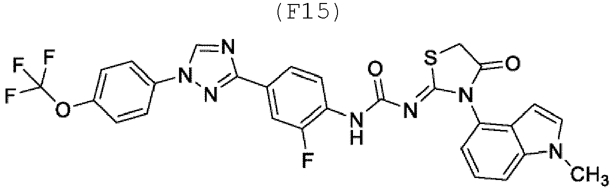

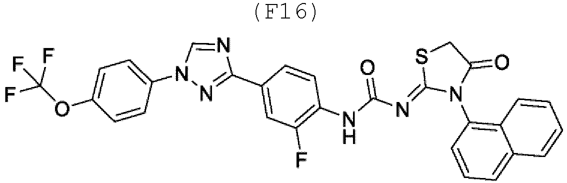

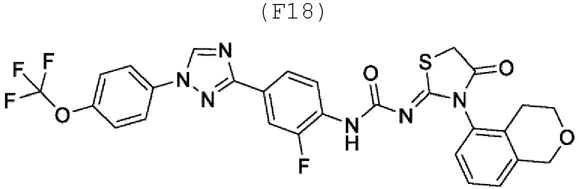

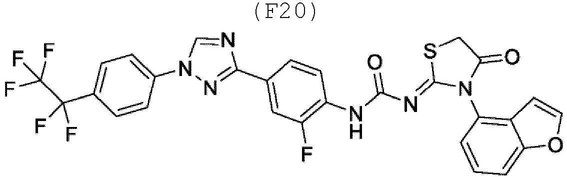

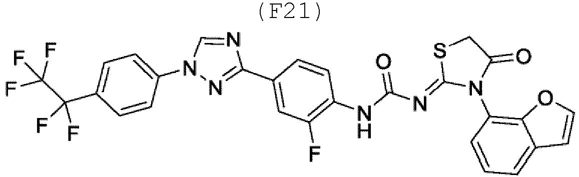

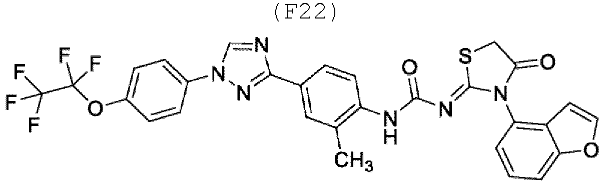

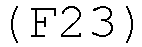

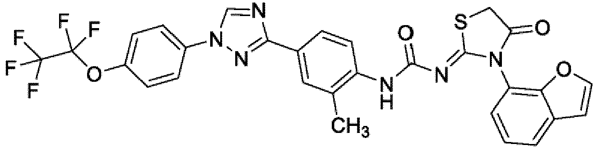

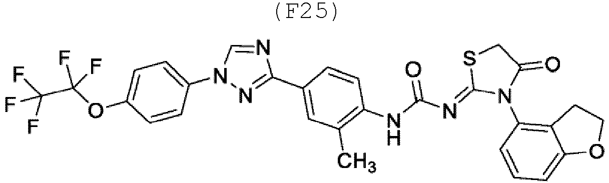

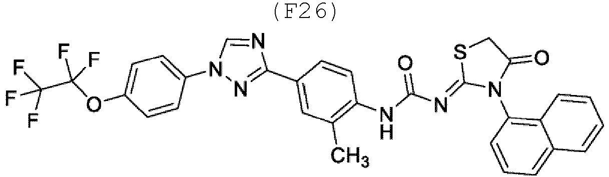

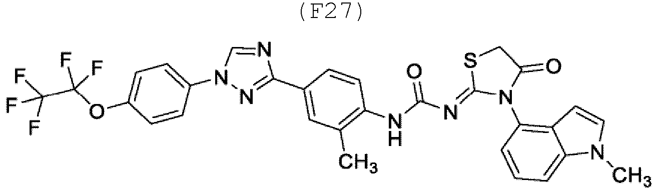

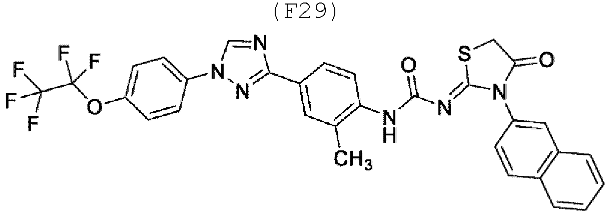

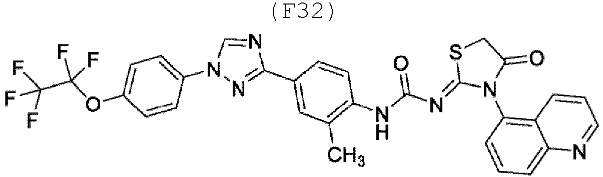

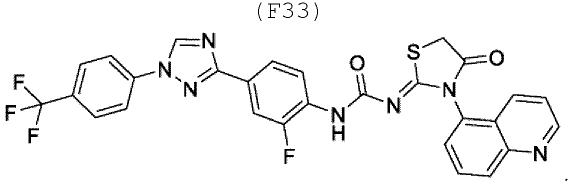

9. Verbindung nach Anspruch 1 zur Verwendung bei der Bekämpfung von Endoparasiten und Ektoparasiten auf dem veterinärmedizinischen Sektor oder bei der menschlichen Gesundheitsfürsorge.

10. Nichttherapeutisches Verfahren, umfassend die Anwendung der Verbindung nach Anspruch 1 an einem Ort zur Bekämpfung eines Schädlings in einer zur Bekämpfung eines solchen Schädlings ausreichenden Menge.

11. Nichttherapeutisches Verfahren nach Anspruch 10, wobei es sich bei dem Schädling um Zuckerrübeneule (Beet Armyworm, BAW), aschgraue Höckereule (Cabbage Looper, CL) oder Gelbfiebermücke (Yellow Fever Mosquito, YFM) handelt.

## Revendications

1. Composé ayant une structure de la formule suivante (« formule un ») 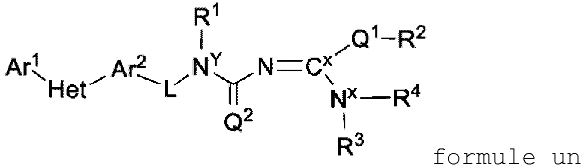
(A) Ar¹ étant choisi parmi
(1) furanyle, phényle, pyridazinyle, pyridinyle, pyridinonyle, pyrimidinyle, thiényle, ou
(2) furanyle substitué, phényle substitué, pyridazinyle substitué, pyridinyle substitué, pyridinonyle substitué, pyrimidinyle substitué, ou thiényle substitué,
lesdits furanyle substitué, phényle substitué, pyridazinyle substitué, pyridinyle substitué, pyridazinyle substitué, pyridinonyle substitué, pyrimidinyle substitué, et thiényle substitué ayant un ou plusieurs substituants indépendamment choisis parmi H, F, Cl, Br, I, CN, OH, SH, SF₅, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S (=O) (C₁-C₈ alkyle), S (=O) (C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)O(C₁-C₈ halogénoalkyle), (C₁-C₈ alkyl) S(C₁-C₈ alkyle), (C₁-C₈ alkyl) S (=O) (C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl)OC(=O)O(C₁-C₈ alkyle), C(=O) (C₁-C₈ alkyl)C(=O)O(C₁-C₈ alkyle), (C₁-C₈ alkyl) C(=O)O(C₁-C₈ alkyle), (C₁-C₈ alkyl) C (=O)(C₁-C₈ alkyle), (C₁-C₈ alkyl)phényle, (C₁-C₈ alkyl)-O-phényle, phényle, phénoxy, Si (C₁-C₈ alkyle)₃, SNR^{x}R^{y}, S (=O) NR^{x}R^{y}, S(=O)₂NR^{x}R^{y}, ou (Het-1),
chaque substituant alkyle, halogénoalkyle, cycloalkyle, halogénocycloalkyle, alcoxy, halogénoalcoxy, alcényle, cycloalcényle, halogénoalcényle, alcynyle, phényle, phénoxy, et (Het-1) pouvant éventuellement être substitués par un ou plusieurs substituants indépendamment choisis parmi H, F, Cl, Br, I, CN, OH, SH, SF₅, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S (C₁-C₈ alkyle), S(C₃-C₈ cycloalkyle), S(C₁-C₈ halogénoalkyle), S(C₃-C₈ halogénocycloalkyle), S (=O) (C₁-C₈ alkyle), S(=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O) (C₁-C₈ alkyle), C(=O)O(C₁-C₈ alkyle), C(=O)(C₁-C₈ halogénoalkyle), C(=O)O(C₁-C₈ halogénoalkyle), C(=O) (C₃-C₈ cycloalkyle), C(=O)O(C₃-C₈ cycloalkyle), C(=O)(C₂-C₈ alcényle), C (=O) O(C₂-C₈ alcényle), (C₁-C₈ alkyl) O(C₁-C₈ alkyle), (C₁-C₈ alkyl)O(C₁-C₈ halogénoalkyle), (C₁-C₈ alkyl)S(C₁-C₈ alkyle), (C₁-C₈ alkyl)S(=O) (C₁-C₈ alkyle), C₁-C₈ alkyl)S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O) (C₁-C₈ alkyl) C(=O)O(C₁-C₈ alkyle), (C₁-C₈ alkyl) C(=O)OC₁-C₈ alkyle), (C₁-C₈ alkyl) C (=O) (C₁-C₈ alkyle), (C₁-C₈ alkyl)phényle, (C₁-C₈ alkyl)-O-phényle, phényle, phénoxy, Si (C₁-C₈ alkyle)₃, SNR^{x}R^{y}, S (=O) NR^{x}R^{y}, S(=O)₂NR^{x}R^{y}, ou (Het-1) ;
chaque substituant alkyle, halogénoalkyle, cycloalkyle, halogénocycloalkyle, alcoxy, halogénoalcoxy, alcényle, cycloalcényle, halogénoalcényle, alcynyle, phényle, phénoxy, et (Het-1) pouvant éventuellement être substitués par un ou plusieurs substituants indépendamment choisis parmi H, F, Cl, Br, I, CN, OH, SH, SF₅, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S (=O) (C₁-C₈ alkyle), S(=O) (C₁-C₈ halogénoalkyle), S(=O)₂(C₁-C₈ alkyle), S(=O)₂(C₁-C₈ halogénoalkyle), OS(=O) ₂(C₁-C₈ alkyle), OS(=O)₂(C₁-C₈ halogénoalkyle), C (=O) NR^{x}R^{y}, (C₁-C₈ alkyl) NR^{x}R^{y}, C(=O) (C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)O(C₁-C₈ halogénoalkyle), (C₁-C₈ alkyl)S(C₁-C₈ alkyle), (C₁-C₈ alkyl)S(=O)(C₁-C₈ alkyle), C₁-C₈ alkyl)S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O) (C₁-C₈ alkyl) C(=O)O(C₁-C₈ alkyle), (C₁-C₈ alkyl) C(=O)OC₁-C₈ alkyle), (C₁-C₈ alkyl) C (=O) (C₁-C₈ alkyle), (C₁-C₈ alkyl)phényle, (C₁-C₈ alkyl)-O-phényle, phényle, phénoxy, Si(C₁-C₈ alkyle)₃, SNR^{x}R^{y}, S (=O) NR^{x}R^{y}, S(=O)₂NR^{x}R^{y}, ou (Het-1) ;
(B) Het étant un cycle hétérocyclique, saturé ou insaturé, à 5 ou 6 chaînons, contenant un ou plusieurs hétéroatomes indépendamment choisis parmi azote, soufre ou oxygène, ledit cycle hétérocyclique pouvant également être substitué par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S (C₃-C₈ halogénocycloalkyle), S (=O) (C₁-C₈ alkyle), S (=O) (C₁-C₈ halogénoalkyle), S(=O)₂(C₁-C₈ alkyle), S(=O)₂(C₁-C₈ halogénoalkyle), OS (=O) ₂(C₁-C₈ alkyle), OS(=O)₂(C₁-C₈ halogénoalkyle), C (=O) NR^{x}R^{y}, (C₁-C₈ alkyl) NR^{x}R^{y}, C(=O) (C₁-C₈ alkyle), C(=O)O(C₁-C₈ alkyle), C(=O) (C₁-C₈ halogénoalkyle), C(=O)O(C₁-C₈ halogénoalkyle), C(=O)(C₃-C₈ cycloalkyle), C(=O)O(C₃-C₈ cycloalkyle), C (=O) (C₂-C₈ alcényle), C(=O)O(C₂-C₈ alcényle), (C₁-C₈ alkyl)O(C₁-C₈ alkyle), (C₁-C₈ alkyl) S(C₁-C₈ alkyle), (C₁-C₈ alkyl) S(=O)(C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl)OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl)C(=O) C(=O)(C₁-C₈ alkyle), (C₁-C₈ alkyl)phényle, (C₁-C₈ alkyl)-O-phényle, phényle, phénoxy, Si (C₁-C₈ alkyle)₃, SNR^{x}R^{y}, S(=O)NR^{x}R^{y}, ou S(=O)₂NR^{x}R^{y},
chaque substituant alkyle, halogénoalkyle, cycloalkyle, halogénocycloalkyle, alcoxy, halogénoalcoxy, alcényle, cycloalcényle, halogénoalcényle, alcynyle, phényle, et phénoxy pouvant éventuellement être substitués par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S (C₃-C₈ halogénocycloalkyle), S (=O) (C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)C(=O)NR^{x}R^{y}, (C₁-C₈ alkyl)NR^{x}R^{y}, C (=O) C (=O) (C₁-C₈ alkyle), C(=O)O(C₁-C₈ alkyle), C (=O) C (=O) (C₁-C₈ halogénoalkyle), C(=O)O(C₁-C₈ halogénoalkyle), C(=O) (C₃-C₈ cycloalkyle), C(=O)O(C₃-C₈ cycloalkyle), C (=O) (C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S (=O) (C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O) (C₁-C₈ alkyl) C(=O)O(C₁-C₈ alkyle), (C₁-C₈ alkyl) C(=O)O(C₁-C₈ alkyle), (C₁-C₈ alkyl) C (=O)(C₁-C₈ alkyle), (C₁-C₈ alkyl)phényle, (C₁-C₈ alkyl)-O-phényle, phényle, phénoxy, Si (C₁-C₈ alkyle)₃, SNR^{x}R^{y}, S (=O) NR^{x}R^{y}, ou S(=O)₂NR^{x}R^{y} ;
(C) Ar² étant choisi parmi
(1) furanyle, phényle, pyridazinyle, pyridinyle, pyrimidinyle, thiényle, ou
(2) furanyle substitué, phényle substitué, pyridazinyle substitué, pyridinyle substitué, pyrimidinyle substitué, ou thiényle substitué,
lesdits furanyle substitué, phényle substitué, pyridazinyle substitué, pyridinyle substitué, pyrimidinyle substitué, et thiényle substitué, ayant un ou plusieurs substituants indépendamment choisis parmi H, F, Cl, Br, I, CN, OH, SH, SF₅, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)O(C₁-C₈ halogénoalkyle), (C₁-C₈ alkyl) S (C₁-C₈ alkyle), (C₁-C₈ alkyl) S (=O) (C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl)C(=O)O(C₁-C₈ alkyle), (C₁-C₈ alkyl) C(=O)O(C₁-C₈ alkyle), (C₁-C₈ alkyl) C (=O)(C₁-C₈ alkyle), (C₁-C₈ alkyl)phényle, (C₁-C₈ alkyl)-O-phényle, phényle, phénoxy, Si(C₁-C₈ alkyle)₃, SNR^{x}R^{y}, S (=O) NR^{x}R^{y}, S (=O)₂NR^{x}R^{y}, ou (Het-1),
chaque substituant alkyle, halogénoalkyle, cycloalkyle, halogénocycloalkyle, alcoxy, halogénoalcoxy, alcényle, cycloalcényle, halogénoalcényle, alcynyle, phényle, phénoxy, et (Het-1) pouvant éventuellement être substitués par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S (C₁-C₈ alkyle), S(C₃-C₈ cycloalkyle), S(C₁-C₈ halogénoalkyle), S(C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O) (C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)O(C₁-C₈ halogénoalkyle), (C₁-C₈ alkyl)S(C₁-C₈ alkyle), (C₁-C₈ alkyl)S(=O)(C₁-C₈ alkyle), (C₁-C₈ alkyl)S(=O)₂(C₁-C₈ alkyle), (C₁-C₈ alkyl)OC(=O) (C₁-C₈ alkyle), (C₁-C₈ alkyl)OC(=O)O(C₁-C₈ alkyle), C(=O) (C₁-C₈ alkyl)C(=O)O(C₁-C₈ alkyle), (C₁-C₈ alkyl) C(=O)O(C₁-C₈ alkyle), (C₁-C₈ alkyl) C (=O) (C₁-C₈ alkyle), (C₁-C₈ alkyl)phényle, (C₁-C₈ alkyl)-O-phényle, phényle, phénoxy, Si (C₁-C₈ alkyle)₃, SNR^{x}R^{y}, S (=O) NR^{x}R^{y}, S (=O) ₂NR^{x}R^{y}, ou (Het-1) ;
(D) R¹ étant choisi parmi H, CN, OH, SH, NO₂, C(=O)H, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S (C₁-C₈ alkyle), S (C₃-C₈ cycloalkyle), S(C₁-C₈ halogénoalkyle), S(C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl) S(C₁-C₈ alkyle), (C₁-C₈ alkyl) S (=O)(C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl) C(=O)O(C₁-C₈ alkyle), (C₁-C₈ alkyl) C (=O)(C₁-C₈ alkyle), (C₁-C₈ alkyl) phényle, et (C₁-C₈ alkyl) -O-phényle, phényle, phénoxy, Si(C₁-C₈ alkyle)₃, SNR^{x}R^{y}, S(=O)NR^{x}R^{y}, S(=O)₂NR^{x}R^{y}, ou (Het-1),
chaque alkyle, halogénoalkyle, cycloalkyle, halogénocycloalkyle, alcoxy, halogénoalcoxy, alcényle, cycloalcényle, halogénoalcényle, alcynyle, phényle, phénoxy, et (Het-1) pouvant éventuellement être substitués par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C (=O)(C₂-C₈ alcényle), C(=O)O(C₂-C₈ alcényle), (C₁-C₈ alkyl)O(C₁-C₈ alkyle), (C₁-C₈ alkyl) S(C₁-C₈ alkyle), (C₁-C₈ alkyl) S (=O)(C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl) C (=O) (C₁-C₈ alkyle), (C₁-C₈ alkyl) phényle, et (C₁-C₈ alkyl)-O-phényle, phényle, phénoxy, Si (C₁-C₈ alkyle)₃, SNR^{x}R^{y}, S(=O)NR^{x}R^{y}, S(=O)₂NR^{x}R^{y}, ou (Het-1) ;
(E) R² étant choisi parmi (J), H, C₁-C₈ alkyle, C₃-C₈ cycloalkyle, C₂-C₈ alcényle, C₂-C₈ alcynyle, C(=O)(C₁-C₈ alkyle), (C₁-C₈ alkyl)O(C₁-C₈ alkyle), (C₁-C₈ alkyl)S(C₁-C₈ alkyle), (C₁-C₈ alkyl)S(=O)(C₁-C₈ alkyle), C₁-C₈ alkyl)S(=O)₂(C₁-C₈ alkyle), (C₁-C₈ alkyl)phényle, (C₁-C₈ alkyl)-O-phényle, C(=O) (Het-1), (Het-1), (C₁-C₈ alkyl)-(Het-1), (C₁-C₈ alkyl)-C(=O)-(C₁-C₈ alkyle), (C₁-C₈ alkyl)-OC (=O) -(C₁-C₈ alkyle), (C₁-C₈ alkyl) -O-C(=O)O-(C₁-C₈ alkyle), (C₁-C₈ alkyl) -O-C (=O) NR^{x}R^{y}, (C₁-C₈ alkyl) - C(=O)N(R^{x})(C₁-C₈ alkyl)-(Het-1), (C₁-C₈ alkyl)-C(=O)(Het-1), (C₁-C₈ alkyl)-C(=O)N(R^{x})(C₁-C₈ alkyl)N(R^{y})C(=O)OH, (C₁-Ce alkyl)-C(=O)N(R^{x})(C₁-C₈ alkyl)N(R^{x})(R^{y}), (C₁-C₈ alkyl) - C(=O)N(R^{x})(C₁-C₈ alkyl)N(R^{y})C (=O) O-(C₁-C₈ alkyle), (C₁-C₈ alkyl)-C(=O)N(R^{x})(C₁-C₈ alkyl)(N(R^{y})C (=O) O-(C₁-C₈ alkyl) C (=O) OH, (C₁-C₈ alkyl) -C (=O) (Het-1) C(=O)O-(C₁-C₈ alkyle), (C₁-C₈ alkyl) -OC (=O) O- (C₁-C₈ alkyle), (C₁-C₈ alkyl) -OC (=O) - (C₁-C₈ alkyle), (C₁-C₈ alkyl) -OC (=O) - (C₃-C₈ cycloalkyle), (C₁-C₈ alkyl) -OC (=O) - (Het-1), (C₁-C₈ alkyl)-OC(=O)-(C₁-C₈ alkyl)N(R^{x})C(=O)O-(C₁-C₈ alkyle), (C₁-C₈ alkyl)-NR^{x}R^{y}, (C₁-C₈ alkyl) -S- (Het-1), (C₁-C₈ alkyl) S (Het-1), (C₁-C₈ alkyl) S(=O) (Het-1), (C₁-C₈ alkyl)S(=O)₂(Het-1), ou (C₁-C₈ alkyl)-O-(Het-1),
chaque alkyle, cycloalkyle, phényle, et (Het-1) étant éventuellement substitués par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S (C₁-C₈ alkyle), S (C₃-C₈ cycloalkyle), S(C₁-C₈ halogénoalkyle), S (C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C(=O)OH, C (=O)NR^{x}R^{y}, (C₁-C₈ alkyl)NR^{x}R^{y}, C(=O)(C₁-C₈ alkyle), C(=O)O(C₁-C₈ alkyle), C (=O)(C₁-C₈ halogénoalkyle), C(=O)O(C₁-C₈ halogénoalkyle), C (=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl) S (C₁-C₈ alkyle), (C₁-C₈ alkyl) S (=O)(C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl) C (=O)(C₁-C₈ alkyle), (C₁-C₈ alkyl)phényle, (C₁-C₈ alkyl) -O-phényle, phényle, phénoxy, Si(C₁-C₈ alkyle)₃, SNR^{x}R^{y}, S(=O)NR^{x}R^{y}, S(=O)₂NR^{x}R^{y}, ou (Het-1) ;
(F) R³ ayant la structure suivante : 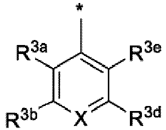
X étant N ou CR^{3c} ;
chaque R^{3a}, R^{3b}, R^{3c}, R^{3d}, et R^{3e} étant indépendamment H, F, Cl, Br, I, CN, OH, SH, NO₂, C₁-C₈ alkyle, C₁-C₈ halogénoalkyle, C₁-C₈ alcoxy, C₁-C₈ halogénoalcoxy, C(=O)(C₁-C₈ alkyle), C(=O)O(C₁-C₈ alkyle), C(=O)(C₁-C₈ halogénoalkyle), C(=O)O(C₁-C₈ halogénoalkyle), (C₁-C₈ alkyl)O(C₁-C₈ alkyle), (C₁-C₈ alkyl) S(C₁-C₈ alkyle), (C₁-C₈ halogénoalkyl)O(C₁-C₈ halogénoalkyle), (C₁-C₈ halogénoalkyl) S(C₁-C₈ halogénoalkyle), (C₁-C₈ alkyl)O(C₁-C₈ halogénoalkyle), (C₁-C₈ halogénoalkyl)O(C₁-C₈ alkyle), (C₁-C₈ alkyl) S(C₁-C₈ halogénoalkyle), (C₁-C₈ halogénoalkyl) S(C₁-C₈ alkyle), (C₁-C₈ alkyl) C (=O) (C₁-C₈ alkyle), (C₁-C₈ alkyl)phényle, (C₁-C₈ alkyl) -O-phényle, phényle, ou phénoxy ;
deux parmi R^{3c}, R^{3d}, et R^{3e} formant un cycle, saturé ou insaturé, à 5 ou 6 chaînons, ayant zéro, un ou deux hétéroatomes choisis parmi azote, soufre et oxygène, et éventuellement substitué par au moins un R¹⁴ ; et
chaque R¹⁴ étant indépendamment H, F, Cl, Br, I, CN, OH, SH, NO₂, C₁-C₈ alkyle, C₁-C₈ halogénoalkyle, C₁-C₈ alcoxy, C₁-C₈ halogénoalcoxy, C(=O)(C₁-C₈ alkyle), C(=O)O(C₁-C₈ alkyle), C (=O) (C₁-C₈ halogénoalkyle), C(=O)O(C₁-C₈ halogénoalkyle), (C₁-C₈ alkyl)O(C₁-C₈ alkyle), (C₁-C₈ alkyl) S(C₁-C₈ alkyle), (C₁-C₈ halogénoalkyl)O(C₁-C₈ halogénoalkyle), (C₁-C₈ halogénoalkyl) S(C₁-C₈ halogénoalkyle), (C₁-C₈ alkyl)O(C₁-C₈ halogénoalkyle), (C₁-C₈ halogénoalkyl)O(C₁-C₈ alkyle), (C₁-C₈ alkyl)S(C₁-C₈ halogénoalkyle), (C₁-C₈ halogénoalkyl)S(C₁-C₈ alkyle), (C₁-C₈ alkyl)C(=O)(C₁-C₈ alkyle), (C₁-C₈ alkyl)phényle, (C₁-C₈ alkyl)-O-phényle, phényle, ou phénoxy ;
(G) R⁴ étant choisi parmi (J), H, OH, SH, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S (C₁-C₈ alkyle), S (C₃-C₈ cycloalkyle), S(C₁-C₈ halogénoalkyle), S (C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C (=O) (C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S (=O)(C₁-C₈ alkyle), (C₁-C₈ alkyl) S(=O)₂(C₁-C₈ alkyle), C(=O)(C₁-C₈ alkyl) C(=O)O(C₁-C₈ alkyle), (C₁-C₈ alkyl) C(=O)O(C₁-C₈ alkyle), phényle, (C₁-C₈ alkyl) phényle, (C₁-C₈ alkyl) -O-phényle, C(=O) (Het-1), (Het-1), (C₁-C₈ alkyl) - (Het-1), (C₁-C₈ alkyl) -C (=O) - (C₁-C₈ alkyle), (C₁-C₈ alkyl) -OC (=O) - (C₁-C₈ alkyle), (C₁-C₈ alkyl) -O-C(=O)O-(C₁-C₈ alkyle), (C₁-C₈ alkyl)-O-C (=O)NR^{x}R^{y}, (C₁-C₈ alkyl) -C (=O)N(R^{x})(C₁-C₈ alkyl) - (Het-1), (C₁-C₈ alkyl)-C(=O)(Het-1), (C₁-C₈ alkyl)-C(=O)N(R^{x})(C₁-C₈ alkyl)N(R^{y})C(=O)OH, (C₁-C₈ alkyl)-C(=O)N(R^{x})(C₁-C₈ alkyl)N(R^{x})(R^{y}), (C₁-C₈ alkyl)-C(=O)N(R^{x})(C₁-C₈ alkyl)N(R^{y})C(=O) O-(C₁-C₈ alkyle), (C₁-C₈ alkyl)-C(=O)N(R^{x})(C₁-C₈ alkyl) (N(R^{y})C (=O) O-(C₁-C₈ alkyl)C(=O)OH, (C₁-C₈ alkyl) -C (=O) (Het-1) C(=O)O-(C₁-C₈ alkyle), (C₁-C₈ alkyl)-OC(=O)-(C₃-C₈ cycloalkyle), (C₁-C₈ alkyl)-OC(=O)-(Het-1), (C₁-C₈ alkyl)-OC(=O)-(C₁-C₈ alkyl)N(R^{x})C(=O)O-(C₁-C₈ alkyle), (C₁-C₈ alkyl)-NR^{x}R^{y}, (C₁-C₈ alkyl)-S-(Het-1), (C₁-C₈ alkyl)S(=O) (Het-1), (C₁-C₈ alkyl)S(=O)₂(Het-1), ou (C₁-C₈ alkyl)-O-(Het-1),
chaque alkyle, halogénoalkyle, cycloalkyle, halogénocycloalkyle, cycloalcoxy, halogénocycloalcoxy, alcoxy, halogénoalcoxy, alcényle, cycloalcényle, halogénoalcényle, alcynyle, phényle, et (Het-1), étant éventuellement substitués par un ou plusieurs substituants indépendamment choisis parmi H, F, Cl, Br, I, CN, OH, SH, NO₂, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C (=O) OH, C(=O)(C₁-C₈ alkyle), C(=O)O(C₁-C₈ alkyle), C (=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C (=O) (C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S(=O) (C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl)C(=O)O(C₁-C₈ alkyle), (C₁-C₈ alkyl) C(=O)O(C₁-C₈ alkyle), (C₁-C₈ alkyl) C (=O)(C₁-C₈ alkyle), (C₁-C₈ alkyl)phényle, (C₁-C₈ alkyl) -O-phényle, phényle, halogénophényle, phénoxy, et (Het-1) ;
(H) chacun parmi Q¹ et Q² étant indépendamment choisi parmi O ou S ;
(I) R^{x} et R^{y} étant indépendamment choisis parmi H, OH, SH, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S (C₃-C₈ cycloalkyle), S(C₁-C₈ halogénoalkyle), S (C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C (=O)(C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S (=O)(C₁-C₈ alkyle), (C₁-C₈ alkyl)S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl)C(=O)(C₁-C₈ alkyle), (C₁-C₈ alkyl)phényle, (C₁-C₈ alkyl)-O-phényle, phényle, (C₁-C₈ alkyl)phényle, (C₁-C₈ alkyl)-O-phényle, C(=O) (Het-1), (Het-1), (C₁-C₈ alkyl) - (Het-1), (C₁-C₈ alkyl) -C (=O) - (C₁-C₈ alkyle), (C₁-C₈ alkyl)-C(=O)(Het-1), (C₁-C₈ alkyl)-C (=O) (Het-1) C(=O)O-(C₁-C₈ alkyle), (C₁-C₈ alkyl) -OC(=O)O-(C₁-C₈ alkyle), (C₁-C₈ alkyl) -OC (=O) -(C₁-C₈ alkyle), (C₁-C₈ alkyl) -OC (=O) -(C₃-C₈ cycloalkyle), (C₁-C₈ alkyl)-OC (=O) - (Het-1), (C₁-C₈ alkyl)-S-(Het-1), (C₁-C₈ alkyl) S (=O) (Het-1), (C₁-C₈ alkyl)S(=O)₂(Het-1), ou (C₁-C₈ alkyl) -O-(Het-1),
chaque alkyle, halogénoalkyle, cycloalkyle, halogénocycloalkyle, cycloalcoxy, halogénocycloalcoxy, alcoxy, halogénoalcoxy, alcényle, cycloalcényle, halogénoalcényle, alcynyle, phényle, et (Het-1), étant éventuellement substitués par un ou plusieurs substituants indépendamment choisis parmi H, F, Cl, Br, I, CN, OH, SH, NO₂, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S (C₃-C₈ halogénocycloalkyle), S (=O)(C₁-C₈ alkyle), S (=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C (=O) OH, C(=O)(C₁-C₈ alkyle), C(=O)O(C₁-C₈ alkyle), C (=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C (=O) O(C₂-C₈ alcényle), (C₁-C₈ alkyl)O(C₁-C₈ alkyle), (C₁-C₈ alkyl) S(C₁-C₈ alkyle), (C₁-C₈ alkyl) S (=O)(C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl)C(=O)O(C₁-C₈ alkyle), (C₁-C₈ alkyl) C(=O)O(C₁-C₈ alkyle), (C₁-C₈ alkyl)C(=O)(C₁-C₈ alkyle), (C₁-C₈ alkyl)phényle, (C₁-C₈ alkyl) -O-phényle, phényle, halogénophényle, phénoxy, et (Het-1),
ou R^{x} et R^{y} ensemble pouvant éventuellement former un groupe cyclique saturé ou insaturé à 5 à 7 chaînons qui peut contenir un ou plusieurs hétéroatomes choisis parmi azote, soufre et oxygène, et ledit groupe cyclique pouvant être substitué par H, F, Cl, Br, I, CN, OH, SH, NO₂, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-Cₑ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S(=O)(C₁-C₈ alkyle), S(=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S(=O)(C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl)OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl) C(=O)(C₁-C₈ alkyle), (C₁-C₈ alkyl)phényle, (C₁-C₈ alkyl) -O-phényle, phényle, phényle substitué, phénoxy, et (Het-1) ;
(J) R² et R⁴ pouvant être un lien hydrocarbyle, saturé ou insaturé à 1 à 4 chaînons, qui peut contenir un ou plusieurs hétéroatomes choisis parmi azote, soufre et oxygène, et conjointement avec C^{X}(Q¹)(N^{X}) formant une structure cyclique à 4 à 7 chaînons, ledit lien hydrocarbyle pouvant éventuellement être substitué par un ou plusieurs substituants indépendamment choisis parmi R⁵, R⁶, et R⁷, chaque R⁵, R⁶, et R⁷ étant choisi parmi H, F, Cl, Br, I, CN, OH, SH, NO₂, NR^{x}R^{y}, C₁-C₈ alkyle, C₁-C₈ alkyle substitué par au moins un OH, C₁-Cₑ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S(=O)(C₁-C₈ alkyle), S(=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C(=O) (C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl)O(C₁-C₈ alkyle), (C₁-C₈ alkyl)S(C₁-C₈ alkyle), (C₁-C₈ alkyl) S(=O)(C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl) C(=O)(C₁-C₈ alkyle), (C₁-C₈ alkyl)phényle, (C₁-C₈ alkyl) -O-phényle, phényle, phényle substitué, phénoxy, ou (Het-1) ;
(K) (Het-1) étant un cycle hétérocyclique, saturé ou insaturé, à 5 ou 6 chaînons, contenant un ou plusieurs hétéroatomes indépendamment choisis parmi azote, soufre ou oxygène, ledit cycle hétérocyclique pouvant également être substitué par un ou plusieurs substituants indépendamment choisis parmi H, F, Cl, Br, I, CN, OH, SH, NO₂, NR^{x}R^{y}, C₁-Cₑ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle) , S(=O)(C₁-C₈ alkyle), S(=O)(C₁-C₈ halogénoalkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C (=O) NR^{x}R^{y}, (C₁-C₈ alkyl) NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O) (C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl) O(C₁-C₈ alkyle), (C₁-C₈ alkyl) S(C₁-C₈ alkyle), (C₁-C₈ alkyl) S(=O)(C₁-C₈ alkyle), (C₁-C₈ alkyl) S(=O)₂(C₁-C₈ alkyle), (C₁-C₈ alkyl)OC(=O)(C₁-C₈ alkyle), (C₁-C₈ alkyl) OC(=O)O(C₁-C₈ alkyle), C(=O)(C₁-C₈ alkyl) C(=O)O(C₁-C₈ alkyle), (C₁-C₈ alkyl)C(=O)O(C₁-C₈ alkyle), (C₁-C₈ alkyl) C(=O)(C₁-C₈ alkyle), (C₁-C₈ alkyl)phényle, (C₁-C₈ alkyl)-O-phényle, phényle, et phénoxy,
chaque alkyle, cycloalkyle, alcoxy, alcényle, alcynyle, phényle, et phénoxy pouvant éventuellement être substitués par un ou plusieurs substituants indépendamment choisis parmi H, F, Cl, Br, I, CN, OH, SH, NO₂, NR^{x}R^{y}, C₁-C₈ alkyle, C₁-C₈ halogénoalkyle, C₃-C₈ cycloalkyle, C₃-C₈ halogénocycloalkyle, C₃-C₈ cycloalcoxy, C₃-C₈ halogénocycloalcoxy, C₁-C₈ alcoxy, C₁-C₈ halogénoalcoxy, C₂-C₈ alcényle, C₃-C₈ cycloalcényle, C₂-C₈ halogénoalcényle, C₂-C₈ alcynyle, S(C₁-C₈ alkyle), S(C₃-C₈ cycloalkyle), S(C₁-C₈ halogénoalkyle), S(C₃-C₈ halogénocycloalkyle), S(=O)(C₁-C₈ alkyle), S(=O)(C₁-C₈ halogénoalkyle), OS(=O)₂(C₁-C₈ alkyle), S(=O)₂(C₁-C₈ alkyle), S(=O)₂(C₁-C₈ halogénoalkyle), OS(=O)₂(C₁-C₈ alkyle), OS(=O)₂(C₁-C₈ halogénoalkyle), C(=O)H, C(=O)NR^{x}R^{y}, (C₁-C₈ alkyl)NR^{x}R^{y}, C(=O)(C₁-C₈ alkyle), C(=O)O(C₁-C₈ alkyle), C(=O)(C₁-C₈ halogénoalkyle), C(=O)O(C₁-C₈ halogénoalkyle), C(=O)(C₃-C₈ cycloalkyle), C(=O)O(C₃-C₈ cycloalkyle), C(=O)(C₂-C₈ alcényle), C(=O)O(C₂-C₈ alcényle), (C₁-C₈ alkyl) O(C₁-C₈ alkyle), (C₁-C₈ alkyl)S(C₁-C₈ alkyle), (C₁-C₈ alkyl)S(=O)(C₁-C₈ alkyle), (C₁-C₈ alkyl) S(=O)₂(C₁-C₈ alkyle), (C₁-C₈ alkyl)OC(=O) (C₁-C₈ alkyle), (C₁-C₈ alkyl) OC(=O)O(C₁-C₈ alkyle), C(=O) (C₁-C₈ alkyl)C(=O)O(C₁-C₈ alkyle), (C₁-C₈ alkyl) C(=O)O(C₁-C₈ alkyle), (C₁-C₈ alkyl)C(=O)(C₁-C₈ alkyle), (C₁-C₈ alkyl)phényle, (C₁-C₈ alkyl)-O-phényle, phényle, et phénoxy ; et
(L) L étant une liaison qui relie Ar² à N^{Y}.

2. Composé selon la revendication 1, R³ ayant une structure de formule R3A ou formule R3B : 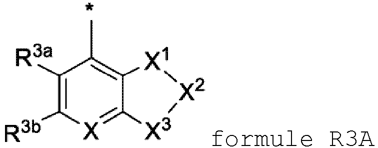 ou 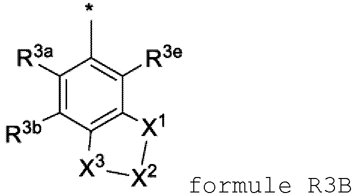
X étant N ou CR^{3c} ;
X¹ étant O, CH, CH₂, N, N(R¹⁴), S, C(O), ou S ;
X² étant O, CH, CH₂, N, N(R¹⁴), S, C(O), ou S ;
X³ étant O, CH, CH₂, N, N(R¹⁴), S, C(O), S, O(CH₂), (CH₂)O, -N=CH-, -CH=N-, C(O)O, OC(O), -N(R¹⁴)-CH2-, -CH₂-N(R¹⁴)-, -N(R¹⁴)-CH-, -CH-N(R¹⁴)-, -CH=CH-, ou -CH₂-CH₂- ;
R^{3a}, R^{3b}, R^{3c}, et R¹⁴ étant indépendamment H, F, Cl, Br, I, CN, OH, SH, NO₂, C₁-Cₑ alkyle, C₁-Cₑ halogénoalkyle, C₁-Cₑ alcoxy, C₁-Cₑ halogénoalcoxy, C(=O)(C₁-C₈ alkyle), C(=O)O(C₁-C₈ alkyle), C(=O)(C₁-C₈ halogénoalkyle), C(=O)O(C₁-C₈ halogénoalkyle), (C₁-C₈ alkyl) O(C₁-C₈ alkyle), (C₁-C₈ alkyl)S(C₁-C₈ alkyle), (C₁-C₈ halogénoalkyl) O(C₁-C₈ halogénoalkyle), (C₁-C₈ halogénoalkyl) S(C₁-C₈ halogénoalkyle), (C₁-C₈ alkyl) O(C₁-C₈ halogénoalkyle), (C₁-C₈ halogénoalkyl)O(C₁-C₈ alkyle), (C₁-C₈ alkyl)S(C₁-C₈ halogénoalkyle), (C₁-C₈ halogénoalkyl)S(C₁-C₈ alkyle), (C₁-C₈ alkyl)C(=O)(C₁-C₈ alkyle), (C₁-C₈ alkyl)phényle, (C₁-C₈ alkyl) -O-phényle, phényle, ou phénoxy.

3. Composé selon la revendication 1,
(A) Ar¹ étant un phényle substitué, un pyridinyle substitué, un pyridazinyle substitué, un pyridinonyle substitué, ou un pyrimidinyle substitué, lesdits phényle substitué, pyridinyle substitué, pyridazinyle substitué, pyridinonyle substitué et pyrimidinyle substitué ayant un ou plusieurs substituants indépendamment choisis parmi H, CN, SF₅, C₁-C₆ alkyle, C₁-C₆ alcoxy, S (C₁-C₆ halogénoalkyle), C₁-C₆ halogénoalkyle et C₁-C₆ halogénoalcoxy ;
(B) Het étant triazolyle ou pyrazolyle ;
(C) Ar² étant phényle, pyridinyle, pyrimidinyle, phényle substitué, pyridinyle substitué ou pyrimidinyle substitué, lesdits phényle substitué, pyridinyle substitué ou pyrimidinyle substitué ayant un ou plusieurs substituants indépendamment choisis parmi H, F, Cl, Br, I, CN, NO₂, NR^{x}R^{y}, OH, C₁-C₆ alcoxy, (C₁-C₆ alkyl)O(C₁-C₆ alkyle), C₁-C₆ halogénoalkyle, et C₁-C₆ alkyle ;
(D) R¹ étant H, C₁-C₆ alkyle, C₃-C₆ cycloalkyle, ou C₂-C₆ alcényle, ledit alkyle, cycloalkyle, ou alcényle étant éventuellement substitué par un C₁-C₆ alcoxy, C₁-C₆ halogénoalcoxy, C₃-C₆ cycloalkyle, ou C₃-C₆ halogénocycloalkyle ;
(E) R² étant choisi parmi (J), H, C₁-C₆ alkyle, ou C₁-C₆ halogénoalkyle ;
(F) R³ étant choisi parmi l'un des groupes suivants R³ 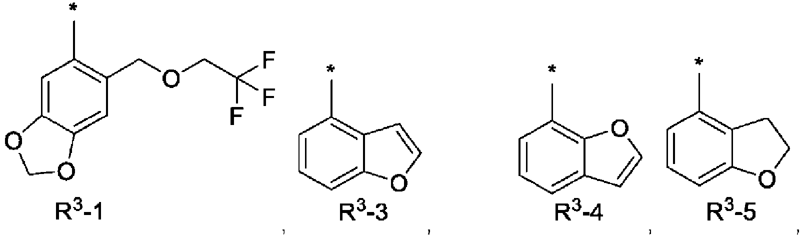 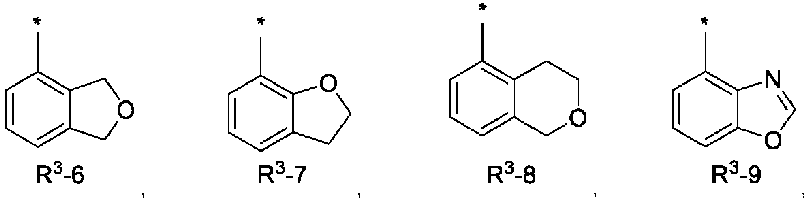 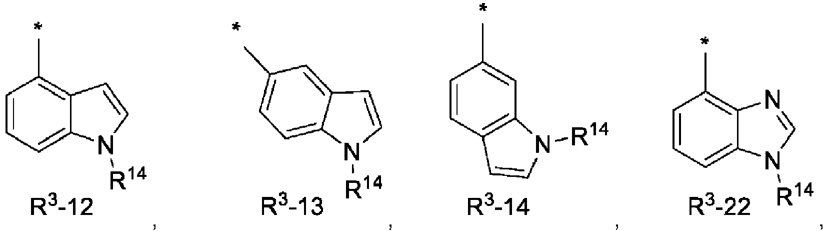 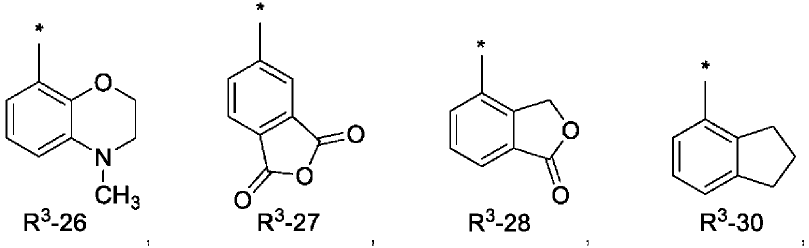 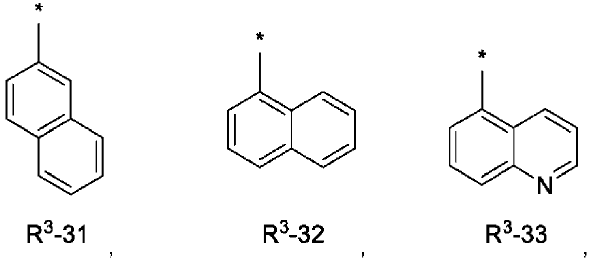 R¹⁴, si cela est applicable au groupe R³, étant choisi dans le groupe constitué par H et C₁-C₆ alkyle ;
(G) R⁴ étant choisi parmi (J), H, C₁-C₆ alkyle, ou C₁-C₆ halogénoalkyle ;
(H) Q¹ étant S et Q² étant O ;
(I) R^{x} et R^{y} étant indépendamment choisis parmi H, C(=O)(C₁-C₆ alkyle), C(=O)O(C₁-C₈ alkyle), C₁-C₆ alkyle, C₁-C₆ halogénoalkyle, C₃-C₆ cycloalkyle, C₃-C₆ halogénocycloalkyle, C₂-C₆ alcényle, C₃-C₆ cycloalcényle, C₂-C₆ halogénoalcényle, C₂-C₆ alcynyle, et phényle ; et
(J) R² et R⁴ pouvant être un lien hydrocarbyle, saturé ou insaturé à 1 à 4 chaînons, qui peut contenir un ou plusieurs hétéroatomes choisis parmi azote, soufre et oxygène, et conjointement avec C^{X}(Q¹)(N^{X}) formant une structure cyclique, ledit lien hydrocarbyle pouvant éventuellement être substitué par un ou plusieurs substituants indépendamment choisis parmi R⁵, R⁶, et R⁷, chaque R⁵, R⁶, et R⁷ étant choisis parmi H, F, Cl, Br, I, CN, NO₂, NR^{x}R^{y}, OH, SH, S(C₁-C₈ alkyle), S(C₁-C₈ halogénoalkyle), C₁-C₆ alkyle, C₁-C₆ alkyle substitué par OH, C₁-C₆ halogénoalkyle, C₁-C₆ alcoxy, C₁-C₆ halogénoalcoxy, C₃-C₆ cycloalkyle, C₃-C₆ halogénocycloalkyle, phényle, et oxo.

4. Composé selon la revendication 1, ayant une structure de formule deux :

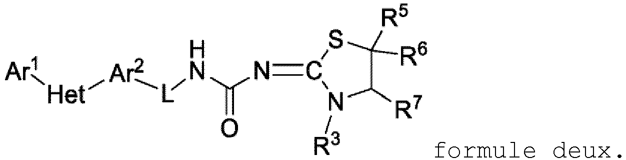

5. Composé selon la revendication 1, ayant une structure choisie parmi les composés listés dans le tableau 1.

6. Composé selon la revendication 1,
(A) Ar¹ étant un phényle substitué, un pyridinyle substitué, ou un pyrimidinyle substitué, lesdits phényle substitué, pyridinyle substitué ou pyrimidinyle substitué ayant un ou plusieurs substituants indépendamment choisis parmi H, CN, SF₅, C₁-C₆ alkyle, C₁-C₆ alcoxy, C₁-C₆ halogénoalkyle et C₁-C₆ halogénoalcoxy ;
(B) Het étant triazolyle ou pyrazolyle ;
(C) Ar² étant phényle, pyridinyle, pyrimidinyle, phényle substitué, pyridinyle substitué ou pyrimidinyle substitué, lesdits phényle substitué, pyridinyle substitué et pyrimidinyle substitué ayant un ou plusieurs substituants indépendamment choisis parmi H, F, Cl, Br, I, CN, NO₂, NR^{x}R^{y}, OH, C₁-C₆ alcoxy, (C₁-C₆ alkyl)O(C₁-C₆ alkyle), C₁-C₆ halogénoalkyle, et C₁-C₆ alkyle ;
(D) R¹ étant H, C₁-C₆ alkyle, C₃-C₆ cycloalkyle, ou C₂-C₆ alcényle, ledit alkyle, cycloalkyle, ou alcényle étant éventuellement substitué par un C₁-C₆ alcoxy, C₁-C₆ halogénoalcoxy, C₃-C₆ cycloalkyle, ou C₃-C₆ halogénocycloalkyle ;
(E) R² étant choisi parmi (J), H, C₁-C₆ alkyle, ou C₁-C₆ halogénoalkyle ;
(F) R³ étant l'un choisi parmi l'un des groupes suivants R³ 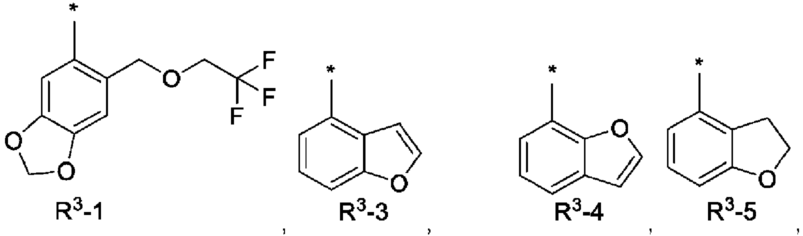 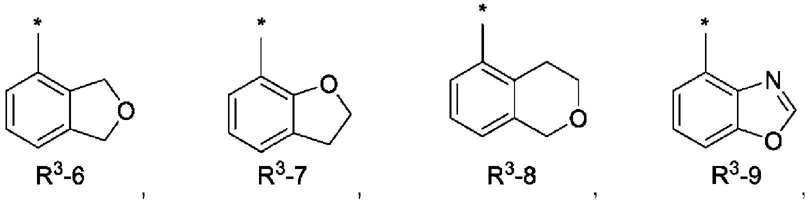 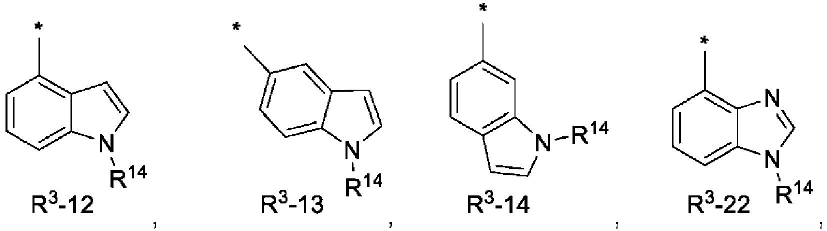 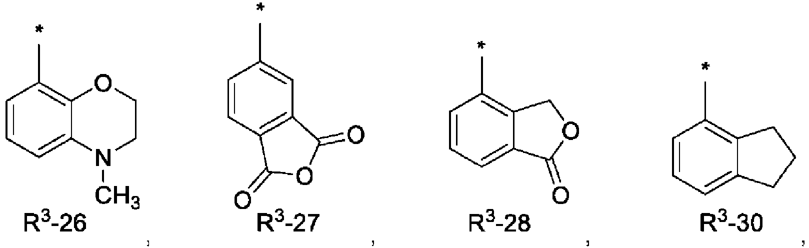 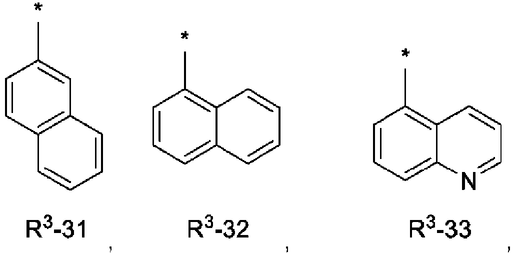 R¹⁴, si cela est applicable au groupe R³, étant choisi dans le groupe constitué par H et CH₃ ;
(G) R⁴ étant choisi parmi (J), H, C₁-C₆ alkyle, ou C₁-C₆ halogénoalkyle ;
(H) Q¹ étant S et Q² étant O ;
(I) R^{x} et R^{y} étant indépendamment choisis parmi H, C(=O) (C₁-C₆ alkyle), C₁-C₆ alkyle, C₁-C₆ halogénoalkyle, C₃-C₆ cycloalkyle, C₃-C₆ halogénocycloalkyle, C₂-C₆ alcényle, C₃-C₆ cycloalcényle, C₂-C₆ halogénoalcényle, C₂-C₆ alcynyle, et phényle ; et
(J) R² et R⁴ pouvant être un lien hydrocarbyle, saturé ou insaturé à 1 à 4 chaînons, qui peut contenir un ou plusieurs hétéroatomes choisis parmi azote, soufre et oxygène, et conjointement avec C^{X}(Q¹)(N^{X}) formant une structure cyclique, ledit lien hydrocarbyle pouvant éventuellement être substitué par un ou plusieurs substituants indépendamment choisis parmi R⁵, R⁶, et R⁷, chaque R⁵, R⁶, et R⁷ étant choisis parmi H, F, Cl, Br, I, CN, NO₂, NR^{x}R^{y}, OH, SH, S(C₁-C₈ alkyle), S(C₁-C₈ halogénoalkyle), C₁-C₆ alkyle, C₁-C₆ halogénoalkyle, C₁-C₆ alcoxy, C₁-C₆ halogénoalcoxy, C₃-C₆ cycloalkyle, C₃-C₆ halogénocycloalkyle, phényle, et oxo.

7. Composé selon la revendication 1, 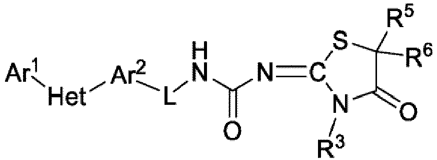
(A) Ar¹ étant un phényle substitué, ledit phényle substitué ayant un ou plusieurs substituants indépendamment choisis parmi H, SF₅, C₁-C₈ halogénoalkyle, et C₁-C₈ halogénoalcoxy ;
(B) Het étant un 1,2,3-triazolyle ;
(C) Ar² étant un phényle substitué, ledit phényle substitué ayant un ou plusieurs substituants indépendamment choisis parmi H, F, Cl, et CH₃ ;
(D) R⁵ et R⁶ étant H ;
(E) R³ étant l'un choisi parmi l'un des groupes suivants R³ 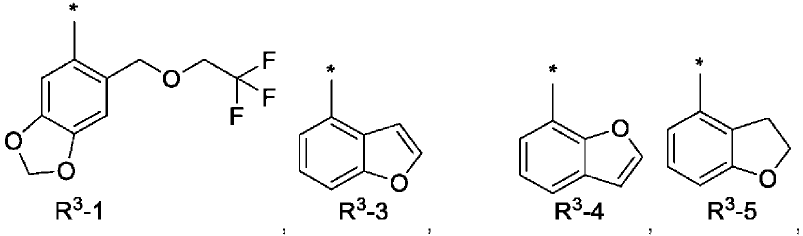 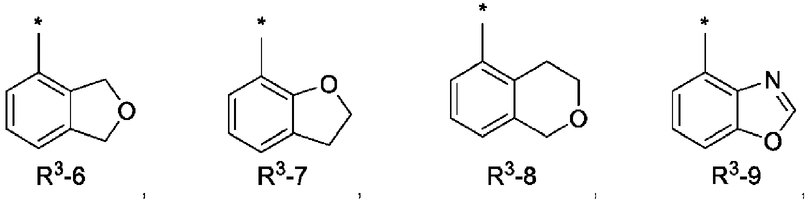 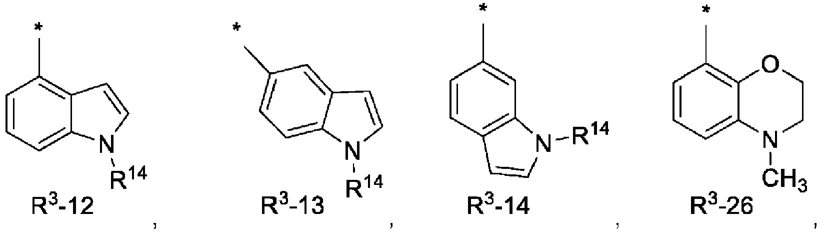 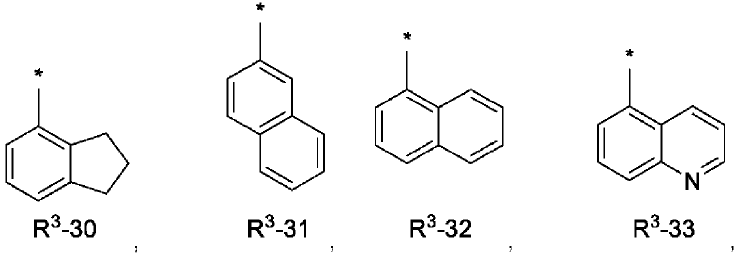 R¹⁴, si cela est applicable au groupe R³, étant choisi dans le groupe constitué par H et CH₃.

8. Composé selon la revendication 1, ayant l'une des structures suivantes

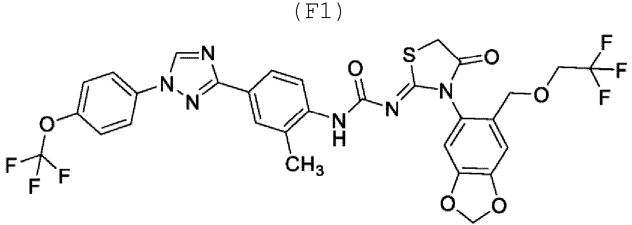

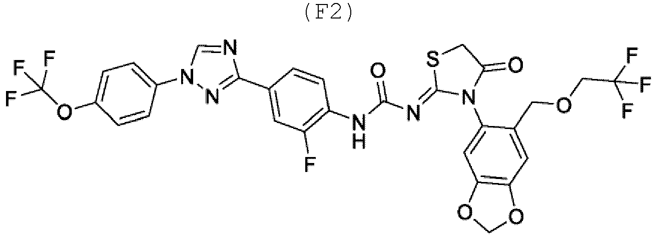

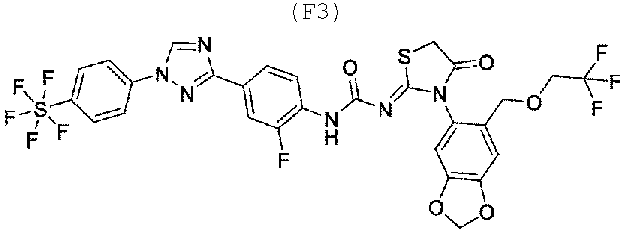

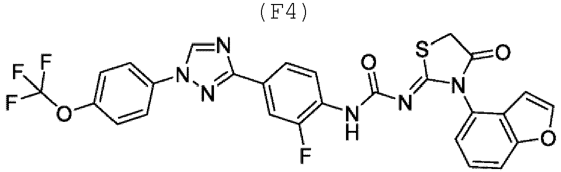

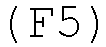

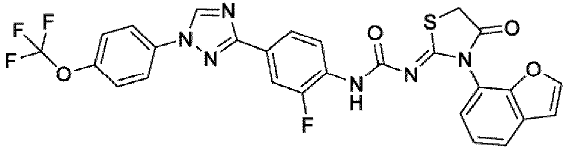

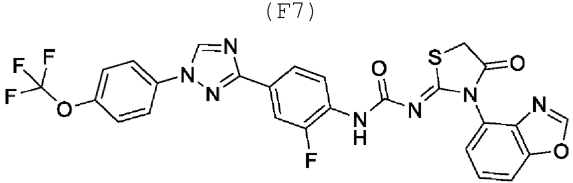

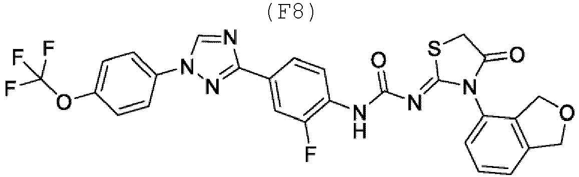

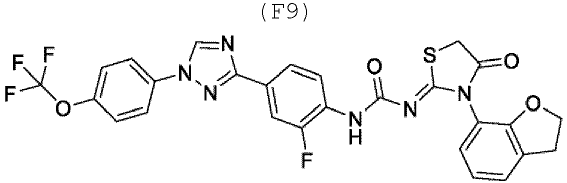

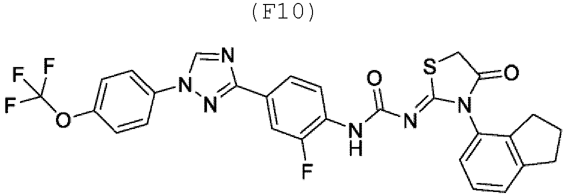

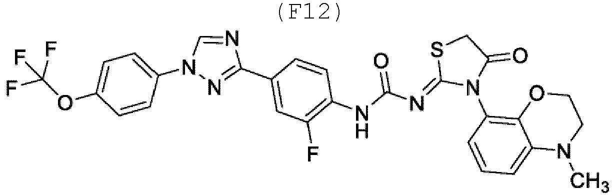

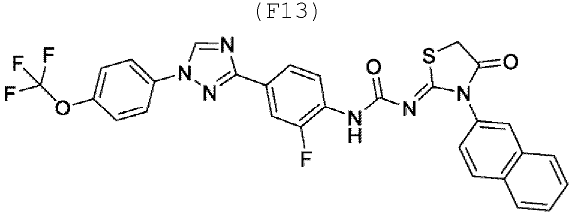

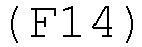

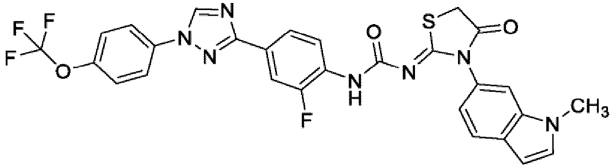

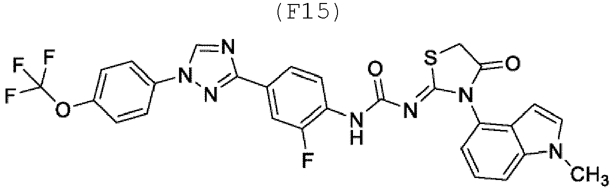

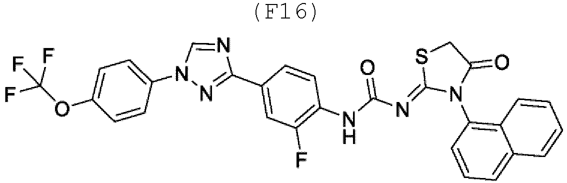

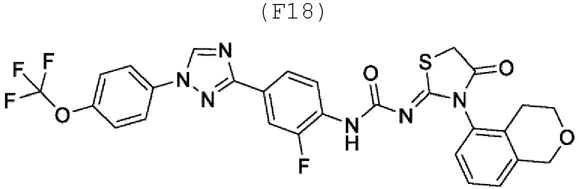

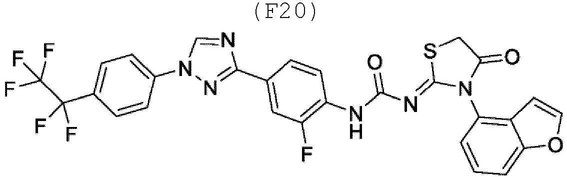

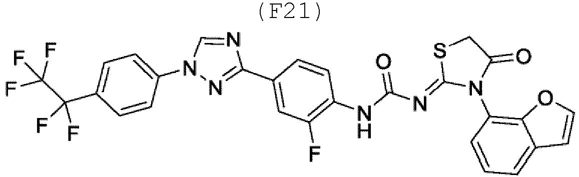

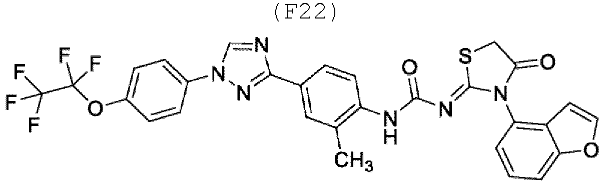

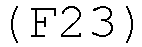

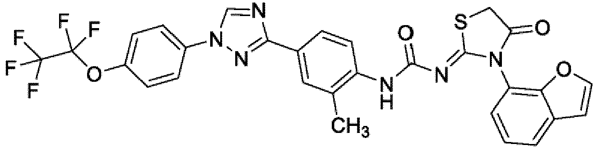

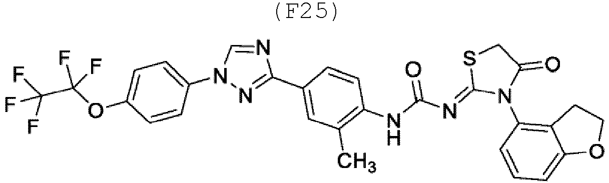

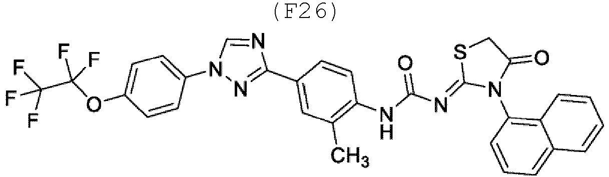

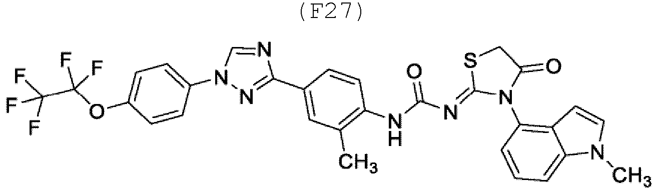

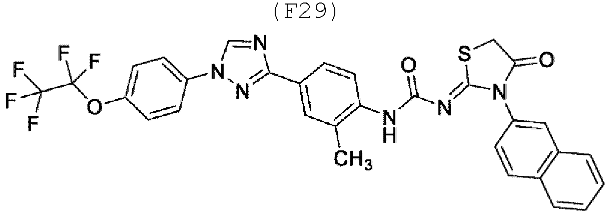

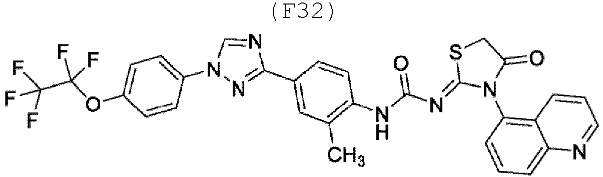

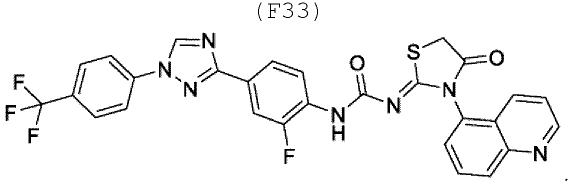

9. Composé selon la revendication 1, destiné à être utilisé dans la lutte contre les endoparasites et les ectoparasites dans le secteur de la médecine vétérinaire ou dans les soins de santé humaine.

10. Procédé non thérapeutique comprenant l'application du composé selon la revendication 1 sur un site pour lutter contre un organisme nuisible, en une quantité suffisante pour lutter contre cet organisme nuisible.

11. Procédé non thérapeutique selon la revendication 10, ledit organisme nuisible étant la noctuelle de la betterave (BAW), la fausse-arpenteuse du chou (CL) ou le moustique de la fièvre jaune (YFM).
